# EUROPEAN PATENT APPLICATION

(11) **EP 3 868 876 A1**
(43) Date of publication of application: **25.08.2021**
(21) Application number: 20158903.3
(22) Date of filing: 21.02.2020
(51) Int. Cl.: C12N 7/00, C12N 15/86, A61K 35/768, A61P 35/00, C07K 14/005, A61K 39/00

(54) **NEW ONCOLYTIC NEWCASTLE DISEASE VIRUSES AND RECOMBINANT NDV STRAINS**

(71) Applicant: Thaller, Arno, 91801 Markt Berolzheim (DE)
(72) Inventor: Thaller, Arno, 91801 Markt Berolzheim (DE)
(74) Representative: Becker, Eberhard

(57) **Abstract**

The invention relates to a novel Newcastle Disease Viruses (NDV) and transgene expressing Newcastle Disease Viruses (NDV), which have been demonstrated to possess significant oncolytic activity against mammalian cancers and an improved safety profile. The invention provides novel oncolytic viruses through the use of genetic engineering, including the transfer of foreign genes or parts thereof. The present invention also provides nucleic acids encoding a reverse genetically engineered (rg-)NDV comprising one or more of these foreign genes and having a mutation in the HN gene, said mutation allowing replication of said rgNDV in a cancer cell to a higher level than replication of an otherwise identical rgNDV not having said mutation in the HN gene, as well as a mutation in the F gene, said mutation resulting in a reduced ICPI value as compared to an otherwise identical rgNDV not having said at least one mutation in the F gene.

## Description

This invention relates to novel oncolytic Newcastle Disease Viruses (NDV) and recombinant Newcastle Disease Viruses, which have been demonstrated to possess significant oncolytic activity against mammalian cancers, especially selected from the specific cancers as described herein and/or mentioned in the claims, and which provide improved viral safety. The invention provides the development of novel and improved oncolytic agents through the use of genetic engineering, including the transfer of genes across species boundaries in order to produce improved genetically modified viruses carrying these transgenes.

### Background

NDV is known as an oncolytic virus that is a virus for use in oncological treatment, preferably in the treatment of human subjects in need thereof. A number of RNA viruses, including NDV, reovirus, measles virus, and vesicular stomatitis virus (VSV), are members of this novel class of viruses being exploited as potential oncolytic agents. These oncolytic viruses are characterized in inherently replicating selectively within and killing tumor cells while leaving non-tumor cells unharmed. Accordingly these oncolytic viruses offer an attractive new tool for cancer therapy.

NDV derives its name from the site of the original outbreak in chickens at a farm near Newcastle-upon-Tyne in England in 1926. The virus is an economically important pathogen in multiple avian species and it is endemic in many countries. NDV is a member of the Avulavirus genus in the Paramyxoviridae family and is a non-segmented, negative-strand RNA virus, whose natural host range is limited to avian species; however, it is known to enter cells by binding to sialic acid residues present on a wide range of human and rodent cancer cells. The oncolytic property of NDV, i.e. to selectively replicate in and destroy tumor cells, while sparing normal cells, is believed to be based in part on defective antiviral responses in tumor cells. Normal cells, which are competent in launching an efficient antiviral response rapidly after infection, are able to inhibit viral replication before viral-mediated cell damage can be initiated. The sensitivity of NDV to interferon (IFN), coupled with defective IFN signalling pathways in tumor cells, provides one plausible mechanism whereby NDV can replicate exclusively within neoplastic tissue. The selective replication of NDV in human tumor cells may also be caused by several other mechanisms, including defects in activation of anti-viral signalling pathways, and activation of Ras signalling and/or expression of Rac1 (Schirrmacher, 2015, Expert Opin. Biol. Ther. 15:17 57-71).

Due to its tumor-specifity NDV is already under investigation as a safe clinical oncolytic virotherapy agent (Cassel et al., 1965, Cancer 1:863-888; Steiner et al., 2004, J. Clin. Oncol. 22:4272-4281; Schulze et al., 2009, Cancer Immunol. Immunother. 58:61-69; Csatary et al., 1999, Anticancer Res. 19:635-638; Pecora et al., 2002, J. Clin. Oncol. 20:2251-2266; Lorence et al., 2003, Curr. Opin. Mol. Ther. 5:618-624; Freeman et al., 2006, Mol. Ther. 13:221-228). The safety margin of NDV is a function of its inability to replicate in and kill normal human cells.

Typically, an oncolytic NDV strain is defined as an NDV for use in oncological virotherapy, preferably in the treatment of human subjects in need thereof. Multiple preclinical model studies have shown significant anti-tumor activity of natural and recombinant oncolytic NDV strains after varying treatment modalities. Promising results were noted in preclinical models of glioblastoma multiforme, anaplastic astrocytoma, leukemia, lymphoma, melanoma, neuroblastoma, osteosarcoma, rhabdomyosarcoma, Ewing's sarcoma, fibrosarcoma, pheochromocytoma, colon carcinoma, lung carcinoma, prostate carcinoma, breast carcinoma, ovary carcinoma, gastric carcinoma, mesothelioma, renal cell carcinoma, and head and neck carcinoma. One typically useful oncolytic NDV that has been extensively explored for use in the treatment of humans, and for which an advantageous safety and efficacy profile has been established in human trials, is a strain named MTH-68/H (Csatary LK, et al., J Neurooncol. 2004 Mar-Apr; 67(1-2):83-93). MTH-68/H therapy has been employed in a range of different tumors with success. MTH-68/H has been developed into a highly purified, lyophilized product, containing live, replication competent viral particles, grown to standardized titers. A Phase II clinical trial in humans was completed where the inhalatory mode of administration was used on patients suffering from a variety of advanced malignancies no longer responding to conventional cancer therapies. The study demonstrated relative efficacy, an overall improved quality of life, and a relatively benign side effect profile (Csatary LK, et al., Cancer detection and Prevention, 1993, 17(6):619-627). Success of treatment with MTH-68/H has also been shown in glioblastomas and anaplastic astrocytomas. Glioblastoma multiforme (GBM) is the most common primary brain tumor and by far the most aggressive form of glial tumors. This neoplasm has a poor prognosis, averaging six months to a year without therapy or about one-and-one-half years with conventional therapy. Four cases of advanced GBM and anaplastic astrocytoma were treated with the NDV viral product MTH-68/H after the conventional modalities of cancer therapy had failed. Results included survival rates of twelve and half to six years post-viral treatment for the surviving patients, and six years for one patient who has since died, after having discontinued treatment. Against all odds, these surviving patients returned to good quality of life and to a lifestyle that resemble their pre-morbid daily routines including giving birth to a healthy child and full professional activity. They have been able to enjoy good clinical health. These patients regularly received the MTH-68/H viral therapy for a number of years without interruption as a form of monotherapy once the classical modalities failed. The MRI and CT results have revealed an objective decrease in size of the tumors, in some cases the near total disappearance of the tumor mass.

NDV strains based on MTH-68/H having a mutation in the HN gene resulting in an increased replication capability in a cancer cell are disclosed in WO 2019/197275 A1. These NDV strains are also capable of expressing a foreign gene.

As reviewed by Zamarin and Palese (Future Microbiol. 2012 Mar; 7(3): 347-367), when compared to other oncolytic agents in development such as poxvirus, HSV-1, adenovirus, measles, and reovirus vectors, NDV as an oncolytic virus exhibits several advantages over other viruses for use in oncological treatment.

The first advantage is that NDV is an avian pathogen. This avoids the problems of preexisting immunity that can neutralize virus infectivity and pathogenicity of the virus in humans. These potential problems exist when using vaccinia, HSV-1, adenovirus, and measles vectors. Based on serological studies it seems that about 96% of the human population is seronegative for NDV, which avoids the problem of pre-existing immunity. The actual level of seropositivity in today's population may be even lower due to low human exposure to NDV, as the outbreaks of which are primarily limited to farm settings. To attest for viral safety, administration of virulent strains to humans has been shown to result in only mild to moderate adverse effects, with mild conjunctivitis, laryngitis, and flu-like symptoms as the only reported side effects. Natural human infections with highly virulent (avian) strains have been reported in the literature in farmers and laboratory workers and have been limited to mild symptoms such as conjunctivitis and laryngitis.

The second advantage offered by using NDV as an oncolytic agent is that NDV, similarly to other oncolytic viruses, possesses strong immunostimulatory properties that are the basis for oncolytic therapy being considered an immunotherapy. These properties include the induction of type I IFN and chemokines, upregulation of MHC and cell adhesion molecules, and facilitation of adhesion of lymphocytes and antigen-presenting cells (APCs) through expression of viral glycoproteins on the surface of infected cells. These properties have been shown to generate effective anti-tumor immune responses, which may persist long after clearance of the primary viral infection.

A third advantage is that the NDV genome has the plasticity to enable the incorporation and stable expression of foreign genes of relatively large size. Since these viruses replicate in the cytoplasm of the cell and not in the nucleus, an unintended integration of the foreign gene into the host genome is avoided. Such an unintended integration of foreign genes carried by an oncolytic agent into the host genome could be a safety problem with some DNA oncolytic vectors. Moreover, the absence of homologous RNA recombination ensures that foreign genes incorporarted in and expressed from the NDV genome are stable for many serial passages in cell culture and in tumor cells.

Lastly, the ubiquitous nature of the NDV receptor allows for utilization of the virus against a wide variety of tumor types. The specificity of NDV for cancer cells due to their defects in antiviral pathways ensures viral safety.

Nevertheless, the full view of all clinical data generated to date in different trials of different NDV strains shows that there is much need for improvement in terms of percentage responders and magnitude of therapeutic effect. Also it is desired to further improve the viral safety. This need for improvement has spurred efforts to design improved recombinant NDV strains, which is the subject of the present patent application.

Similar to other paramyxoviruses in its family, the 15186, 15192 or 15198 nucleotide(nt)-long negative single-strand RNA genome of NDV encodes six genes including the nucleocapsid protein (NP), phosphoprotein (P), matrix protein (M), fusion protein (F), hemagglutinin-neuraminidase (HN), and RNA-dependent RNA polymerase (L) (Lamb R, Paramyxoviridae: the viruses and their replication. In: Knipe D, editor. Fields Virology, Lippincott Williams & Wilkins; 2007, pp. 1449-1496). The genes are separated by junction sequences that consist of three elements, known as gene start (GS), intergenic (IG), and gene-end (GE) motifs, which regulate mRNA transcription. In the P gene, a unique RNA editing mechanism adds non-templated G residues, resulting in the expression of V and W proteins that are colinear to the P protein in the amino-terminal end. The genomic RNA is bound in a ribonucleotide protein complex (RNP) consisting of NP, P, and L proteins and is surrounded by a lipid envelope containing three virus glycoprotein spikes, HN, M and F. An important factor that influences the extent of virulence of NDV in birds is the cleavage site of the F protein.

Pathogenic classification and virulence of NDV strains in birds generally correlates with their oncolytic properties in human cancer cells. Velogenic (high virulence) strains produce severe respiratory and nervous system signs, spread rapidly through chicken flocks, and can cause up to 90% mortality. Mesogenic (intermediate virulence) strains cause coughing, affect egg production and quality, and can result in up to 10% mortality. Lentogenic (low virulence) strains produce only mild symptoms with little if any mortality. Correspondingly, velogenic strains can efficiently carry out multicycle replication in multiple human cancer cells with effective and efficient cell lysis, lentogenic strains are more attenuated due to lack of activation of the F0 protein, and mesogenic strains convey intermediate effects. On the basis of these observations in human cancers, NDV strains have been classified as either lytic or non-lytic, with velogenic and mesogenic viruses being lytic (high and low respectively), and lentogenic viruses in general being non-lytic. Early studies demonstrated that the lytic abilities of lentogenic NDV strains could be enhanced by the introduction of a polybasic cleavage site into their F proteins (Peeters et al, J. Virol. 1999; 73:5001-5009). Other NDV proteins including NP, P, V, HN, and L have also been shown to be implicated in virulence in birds. A deletion (18nt) introduced in the NP gene (Mebatsion T et al., J Virol. 2002 Oct; 76 (20):10138-46) resulted in the deletion of NP residues 443-460. The resulting mutant NDV propagated in embryonated specific-pathogen-free (SPF) chicken eggs to a level fully comparable to that of the parent virus.

In addition, a B-cell epitope of the S2 glycoprotein of murine hepatitis virus (MHV) was inserted in-frame. Recombinant NDV viruses properly expressing the introduced MHV epitope were successfully generated, demonstrating that the NP can be used as the insertion point in the NDV genome to insert foreign or transgenic sequences to be co-expressed with NDV during virus infection.

Several studies showed that type I IFN antagonist activity of NDV V protein is associated with pathogenicity in birds. In recombinant viruses possessing attenuating mutations or deletions in the V protein or in viruses expressing no or lower levels of V protein decreased pathogenicity in birds was observed (Huang et al., 2003, J Virol. 77:8676-8685; Mebatsion et al., 2001, J Virol. 75:420-428; Qiu et al., 2016, PLoS ONE 11(2): e0148560. doi: 10.1371/journal.pone.0148560; Elankumaran et al., 2010, J Virol. 84:3835-3844; Jang et al., 2010, Appl Microbiol Biotechnol. 85:1509-1520; Alamares et al., 2010, Virus Res. 147:153-157; Park et al., 2003, J Virol.77:9522-9532.). It was found to be sufficient to introduce a small (6 nt) deletion in the P gene to abolish expression of the V protein (Mebatsion et al., 2001).

Recently, two studies showed the role of the polymerase complex in viral pathogenesis, with L protein contributing to pathogenicity of the mesogenic Beaudette C strain, and NP, P, and L proteins contributing to pathogenicity of the velogenic Herts strain (Rout & Samal, 2008, J Virol. 82:7828-7836; Dortmans et al., 2010, J Virol. 84:10113-10120). In addition, several studies noted the contribution of the HN protein to virulence in birds (Römer-Oberdörfer et al., 2006, Avian Dis. 50:259-263; Paldurai et al., 2014, J Virol. 88: 8579-8596.) The HN (hemagglutinin-neuraminidase) protein of NDV is a multifunctional protein with receptor-recognition, hemagglutinin (HA) and receptor-destroying neuraminidase (NA) activities associated with the virus. HN is thought to possess both the receptor recognition of sialic acid at the termini of host glycoconjugates and the neuraminidase activity to hydrolyze sialic acid from progeny virion particles in order to prevent viral self-aggregation. It also recognizes sialic acid-containing receptors on cell surfaces and promotes the fusion activity of the F protein, thereby allowing the virus to penetrate the cell surface. Thus, the HN protein plays a critical role in viral infection of birds.

A recombinant NDV vector virus can be provided by a reverse genetics (rg) system, which has been known since 1999 (Peeters et al., 1999, J. Virol. 73:5001-5009). This system allows desired modification and engineering of NDV genomes. Indeed, various reports have shown the successful use of recombinant NDV vectors engineered to express various transgenes, i.e. foreign genes incorporated in the genome of the virus, with the goal of improving viral oncolytic efficacy (Vigil et al. Cancer Res. 2007, 67:8285-8292; Janke et al., 2007, Gene Ther. 14:1639-1649). As discussed above, the viral F protein of NDV is responsible for viral fusion with the cell membrane and for viral spread from host cell to host cell via formation of syncytia. The presence of the multi-basic amino acid cleavage site in the F protein enables protein cleavage and activation by a broad range or proteases and is known to be a determinant of virulence in velogenic NDV strains. In order to increase the oncolytic potency of a highly attenuated lentogenic Hitchner B1 NDV strain, a polybasic cleavage site was introduced into the F protein to generate rNDV/F3aa with mutated F protein. Altomonte et al. (Mol Ther. 2010 18:275-84) reported an oncolytic NDV vector virus with the mutated F-protein rNDV/F(L289A), harboring an L289A mutation within the F protein, which is required for virus entry and cell-cell fusion.

Due to compelling preclinical data implicating oncolytic NDV as an ideal candidate for cancer therapy, phase I and II clinical trials have been initiated. Several trials have shown that NDV is a safe and potentially therapeutically useful therapeutic agent, with no reports of significant adverse effects in patients beyond conjunctivitis or mild flu-like symptoms (Csatary et al., 1999, Anticancer Res. 19:635-638; Pecora et al., 2002, J. Clin. Oncol. 20:2251-2266; Lorence et al., 2003, Curr. Opin. Mol. Ther. 5, 618-624; Freeman et al., 2006, Mol. Ther. 13:221-228). Although the results of these clinical trials have been encouraging, the extent of clinical responses has not been strong and robust enough to consider bringing one of these initial NDV strains into advanced clinical development. Thus, strategies for improving therapeutic effectiveness while maintaining an acceptable safety profile of oncolytic NDV strains are needed. Thus there remains a clear need for improvement in therapeutic outcome by providing improved NDV strains, in particular NDV strains with enhanced killing effect without affecting - or even improving - its safety toward normal cells.

### Description of the present invention

In a first aspect the present invention provides a novel Newcastle disease virus or novel NDV strains.

The NDV strains according to the present invention comprise a mutation in the viral HN gene, i.e. the nucleic acid sequence encoding the hemagglutinin-neuraminidase protein (HN proteine), leading to a change in the amino acid sequence of the HN protein and providing the NDV with an increased replication capability in a cancer cell, preferably in a human cancer cell, as compared to an otherwise identical NDV not having the said mutation in the HN gene.

Preferably the mutation in the HN gene results in an unexpected beneficially increased replication capability of the NDV strain in cancer cells, preferably in human cancer cells, and more preferably results in a replication of said oncolytic NDV in a human cancer cell which is at least 2-fold higher, more preferably 2- to 10-fold higher, still more preferably 5- to 10-fold higher than an NDV strain not having said mutation in its viral HN gene. Preferably, said NDV strain comprises or is an oncolytic NDV already having an advantageous safety and efficacy profile in humans, such as the NDV strain MTH-68/H.

According to a preferred embodiment the NDV strain comprises a mutated HN gene and the encoded hemagglutinin-neuramidase (HN protein) with an amino acid substitution at position 277 to an amino acid with a hydrophobic side chain other than phenylalanine. Still more preferred is the amino acid phenylalanine (F) substituted to leucine (L) at amino acid position 277. Herein an oncolytic NDV having such a mutation is also identified as NDV^{F277L} to discern it from a parent NDV having a phenylalanine at amino acid position 277 of the HN gene.

The mutation in the HN gene is also identified as HN(F277L), wherein "HN" identifies the gene/nucleic acid sequence of the viral genome (i.e. in this example the HN gene), "277" identifies the amino acid position in the respective gene product (i.e. in this example the amino acid at position 277 of the HN protein), "F" or in general the letter before the number, identifies the amino acid originally present in the respective wild type sequence, and "L" or in general the letter following the number identifies the amino acid present in the NDV strain of the present invention. That is the amino acid at amino acid position 277 of the HN protein is "L", which stands for leucine. A respective nomenclature is used for all other mutations described in the following.

In describing a protein or peptide formulation, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal (left terminal to right terminal), the N-terminal being identified as a first residue. Amino acids are designated by their 3-letter abbreviation, 1-letter abbreviation, or full name, as follows. Ala : A : alanine; Asp : D : aspartic acid; Glu : E : glutamic acid ; Phe : F : phenylalanine; Gly : G : glycine; His : H : histidine; Ile : I : isoleucine; Lys : K : lysine; Leu : L : leucine; Met: M : methionine; Asn : N : asparagine; Pro : P: proline; Gln : Q : glutamine; Arg : R : arginine; Ser : S : serine; Thr : T : threonine; Val : V : valine; Trp : W : tryptophan; Tyr : Y : tyrosine; Cys : C : cysteine.

According to the present invention the novel NDV strains comprise in addition to the above described mutation in the HN gene, mutations in the F gene and the thus encoded fusion protein (F protein). The mutated F gene encodes a fusion protein with an amino acid substitution at amino acid position 117 to an amino acid with a hydroxylated side chain, preferably where phenylalanine (F) is substituted to serine (S) at position 117 of the F gene product (also referred to as F(F117S) in the following), and/or with an amino acid substitution at amino acid position 190 to an amino acid with an aliphatic amino acid side chain, preferably where phenylalanine (F) is substituted to leucine (L) at position 190 of the F gene product (also referred to as F(F190L) in the following). The combination of both mutations at amino acid position 117 and 190 of the F gene is particularly preferred. In any case the nucleic acid sequence encoding the fusion protein encodes an amino acid substitution at position 289. This mutation is capable of improving the oncolytic potential of the respective NDV strain. Preferably the amino acid at position 289 of the F protein is an amino acid with an aliphatic side chain other than leucine (L). More preferably leucine (L) is substituted to alanine (A) at position 289 of the F protein (also referred to as F(L289A) in the following). Thus, the following mutations in the F protein are encompassed by the present invention: F(F117S) + F(L289A), F(F190L) + F(L289A, and F(F117S) + F(F190L) + F(L289A). I.e. the F(L289A) mutation is comprised in combination with at least one further amino acid substitution at position 117 or 190.

Within the meaning of the present invention the term "Newcastle disease virus" or "NDV" includes all known velogenic, mesogenic and lentogenic NDV strains. According to the present invention mesogenic and lentogenic strains are preferred. Still more preferred is the NDV derived from NDV strain MTH-68/H.

A NDV strain derived from NDV strain MTH-68/H having a mutation in the HN gene product or HN protein at position 277, wherein the amino acid phenylalanine (F) is substituted to leucine (L) at position 277, and having mutations in the F gene resulting in a substitution of the amino acid phenylalanine (F) to the amino acid serine (S) at position 117 of the F gene product and a substitution of the amino acid leucine (L) to the amino acid alanine (A) at position 289 of the F gene product is referred to herein also as NDV-Mut HN(F277L)/F(F117S)/F(L289A). A NDV strain derived from NDV strain MTH-68/H having a mutation in the HN gene product or HN protein at position 277, wherein the amino acid phenylalanine (F) is substituted to leucine (L) at position 277, and having mutations in the F gene resulting in a substitution of the amino acid phenylalanine (F) to the amino acid leucine (L) at position 190 of the F gene product and a substitution of the amino acid leucine (L) to the amino acid alanine (A) at position 289 of the F gene product is referred to herein also as NDV-Mut HN(F277L)/F(F190L)/F(L289A). A NDV strain having the mutation in the HN gene and all three amino acid mutations in the F gene is referred to herein also as NDV-Mut HN(F277L)/F(F117S)/F(F190L)/F(L289A).

The following NDV strains derived from NDV strain MTH-68/H are provided by the present invention in a preferred embodiment:
- NDV-Mut HN(F277L)/F(F117S)/F(L289A)
- NDV-Mut HN(F277L)/F(F190L)/F(L289A)
- NDV-Mut HN(F277L)/F(F117S)/F(F190L)/F(L289A)

Within the meaning of the present invention "derived from NDV strain MTH-68/H" is intended to mean that the derived NDV strain is encoded by a viral genome or comprises a viral genome having a nucleic acid sequence with a sequence identity of at least 70 %, preferably of at least 75 %, more preferably of at least 90 %, more preferred of at least 95 % to the nucleic acid sequence of the NDV strain MTH-68/H. In one embodiment the sequence identity is at least 99 % or 100 %. Any inserted sequences / transgenic constructs comprising nucleic acid sequences encoding one or more foreign genes comprised in the recombinant NDV strains are not considered in the sequence alignment, and the reference sequence for the sequence alignment is that of SEQ ID No. 1 of the sequence listing. A sequence variation is for example the substitution, insertion or deletion of at least one nucleotide, preferably the substitution, insertion or deletion of up to 20 nucleotides, still more preferably of up to 15 nucleotides. Any combination of substitution, insertion and deletion is also comprised by that definition, provided that the final construct possesses the desired characteristics. The variation may result in at least one amino acid substitution, wherein each amino acid substitution can be a conservative or a non-conservative amino acid substitution.

It has been surprisingly shown that the mutation in the HN gene results in a beneficially improved replication capability of the respective strain as compared to an otherwise identical NDV not having said mutation in the HN gene. The beneficially increased replication rate of the NDV not only results in higher number of virus particles capable of infecting and destroying cancer cells, but also results in a higher expression of a foreign gene carried by the respective recombinant NDV strains. As explained earlier, the respective gene products of the foreign gene results in an improved immunological response and thus an improved destruction of cancer cells.

Also it has been surprisingly shown that the at least one mutation, preferably both mutations (also referred to as "F2" herein), in the F gene at position 117 and 190 of the amino acid sequence of the resulting F protein beneficially improves the oncolytic potential of the respective NDV strains by improving the safety profile of the viruses in humans. Moreover, it has been surprisingly shown that the additional amino acid substitution at position 289 of the F protein in combination with at least one of the other mutations, preferably both mutations (also referred to as "F3" (= "F2" + L289A) herein) further improves the oncolytic potential of the NDV strain, i.e. the ability to infect and lyse cancer cells. That is the new NDV strains show improved oncolytic power and improved safety. The amino acid substitutions at the respective positions are as specified supra.

With regard to the viral safety profile, this in particular means that the intracerebral pathogenicity index (ICPI) is reduced by the mutations in the F gene at position 117 and/or 190, preferably by F2. The ICPI value indicates the the pathogenicity of a Newcastle disease virus. Preferably, the ICPI is reduced by at least 10 %, more preferably by at least 20 % and still more preferred by at least 30 %, as compared to the identically determined ICPI value of a NDV strain not having the respective mutation(s) in the F gene. The ICPI can be determined according to the method set forth in World Organization for Animal Health (OIE) 2009, Chapter 2.3.14. Newcastle disease, pp. 576-589, in: Manual of Diagnostic Tests and Vaccines for Terrestrial Animals 2009, World Organization for Animal Health (OIE), Paris.

In a preferred embodiment of the present invention the mutated NDV strains comprise in addition to the above described mutation in the HN gene, and the mutations in the F gene at amino acid position 117 and/or 190 and 289, a mutation in the M gene and the thus encoded matrix protein (M protein). Still more preferred the mutated M gene encodes a matrix protein with an amino acid substitution at position 165 to an amino acid with an aromatic side chain. Still more preferred the amino acid glycine (G) at amino acid position 165 of the M gene is substituted to the amino acid tryptophane (W).

NDV strains comprising both a mutation in the HN gene and the M gene, as described supra, have been shown as being particularly suitable to infect cancer cells and to provide an enhanced replication rate within the cancer cells.

In a preferred embodiment of the present invention the mutated NDV strains comprise in addition to the above described mutation in the HN gene and the mutations in the F gene at amino acid positions 117 and/or 190 and 289 as specified above, at least one mutation in the L gene and the thus encoded RNA-dependent RNA polymerase protein, also referred to as L protein or L gene product herein. Still more preferred the mutated L gene encodes a RNA-dependent RNA polymerase protein with an amino acid substitution at position 757 to an amino acid with an aliphatic amino acid side chain other than valine (V), wherein preferably valine (V) is substituted to isoleucine (I) at position 757 of the L gene product, and/or with an amino acid substitution at position 1551 to an amino acid with a hydroxylated side chain, wherein preferably phenylalanine (F) is substituted to serine (S) at position 1551 of the L gene product, and/or with an amino acid substitution at position 1700 to an amino acid with an aliphatic side chain, preferably wherein arginine (R) is substituted to leucine (L) at position 1700 of the L gene product. Preferably the NDV genome comprises a mutated L gene encoding a L protein with the amino acid substitution at position 757, position 1551 and position 1700. These NDV strains may in addition also comprise the above described mutation in the M gene.

A NDV strain derived from NDV strain MTH-68/H having a mutation in the HN gene, wherein the amino acid phenylalanine (F) is substituted to leucine (L) at amino acid position 277, having a mutation in the M gene, wherein the amino acid glycine (G) is substituted to the amino acid tryptophane (W) at position 165 of the M gene product, having three mutations in the F gene, wherein the amino acid phenylalanine (F) is substituted to the amino acid serine (W) at position 117, the amino acid phenylalanine (F) is substituted to the amino acid leucine (L) at position 190, and the amino acid leucine (L) is substituted to the amino acid alanine (A) at position 289 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product is herein referred to also as NDV-Mut HN(F277L)/M(G165W)/ F(F117S)/F(F190L)/F(L289A)/L(V757I)/L(F1551S)/L(R1700L) or MutHu2b or NDV-NoThaBene-2b. This NDV strain derived from NDV strain MTH-68/H is provided by the present invention in a preferred embodiment.

The three mutations in the F gene at position 117, 190 and 289 of the amino acid sequence of the resulting F protein and the three mutations in the L gene at position 757, 1551 and 1700 of the amino acid sequence of the resulting L protein, either alone or in combination, have been shown to beneficially improve the oncolytic potential of the respective NDV strains and the safety profile of these viruses in humans. The combinations include any combination of the referenced mutations in the F gene and L gene, and the F protein and L protein, respectively. That is any combination from three to six mutations. Particularly preferred is a combination of three mutations at amino acid position 117, 190 and 289 of the F gene (also referred to as "F3" herein) and a combination of all six mutations, that is the mutations at positions 117, 190 and 289 of the F gene product and at positions 757, 1551 and 1700 of the L gene product (also referred to as "F3L3" herein). The respective amino acid substitutions are as specified supra. The F3 and F3L3 mutations result in an improved onlytic potential and a reduced intracerebral pathogenicity index (ICPI). Preferably, the ICPI is reduced by at least 10 %, more preferably by at least 20 % and still more preferred by at least 30 %, as compared to the identically determined ICPI value of a NDV strain not having the respective mutations in the F gene and the L gene.

In addition to the mutation at position 277 of the HN gene product, the at least two mutations at positions 117 and/or 190 and 289 of F gene product, and preferably one or more of the above specified mutations at position 165 of the M gene product, and at positions 757 and/or 1551 and/or 1700 of the L gene product, a NDV according to the present invention may also comprise at least one further mutation in the L gene and the encoded L protein having an amino acid substitution at position 1717 to an amino acid with aromatic side chain other than tyrosine (Y), preferably where tyrosine (Y) is substituted to histidine (H) at position 1717 of the L gene product, and/or at position 1910 to an amino acid with a basic side chain, preferably where glutamic acid (E) is substituted to lysine (K) at position 1910 of the L gene product.

Alternatively or in addition a NDV according to the present invention may comprise a mutation in the RNA-editing sequence of the P gene. More preferably, the mutation abolishes and/or decreases the expression of the V protein.

Table 1a shows a list of mutations for obtaining NDV variants with increased titer production and/or good or improved safety profile according to the present invention. The mutation in the HN gene and the at least two mutations in the F gene at amino acid position 117 and/or 190 and 289 is comprised in all NDV strains according to the present invention, while the mentioned mutations in the M gene, and L gene may or may not be present. If present, any possible combination of these mutations is encompassed by the present invention.

**Table 1a:**

| Gene | Relevant amino acid |
|---|---|
| HN | F277L |
| M | G165W |
| F | F117S |
| F | F190L |
| F | L289A |
| L | V757I |
| L | F1551S |
| L | R1700L |
| L | Y1717H |
| L | E1910K |

Table 1b shows particulary preferred NDV variants with increased titer production and/or improved safety profile, and the parent NDV strain MTH-68/H. A "+" means that at the mentioned amino acid position the original (first mentioned) amino acid is substituted by the last mentioned amino acid, while "-" means that there is no amino acid substitution at the mentioned position, i.e. the first mentioned amino acid is present. For example and with regard to the HN gene "+" means that at amino acid position 277 phenylalanine (F) is substituted to leucine (L).

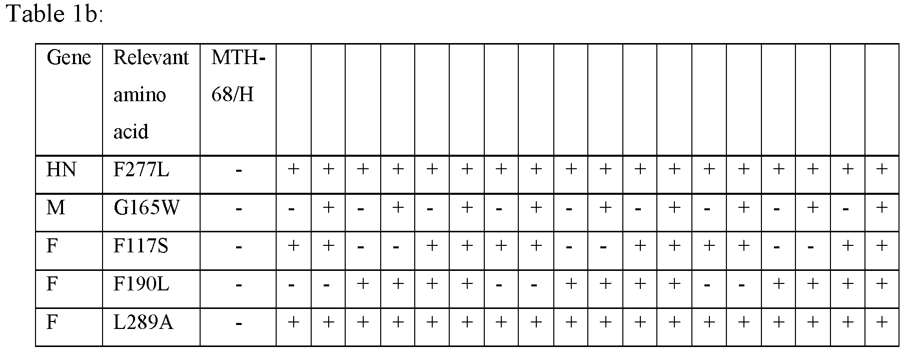

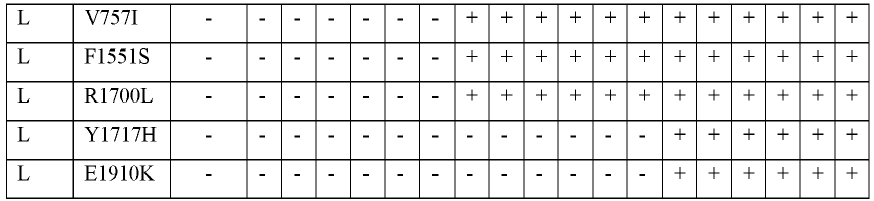

Table 1c shows all mutations in the F gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present. According to the present invention at least one of the mutations at amino acid position 117 or 190 is mandatory in addition to the mutation at amino acid position 289. The combination of both mutations at position 117 and 190 is particularly preferred.

**Table 1c:**

| Gene | Relevant amino acid | 2 | 2 | 3 |
|---|---|---|---|---|
| F | F117S | + | - | + |
| F | F190L | - | + | + |
| F | L289A | + | + | + |

Table 1d shows preferred mutations in the L gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present.

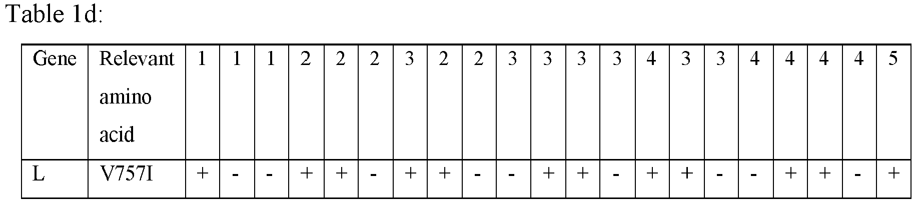

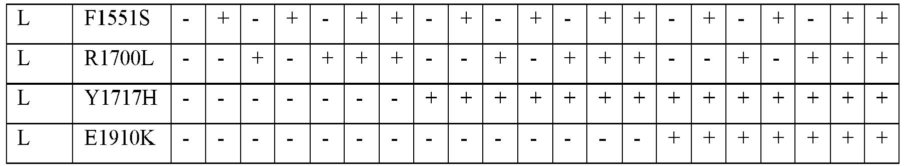

In a preferred embodiment the parent NDV strain is encoded by and/or comprises the nucleic acid sequence according to SEQ ID No. 1 or parts thereof. The novel NDV strain according to the present invention can be encoded by and/or comprises the nucleic acids according to SEQ ID No. 2 or SEQ ID No. 3 or parts thereof. According to another preferred embodiment the parent NDV strain from which the novel NDV strains of the present invention are derived and the NDV strains of the present invention have a sequence identity of at least 70 %, more preferably of at least 75 %, more preferred of at least 90 %, still more preferred of at least 95 % to any one of SEQ ID No. 1 to 3. The sequences given by these SEQ ID numbers are as follows:

| | |
|---|---|
| SEQ ID No. 1: | Nucleic acid (cDNA) sequence of the Newcastle disease virus (NDV) strain MTH-68/H genome; |
| SEQ ID No. 2: | Nucleic acid (cDNA) sequence of Newcastle disease virus strain Mut HN(F277L)/M(G165W)/F(F117S)/F(F190L)/F(L289A)/L(V 757I)/L(F1551S)/L(R1700L) genome having a mutation in the HN gene, a mutation in the M gene, three mutations in the F gene (F3) and three mutations in the L gene (L3) (= NDV-NoThaBene-2b genome). |
| SEQ ID No. 3: | Nucleic acid (cDNA) sequence of Newcastle disease virus strain according to SEQ ID No. 2, but comprising in addition a 12 bp insert containing a AfeI restriction site between nucleotide positions 3134 and 3135 of SEQ ID No. 2. |

The nucleic acid sequence of SEQ ID No. 3 differs from the nucleic acid sequence of SEQ ID No. 2 therein that a 12 bp insert containing unique AfeI restriction site between the P and M genes (between nucleotide position 3134-3135 of SEQ ID No. 2) has been inserted. This 12 bp sequence is for inserting a transgenic construct between the AGC and the GCT sequence. The sequence of the AfeI-site is AGCGCT. The nucleic acid sequence of this 12 bp insert is shown in SEQ ID No. 4 of the sequence listing.

A person skilled in the art is well aware of the fact that the nucleobase thymine in the nucleic acid sequence of DNA is replaced by the nucleobase uracil in the nucleic acid sequence of RNA.

In a preferred embodiment of the present invention the viral HN gene product has or comprises an amino acid sequence as identified in SEQ ID No. 32, and/or the viral M gene product preferably has or comprises an amino acid sequence as identified in SEQ ID No. 34. In addition it is preferred that a NDV strain according to the present invention comprises beside the viral HN gene product having an amino acid sequence as identified in SEQ ID No. 32, and/or the viral M gene product preferably having an amino acid sequence as identified in SEQ ID No. 34, a viral F gene product having or comprising an amino acid sequence as identified in SEQ ID No. 33 and/or a viral L gene product having or comprising an amino acid sequence as identified in SEQ ID No. 35. Also encompassed by the present invention are NDV strains comprising variants of the gene products according to SEQ ID No. 32 to 35.

The NDV as described supra can be a recombinant virus comprising a viral genome comprising a nucleic acid comprising a nucleic acid sequence encoding at least one foreign gene. The at least one foreign gene can be selected from the group consisting of:
- A gene encoding an antibody directed to the protein PD-L1 or an antigen-binding part directed to the protein PD-L1 (anti-PD-L1), such as Atezolizumab or a variant or an antigen-binding part thereof. In one embodiment the antibody is Atezolizumab, an antigen-binding part of Atezolizumab, a variant of Atezolizumab or a variant of an antigen-binding part of Atezolizumab.
- A gene encoding an antibody directed to the growth factor protein VEGF-A or an antigen-binding part directed to the growth factor protein VEGF-A (anti-VEGF-A), such as Bevacizumab or a variant or an antigen-binding part thereof.VEGF-A is a growth factor protein that stimulates angiogenesis in a variety of diseases, especially in cancer. Thus the antibody is capable of blocking angiogenesis. In one embodiment the antibody is Bevacizumab, an antigen-binding part of Bevacizumab, a variant of Bevacizumab or a variant of an antigen-binding part of Bevacizumab.
- A gene encoding an antibody directed to the killer-cell immunoglobulin-like receptors (KIRs), in particular KIR2DL1/2L3, thereby regulating the killing function of the cells expressing the receptors, or an antigen-binding part directed to killer-cell immunoglobulin-like receptors (KIRs), in particular KIR2DL1/2L3, (anti-KIR). They work by regulating the killing function of the cells expressing the receptors. In one embodiment the antiboby is Lirilumab, an antigen-binding part of Lirilumab, a variant of Lirilumab or a variant of an antigen-binding part of Lirilumab.
- A gene encoding an antibody directed to and inhibiting LAG-3 or an antigen-binding part directed to directed to and inhibiting LAG-3 (anti-LAG-3). LAG-3 is a protein, which is present on the surface of T-cells. When the antibody or its antigen-binding part is bound to LAG-3 on T-cells, the T-cells are stimulated to attack cancer cells. In one embodiment the antiboby is Relatlimab, an antigen-binding part of Relatlimab, a variant of Relatlimab or a variant of an antigen-binding part of Relatlimab. When bound to LAG-3 on T-cells, the T-cells are stimulated to attack cancer cells.
- A gene encoding an antibody directed to NKG2A or an antigen-binding part directed to protein NKG2A (anti-NKG2A). Binding of the antibody to NKG2a prevents the binding of NKG2A to its ligand human leukocyte antigen-E (HLA-E), which induces an immune response against the cancer cells leading to their destruction. In one embodiment the antiboby is Monalizumab, an antigen-binding part of Monalizumab, a variant of Monalizumab or a variant of an antigen-binding part of Monalizumab.
- A gene encoding an antibody directed to the glucocorticoid-induced TNF-superfamily receptor (GITR), thereby blocking the interaction of GITR with its ligand, or an antigen-binding part directed to the glucocorticoid-induced TNF-superfamily receptor (GITR) (anti-GITR). In one embodiment the antibody is TRX518, an antigen-binding part of TRX518, a variant of TRX518 or a variant of an antigen-binding part of TRX518.
- A gene encoding an antibody directed to and activating the protein OX40 (also known as CD134 or TNFRSF4), thereby increasing the number of T-cells, or an antigen-binding part directed to protein OX40 (anti-OX40). In one embodiment the antibody is BMS 986178, an antigen-binding part of BMS 986178, a variant of BMS 986178 or a variant of an antigen-binding part of BMS 986178.
- A gene encoding the protein CD40 (cluster of differentiation 40), a part of CD40, a variant of CD40 or a variant of a part of CD40.
- A gene encoding a membrane protein which is the receptor for the protein CD28, and which membrane protein is involved in the costimulatory signal essential for T-lymphocyte activation, or a part thereof which is the receptor for the protein CD28, and which membrane protein is involved in the costimulatory signal essential for T-lymphocyte activation. In one embodiment the membrane protein is CD80 (cluster of differentiation 80), a part of CD80, a variant of CD80 or a variant of a part of CD80.
- A gene encoding a human interleukin 12 (hIL-12) protein, or a part thereof. In one embodiment the protein is (ns)hIL-12, a part of (ns)hIL-12, a variant of (ns)hIL-12 or a variant of a part of (ns)hIL-12. As used herein "(ns)" is intended to mean "non secreting". That is the interleukin is preferred to be a non secreting human interleukin 12. A non secreting interleukin may be advantageous over a normal interleukin, as it may prevent that there is an uncontrolled immune response, for example in the form of a massive cytokine secretion, triggered by administration of the recombinant NDV strain.
- A gene encoding a green fluorescent protein, which can be used for the localization and monitoring of cells, in particular cancer cells, or a part thereof. In one embodiment the protein is enhanced green fluorescent protein (eGFP), a part of enhanced green fluorescent protein (eGFP), a variant of enhanced green fluorescent protein (eGFP) or a variant of a part of eGFP.
- A gene encoding an antibody capable of blocking checkpoint inhibition, such as an antibody directed against checkpoint inhibition protein PD-1 (programmed cell death protein 1, also known as CD279) or an antigen-binding part directed to protein PD-1 (anti-PD-1). They work by blocking a signal that prevents activated T cells from attacking the cancer upon expression in rNDV-expresser cells, thus allowing the immune system to clear the cancer. In one embodiment the antibody is Nivolumab, an antigen-binding part of Nivolumab, a variant of Nivolumab or a variant of an antigen-binding part of Nivolumab.
- A gene encoding an antibody capable of blocking checkpoint inhibition, such as an antibody directed to the surface protein CTLA-4 or an antigen-binding part directed to protein CTLA-4 (anti-CTLA-4). In one embodiment the antiboby is Ipilimumab, an antigen-binding part of Ipilimumab or a variant of Ipilimumab or a variant of an antigen-binding part of Ipilimumab.
- A gene encoding a protein which improves the cellular immune response and improves the ability of T cells to enter tumor cells, or a part thereof which improves the cellular immune response and improves the ability of T cells to enter tumor cells. In one embodiment the protein is interleukin-12 (IL-12) or a part of interleukin-12 or a variant of interleukin-12 or a variant of a part of interleukin-12.
- A gene encoding a protein with the ability to modulate the virus replication cycle, or a part thereof with the ability to modulate the virus replication cycle. In one embodiment the protein is the non-structural protein NS1 of influenza A virus or a part of the non-structural protein NS1 of influenza A virus or a variant of the non-structural protein NS1 of influenza A virus or a variant of a part of the non-structural protein NS1 of influenza A virus.
- A gene encoding a protein with the ability to selectively induce apoptosis in human tumor cells, but not in normal human cells, or a part thereof with the ability to selectively induce apoptosis in human tumor cells, but not in normal human cells. In one embodiment the protein is Apoptin (VP3 from chicken anemia virus) or a part of Apoptin or a variant of Apoptin or a variant of a part of Apoptin.
- A gene encoding a protein that reduces or inhibits IFN expression such as an IFN-beta receptor, or a part thereof that reduces or inhibits IFN expression such as an IFN-beta receptor. In one embodiment the protein is the viral protein B18R from vaccinia virus or a part of the viral protein B18R from vaccinia virus or a variant of the viral protein B18R from vaccinia virus or a variant of a part of the viral protein B18R from vaccinia virus. B18R is a homologue of the human IFN-β receptor. B18R acts as a decoy for IFN-β, thereby inhibiting activation by cell signaling of the antiviral host response in native cells, resulting in increased lytic activity in cancer cells.
- A gene encoding an antibody, preferably an agonistic antibody, directed to CD28 or an antigen-binding part directed to CD28 (anti-CD28). CD28 (cluster of differentiation 28) is one of the proteins expressed on T cells that provide costimulatory signals required for T cell activation and survival. T cell stimulation through CD28 in addition to the T-cell receptor (TCR) can provide a potent signal for the production of various interleukins. In one embodiment the antibody is Theralizumab, an antigen-binding part of Theralizumab, a variant of Theralizumab or a variant of an antigen-binding part of Theralizumab.
- A gene encoding an antibody, preferably an agonistic antibody, directed to CD40 or an antigen-binding part directed to CD40 (anti-CD40). CD40 (cluster of differentiation 40) is a costimulatory protein found on antigen-presenting cells and is required for their activation. Thus, the antidody or its antigen-binding part is used to activate an anti-tumor T cell response via activation of the antigen-presenting cells.
- A gene encoding an antibody, preferably an agonistic antibody, directed to CD80 or an antigen-binding part directed to CD80 (anti-CD80). CD80 (cluster of differentiation 80) is amembrane protein which is the receptor for the proteins CD28 and CTLA-4, and which membrane protein is involved in the costimulatory signal essential for T-lymphocyte activation. Thus, the antidody or its antigen-binding part is used to activate T-lymphocytes.
- A gene encoding an antibody directed to CA 15-3 or an antigen-binding part directed to CA 15-3 (anti-CA 15-3). CA 15-3 is also known as the human gene MUC1.
- A gene encoding an antibody directed to CA 19-9 or an antigen-binding part directed to CA 19-9 (anti-CA 19-9). CA 19-9 is a muzin which corresponds to the sialylated Lewisa blood group antigen.
- A gene encoding an antibody directed to CA 125 or an antigen-binding part directed to CA 125 (anti-CA 125). CA 125 is also known as the human gene MUC16. An antibody or its antigen-binding part targeting the MUC16 protein results in a G2/M-phase growth arrest and tumour cell apoptosis. In one embodiment the antibody is Sofituzumab, an antigen-binding part of Sofituzumab, a variant of Sofituzumab or a variant of an antigen-binding part of Sofituzumab.
- A gene encoding an antibody, preferably an antagonistic antibody, directed to EGFR or an antigen-binding part directed to EGFR (anti-EGFR). The epidermal growth factor receptor (EGFR) is a transmembrane protein. Permanent activation of the receptor results in an uncontrolled cell proliferation and prevents controlled cell death (apoptosis), which contributes to the malignant transformation of cells. The antagonistic antibody is directed against the extracellular domain of the EGFR and leads to an inhibition of ligand binding.. In one embodiment the antibody is Cetuximab, an antigen-binding part of Cetuximab, a variant of Cetuximab or a variant of an antigen-binding part of Cetuximab.
- A gene encoding an antibody, preferably an antagonistic antibody, directed to HER2 or an antigen-binding part directed to HER2 (anti-HER2). The antibody or its antigen-binding part is targeting the extracellular part of the HER2 receptor, which is overexpressen in certain cancers, and is intended to interrupt the growth-promoting signals. In one embodiment the antibody is Trastuzumab, an antigen-binding part of Trastuzumab, a variant of Trastuzumab or a variant of an antigen-binding part of Trastuzumab.
- A gene encoding an antibody directed to nfP2X₇ or an antigen-binding part directed to nfP2X₇ (anti-nfP2X₇). The non-functioning P2X₇ receptor is a form of the ATP-gated P2X₇ receptor in which the E200 epitope is exposed for antibody binding. The nfP2X₇ is able to form a Ca²⁺channel but not an apoptotic pore and is found on every type of cancer cell but no normal cells. In one embodiment the antibody is BIL=3s, an antigen-binding part of BIL=3s, a variant of BIL=3 s or a variant of an antigen-binding part of BIL=3 s.
- A gene encoding an antibody directed to PSMA or an antigen-binding part directed to PSMA (anti-PSMA). The prostate specific membrane antigen (PSMA) is a cell surface peptidase highly expressed by malignant prostate epithelial cells and vascular endothelial cells of numerous solid tumor malignancies. In one embodiment the antibody is J591, an antigen-binding part of J591, a variant of J591 or a variant of an antigen-binding part of J591.
- A gene encoding an antibody, preferably an antagonistic antibody, directed to VEGFR or an antigen-binding part directed to VEGFR (anti-VEGFR). The vascular endothelial growth factor (VEGF) is involved in angiogenesis. The natural ligands comprise VEGF-A, VEGF-C and VEGF-D. Antagonictic antibodies targeting VEGF lead to to inhibition of VEGF-mediated tumor angiogenesis. In one embodiment the antibody is Ramucirumab, an antigen-binding part of Ramucirumab, a variant of Ramucirumab or a variant of an antigen-binding part of Ramucirumab.
- A gene encoding a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5. In one embodiment the protein is TRAIL, a part of TRAIL, a variant of TRAIL or a variant of a part of TRAIL.
- Any combination of these genes, parts or variants of these genes or parts thereof.

In following all these genes encoding the respective antibodies, antigen-binding parts thereof, proteins or parts thereof as well as any of the preferably mentioned antibodys, antigen-bindings parts, proteins, parts or variants thereof, are referred to in the following as "the foreign genes according to group A". This group A also encompasses any combination of these genes, parts or variants of these genes or parts thereof.

The respective proteins or antibodies or parts thereof (i.e. the respective gene products) are also referred to in the following as "the foreign gene products according to group A". This group also encompasses any combination of these gene products, parts or variants thereof.

An agonist is intended to mean a substance that activates signal transduction in the associated cell by occupying a receptor. Chemical compounds that bind to a receptor, but do not activate it, and thus block and inhibit it, are called antagonists.

The antibodies as mentioned above can be monoclonal antibodies, still more preferred monoclonal humanized antibodies, in a preferred embodiment.

The nucleic acid sequence comprising a nucleic acid sequence encoding at least one foreign gene, also referred to as "transgenic construct" or "inserted sequence" herein, can can be or comprises a nucleic acid according to SEQ ID No. 5 or SEQ ID No. 30 or parts thereof. In another embodiment the transgenic construct can have a sequence identity of at least 70 %, more preferably of at least 75 %, more preferred of at least 90 %, still more preferred of at least 95 % to the respective sequence of any one of SEQ ID No. 5 to 30. The sequences given by these SEQ ID numbers are as follows:

| | |
|---|---|
| SEQ ID No. 5: | Nucleic acid sequence of a transgenic construct comprising a nucleic acid sequence encoding the anti-PD-Ll antibody Atezolizumab; |
| SEQ ID No. 6: | Nucleic acid sequence of a transgenic construct comprising a nucleic acid sequence encoding an anti-VEGF-A antibody; |
| SEQ ID No. 7: | Nucleic acid sequence of a transgenic construct comprising a nucleic acid sequence encoding an anti-KIR antibody; |
| SEQ ID No. 8: | Nucleic acid sequence of a transgenic construct comprising a nucleic acid sequence encoding an anti-LAG-3 antibody; |
| SEQ ID No. 9: | Nucleic acid sequence of a transgenic construct comprising a nucleic acid sequence encoding an anti-NKG2A antibody; |
| SEQIDNo. 10: | Nucleic acid sequence of a transgenic construct comprising a nucleic acid sequence encoding an anti-GITR antibody; |
| SEQ ID No. 11: | Nucleic acid sequence of a transgenic construct comprising a nucleic acid sequence encoding an anti-OX40 antibody; |
| SEQIDNo. 12: | Nucleic acid sequence of a transgenic construct comprising a nucleic acid sequence encoding a CD80 protein; |
| SEQIDNo. 13: | Nucleic acid sequence of a transgenic construct comprising a nucleic acid sequence encoding a (ns)hIL-12 protein; |
| SEQIDNo. 14: | Nucleic acid sequence of a transgenic construct comprising a nucleic acid sequence encoding a eGFP; |
| SEQIDNo. 15: | Nucleic acid sequence of a transgenic construct comprising a nucleic acid sequence encoding Nivolumab; |
| SEQIDNo. 16: | Nucleic acid sequence of a transgenic construct comprising a nucleic acid sequence encoding an anti-CTLA-4 antibody; |
| SEQIDNo. 17: | Nucleic acid sequence of a transgenic construct comprising a nucleic acid sequence encoding an interleukin-12 protein; |
| SEQIDNo. 18: | Nucleic acid sequence of a transgenic construct comprising a nucleic acid sequence encoding a non-structural protein NS1; |
| SEQIDNo. 19: | Nucleic acid sequence of a transgenic construct comprising a nucleic acid sequence encoding Apoptin; |
| SEQ ID No. 20: | Nucleic acid sequence of a transgenic construct comprising a nucleic acid sequence encoding B18R; |
| SEQIDNo. 21: | Nucleic acid sequence of a transgenic construct comprising a nucleic acid sequence encoding an anti-CD28 antibody; |
| SEQ ID No. 22: | Nucleic acid sequence of a transgenic construct comprising a nucleic acid sequence encoding an anti-CA 15-3 antibody; |
| SEQ ID No. 23: | Nucleic acid sequence of a transgenic construct comprising a nucleic acid sequence encoding an anti-CA 19-9 antibody; |
| SEQ ID No. 24: | Nucleic acid sequence of a transgenic construct comprising a nucleic acid sequence encoding an anti-CA 125 antibody; |
| SEQ ID No. 25: | Nucleic acid sequence of a transgenic construct comprising a nucleic acid sequence encoding an anti-EGFR antibody; |
| SEQ ID No. 26: | Nucleic acid sequence of a transgenic construct comprising a nucleic acid sequence encoding an anti-HER2 antibody; |
| SEQ ID No. 27: | Nucleic acid sequence of a transgenic construct comprising a nucleic acid sequence encoding an anti-nfP2X₇ antibody; |
| SEQ ID No. 28: | Nucleic acid sequence of a transgenic construct comprising a nucleic acid sequence encoding an anti-PSMA antibody; |
| SEQ ID No. 29: | Nucleic acid sequence of a transgenic construct comprising a nucleic acid sequence encoding an anti-VEGFR antibody; |
| SEQ ID No. 30: | Nucleic acid sequence of a transgenic construct comprising a nucleic acid sequence encoding an anti-(ns)hIL-12; |

The SEQ ID No. 31 of the sequence listing shows by way of example a transgenic NDV strain according to the present invention. Shown is a nucleic acid (cDNA) sequence of Newcastle disease virus strain Mut HN(F277L)/M(G165W)/F(F117S) /F(F190L)/F(L289A)/L(V757I)/L(F1551S)/L(RI700L)-Sofituzumab genome created by reverse genetics as disclosed herein, wherein the nucleic acid comprises a mutation in the HN gene, a mutation in the M gene, three mutations in the F gene (F3) and three mutations in the L gene (L3), and additionally encodes Sofituzumab.

The transgenic construct comprising the nucleic acid sequence encoding Sofituzumab is in this example inserted in the 12 nucleotide long sequence according to SEQ ID No. 4 of the sequence listing in the AfeI-site.

Instead of the nucleic acid sequence comprising a nucleic acid sequence encoding anti-CA 125, preferably Sofituzumab, any other nucleic acid sequence according to any one of SEQ ID No. 5 to 24 and SEQ ID No. 25 to 30 can be inserted to obtain a preferred transgenic NDV strain according to the present invention.

The foreign gene or part or the respective variant is/are expressed in the recombinant NDV strain of the present invention, when the virus is replicating in a suitable host or host cell. An expression of a gene means that the nucleic acid information encoded by the gene is translated in a respective amino acid sequence, which is the primary sequence for building up the respective protein or gene product. By way of example, the gene product of the HN gene is the HN protein.

In one embodiment the recombinant NDV can comprise any one of the foreign genes or parts or variants thereof according to group A. In another embodiment of the present invention a recombinant NDV can comprise in its viral genome at least two foreign genes or parts or variants thereof, which foreign genes or parts or variants are selected from the above mentioned foreign genes according to group A. Still more preferred the sequences comprising a nucleic acid sequence encoding a foreign gene can be any sequence according to SEQ ID No. 5 to 30 of the sequence listing or a variant thereof. A "variant" of a starting nucleic acid is a nucleic acid that comprises a nucleic acid sequence different from that of the starting nucleic acid. Generally, a variant will possess at least 75 % sequence identity, preferably at least 90 % sequence identity, more preferably at least 95 % sequence identity, and most preferably at least 98 % sequence identity with the native nucleic acid. Variants of a nucleic acid may be prepared by introducing appropriate nucleotide changes into the nucleic acid. Such variants include, for example, deletions from, and/or insertions into and/or substitutions of residues within the nucleic acid sequence of the gene of interest. Any combination of deletion, insertion, and substitution is possibly made to arrive at the final construct, provided that the final construct possesses the desired characteristics. Methods for generating nucleic acid sequence variants are known to the skilled person.

A "variant" of a starting polypeptide or protein, e.g. an antibody or another protein, is a polypeptide or protein that comprises an amino acid sequence different from that of the starting polypeptide or protein. Generally, a variant will possess at least 75 % sequence identity, preferably at least 90 % sequence identity, more preferably at least 95 % sequence identity, and most preferably at least 98 % sequence identity with the native polypeptide or protein. Variants of a polypeptide or a proteine may be prepared by introducing appropriate nucleotide changes into the nucleic acid encoding the polypeptide or protein. Such variants include, for example, deletions from, and/or insertions into and/or substitutions of residues within the amino acid sequence of the polypeptide/proteine of interest. Any combination of deletion, insertion, and substitution is possibly made to arrive at the final construct, provided that the final construct possesses the desired characteristics. The amino acid changes also may alter post-translational processes of the polypeptide, such as changing the number or position of glycosylation sites. Methods for generating amino acid sequence variants of polypeptides are known to the skilled person.

Percentage sequence identity is determined after the best alignment of the sequence of interest with the respective reference sequence. The best alignment of the sequences may for example be produced by means of the Fitchet al., Proc. Natl. Acad. Sci. USA 80:1382-1386 (1983), version of the algorithm described by Needleman et al., J. Mol. Biol.48:443-453 (1970), after aligning the sequences to provide for best homology, by means of the similarity search method of Pearson and Lipman, Proc. Natl Acad. Sci. USA 85, 2444 (1988), or by means of computer programs which use these algorithms (in particular BLASTN in Wisconsin Genetics Software Package, Genetics Computer Group, 575 Science Drive, Madison, Wis.). The percentage identity is calculated by determining the number of identical positions between the two sequences being compared, dividing this number by the number of positions compared and multiplying the result obtained by 100 so as to obtain the percentage identity between these two sequences. Preferably, sequence identity of a nucleotide sequence is determined using BLASTN, preferably BLASTN in standard settings as provided by the website of the U.S. National Library of Medicine "https://blast.ncbi.nlm.nih.gov". Also preferably, sequence identity of an amino acid sequence is determined using BLASTP, preferably BLASTP in standard settings as provided by the website of the U.S. National Library of Medicine "https://blast.ncbi.nlm.nih.gov". It is further preferred that the sequence identity is calculated over the entire length of the respective sequence.

As used herein, the term "part" means a fragment of a starting nucleic acid or amino acid sequence, which fragment is a made up of a consecutive sequence of the nucleotides or amino acids of the starting sequence. The part or fragment preferably comprises at least 10, more preferably at least 20 and preferably up to 500 nucleotides or amino acids. In any case a part according to the present invention is a functional part. That is nucleic acid or proteine encoded by that nucleic acid must provide the desired function, e.g. an antigen-binding function or expression inhibiting function as specified herein, of the starting gene or protein.

The parent NDV strain carrying the at least one foreign gene comprises the mutations in the HN gene, the F gene and optionally the M gene and L gene as specified above. The parent NDV strain carrying the at least one foreign gene can be derived from the NDV strain MTH-68/H.

As mentioned above a NDV strain derived from NDV strain MTH-68/H having the respective mutations in the HN gene product or HN protein at position 277 and in the F gene resulting in a substitution of the amino acids at position 117 and/or 190 and 289 are referred to herein also as NDV-Mut HN(F277L)/F(F117S)/F(L289A), NDV-Mut HN(F277L)/F(F190L)/F(L289A) or NDV-Mut HN(F277L)/F(F117S)/(F190L)/ F(L289A). The foreign gene or part or respective variant thereof carried by one of these strain is referred to as Atezolizumab, Bevacizumab, Lirilumab, Relatlimab, Monalizumab, TRX518, BMS 986178, CD40, CD80, (ns)hIL-12, eGFP, Nivolumab, Ipilimumab, IL-12, NS1, Apoptin, B18R, Theralizumab, anti-CD40, anti-CD80, anti-CA 15-3, anti CA 19-9, Sofituzumab, Cetuximab, Trastuzumab, BIL=3s, J591, Ramucircumab or TRAIL in this shortcut for the strain.

Within the shortcut for the recombinant NDV strains as used herein a reference to e.g. Atezolizumab is intended to comprise the complete antibody, antigen-binding parts, and variants of the antibody or of antigen-binding parts, but also encompasses the more general form of an anti-PD-Ll-antibody and antigen-binding parts of this antibody. A reference to Bevacizumab is again intended to comprise the complete antibody, antigen-binding parts and variants of the antibody or antigen-binding parts, but also the more general form of an antibody to the growth factor protein VEGF-A, and antigen-binding parts of this antibody. A reference to Lirilumab is intended to comprise the complete antibody, antigen-binding parts, and variants of the antibody or of antigen-binding parts, but also encompasses the more general form of an anti-KIR-antibody and antigen-binding parts of this antibody. A reference to Relatlimab is intended to comprise the complete antibody, antigen-binding parts, and variants of the antibody or of antigen-binding parts, but also encompasses the more general form of an anti-LAG-3-antibody and antigen-binding parts of this antibody. A reference to Monalizumab is intended to comprise the complete antibody, antigen-binding parts, and variants of the antibody or of antigen-binding parts, but also encompasses the more general form of an anti-NKG2A-antibody and antigen-binding parts of this antibody. A reference to TRX518 is intended to comprise the complete antibody, antigen-binding parts, and variants of the antibody or of antigen-binding parts, but also encompasses the more general form of an anti-GITR-antibody and antigen-binding parts of this antibody. A reference to BMS 986178 is intended to comprise the complete antibody, antigen-binding parts, and variants of the antibody or of antigen-binding parts, but also encompasses the more general form of an anti-OX40-antibody and antigen-binding parts of this antibody. A reference to CD40 is intended to comprise the complete protein, parts and variants of the protein. A reference to CD80 is intended to comprise the complete protein, parts and variants of the protein, as well as the more general form of a membrane protein which is the receptor for the protein CD28, and which membrane protein is involved in the costimulatory signal essential for T-lymphocyte activation, or a respective part thereof. A reference to (ns)hIL-12 is intended to comprise the complete protein, parts and variants of the protein, as well as the more general form of a human interleukin 12 (hIL-12), or a respective part thereof. A reference to eGFP is intended to comprise the complete protein, parts and variants of the protein, as well as the more general form of a green fluorescent protein, or a respective part thereof. A reference to Nivolumab is intended to comprise the complete antibody, antigen-binding parts, and variants of the antibody or of antigen-binding parts, but also encompasses the more general form of an anti-PD-1-antibody and antigen-binding parts of this antibody. A reference to Ipilimumab is again intended to comprise the complete antibody, antigen-binding parts and variants of the antibody or antigen-binding parts, but also the more general form of an anti-CTLA-4-antibody, and antigen-binding parts of this antibody. A reference to IL-12 is intended to comprise the complete protein, parts and variants of the protein, as well as the more general form of a protein which improves the cellular immune response and the ability of T cells to enter tumor cells, or a respective part thereof. A reference to NS1 is intended to comprise the complete protein, parts and variants of the protein, as well as the more general form of a protein with the ability to modulate the virus replication cycle, or a respective part thereof. A reference to Apoptin is intended to comprise the complete protein, parts and variants of the protein, as well as the more general form of a protein with the ability to selectively induce apoptosis in human tumor cells, but not in normal human cells, or a respective part thereof. A reference to B18R in this shortcut is intented to comprise the complete protein, parts and variants of the protein, as well as the more general form of a protein that reduces or inhibits IFN expression such as an IFN-beta receptor, or a respective part thereof. A reference to Theralizumab is intended to comprise the complete antibody, antigen-binding parts, and variants of the antibody or of antigen-binding parts, but also encompasses the more general form of an anti-CD28-antibody and antigen-binding parts of this antibody. A reference to anti-CD40 is intended to comprise the complete antibody, antigen-binding parts, and variants of the antibody or of antigen-binding parts. A reference to anti-CD80 is intended to comprise the complete antibody, antigen-binding parts, and variants of the antibody or of antigen-binding parts. A reference to anti-CA 15-3 is intended to comprise the complete antibody, antigen-binding parts, and variants of the antibody or of antigen-binding parts according to SEQ ID. 22, but also encompasses the more general form of an anti-CA 15-3-antibody and antigen-binding parts of this antibody. A reference to anti-CA 19-9 is intended to comprise the complete antibody, antigen-binding parts, and variants of the antibody or of antigen-binding parts according to SEQ ID. 23, but also encompasses the more general form of an anti-CA 19-9-antibody and antigen-binding parts of this antibody. A reference to Sofituzumab is intended to comprise the complete antibody, antigen-binding parts, and variants of the antibody or of antigen-binding parts, but also encompasses the more general form of an anti-CA 125-antibody and antigen-binding parts of this antibody. A reference to Cetuximab is intended to comprise the complete antibody, antigen-binding parts, and variants of the antibody or of antigen-binding parts, but also encompasses the more general form of an anti-EGFR-antibody and antigen-binding parts of this antibody. A reference to Trastuzumab is intended to comprise the complete antibody, antigen-binding parts, and variants of the antibody or of antigen-binding parts, but also encompasses the more general form of an anti-HER2-antibody and antigen-binding parts of this antibody. A reference to BIL=3s is intended to comprise the complete antibody, antigen-binding parts, and variants of the antibody or of antigen-binding parts, but also encompasses the more general form of an anti-nfP2X₇-antibody and antigen-binding parts of this antibody. A reference to J591 is intended to comprise the complete antibody, antigen-binding parts, and variants of the antibody or of antigen-binding parts, but also encompasses the more general form of an anti-PSMA-antibody and antigen-binding parts of this antibody. A reference to Ramucirumab is intended to comprise the complete antibody, antigen-binding parts, and variants of the antibody or of antigen-binding parts, but also encompasses the more general form of an anti-VEGFR-antibody and antigen-binding parts of this antibody. A reference to TRAIL is intended to comprise the complete protein, parts and variants of the protein, as well as the more general form of a protein with the ability to induce the process of apoptosis by binding to a death receptor, such as death receptor DR4 and/or death receptor DR5, or a respective part thereof. A reference to the foreign gene in this shortcut is also intended to comprise the sequence of the transgenic construct comprising the respective foreign gene as identified in any of the nucleic acid sequences according to SEQ ID No. 5 to 30 of the sequence listing or a variant thereof.

The following recombinant NDV strains are provided by the present invention in a preferred embodiment:
- NDV-Mut HN(F277L)/F(F117S)/F(F190L)/F(L289A)-Atezolizumab
- NDV-Mut HN(F277L)/F(F117S)/F(F190L)/F(L289A)-Bevacizumab
- NDV-Mut HN(F277L)/F(F117S)/F(F190L)/F(L289A)-Lirilumab
- NDV-Mut HN(F277L)/F(F117S)/F(F190L)/F(L289A)-Relatlimab
- NDV-Mut HN(F277L)/F(F117S)/F(F190L)/F(L289A)-Monalizumab
- NDV-Mut HN(F277L)/F(F117S)/F(F190L)/F(L289A)-TRX518
- NDV-Mut HN(F277L)/F(F117S)/F(F190L)/F(L289A)-BMS 986178
- NDV-Mut HN(F277L)/F(F117S)/F(F190L)/F(L289A)-CD40
- NDV-Mut HN(F277L)/F(F117S)/F(F190L)/F(L289A)-CD80
- NDV-Mut HN(F277L)/F(F117S)/F(F190L)/F(L289A)-(ns)hIL-12
- NDV-Mut HN(F277L)/F(F117S)/F(F190L)/F(L289A)-eGFP
- NDV-Mut HN(F277L)/F(F117S)/F(F190L)/F(L289A)-Nivolumab
- NDV-Mut HN(F277L)/F(F117S)/F(F190L)/F(L289A)-Ipilimumab
- NDV-Mut HN(F277L)/F(F117S)/F(F190L)/F(L289A)-IL-12
- NDV-Mut HN(F277L)/F(F117S)/F(F190L)/F(L289A)-NS1
- NDV-Mut HN(F277L)/F(F117S)/F(F190L)/F(L289A)-Apoptin
- NDV-Mut HN(F277L)/F(F117S)/F(F190L)/F(L289A)-B18R
- NDV-Mut HN(F277L)/F(F117S)/F(F190L)/F(L289A)-Theralizumab
- NDV-Mut HN(F277L)/F(F117S)/F(F190L)/F(L289A)-anti-CD40
- NDV-Mut HN(F277L)/F(F117S)/F(F190L)/F(L289A)-anti-CD80
- NDV-Mut HN(F277L)/F(F117S)/F(F190L)/F(L289A)-anti-CA 15-3
- NDV-Mut HN(F277L)/F(F117S)/F(F190L)/F(L289A)-anti CA 19-9
- NDV-Mut HN(F277L)/F(F117S)/F(F190L)/F(L289A)-Sofituzumab
- NDV-Mut HN(F277L)/F(F117S)/F(F190L)/F(L289A)-Cetuximab
- NDV-Mut HN(F277L)/F(F117S)/F(F190L)/F(L289A)-Trastuzumab
- NDV-Mut HN(F277L)/F(F117S)/F(F190L)/F(L289A)-BIL=3 s
- NDV-Mut HN(F277L)/F(F117S)/F(F190L)/F(L289A)-J591
- NDV-Mut HN(F277L)/F(F117S)/F(F190L)/F(L289A)-Ramucircumab
- NDV-Mut HN(F277L)/F(F117S)/F(F190L)/F(L289A)-TRAIL

Thus, according to one embodiment of the present invention a recombinant NDV strain, which can be derived from NDV strain MTH-68/H, comprises as a foreign gene at least one gene selected from the foreign genes according to group A, and has a mutation in the HN gene, preferably resulting in a substitution of the amino acid phenylalanine (F) to leucine (L) at position 277 of the HN gene product, and at least two mutation in the F gene, resulting in a substitution of the amino acid phenylalanine (F) to the amino acid serine (S) at position 117 of the F gene product, and/or resulting in a substitution of the amino acid phenylalanine (F) to the amino acid leucine (L) at position 190 of the F gene product and resulting in a substitution of the amino acid leucine (L) to the amino acid alanine (A) at position 289 of the F gene product. The combination of both mutations at amino acid position 117 and 190 of the F gene product is particularly preferred. As explained supra, other substitutions at these amino acid positions are also comprised within the present invention. In one embodiment the recombinant NDV strain can additionally comprise a mutation in the M gene, preferably resulting in a substitution of the amino acid glycine (G) to the amino acid tryptophane (W) at position 165 of the M gene product.

The recombinant and mutated NDV strains can comprise in addition to the above described mutation in the HN gene and the at least two mutations in the F gene at amino acid positions 117 and/or 190 and 289 as specified above, at least one mutation in the L gene and the thus encoded RNA-dependent RNA polymerase protein at amino acid positions 757 and/or 1551 and/or 1700 as specified above. These recombinant NDV strains may in addition also comprise the above described mutation in the M gene. As it relates to preferred and more preferred embodiments of the substiutions at amino acid positions 117, 190 and 289 of the F gene, and amino acid positions 757, 1551 and 1700 of the L gene reference to above disclosure with regard to the novel NDV strains per se is made. The same preferred and more preferred embodiments or amino acids apply for the the recombinant NDV strains.

According to one embodiment of the present invention a recombinant NDV strain comprises as a foreign gene at least one gene selected from the foreign genes according to group A, and comprises a mutation in the HN gene, preferably resulting in a substitution of the amino acid phenylalanine (F) to leucine (L) at amino acid position 277 of the HN gene, two or more of the above described mutations in the F gene, preferably resulting in a substitution of the amino acid phenylalanine (F) to the amino acid serine (S) at position 117 of the gene product and/or resulting in a substitution of the amino acid phenylalanine (F) to the amino acid leucine (L) at position 190 of the F gene product and resulting in a substitution of the amino acid leucine (L) to the amino acid alanine (A) at position 289 of the F gene product, and at least one mutation in the L gene, preferably one resulting at amino acid position 757 to an amino acid with an aliphatic amino acid side chain other than valine (V), preferably where valine (V) is substituted to isoleucine (I) at position 757 of the L gene product, and/or with an amino acid substitution at position 1551 to an amino acid with a hydroxylated side chain, preferably where phenylalanine (F) is substituted to serine (S) at position 1551 of the L gene product, and/or with an amino acid substitution at position 1700 to an amino acid with an aliphatic side chain, preferably where arginine (R) is substituted to leucine (L) at position 1700 of the L gene product, preferably wherein the NDV comprises a mutated L gene and the encoded L protein with the amino acid substitution at position 757, position 1551 and position 1700. These recombinant NDV strains may in addition also comprise the above described mutation in the M gene, preferably resulting in a substitution of the amino acid glycine (G) to the amino acid tryptophane (W) at position 165 of the M gene product. As explained supra, other substitutions at these amino acid positions are also comprised within the present invention.

A NDV strain derived from NDV strain MTH-68/H having a mutation in the HN gene, wherein the amino acid phenylalanine (F) is substituted to leucine (L) at amino acid position 277, having a mutation in the M gene, wherein the amino acid glycine (G) is substituted to the amino acid tryptophane (W) at position 165 of the M gene product, having three mutations in the F gene, wherein the amino acid phenylalanine (F) is substituted to the amino acid serine (W) at position 117, the amino acid phenylalanine (F) is substituted to the amino acid leucine (L) at position 190 and the amino acid leucine (L) is substituted to the amino acid alanine (A) at position 289 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product is herein referred to also as NDV-Mut HN(F277L)/M(G165W)/ F(F117S)/F(F190L)/F(L289A)/L(V757I)/L(F1551S)/L(R1700L) or MutHu2b or NDV-NoThaBene-2b. The foreign gene or part or variant thereof carried by this strain is referred to as Atezolizumab, Bevacizumab, Lirilumab, Relatlimab, Monalizumab, TRX518, BMS 986178, CD40, CD80, (ns)hIL-12, eGFP, Nivolumab, Ipilimumab, IL-12, NS1, Apoptin, B18R, Theralizumab, anti-CD40, anti-CD80, anti-CA 15-3, anti CA 19-9, Sofituzumab, Cetuximab, Trastuzumab, BIL=3s, J591, Ramucircumab or TRAIL in this shortcut for the strain. Within these shortcuts for the recombinant NDV strains a reference to the respective foreign gene is intended to have the meaning as already defined above. The following NDV strains are provided by the present invention in a particularly preferred embodiment:
- NDV-Mut HN(F277L)/M(G165W)/F(F117S)/F(F190L)/F(L289A)/L(V757I)/ L(F1551S)/L(R1700L)-Atezolizumab
- NDV-Mut HN(F277L)/M(G165W)/F(F117S)/F(F190L)/F(L289A)/L(V757I)/ L(F1551S)/ L(R1700L)-Bevacizumab
- NDV-Mut HN(F277L)/M(G165W)/F(F117S)/F(F190L)/F(L289A)/L(V757I)/ L(F1551S)/ L(R1700L)-Linlumab
- NDV-Mut HN(F277L)/M(G165W)/F(F117S)/F(F190L)/F(L289A)/L(V757I)/ L(F1551S)/ L(R1700L)-Relatlimab
- NDV-Mut HN(F277L)/M(G165W)/F(F117S)/F(F190L)/F(L289A)/L(V757I)/ L(F1551S)/ L(R1700L)-Monalizumab
- NDV-Mut HN(F277L)/M(GI65W)/F(FI17S)/F(FI90L)/F(L289A)/L(V7571)/ L(F1551S)/L(R1700L)-TRX518
- NDV-Mut HN(F277L)/M(G165W)/F(F117S)/F(F190L)/F(L289A)/L(V757I)/ L(F1551S)/L(R1700L)-BMS 986178
- NDV-Mut HN(F277L)/M(G165W)/F(F117S)/F(F190L)/F(L289A)/L(V757I)/ L(F1551S)/L(R1700L)-CD40
- NDV-Mut HN(F277L)/M(GI65W)/F(FI17S)/F(FI90L)/F(L289A)/L(V7571)/ L(F1551S)/L(R1700L)-CD80
- NDV-Mut HN(F277L)/M(GI65W)/F(FI17S)/F(FI90L)/F(L289A)/L(V7571)/ L(F1551S)/L(R1700L)-(ns)hIL-12
- NDV-Mut HN(F277L)/M(GI65W)/F(FI17S)/F(FI90L)/F(L289A)/L(V7571)/ L(F1551S)/L(R1700L)-eGFP
- NDV-Mut HN(F277L)/M(GI65W)/F(FI17S)/F(FI90L)/F(L289A)/L(V7571)/ L(F1551S)/ L(R1700L)-Nivolumab
- NDV-Mut HN(F277L)/M(G165W)/F(F117S)/F(F190L)/F(L289A)/L(V757I)/ L(F1551S)/ L(R1700L)-Ipilimumab
- NDV-Mut HN(F277L)/M(G165W)/F(F117S)/F(F190L)/F(L289A)/L(V757I)/ L(F1551S)/ L(R1700L)-IL-12
- NDV-Mut HN(F277L)/M(G165W)/F(F117S)/F(F190L)/F(L289A)/L(V757I)/ L(F1551S)/L(R1700L)-NS1
- NDV-Mut HN(F277L)/M(G165W)/F(F117S)/F(F190L)/F(L289A)/L(V757I)/ L(F1551S)/ L(R1700L)-Apoptin
- NDV-Mut HN(F277L)/M(G165W)/F(F117S)/F(F190L)/F(L289A)/L(V757I)/ L(F1551S)/L(R1700L)-B18R
- NDV-Mut HN(F277L)/M(G165W)/F(F117S)/F(F190L)/F(L289A)/L(V757I)/ L(F1551S)/L(R1700L)-Theralizumab
- NDV-Mut HN(F277L)/M(G165W)/F(F117S)/F(F190L)/F(L289A)/L(V757I)/ L(F1551S)/L(R1700L)-anti-CD40
- NDV-Mut HN(F277L)/M(G165W)/F(F117S)/F(F190L)/F(L289A)/L(V757I)/ L(F1551S)/L(R1700L)-anti-CD80
- NDV-Mut HN(F277L)/M(GI65W)/F(FI17S)/F(FI90L)/F(L289A)/L(V7571)/ L(F1551S)/L(R1700L)-anti-CA 15-3
- NDV-Mut HN(F277L)/M(GI65W)/F(FI17S)/F(FI90L)/F(L289A)/L(V7571)/ L(F1551S)/L(R1700L)-anti CA 19-9
- NDV-Mut HN(F277L)/M(GI65W)/F(FI17S)/F(FI90L)/F(L289A)/L(V7571)/ L(F1551S)/L(R1700L)-Sofituzumab
- NDV-Mut HN(F277L)/M(G165W)/F(F117S)/F(F190L)/F(L289A)/L(V757I)/ L(F1551S)/L(R1700L)-Cetuximab
- NDV-Mut HN(F277L)/M(G165W)/F(F117S)/F(F190L)/F(L289A)/L(V757I)/ L(F1551S)/L(R1700L)-Trastuzumab
- NDV-Mut HN(F277L)/M(G165W)/F(F117S)/F(F190L)/F(L289A)/L(V757I)/ L(F1551S)/L(R1700L)-BIL=3s
- NDV-Mut HN(F277L)/M(G165W)/F(F117S)/F(F190L)/F(L289A)/L(V757I)/ L(F1551S)/L(R1700L)-J591
- NDV-Mut HN(F277L)/M(G165W)/F(F117S)/F(F190L)/F(L289A)/L(V757I)/ L(F1551S)/L(R1700L)-Ramucircumab
- NDV-Mut HN(F277L)/M(G165W)/F(F117S)/F(F190L)/F(L289A)/L(V757I)/ L(F1551S)/L(R1700L)-TRAIL.

According to another embodiment of the present invention a recombinant NDV according to the present invention may also comprise at least one further mutation in the L gene and the encoded L protein having an amino acid substitution at position 1717 to an amino acid with aromatic side chain other than tyrosine (Y), preferably where tyrosine (Y) is substituted to histidine (H) at position 1717 of the L gene product, and/or at position 1910 to an amino acid with a basic side chain, preferably where glutamic acid (E) is substituted to lysine (K) at position 1910 of the L gene product. Alternatively or in addition a recombinant NDV according to the present invention may comprise a mutation in the RNA-editing sequence of the P gene. More preferably, the mutation abolishes and/or decreases the expression of the V protein. For further details reference to the above disclosure, for example tables 1a to 1d, concerning the novel NDV strain per se is made.

A person skilled in the art is well aware of the fact that the nucleobase thymine in the nucleic acid sequence of DNA is replaced by the nucleobase uracil in the nucleic acid sequence of RNA.

The present invention also provides a nucleic acid comprising the nucleic acid sequence encoding the NDV strains of the present invention.

In describing nucleic acid formulation, structure and function herein, reference is made to any of a group of polymeric nucleotides such as DNA or RNA. Nucleic acid is composed of either one or two chains of repeating units called nucleotides, which nucleotides consist of a nitrogen base (a purine or pyrimidine) attached to a sugar phosphate. In the present specification, nucleotide residues are identified by using the following abbreviations. Adenine residue: A; guanine residue: G; thymine residue: T; cytosine residue: C; uracil residue: U. Also, unless explicitly otherwise indicated, the nucleotide sequences of nucleic acid are identified from 5'-terminal to 3'-terminal (left to right terminal), the 5'-terminal being identified as a first residue.

The nucleic acid according to the present invention comprises a nucleic acid sequence (HN gene) encoding a HN protein having a mutation allowing replication of the NDV in a cancer cell to a higher level than replication of an otherwise identical NDV not having said the mutation in the HN gene.

In a preferred embodiment of the present invention the nucleic acid comprises a nucleic acid sequence encoding a hemagglutinin-neuramidase protein with an amino acid substitution at position 277 to an amino acid with a hydrophobic side chain other than phenylalanine. Still more preferred the nucleic acid sequence encodes for a hemagglutinin-neuramidase protein referred to as HN^{F277L}. That is the amino acid phenylalanine (F) at position 277 of the hemagglutinin-neuramidase is substituted to leucine (L).

In addition a nucleic acid according to the present invention comprises a nucleic acid sequence (F gene) encoding a fusion protein with an amino acid substitution at position 117 and/or position 190 and position 289. With regard to amino acid position 117 the amino acid is substituted to an amino acid with a hydroxylated side chain. Most preferred the mutated F gene encodes F^{F117S}. That is the amino acid phenylalanine (F) at position 117 of the F gene product is substituted to the amino acid serine (S) at the respective position. With regard to the amino acid substitution at position 190 the amino acid is changed to an amino acid with an aliphatic side chain. Most preferred the mutated F gene encodes F^{F190L}. That is the amino acid phenylalanine (F) at position 190 of the F gene product is substituted to the amino acid leucine (L) at the respective position. The combination of boths mutations at amino acid position 117 (F^{F117S}) and 190 (F^{F190L}) is particularly preferred. With regard to amino acid position 289 the amino acid is substituted to an amino acid with an aliphatic side chain other than leucine (L). Preferrably the mutated F gene encodes F^{L289A}. That is the amino acid leucine (L) at position 289 of the F gene product is substituted to the amino acid alanine (A) at the respective position.

In a particular preferred embodiment the nucleic acid is derived from the NDV strain MTH-68/H.

The nucleic acid can also comprise a nucleic acid sequence (M gene) encoding a mutated M protein. Preferably the mutated M gene encodes a matrix protein with an amino acid substitution at position 165 to an amino acid with an aromatic side chain. Most preferred the mutated M gene encodes M^{G165W}. That is the amino acid glycine (G) at position 165 of the M gene product is substituted to the amino acid tryptophane (W) at the respective position. In one embodiment of the present invention the mutation in the M gene is comprised in combination with the mutations in the HN gene and in the F gene as specified supra.

In a particular preferred embodiment the nucleic acid is derived from the NDV strain MTH-68/H and comprises a mutation in the HN gene resulting in an amino acid with a hydrophobic side chain other than phenylalanine at amino acid position 277, a mutation in the M gene resulting in a matrix protein with an amino acid substitution at position 165 to an amino acid with an aromatic side chain, and at least two mutation in the F gene resulting in a fusion protein with an amino acid substitution at position 117 to an amino acid with a hydroxylated side chain and/or an amino acid substitution at position 190 to an amino acid with an aliphatic side chain and an amino acid substitution at position 289 to an amino acid with an aliphatic side chain other than leucine (L). Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), and the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L) and the amino acid leucine (L) at position 289 is substituted to the amino acid alanine (A). The combination of the three mutations at amino acid position 117 (F^{F117S}), position 190 (F^{F190L}) and position 289 (F^{L289A}) of the F gene is particularly preferred.

The nucleic acid can of the present invention can, in addition to the above described mutation in the HN gene, particulary the mutation resulting in an amino acid substitution at position 277 of the HN gene product as specified supra, and in addition to the above described at least two mutations in the F gene, particulary the mutation resulting in an amino acid substitution at position 117 (F^{F117S}) and/or at position 190 (F^{F190L}) and position 289 (F^{L289A}) of the F gene product as specified supra, optionally in combination with the above described mutation on the M gene, particulary the mutation resulting in an amino acid exchange at position 165 of the M gene product as specified supra, also comprises a nucleic acid sequence (L gene) having at least one mutation. Preferably the mutated L gene encodes for a L protein with at least one amino acid substitution at amino acid position 757, 1551 and/or 1700. At position 757 the amino acid may be changed to an amino acid with an aliphatic side chain other than valine (V). Most preferred the mutated L gene encodes L^{V7571}. That is the amino acid valine (V) at position 757 of the L gene product is substituted to the amino acid isoleucine (I) at the respective position. At position 1551 the amino acid may be changed to an amino acid with a hydroxylated side chain. Most preferred the mutated L gene encodes L^{F1551S}. That is the amino acid phenylalanine (F) at position 1551 of the L gene product is substituted to the amino acid serine (S) at the respective position. At position 1700 the amino acid may be changed to an amino acid with an aliphatic side chain. Most preferred the mutated L gene encodes L^{R1700L}. That is the amino acid arginine (R) at position 1700 of the L gene product is substituted to the amino acid leucine (L) at the respective position. The mutations at amino acid positions 757, 1551 and 1700 of the L gene may be present alone or in each possible combination, i.e. position 757 and/or position 1551 and/or position 1700. The combination of all three mutations in the L gene (L3) is particularly preferred. In one embodiment of the present invention the at least one mutation in the L gene, preferably all three mutation at the respective amino acid positions 757, 1551 and 1700, is/are comprised in combination with the mutation in the HN gene as specified supra, the two or three mutations in the F gene as specified supra, and optionally, also in combination with the mutation in the M gene as specified supra.

In a particular preferred embodiment the nucleic acid is derived from the NDV strain MTH-68/H and comprises a mutation in the HN gene resulting in an amino acid with a hydrophobic side chain other than phenylalanine at position 277 of the respective amino acid sequence, a mutation in the M gene resulting in a matrix protein with an amino acid substitution at position 165 to an amino acid with an aromatic side chain, mutations of the F gene resulting in a fusion protein with an amino acid substitution at position 117 to an amino acid with a hydroxylated side chain and/or an amino acid substitution at position 190 to an amino acid with an aliphatic side chain and an amino acid substitution at position 289 to an amino acid with an aliphatic side chain other than leucine (L), and at least one mutation in the L gene resulting in a L protein with an amino acid substitution at position 757 to an amino acid with an aliphatic side chain other than valine (V), and/or an amino acid substitution at position 1551 to an amino acid with a hydroxylated side chain, and/or an amino acid substitution at position 1700 to an amino acid with an aliphatic side chain. More preferred, the mutated L gene results in a L protein comprising the three amino acid substitutions at positions 757, 1551 and 1700. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L) and the amino acid leucine (L) at position 289 is substituted to the amino acid alanine (A), and the mutated L gene encodes for a L protein in which the amino acid valine (V) at position 757 is substituted to the amino acid isoleucine (I), and/or the amino acid phenylalanine (F) at position 1551 is substituted to the amino acid serine (S), and/or the amino acid arginine (R) at position 1700 is substituted to the amino acid leucine (L). Still more preferred the L protein comprises all three mutations at positions 757, 1551 and 1700.

Also within the scope of the present invention are those nucleic acids comprising, in addition to the mutated HN gene and to the mutated F gene encoding at least two mutations at amino acid position 117 and/or 190 and 289, preferably in combination with a mutated M gene with one or more mutation, and the mutated L gene, as specified supra, a nucleic acid sequence (L gene) encoding, in addition to the at least one mutation at amino acid position 757 and/or 1551 and/or 1700, at least one an amino acid substitution at position 1717 and/or at position 1910 of the L proteine, and/or a mutated P gene as specified supra.

In one embodiment of the present invention the nucleic acid consists of or comprises at least one of the nucleic acids according to SEQ ID No. 2 or 3 or parts thereof. In another preferred embodiment the nucleic acid of the present invention has a sequence identity of at least 75 %, particularly of at least 90 %, more particularly of at least 95 % to any of the sequences according to SEQ ID No. 2 or 3.

The nucleic acid as described supra can additionally comprise a transgenic construct or inserted nucleic acid sequence comprising a nucleic acid sequence encoding at least one protein or antibody or a part thereof. Such a nucleic acid is also referred to as a recombinant nucleic acid herein. The at least one protein or antibody or antigen-binding part can be selected from the foreign gene products according to group A. The transgenic construct can be a nucleic acid sequence according to any of SEQ ID No. 5 to 30 of the sequence listing or a variant or part thereof.

In one embodiment of the present invention the nucleic acid or recombinant nucleic acid is derived from a NDV as described supra.

The skilled artisan knows how to use the disclosed sequences to produce synthetic DNAs (e.g. vectors, in particular in rg-NDV vectors), and how to use the synthetic DNAs to reconstruct and express the viral genome. Thus, the skilled artisan is enabled to produce virus particles and the respective nucleic acids by using the information disclosed herein.

In the following a method for obtaining recombinant viral genomes will be described in detail. A person skilled in the art knows how to transfer the disclosed method for obtaining mutated viral genomes not comprising a transgenic construct.

Recombinant viral genomes, which can be used to rescue virus particles, can for example be obtained by a method called 'reverse genetics'. Therefore, in another aspect the present invention also provides a method (herein also called 'reverse genetics') for providing cloned full-length cDNA of the NDV strains and recombinant NDV strains according to the present invention. In a further embodiment, the invention also provides infectious virus particles obtained from said full-length cDNA of the NDV strains or recombinant NDV strains according to the present invention. These NDV strains have an unexpected beneficial replication capability in cell cultures, eggs or animals that are used to propagate the virus for pharmaceutical purposes, and have good or even an improved viral safety profile, giving production advantages to such improved rgNDV strains. Furthermore, the higher replication rate is also particularly useful to obtain the desired oncolytic effects in vivo in that more virus particles will be produced for subsequent rounds of infection of cancer cells that were missed in the first round of infection. What is more, if the NDV strain is recombinant, higher levels (yields) of the foreign gene or the foreign genes encoded by the transgenic construct will be achieved for providing the desired enhanced oncolytic effects in vivo. Thus, by modifying the HN protein within a transgene-expressing NDV higher yields of the transgenic construct protein can be obtained, which is very useful in anti-tumor immunotherapy. By modifying the F protein, and preferably also the L protein, as specified supra, beside the oncolytic potential also the viral safety can be improved.

The NDV strains according to the present invention can be obtained by a reverse genetic method for preparing an rgNDV, optionally encoding at least one foreign gene, preferably at least one foreign gene according to group A or comprising a nucleic acid sequence according to any of SEQ ID No. 5 to 30 of the sequence listing, and having improved replication in a cancer cell, and good or even improved viral safety, over a parent NDV.

The method according to the present invention comprises providing a nucleic acid construct comprising a HN gene with a mutation, particularly wherein the mutation in the HN gene leads to a change in the expression of the hemagglutinin-neuraminidase, wherein the amino acid, particularly phenylalanine (F), in position 277 is substituted, particularly wherein the amino acid in position 277 is substituted to an amino acid with a hydrophobic side chain, more particularly wherein the amino acid in position 277 is substituted to leucine (L) at position 277 of the HN gene product / HN protein, and comprising a F gene encoding a fusion protein / F protein with an amino acid substitution at position 117 to an amino acid with a hydroxylated side chain and/or with an amino acid substitution at position 190 to an amino acid with an aliphatic side chain and an amino acid substitution at position 289 to an amino acid with an aliphatic side chain other than leucine. Still more preferred is a substitution of the amino acid phenylalanine (F) at position 117 of the F gene product to the amino acid serine (S) and/or a substitution of the amino acid phenylalanine (F) at position 190 of the F gene product to the amino acid leucine (L) and a substitution of the amino acid leucine (L) at position 289 of the F gene product to the amino acid alanine (A). The combination of all three mutations in the F gene is particularly preferred.

The method further comprises a step of providing a nucleic acid encoding a rgNDV further comprising a transgenic construct encoding at least one of the foreign gene products according to group A or or comprising a nucleic acid sequence according to any of SEQ ID No. 5 to 30 of the sequence listing.

In the next step of the claimed method the nucleic acid construct with said mutations in the HN gene and the at least two mutations in the F gene at amino acid position 117 and/or position 190 and position 289 (optionally with further mutations, in particular those as specified herein) is incorporated in the nucleic acid encoding a rgNDV comprising the transgenic construct in order to obtain a full length cDNA of the NDV, which additionally comprises a transgenic construct as specified supra. In a preferred embodiment this method step results in a nucleic acid as disclosed supra.

The nucleic acid encoding a rgNDV further comprising a transgenic construct as specified supra can be obtained by generating sub-genomic cDNA fragments and assembling the full length cDNA of the recombinant NDV from these fragments.

Following these method steps the thus obtained nucleic acid encoding a mutated and optionally recombinant rgNDV according to the present invention is used to produce infectious rgNDV particles, which replication characteristics and expression rates of the encoded foreign protein(s) in cancer cells are compared with the replication characteristics and expressions rates of the parent NDV used for designing the recombinant and mutated NDV strain. Also the ICPI values of the produced infectious rgNDV particles are compared with the parent NDV.

Finally, said rgNDV is selected for further use, when it shows an improved replication characteristic over the parent NDV and good or even an improved viral safety, as well as a sufficient expression of the gene product or gene products of the at least one foreign genes.

If it is intended to introduce further to the mutation in the HN gene and the at least two mutations in the F gene at amino acid position 117 and/or position 190 and position 289, at least one further mutation in a viral gene, preferably the M gene, the L gene and/or the P gene, the selected recombinant and mutated rgNDV comprising a transgenic construct as specified supra and a mutated HN gene is used for incorporating a nucleic acid construct encoding a mutated M gene, L gene or P gene, and the method for introducing the mutations in the HN gene and F gene is repeated respectively. Alternatively there is already provided a nucleic acid encoding a rgNDV with the transgenic construct and carrying a mutation in the M gene, F gene, L gene or P gene, particularly wherein said mutation is capable of improving oncolytic potential of said rgNDV and/or viral safety.

According to a preferred embodiment of the present invention the mutation in the M gene comprises or is a mutation which encodes a matrix protein with an amino acid substitution at position 165 to an amino acid with an aromatic side chain. Still more preferred is a substitution of the amino acid glycine (G) at position 165 of the M gene product to the amino acid tryptophane (W).

According to a still further preferred embodiment of the present invention the at least one mutation in the L gene comprises or is a mutation which encodes a L protein with an amino acid substitution at position 757 to an amino acid with an aliphatic side chain other than valine, and/or with an amino acid substitution at position 1551 to an amino acid with a hydroxylated side chain, and/or with an amino acid substitution at position 1700 to an amino acid with an aliphatic side chain. Still more preferred is a substitution of the amino acid valine (V) at position 757 of the L gene product to the amino acid isoleucine (I) and/or a substitution of the amino acid phenylalanine (F) at position 1551 of the L gene product to the amino acid serine (S), and/or a substitution of the amino acid arginine (R) at position 1700 of the L gene product to the amino acid leucine (L). Optionally, the L gene may also comprise a mutation which encodes a L protein with an amino acid substitution at position 1717 to an amino acid with an aromatic side chain other than tyrosine, preferably a substitution of the amino acid tyrosine (Y) at position 1717 of the L gene product to the amino acid histidine (H), and/or a mutation which encodes a L protein with an amino acid substitution at position 1910 to an amino acid with a basic side chain, preferably a substitution of the amino acid glutamic acid (E) at position 1910 of the L gene product to the amino acid lysine (K).

According to another preferred embodiment of the present invention the mutation is in the RNA-editing sequence of the P gene. More preferably, the mutation abolishes and/or decreases the expression of the V protein.

It is also within the scope of the present invention that the viral genome comprises nucleic acid sequences comprising a combination of two or more mutations, which are selected from a mutation in the HN gene, a mutation in the M gene, at least two mutations in the F gene, at least one mutation in the L gene, and a mutation in the P gene. For the details of the respective mutations, reference to the detailed disclosure of each of these mutations given supra and in tables 1 is made.

The invention also provides an isolated, recombinant nucleic acid encoding a rgNDV obtainable by a method for preparing an rgNDV having improved replication in a cancer cell in comparison to that of a parent NDV, as described supra.

In one preferred embodiment the isolated and recombinant nucleic acid encodes a rgNDV provided with a mutation in the HN gene resulting in a change of the amino acid phenylalanine (F) at amino acid position 277 of the hemagglutinin-neuraminidase into a leucine (L), said mutation (a F277L mutation) allowing replication of said rgNDV in a human cancer cell to a higher level than replication of an otherwise identical rgNDV having the amino acid phenylalanine (F) at position 277 in the HN gene product, and with mutations in the F gene resulting in a change of the amino acid phenylalanine (F) at amino acid position 117 of the fusion protein into serine (S) and/or resulting in a change of the amino acid phenylalanine (F) at amino acid position 190 of the fusion protein into leucine (L) and resulting in a change of the amino acid leucine (L) at amino acid position 289 of the fusion protein into alanine (A). These mutations are provided in combination with a transgenic construct encoding any of the foreign genes or parts or variants as specified supra. The rgNDV may further comprise at least one or more mutation(s) selected from a mutation in the M gene, preferably G165W, a mutation at amino acid position 757, 1551, 1700, 1717 and/or 1910 in the L gene, preferably V757I, F1551S, R1700L, Y1717H and/or E1910K, and a mutation in the P gene, preferably the mutation in the P gene abolishes and/or decreases the expression of the V protein.

According to a particularly preferred embodiment the nucleic acid used to engineer a NDV genome or a recombinant NDV genome is derived from the NDV strain MTH-68/H. Thus the present invention provides in one embodiment nucleic acids, preferably obtainable by the above specified method, which are or comprise
- rgNDV-Mut HN(F277L)/F(F117S)/F(F190L)/F(L289A)
- rgNDV-Mut HN(F277L)/F(F117S)/F(F190L)/F(L289A)-Atezolizumab
- rgNDV-Mut HN(F277L)/F(F117S)/F(F190L)/F(L289A)-Bevacizumab
- rgNDV-Mut HN(F277L)/F(F117S)/F(F190L)/F(L289A)-Linlumab
- rgNDV-Mut HN(F277L)/F(F117S)/F(F190L)/F(L289A)-Relatlimab
- rgNDV-Mut HN(F277L)/F(F117S)/F(F190L)/F(L289A)-Monalizumab
- rgNDV-Mut HN(F277L)/F(F117S)/F(F190L)/F(L289A)-TRX518
- rgNDV-Mut HN(F277L)/F(F117S)/F(F190L)/F(L289A)-BMS 986178
- rgNDV-Mut HN(F277L)/F(F117S)/F(F190L)/F(L289A)-CD40
- rgNDV-Mut HN(F277L)/F(F117S)/F(F190L)/F(L289A)-CD80
- rgNDV-Mut HN(F277L)/F(F117S)/F(F190L)/F(L289A)-(ns)hIL-12
- rgNDV-Mut HN(F277L)/F(F117S)/F(F190L)/F(L289A)-eGFP
- rgNDV-Mut HN(F277L)/F(F117S)/F(F190L)/F(L289A)-Nivolumab
- rgNDV-Mut HN(F277L)/F(F117S)/F(F190L)/F(L289A)-Ipilimumab
- rgNDV-Mut HN(F277L)/F(F117S)/F(F190L)/F(L289A)-IL-12
- rgNDV-Mut HN(F277L)/F(F117S)/F(F190L)/F(L289A)-NS1
- rgNDV-Mut HN(F277L)/F(F117S)/F(F190L)/F(L289A)-Apoptin
- rgNDV-Mut HN(F277L)/F(F117S)/F(F190L)/F(L289A)-B18R
- rgNDV-Mut HN(F277L)/F(F117S)/F(F190L)/F(L289A)-Theralizumab
- rgNDV-Mut HN(F277L)/F(F117S)/F(F190L)/F(L289A)-anti-CD40
- rgNDV-Mut HN(F277L)/F(F117S)/F(F190L)/F(L289A)-anti-CD80
- rgNDV-Mut HN(F277L)/F(F117S)/F(F190L)/F(L289A)-anti-CA 15-3
- rgNDV-Mut HN(F277L)/F(F117S)/F(F190L)/F(L289A)-anti CA 19-9
- rgNDV-Mut HN(F277L)/F(F117S)/F(F190L)/F(L289A)-Sofituzumab
- rgNDV-Mut HN(F277L)/F(F117S)/F(F190L)/F(L289A)-Cetuximab
- rgNDV-Mut HN(F277L)/F(F117S)/F(F190L)/F(L289A)-Trastuzumab
- rgNDV-Mut HN(F277L)/F(F117S)/F(F190L)/F(L289A)-BIL=3s
- rgNDV-Mut HN(F277L)/F(F117S)/F(F190L)/F(L289A)-J591
- rgNDV-Mut HN(F277L)/F(F117S)/F(F190L)/F(L289A)-Ramucircumab
- rgNDV-Mut HN(F277L)/F(F117S)/F(F190L)/F(L289A)-TRAIL.

In another embodiment the present invention provides nucleic acids, preferably obtainable by the above specified method, which are or comprise
- rgNDV-Mut HN(F277L)/M(G165W)/F(F117S)/F(F190L)/F(L289A)/L(V757I)/ L(F1551S)/L(R1700L) (= rgNDV-NoThaBene-2b)
- rgNDV-Mut HN(F277L)/M(G165W)/F(F117S)/F(F190L)/F(L289A)/L(V757I)/ L(F1551S)/L(R1700L)-Atezolizumab (= rgNDV-NoThaBene-2b-Atezolizumab)
- rgNDV-Mut HN(F277L)/M(G165W)/F(F117S)/F(F190L)/F(L289A)/L(V757I)/ L(F1551S)/L(R1700L)-Bevacizumab (= rgNDV-NoThaBene-2b-Bevacizumab)
- rgNDV-Mut HN(F277L)/M(G165W)/F(F117S)/F(F190L)/F(L289A)/L(V757I)/ L(F1551S)/L(R1700L)-Lirilumab (= rgNDV-NoThaBene-2b-Lirilumab)
- rgNDV-Mut HN(F277L)/M(G165W)/F(F117S)/F(F190L)/F(L289A)/L(V757I)/ L(F1551S)/L(R1700L)-Relatlimab(=rgNDV-NoThaBene-2b-Relatlimab)
- rgNDV-Mut HN(F277L)/M(G165W)/F(F117S)/F(F190L)/F(L289A)/L(V757I)/ L(F1551S)/L(R1700L)-Monalizumab (= rgNDV-NoThaBene-2b-Monalizumab)
- rgNDV-Mut HN(F277L)/M(G165W)/F(F117S)/F(F190L)/F(L289A)/L(V757I)/ L(F1551S)/L(R1700L)-TRX518 (= rgNDV-NoThaBene-2b-TRX518)
- rgNDV-Mut HN(F277L)/M(G165W)/F(F117S)/F(F190L)/F(L289A)/L(V757I)/ L(F1551S)/L(R1700L)-BMS 986178 (= rgNDV-NoThaBene-2b-BMS 986178)
- rgNDV-Mut HN(F277L)/M(G165W)/F(F117S)/F(F190L)/F(L289A)/L(V757I)/ L(F1551S)/L(R1700L)-CD40 (= rgNDV-NoThaBene-2b-CD40)
- rgNDV-Mut HN(F277L)/M(G165W)/F(F117S)/F(F190L)/F(L289A)/L(V757I)/ L(F1551S)/L(R1700L)-CD80 (= rgNDV-NoThaBene-2b-CD80)
- rgNDV-Mut HN(F277L)/M(G165W)/F(F117S)/F(F190L)/F(L289A)/L(V757I)/ L(F1551S)/L(R1700L)-(ns)hIL-12 (= rgNDV-NoThaBene-2b-(ns)hIL-12)
- rgNDV-Mut HN(F277L)/M(G165W)/F(F117S)/F(F190L)/F(L289A)/L(V757I)/ L(F1551S)/L(R1700L)-eGFP (= rgNDV-NoThaBene-2b-eGFP)
- rgNDV-Mut HN(F277L)/M(G165W)/F(F117S)/F(F190L)/F(L289A)/L(V757I)/ L(F1551S)/L(R1700L)-Nivolumab (= rgNDV-NoThaBene-2b-Nivolumab)
- rgNDV-Mut HN(F277L)/M(G165W)/F(F117S)/F(F190L)/F(L289A)/L(V757I)/ L(F1551S)/L(R1700L)-Ipilimumab (= rgNDV-NoThaBene-2b-Ipilimumab)
- rgNDV-Mut HN(F277L)/M(G165W)/F(F117S)/F(F190L)/F(L289A)/L(V757I)/ L(F1551S)/L(R1700L)-IL-12 (= rgNDV-NoThaBene-2b-IL-12)
- rgNDV-Mut HN(F277L)/M(G165W)/F(F117S)/F(F190L)/F(L289A)/L(V757I)/ L(F1551S)/L(R1700L)-NS1 (= rgNDV-NoThaBene-2b-NS1)
- rgNDV-Mut HN(F277L)/M(G165W)/F(F117S)/F(F190L)/F(L289A)/L(V757I)/ L(F1551S)/L(R1700L)-Apoptin (= rgNDV-NoThaBene-2b-Apoptin)
- rgNDV-Mut HN(F277L)/M(G165W)/F(F117S)/F(F190L)/F(L289A)/L(V757I)/ L(F1551S)/L(R1700L)-B18R(=rgNDV-NoThaBene-2b-B18R)
- rgNDV-Mut HN(F277L)/M(G165W)/F(F117S)/F(F190L)/F(L289A)/L(V757I)/ L(F1551S)/L(R1700L)-Theralizumab (= rgNDV-NoThaBene-2b-Theralizumab)
- rgNDV-Mut HN(F277L)/M(G165W)/F(F117S)/F(F190L)/F(L289A)/L(V757I)/ L(F1551S)/L(R1700L)-anti-CD40 (= rgNDV-NoThaBene-2b-anti-CD40)
- rgNDV-Mut HN(F277L)/M(G165W)/F(F117S)/F(F190L)/F(L289A)/L(V757I)/ L(F1551S)/L(R1700L)-anti-CD80 (= rgNDV-NoThaBene-2b-anti-CD80)
- rgNDV-Mut HN(F277L)/M(G165W)/F(F117S)/F(F190L)/F(L289A)/L(V757I)/ L(F1551S)/L(R1700L)-anti-CA 15-3 (= rgNDV-NoThaBene-2b-anti-CA 15-3)
- rgNDV-Mut HN(F277L)/M(G165W)/F(F117S)/F(F190L)/F(L289A)/L(V757I)/ L(F1551S)/L(R1700L)-anti CA 19-9 (= rgNDV-NoThaBene-2b-anti CA 19-9)
- rgNDV-Mut HN(F277L)/M(G165W)/F(F117S)/F(F190L)/F(L289A)/L(V757I)/ L(F1551S)/L(R1700L)-Sofituzumab (= rgNDV-NoThaBene-2b-Sofituzumab)
- rgNDV-Mut HN(F277L)/M(G165W)/F(F117S)/F(F190L)/F(L289A)/L(V757I)/ L(F1551S)/L(R1700L)-Cetuximab (= rgNDV-NoThaBene-2b-Cetuximab)
- rgNDV-Mut HN(F277L)/M(G165W)/F(F117S)/F(F190L)/F(L289A)/L(V757I)/ L(F1551S)/L(R1700L)-Trastuzumab(=rgNDV-NoThaBene-2b-Trastuzumab)
- rgNDV-Mut HN(F277L)/M(G165W)/F(F117S)/F(F190L)/F(L289A)/L(V757I)/ L(F1551S)/L(R1700L)-BIL=3s (= rgNDV-NoThaBene-2b-BIL=3s)
- rgNDV-Mut HN(F277L)/M(G165W)/F(F117S)/F(F190L)/F(L289A)/L(V757I)/ L(F1551S)/L(R1700L)-J591 (= rgNDV-NoThaBene-2b-J591)
- rgNDV-Mut HN(F277L)/M(G165W)/F(F117S)/F(F190L)/F(L289A)/L(V757I)/ L(F1551S)/L(R1700L)-Ramucircumab (= rgNDV-NoThaBene-2b-Ramucircumab)
- rgNDV-Mut HN(F277L)/M(G165W)/F(F117S)/F(F190L)/F(L289A)/L(V757I)/ L(F1551S)/L(R1700L)-TRAIL (= rgNDV-NoThaBene-2b-TRAIL).

A NDV or rgNDV according to the present invention can be provided in a suitable cell or cell line, for example a HeLa cell line or in cancer cells, or an embryonated egg that is susceptible to a NDV infection.

In these infected cells the virus is grown to sufficient quantities, and preferably under sufficient conditions such that the virus is free from exogenous contamination, and the progeny virus particles are collected by suitable methods well known to those skilled in the art.

Even though the NDV strains as described above, for example the F3L3 strains, are particularly beneficial for expressing the foreign genes as mentioned above, also F2L3 strains of NDV can be advantageously used to express these foreign genes. Even more general, NDV strains having the mutation in the HN gene relating to the amino acid position 277 as specified above and having at least one mutation in the F gene concerning amino acid position 117 and/or amino acid position 190 as specified, but not the mutation at amino acid position 289, above can be beneficially used.

Thus, in a second aspect the present invention also encompasses the recombinant NDV strains comprising a viral genome comprising a nucleic acid comprising a nucleic acid sequence encoding at least one foreign gene. The at least one foreign gene being selected from the following "group B":
- A gene encoding an antibody capable of blocking checkpoint inhibition, such as an antibody directed against checkpoint inhibition protein PD-1 (programmed cell death protein 1, also known as CD279) or an antigen-binding part directed to protein PD-1 (anti-PD-1). They work by blocking a signal that prevents activated T cells from attacking the cancer upon expression in rNDV-expresser cells, thus allowing the immune system to clear the cancer. In one embodiment the antibody is Nivolumab, an antigen-binding part of Nivolumab, a variant of Nivolumab or a variant of an antigen-binding part of Nivolumab.
- A gene encoding an antibody capable of blocking checkpoint inhibition, such as an antibody directed to the surface protein CTLA-4 or an antigen-binding part directed to protein CTLA-4 (anti-CTLA-4). In one embodiment the antiboby is Ipilimumab, an antigen-binding part of Ipilimumab or a variant of Ipilimumab or a variant of an antigen-binding part of Ipilimumab.
- A gene encoding a protein which improves the cellular immune response and improves the ability of T cells to enter tumor cells, or a part thereof which improves the cellular immune response and improves the ability of T cells to enter tumor cells. In one embodiment the protein is interleukin-12 (IL-12) or a part of interleukin-12 or a variant of interleukin-12 or a variant of a part of interleukin-12.
- A gene encoding a protein with the ability to modulate the virus replication cycle, or a part thereof with the ability to modulate the virus replication cycle. In one embodiment the protein is the non-structural protein NS1 of influenza A virus or a part of the non-structural protein NS1 of influenza A virus or a variant of the non-structural protein NS1 of influenza A virus or a variant of a part of the non-structural protein NS1 of influenza A virus.
- A gene encoding a protein with the ability to selectively induce apoptosis in human tumor cells, but not in normal human cells, or a part thereof with the ability to selectively induce apoptosis in human tumor cells, but not in normal human cells. In one embodiment the protein is Apoptin (VP3 from chicken anemia virus) or a part of Apoptin or a variant of Apoptin or a variant of a part of Apoptin.
- A gene encoding a protein that reduces or inhibits IFN expression such as an IFN-beta receptor, or a part thereof that reduces or inhibits IFN expression such as an IFN-beta receptor. In one embodiment the protein is the viral protein B 18R from vaccinia virus or a part of the viral protein B18R from vaccinia virus or a variant of the viral protein B18R from vaccinia virus or a variant of a part of the viral protein B18R from vaccinia virus. B18R is a homologue of the human IFN-β receptor. B18R acts as a decoy for IFN-β, thereby inhibiting activation by cell signaling of the antiviral host response in native cells, resulting in increased lytic activity in cancer cells.
- A gene encoding the protein CD40 (cluster of differentiation 40), a part of CD40, a variant of CD40 or a variant of a part of CD40.
- A gene encoding an antibody, preferably an agonistic antibody, directed to CD28 or an antigen-binding part directed to CD28 (anti-CD28). CD28 (cluster of differentiation 28) is one of the proteins expressed on T cells that provide costimulatory signals required for T cell activation and survival. T cell stimulation through CD28 in addition to the T-cell receptor (TCR) can provide a potent signal for the production of various interleukins. In one embodiment the antibody is Theralizumab, an antigen-binding part of Theralizumab, a variant of Theralizumab or a variant of an antigen-binding part of Theralizumab.
- A gene encoding an antibody, preferably an agonistic antibody, directed to CD40 or an antigen-binding part directed to CD40 (anti-CD40). CD40 (cluster of differentiation 40) is a costimulatory protein found on antigen-presenting cells and is required for their activation. Thus, the antidody or its antigen-binding part is used to activate an anti-tumor T cell response via activation of the antigen-presenting cells.
- A gene encoding an antibody, preferably an agonistic antibody, directed to CD80 or an antigen-binding part directed to CD80 (anti-CD80). CD80 (cluster of differentiation 80) is amembrane protein which is the receptor for the proteins CD28 and CTLA-4, and which membrane protein is involved in the costimulatory signal essential for T-lymphocyte activation. Thus, the antidody or its antigen-binding part is used to activate T-lymphocytes.
- A gene encoding an antibody directed to CA 15-3 or an antigen-binding part directed to CA 15-3 (anti-CA 15-3). CA 15-3 is also known as the human gene MUC1.
- A gene encoding an antibody directed to CA 19-9 or an antigen-binding part directed to CA 19-9 (anti-CA 19-9). CA 19-9 is a muzin which corresponds to the sialylated Lewisa blood group antigen.
- A gene encoding an antibody directed to CA 125 or an antigen-binding part directed to CA 125 (anti-CA 125). CA 125 is also known as the human gene MUC16. An antibody or its antigen-binding part targeting the MUC16 protein results in a G2/M-phase growth arrest and tumour cell apoptosis. In one embodiment the antibody is Sofituzumab, an antigen-binding part of Sofituzumab, a variant of Sofituzumab or a variant of an antigen-binding part of Sofituzumab.
- A gene encoding an antibody, preferably an antagonistic antibody, directed to EGFR or an antigen-binding part directed to EGFR (anti-EGFR). The epidermal growth factor receptor (EGFR) is a transmembrane protein. Permanent activation of the receptor results in an uncontrolled cell proliferation and prevents controlled cell death (apoptosis), which contributes to the malignant transformation of cells. The antagonistic antibody is directed against the extracellular domain of the EGFR and leads to an inhibition of ligand binding. In one embodiment the antibody is Cetuximab, an antigen-binding part of Cetuximab, a variant of Cetuximab or a variant of an antigen-binding part of Cetuximab.
- A gene encoding an antibody, preferably an antagonistic antibody, directed to HER2 or an antigen-binding part directed to HER2 (anti-HER2). The antibody or its antigen-binding part is targeting the extracellular part of the HER2 receptor, which is overexpressen in certain cancers, and is intended to interrupt the growth-promoting signals. In one embodiment the antibody is Trastuzumab, an antigen-binding part of Trastuzumab, a variant of Trastuzumab or a variant of an antigen-binding part of Trastuzumab.
- A gene encoding an antibody directed to nfP2X₇ or an antigen-binding part directed to nfP2X₇ (anti-nfP2X₇). The non-functioning P2X₇ receptor is a form of the ATP-gated P2X₇ receptor in which the E200 epitope is exposed for antibody binding. The nfP2X₇ is able to form a Ca²⁺channel but not an apoptotic pore and is found on every type of cancer cell but no normal cells. In one embodiment the antibody is BIL=3s, an antigen-binding part of BIL=3s, a variant of BIL=3s or a variant of an antigen-binding part of BIL=3 s.
- A gene encoding an antibody directed to PSMA or an antigen-binding part directed to PSMA (anti-PSMA). The prostate specific membrane antigen (PSMA) is a cell surface peptidase highly expressed by malignant prostate epithelial cells and vascular endothelial cells of numerous solid tumor malignancies. In one embodiment the antibody is J591, an antigen-binding part of J591, a variant of J591 or a variant of an antigen-binding part of J591.
- A gene encoding an antibody, preferably an antagonistic antibody, directed to VEGFR or an antigen-binding part directed to VEGFR (anti-VEGFR). The vascular endothelial growth factor (VEGF) is involved in angiogenesis. The natural ligands comprise VEGF-A, VEGF-C and VEGF-D. Antagonictic antibodies targeting VEGF lead to to inhibition of VEGF-mediated tumor angiogenesis. In one embodiment the antibody is Ramucirumab, an antigen-binding part of Ramucirumab, a variant of Ramucirumab or a variant of an antigen-binding part of Ramucirumab.
- A gene encoding a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5. In one embodiment the protein is TRAIL, a part of TRAIL, a variant of TRAIL or a variant of a part of TRAIL.
- Any combination of these genes, parts or variants of these genes or parts thereof.

In the following all these genes encoding the respective antibodies, antigen-binding parts, proteins or parts thereof as well as any of the preferably mentioned antibodys, antigen-bindings parts, proteins, parts or variants thereof, are referred to in the following as "the foreign genes according to group B". This group B also encompasses any combination of these genes, parts or variants of these genes or parts thereof.

The respective proteins or antibodies or parts thereof are also referred to in the following as "the foreign gene products according to group B". This group also encompasses any combination of these gene products, parts or variants thereof.

In another embodiment of the present invention a recombinant NDV comprises in its viral genome at least two foreign genes or parts or variants thereof, which foreign genes or parts or variants are selected from the above mentioned foreign genes according to group B.

Preferred embodiments of a nucleic acid comprising a nucleic acid sequence encoding at least one foreign gene according to group B are given in SEQ ID No. 15 to 30 of the sequence listing. The transgenic construct can also be a variant or part of any of the sequences according to SEQ ID No. 15 to 30 of the sequence listing.

In addition to comprising a transgenic construct comprsing a nucleic acid sequence encoding at least one foreign gene according to group B, the viral genome of the recombinant NDV strains according to the present invention further comprise a nucleic acid comprising a nucleic acid sequence (HN gene) encoding a HN protein having a change in the amino acid sequence of the HN protein and providing the NDV with an increased replication capability in a cancer cell, preferably in a human cancer cell, as compared to an otherwise identical NDV not having the said mutation in the HN gene.

Preferably the mutation in the HN gene results in an unexpected beneficially increased replication capability of the recombinant NDV strain in cancer cells, preferably in human cancer cells, and more preferably results in a replication of said oncolytic NDV in a human cancer cell which is at least 2-fold higher, more preferably 2- to 10-fold higher, still more preferably 5- to 10-fold higher than an NDV parent strain not having said mutation in its viral HN gene. Preferably, said parent strain comprises or is an oncolytic NDV already having an advantageous safety and efficacy profile in humans, such as the NDV strain MTH-68/H.

According to a preferred embodiment the recombinant NDV strain comprises a mutated HN gene encoding a hemagglutinin-neuramidase (HN proteine) with an amino acid substitution at position 277 to an amino acid with a hydrophobic side chain other than phenylalanine. As it relates to preferred and more preferred embodiments of the substitution at amino acid position 277 of the HN protein reference to above disclosure with regard to the novel NDV strains is made. The same preferred and more preferred embodiments / amino acids apply for these recombinant NDV strains according to the second aspect of the present invention.

The oncolytic NDV strain having this mutation in the HN gene and carrying at least one of the foreign genes according to group B can be derived from NDV strain MTH-68/H.

According to the present invention the recombinant and mutated NDV strains comprise in addition to the above described mutated HN gene, a nucleic acid sequence (F gene) encoding a F protein comprising at least one mutation at amino acid position 117 and/or amino acid position 190. The combination of both mutations at amino acid position 117 and 190 of the F gene is particularly preferred. As it relates to preferred and more preferred embodiments of the substitutions at amino acid position 117 and amino acid position 190 of the F gene reference to above disclosure with regard to the novel NDV strains is made. The same preferred and more preferred embodiments / amino acids apply for these recombinant NDV strains according to the second aspect of the present invention. The recombinant NDV strain can be a NDV strain derived from NDV strain MTH-68/H having the mutation in the HN gene product or HN protein at position 277 and having at least one mutation in the F gene at amino acid position 117 and/or 190. A strain comprising the mutation at amino acid position 117 is also referred to as NDV-Mut HN(F277L)/F(F117S). That is, the same nomenclature as explained above is used. The strain can also be NDV-Mut HN(F277L)/F(F190L) or NDV-Mut HN(F277L)/F(F117S)/F(F190L). The transgenic construct comprising the foreign gene or part or respective variant thereof carried by these strains is referred to as Nivolumab, Ipilimumab, IL-12, NS1, Apoptin, B18R, CD40, Theralizumab, anti-CD40, anti-CD80, anti-CA 15-3, anti CA 19-9, Sofituzumab, Cetuximab, Trastuzumab, BIL=3s, J591, Ramucircumab or TRAIL in this shortcut for the strain. Within these shortcuts the meaning of the respective foreign gene is as explaine above.

The following NDV strains are provided by the present invention in a preferred embodiment:
- NDV-Mut HN(F277L)/F(F117S)/F(F190L)-Nivolumab
- NDV-Mut HN(F277L)/F(F117S)/F(F190L)-Ipilimumab
- NDV-Mut HN(F277L)/F(F117S)/F(F190L)-IL-12
- NDV-Mut HN(F277L)/F(F117S)/F(F190L)-NS1
- NDV-Mut HN(F277L)/F(F117S)/F(F190L)-Apoptin
- NDV-Mut HN(F277L)/F(F117S)/F(F190L)-B18R
- NDV-Mut HN(F277L)/F(F117S)/F(F190L)-CD40
- NDV-Mut HN(F277L)/F(F117S)/F(F190L)-Theralizumab
- NDV-Mut HN(F277L)/F(F117S)/F(F190L)-anti-CD40
- NDV-Mut HN(F277L)/F(F117S)/F(F190L)-anti-CD80
- NDV-Mut HN(F277L)/F(F117S)/F(F190L)-anti-CA 15-3
- NDV-Mut HN(F277L)/F(F117S)/F(F190L)-anti-CA 19-9
- NDV-Mut HN(F277L)/F(F117S)/F(F190L)-Sofituzumab
- NDV-Mut HN(F277L)/F(F117S)/F(F190L)-Trastuzumab
- NDV-Mut HN(F277L)/F(F117S)/F(F190L)-BIL=3s
- NDV-Mut HN(F277L)/F(F117S)/F(F190L)-J591
- NDV-Mut HN(F277L)/F(F117S)/F(F190L)-Ramucircumab
- NDV-Mut HN(F277L)/F(F117S)/F(F190L)-TRAIL.

It has been surprisingly shown that the at least one mutation, preferably both mutations (also referred to as "F2" herein), in the F gene at position 117 and 190 of the amino acid sequence of the resulting F protein beneficially improves the oncolytic potential of the respective NDV strains by improving the safety profile of the viruses in humans. The respective amino acid substitutions are as specified supra.

With regard to the viral safety profile, this in particular means that the intracerebral pathogenicity index (ICPI) is reduced by these mutations in the F gene, preferably F2, The ICPI value indicates the the pathogenicity of a Newcastle disease virus. Preferably, the ICPI is reduced by at least 10 %, more preferably by at least 20 % and still more preferred by at least 30 %, as compared to the identically determined ICPI value of a NDV strain not having the respective mutation(s) in the F gene. The ICPI can be determined according to the method set forth in World Organization for Animal Health (OIE) 2009, Chapter 2.3.14. Newcastle disease, pp. 576-589, in: Manual of Diagnostic Tests and Vaccines for Terrestrial Animals 2009, World Organization for Animal Health (OIE), Paris.

The recombinant and mutated NDV strains according to the second aspect of the present invention can comprise in addition to the above described mutation in the HN gene, and the at least one mutation in the F gene at amino acid position 117 and/or 190, a mutation in the M gene and the thus encoded matrix protein, preferably at amino acid position 165. Again, as it relates to preferred and more preferred embodiments of the substitution at amino acid position 165 of the M gene reference to above disclosure with regard to the novel NDV strains is made. The same preferred and more preferred embodiments / amino acids apply for these recombinant NDV strains according to the second aspect of the present invention.

According to one embodiment of the present invention a recombinant NDV strain comprises as a foreign gene at least one gene selected from the foreign genes according to group B, and has a mutation in the HN gene, preferably resulting in a substitution of the amino acid phenylalanine (F) to leucine (L) at position 277 of the HN gene product, a mutation in the M gene, preferably resulting in a substitution of the amino acid glycine (G) to the amino acid tryptophane (W) at position 165 of the M gene product, and at least one mutation in the F gene, preferably resulting in a substitution of the amino acid phenylalanine (F) to the amino acid serine (S) at position 117 of the F gene product, and/or resulting in a substitution of the amino acid phenylalanine (F) to the amino acid leucine (L) at position 190 of the F gene product. The combination of both mutations at amino acid position 117 and 190 of the F gene is particularly preferred. As explained supra, other substitutions at these amino acid positions are also comprised within the present invention.

In a preferred embodiment of the present invention the recombinant and mutated NDV strains comprise in addition to the above described mutation in the HN gene and the at least one mutation in the F gene at amino acid positions 117 and/or 190 as specified above, at least one mutation in the L gene and the thus encoded RNA-dependent RNA polymerase protein, also referred to as L protein or L gene product herein. Still more preferred the mutated L gene encodes a RNA-dependent RNA polymerase protein with an amino acid substitution at position 757 and/or position 1551 and/or 1700. Preferably the NDV comprises a mutated L gene and the encoded L protein with the amino acid substitution at position 757, position 1551 and position 1700. Again, as it relates to preferred and more preferred embodiments of the substitutions at amino acid positions 757, 1551 and 1700 of the L gene reference to above disclosure with regard to the novel NDV strains is made. The same preferred and more preferred embodiments / amino acids apply for these recombinant NDV strains according to the second aspect of the present invention. These recombinant NDV strains may in addition also comprise the above described mutation in the M gene.

A NDV strain derived from NDV strain MTH-68/H having a mutation in the HN gene, wherein the amino acid phenylalanine (F) is substituted to leucine (L) at amino acid position 277, having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (W) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product is herein referred to also as NDV-Mut HN(F277L)/M(G165W)/F(F117S)/F(F190L) /L(V757I)/L(F1551S)/L(R1700L) or MutHu2 or NDV-NoThaBene-2. The foreign gene or part or variant thereof carried by this strain is referred to as Nivolumab, Ipilimumab, IL-12, NS1, Apoptin, B18R, CD40, Theralizumab, anti-CD40, anti-CD80, anti-CA 15-3, anti CA 19-9, Sofituzumab, Cetuximab, Trastuzumab, BIL=3s, J591, Ramucircumab or TRAIL. Within these shortcuts for the recombinant NDV strains a reference to the respective foreign gene is intended to have the meaning as already defined above. The following NDV strains are provided by the present invention in a particularly preferred embodiment:
- NDV-Mut HN(F277L)/M(G165W)/F(F117S)/F(F190L)/L(V757I)/L(F1551S)/ L(R1700L)-Nivolumab,
- NDV-Mut HN(F277L)/M(G165W)/F(F117S)/F(F190L)/L(V757I)/L(F1551S)/ L(R1700L)-Ipilimumab,
- NDV-Mut HN(F277L)/M(G165W)/F(F117S)/F(F190L)/L(V757I)/L(F1551S)/ L(R1700L)-IL-12,
- NDV-Mut HN(F277L)/M(G165W)/F(F117S)/F(F190L)/L(V757I)/L(F1551S)/ L(R1700L)-NS1,
- NDV-Mut HN(F277L)/M(G165W)/F(F117S)/F(F190L)/L(V757I)/L(F1551S)/ L(R1700L)-Apoptin,
- NDV-Mut HN(F277L)/M(G165W)/F(F117S)/F(F190L)/L(V757I)/L(F1551S)/ L(R1700L)-B18R,
- NDV-Mut HN(F277L)/M(G165W)/F(F117S)/F(F190L)/L(V757I)/L(F1551S)/ L(R1700L)-CD40,
- NDV-Mut HN(F277L)/M(G165W)/F(F117S)/F(F190L)/L(V757I)/L(F1551S)/ L(R1700L)-Theralizumab,
- NDV-Mut HN(F277L)/M(G165W)/F(F117S)/F(F190L)/L(V757I)/L(F1551S)/ L(R1700L)-anti-CD40,
- NDV-Mut HN(F277L)/M(G165W)/F(F117S)/F(F190L)/L(V757I)/L(F1551S)/ L(R1700L)-anti-CD80,
- NDV-Mut HN(F277L)/M(G165W)/F(F117S)/F(F190L)/L(V757I)/L(F1551S)/ L(R1700L)-anti-CA 15-3,
- NDV-Mut HN(F277L)/M(G165W)/F(F117S)/F(F190L)/L(V757I)/L(F1551S)/ L(R1700L)-anti CA 19-9,
- NDV-Mut HN(F277L)/M(G165W)/F(F117S)/F(F190L)/L(V757I)/L(F1551S)/ L(R1700L)-Sofituzumab,
- NDV-Mut HN(F277L)/M(G165W)/F(F117S)/F(F190L)/L(V757I)/L(F1551S)/ L(R1700L)-Cetuximab,
- NDV-Mut HN(F277L)/M(G165W)/F(F117S)/F(F190L)/L(V757I)/L(F1551S)/ L(R1700L)-Trastuzumab,
- NDV-Mut HN(F277L)/M(G165W)/F(F117S)/F(F190L)/L(V757I)/L(F1551S)/ L(R1700L)-BIL=3s,
- NDV-Mut HN(F277L)/M(G165W)/F(F117S)/F(F190L)/L(V757I)/L(F1551S)/ L(R1700L)-J591,
- NDV-Mut HN(F277L)/M(G165W)/F(F117S)/F(F190L)/L(V757I)/L(F1551S)/ L(R1700L)-Ramucircumab,
- NDV-Mut HN(F277L)/M(G165W)/F(F117S)/F(F190L)/L(V757I)/L(F1551S)/ L(R1700L)-TRAIL.

The two mutations in the F gene at position 117 and 190 of the amino acid sequence of the resulting F protein and the three mutations in the L gene at position 757, 1551 and 1700 of the amino acid sequence of the resulting L protein, either alone or in combination, have been shown to beneficially improve the oncolytic potential of the respective NDV strains by improving the safety profile of the viruses in humans. The combinations include any combination of the referenced mutations in the F gene and L gene, and the F protein and L protein, respectively. That is any combination from two to five mutations. Particularly preferred is a combination of both mutations at amino acid position 117 and 190 of the F gene (also referred to as "F2" herein) and a combination of all five mutations, that is mutations at positions 117 und 190 of the F gene product and at positions 757, 1551 and 1700 of the L gene product (also referred to as "F2L3" herein). The respective amino acid substitutions are as specified supra. The F2 and F2L3 mutations result in a reduced intracerebral pathogenicity index (ICPI). Preferably, the ICPI is reduced by at least 10 %, more preferably by at least 20 % and still more preferred by at least 30 %, as compared to the identically determined ICPI value of a NDV strain not having the respective mutations in the F gene and the L gene.

According to another embodiment of the present invention a recombinant NDV according to the present invention may also comprise at least one further mutation in the L gene and the encoded L protein having an amino acid substitution at position 1717 to an amino acid with aromatic side chain other than tyrosine (Y), preferably where tyrosine (Y) is substituted to histidine (H) at position 1717 of the L gene product, and/or at position 1910 to an amino acid with a basic side chain, preferably where glutamic acid (E) is substituted to lysine (K) at position 1910 of the L gene product.

Alternatively or in addition a recombinant NDV according to the present invention may comprise a mutation in the RNA-editing sequence of the P gene. More preferably, the mutation abolishes and/or decreases the expression of the V protein.

Table 1a shown above gives a list of mutations for obtaining NDV variants with increased titer production and/or good or improved safety profile according to the present invention. The mutation in the HN gene and at least one mutation in the F gene at amino acid position 117 or 190 is comprised in all these NDV strains according to the present invention, while the mentioned mutations in the M gene and L gene may or may not be present. As it relates to the third mutation in the F gene at amino acid position 289, this mutation is not mandatory for the recombinant NDV strains according to the second aspect of the present invention.

Table 1e shows particulary preferred NDV variants with increased titer production and/or improved safety profile in line with the second aspect of the present invention, and the parent NDV strain MTH-68/H. "+" means that at the mentioned amino acid position the original (first mentioned) amino acid is substituted by the last mentioned amino acid, while "-" means that there is no amino acid substitution at the mentioned position, i.e. the first mentioned amino acid is present. For example and with regard to the HN gene "+" means that at amino acid position 277 phenylalanine (F) is substituted to leucine (L).

**Table 1e:**

| Gene | Relevant amino acid | MTH-68/H | | | | | | | | |
|---|---|---|---|---|---|---|---|---|---|---|
| HN | F277L | - | + | + | + | + | + | + | + | + |
| M | G165W | - | - | + | - | + | - | + | - | + |
| F | F117S | - | + | + | + | + | + | + | + | + |
| F | F190L | - | + | + | + | + | + | + | + | + |
| F | L289A | - | - | - | + | + | - | - | + | + |
| L | V757I | - | - | - | - | - | + | + | + | + |
| L | F1551S | - | - | - | - | - | + | + | + | + |
| L | R1700L | - | - | - | - | - | + | + | + | + |
| L | Y1717H | - | - | - | - | - | - | - | + | + |
| L | E1910K | - | - | - | - | - | - | - | + | + |

Table If shows all mutations in the F gene encompassed by the second aspect of the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present. According to the second aspect of the present invention at least one mutation at amino acid position 117 or 190 is mandatory. The combination of both mutations is particularly preferred.

**Table If:**

| Gene | Relevant amino acid | 1 | 1 | 2 | 2 | 2 | 3 |
|---|---|---|---|---|---|---|---|
| F | F117S | + | - | + | + | - | + |
| F | F190L | - | + | + | - | + | + |
| F | L289A | - | - | - | + | + | + |

As it relates to preferred mutations in the L gene reference to Table 1d shown above is made.

In a preferred embodiment the NDV and the recombinant NDV resulting thereof is encoded by and/or comprises at least one of the nucleic acids according to SEQ ID No. 1 or any one of SEQ ID No. 36 to 38 or parts thereof. According to another preferred the recombinant NDV strains of the present invention have a sequence identity of at least 75 %, particularly of at least 90 %, more particularly of at least 95 % to any one of SEQ ID No. 1 or SEQ ID No. 36 to 38. The sequences given by these SEQ ID numbers are as follows:

| | |
|---|---|
| SEQ ID No. 1: | Nucleic acid (cDNA) sequence of the Newcastle disease virus (NDV) strain MTH-68/H genome; |
| SEQ ID No. 36: | Nucleic acid (cDNA) sequence of Newcastle disease virus strain Mut HN(F277L)/M(G165W)/F(F117S)/F(F190L)/L(V757I)/L(F1 551S)/L(R1700L) genome having a mutation in the HN gene, a mutation in the M gene, two mutations in the F gene (F2) and three mutations in the L gene (L3) (= NDV-NoThaBene-2 genome). |
| SEQ ID No. 37: | Nucleic acid (cDNA) sequence of Newcastle disease virus strain according to SEQ ID No.35, but comprising in addition a 12 bp insert containing a AfeI restriction site according to SEQ ID no. 4 between nucleotide positions 3134 and 3135 of SEQ ID No.35. |
| SEQ ID No. 38: | Nucleic acid (cDNA) sequence of Newcastle disease virus strain Mut HN(F277L)/M(G165W)/F(F117S)/F(F190L)/L(V757I)/L(F1 551S)/L(R1700L)-Lirilumab genome created by reverse genetics as disclosed herein, wherein the nucleic acid comprises a mutation in the HN gene, a mutation in the M gene, two mutations in the F gene (F2) and three mutations in the L gene (L3), and additionally encodes Lirilumab. |

The SEQ ID No. 38 of the sequence listing shows by way of example a transgenic NDV strain according to the present invention. Shown is a nucleic acid (cDNA) sequence of Newcastle disease virus strain Mut HN(F277L)/M(G165W)/F(F117S) /F(F190L)/L(V757I)/L(F1551S)/L(R1700L)-Lirilumab genome created by reverse genetics as disclosed herein, wherein the nucleic acid comprises a mutation in the HN gene, a mutation in the M gene, two mutations in the F gene (F2) and three mutations in the L gene (L3), and additionally encodes Lirilumab.

The transgenic construct comprising the nucleic acid sequence encoding Lirilumab is in this example inserted in the 12 nucleotide long sequence according to SEQ ID No. 4 of the sequence listing in the Afel-site.

Instead of the nucleic acid sequence comprising a nucleic acid sequence encoding anti-KIR, preferably Lirilumab, any other nucleic acid sequence according to any one of SEQ ID No. 5 to 25 and SEQ ID No. 27 to 30 can be inserted to obtain a preferred transgenic NDV strain according to the present invention.

In a preferred embodiment of the present invention the viral HN gene product has an amino acid sequence as identified in SEQ ID No. 32, and/or the viral M gene product preferably has an amino acid sequence as identified in SEQ ID No. 34. In addition it is preferred that a NDV strain according to the present invention comprises beside the viral HN gene product having an amino acid sequence as identified in SEQ ID No. 32, and/or the viral M gene product preferably having an amino acid sequence as identified in SEQ ID No. 34, a viral F gene product having an amino acid sequence as identified in SEQ ID No. 39 and/or a viral L gene product having an amino acid sequence as identified in SEQ ID No. 35. Also encompassed by the present invention are NDV strains comprising variants of the gene products according to SEQ ID No. 32, 34, 35 or 39.

The present invention also provides a recombinant nucleic acid comprising the nucleic acid sequence of the recombinant NDV strains according to the second aspect of the present invention. The nucleic acid comprises a transgenic construct. The transgenic construct comprises a nucleic acid sequence encoding at least one foreign gene or transgene. The foreign gene can encode a gene product according to group B. The transgenic construct comprised in the nucleic acid can also be a combination of two or more of the foreign genes according to group B. The transgenic construct can be or comprise a nucleic acid sequence according to any one of SEQ ID No. 15 to 30 of the sequence listing or a part or variant thereof.

In addition to the transgenic construct a nucleic acid according to the present invention comprises a nucleic acid sequence (HN gene) encoding a mutated HN protein. The encoded HN protein is preferably mutated at amino acid position 277. As it relates to preferred and more preferred embodiments of the substitution at amino acid position 277 of the HN gene reference to above disclosure with regard to the novel NDV strains according to the first aspect of the present invention is made. The same preferred and more preferred embodiments / amino acids apply for the recombinant NDV strains according to the second aspect of the present invention.

In addition a nucleic acid according to the present invention comprises a nucleic acid sequence (F gene) having at least one mutation. Preferably the mutated F gene encodes a fusion protein with an amino acid substitution at position 117 and/or position 190. As it relates to preferred and more preferred embodiments of the substitutions at amino acid positions 117 and 190 of the F gene reference to above disclosure with regard to the novel NDV strains according to the first aspect of the present invention is made. The same preferred and more preferred embodiments apply for the recombinant NDV strains according to the second aspect of the present invention.

In a particular preferred embodiment the nucleic acid is derived from the NDV strain MTH-68/H.

The nucleic acid sequence encoding the recombinant NDV can also comprise a nucleic acid sequence (M gene) having a mutation. Preferably the mutated M gene encodes a matrix protein with an amino acid substitution at position 165 to an amino acid with an aromatic side chain. As it relates to preferred and more preferred embodiments of the substitution at amino acid position 165 of the M gene reference to above disclosure with regard to the novel NDV strains according to the first aspect of the present invention is made. The same preferred and more preferred embodiments / amino acids apply for the recombinant NDV strains according to the second aspect of the present invention.

In a particular preferred embodiment the nucleic acid is derived from the NDV strain MTH-68/H and comprises a mutation in the HN gene resulting in an amino acid with a hydrophobic side chain other than phenylalanine at amino acid position 277, a mutation in the M gene resulting in a matrix protein with an amino acid substitution at position 165 to an amino acid with an aromatic side chain, and at least one mutation in the F gene resulting in a fusion protein with an amino acid substitution at position 117 to an amino acid with a hydroxylated side chain and/or an amino acid substitution at position 190 to an amino acid with an aliphatic side chain. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), and the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L). The combination of boths mutations at amino acid position 117 (F^{F117S}) and 190 (F^{F190L}) of the F gene is particularly preferred.

According to a further preferred embodiment of the present invention the sequence encoding the recombinant NDV comprises, in addition to the above described mutation in the HN gene, particulary the mutation resulting in an amino acid substitution at position 277 of the HN gene product as specified supra, and in addition to the above described at least one mutation in the F gene, particulary the mutation resulting in an amino acid substitution at position 117 (F^{F117S}) and/or at position 190 (F^{F190L}) of the F gene product as specified supra, optionally in combination with the above described mutation on the M gene, particulary the mutation resulting in an amino acid exchange at position 165 of the M gene product as specified supra, also comprises at least one mutation in the L gene. Preferably the mutated L gene encodes for a L protein with at least one amino acid substitution at amino acid position 757, 1551 and/or 1700. As it relates to preferred and more preferred embodiments of the substitutions at amino acid positions 757, 1551 and/or 1700 of the L gene reference to above disclosure with regard to the novel NDV strains according to the first aspect of the present invention is made. The same preferred and more preferred embodiments apply for the recombinant NDV strains according to the second aspect of the present invention. The mutations at amino acid positions 757, 1551 and 1700 of the L gene may be present alone or in each possible combination, i.e. position 757 and/or position 1551 and/or position 1700. The combination of all three mutations in the L gene (L3) is particularly preferred. In one embodiment of the present invention the at least one mutation in the L gene, preferably all three mutation at the respective amino acid positions 757, 1551 and 1700, is/are comprised in combination with the mutation in the HN gene as specified supra, the one or two mutations in the F gene as specified supra, and optionally, also in combination with the mutation in the M gene as specified supra.

In a particular preferred embodiment the nucleic acid is derived from the NDV strain MTH-68/H and comprises a mutation in the HN gene resulting in an amino acid with a hydrophobic side chain other than phenylalanine at position 277 of the respective amino acid sequence, a mutation in the M gene resulting in a matrix protein with an amino acid substitution at position 165 to an amino acid with an aromatic side chain, at least one mutation of the F gene resulting in a fusion protein with an amino acid substitution at position 117 to an amino acid with a hydroxylated side chain and/or an amino acid substitution at position 190 to an amino acid with an aliphatic side chain, and at least one mutation in the L gene resulting in a L protein with an amino acid substitution at position 757 to an amino acid with an aliphatic side chain other than valine (V), and/or an amino acid substitution at position 1551 to an amino acid with a hydroxylated side chain, and/or an amino acid substitution at position 1700 to an amino acid with an aliphatic side chain. More preferred, the mutated L gene results in a L protein comprising the three amino acid substitutions at positions 757, 1551 and 1700. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L), and the mutated L gene encodes for a L protein in which the amino acid valine (V) at position 757 is substituted to the amino acid isoleucine (I), and/or the amino acid phenylalanine (F) at position 1551 is substituted to the amino acid serine (S), and/or the amino acid arginine (R) at position 1700 is substituted to the amino acid leucine (L). Still more preferred the L protein comprises all three mutations at positions 757, 1551 and 1700.

Also within the scope of the present invention are those nucleic acids comprising, in addition to the mutation in the HN gene and to the at least one mutation in the F gene at amino acid position 117 or 190, preferably in combination with one or more mutation in the M gene, and the L gene, as specified supra, at least one mutation in the L gene resulting in an amino acid substitution at position 1717 and/or at position 1910 of the L proteine, and/or a mutation in the P gene as specified supra.

In one embodiment of the present invention the nucleic acid consists of or comprises at least one of the nucleic acids according to SEQ ID No. 36 or 37 or 38 or parts thereof. In another preferred embodiment the nucleic acid of the present invention has a sequence identity of at least 75 %, particularly of at least 90 %, more particularly of at least 95 % to any of the sequences according SEQ ID No. 36 or 37 or 38.

In one embodiment of the present invention the nucleic acid is derived from a recombinant NDV as described supra.

As already mentioned above with regard to the first aspect of the present invention, a person skilled in the art knows how to use the disclosed sequences to produce synthetic DNAs (e.g. vectors, in particular in rg-NDV vectors), and how to use the synthetic DNAs to reconstruct and express the viral genome. Thus, the skilled artisan is enabled to produce virus particles by using the information disclosed herein. Reference is made to the 'reverse genetics' method as explained above. The difference with regard to the first aspect of the invention is that the amino acid substitution at position 289 of the F protein is not mandatory and that the transgenic construct is according to group B.

Therefore, the present invention also provides a method (herein also called 'reverse genetics') for providing cloned full-length cDNA of the recombinant NDV strains according to the second aspect of the present invention, and, in a further embodiment, the invention also provides infectious virus particles obtained from said full-length cDNA of the recombinant NDV strains according to the second aspect of the present invention. These recombinant NDV strains have an unexpected beneficial replication capability in cell cultures, eggs or animals that are used to propagate the virus for pharmaceutical purposes, and have good or even an improved viral safety profile, giving production advantages to such improved rgNDV strains. Furthermore, the higher replication rate is also particularly useful to obtain the desired oncolytic effects in vivo in that more virus particles will be produced for subsequent rounds of infection of cancer cells that were missed in the first round of infection. What is more, higher levels (yields) of the foreign gene or the foreign genes encoded by the transgenic construct will be achieved for providing the desired enhanced oncolytic effects in vivo. Thus, by modifying the HN protein within a transgene-expressing NDV higher yields of the transgenic construct protein can be obtained, which is very useful in anti-tumor immunotherapy. By modifying the F protein, and preferably also the L protein, as specified supra, also the viral safety can be improved.

The invention also provides an isolated, recombinant nucleic acid encoding a rgNDV obtainable by a method for preparing an rgNDV having improved replication in a cancer cell in comparison to that of a parent NDV, as described supra.

In one preferred embodiment the isolated and recombinant nucleic acid encodes a rgNDV provided with a mutation in the HN gene resulting in a change of the amino acid phenylalanine (F) at amino acid position 277 of the hemagglutinin-neuraminidase into a leucine (L), said mutation (a F277L mutation) allowing replication of said rgNDV in a human cancer cell to a higher level than replication of an otherwise identical rgNDV having the amino acid phenylalanine (F) at position 277 in the HN gene product, and with at least one mutation in the F gene resulting in a change of the amino acid phenylalanine (F) at amino acid position 117 of the fusion protein into serine (S), and/or resulting in a change of the amino acid phenylalanine (F) at amino acid position 190 of the fusion protein into leucine (L), said at least one mutation providing a decreased ICPI value than the ICPI of an otherwise identical rgNDV strain not having the at least one mutation in the F gene. These mutations are provided in combination with a transgenic construct encoding any of the foreign genes according to group B. The rgNDV may further comprise at least one or more mutation(s) selected from a mutation in the M gene, preferably G165W, a mutation at amino acid position 757, 1551, 1700, 1717 and/or 1910 in the L gene, preferably V757I, F1551S, R1700L, Y1717H and/or E1910K, and a mutation in the P gene, preferably the mutation in the P gene abolishes and/or decreases the expression of the V protein.

According to a particularly preferred embodiment the nucleic acid used to engineer a recombinant NDV genome is derived from the NDV strain MTH-68/H. Thus the present invention provides in one embodiment nucleic acids, preferably obtainable by the above specified method, which are or comprise
- rgNDV-Mut HN(F277L)/F(F117S)/F(F190L)-Nivolumab,
- rgNDV-Mut HN(F277L)/F(F117S)/F(F190L)-Ipilimumab,
- rgNDV-Mut HN(F277L)/F(F117S)/F(F190L)-IL-12,
- rgNDV-Mut HN(F277L)/F(F117S)/F(F190L)-NS1,
- rgNDV-Mut HN(F277L)/F(F117S)/F(F190L)-Apoptm,
- rgNDV-Mut HN(F277L)/F(F117S)/F(F190L)-B18R,
- rgNDV-Mut HN(F277L)/F(F117S)/F(F190L)-CD40,
- rgNDV-Mut HN(F277L)/F(F117S)/F(F190L)-Theralizumab,
- rgNDV-Mut HN(F277L)/F(F117S)/F(F190L)-anti-CD40,
- rgNDV-Mut HN(F277L)/F(F117S)/F(F190L)-anti-CD80,
- rgNDV-Mut HN(F277L)/F(F117S)/F(F190L)-anti-CA 15-3,
- rgNDV-Mut HN(F277L)/F(F117S)/F(F190L)-anti CA 19-9,
- rgNDV-Mut HN(F277L)/F(F117S)/F(F190L)-Sofituzumab,
- rgNDV-Mut HN(F277L)/F(F117S)/F(F190L)-Cetuximab,
- rgNDV-Mut HN(F277L)/F(F117S)/F(F190L)-Trastuzumab,
- rgNDV-Mut HN(F277L)/F(F1 17S)/F(F190L)-BIL=3 s,
- rgNDV-Mut HN(F277L)/F(F117S)/F(F190L)-J591,
- rgNDV-Mut HN(F277L)/F(F117S)/F(F190L)-Ramucircumab,
- rgNDV-Mut HN(F277L)/F(F117S)/F(F190L)-TRAIL.

In another enmbodiment the present invention provides nucleic acids, preferably obtainable by the above specified method, which are or comprise
- rgNDV-Mut HN(F277L)/M(G165W)/F(F117S)/F(F190L)/L(V757I)/ L(F1551S)/L(R1700L)-Nivolumab (= rgNDV-NoThaBene-2-Nivolumab,
- rgNDV-Mut HN(F277L)/M(G165W)/F(F117S)/F(F190L)/L(V757I)/ L(F1551S)/L(R1700L)-Ipilimumab (= rgNDV-NoThaBene-2-Ipilimumab,
- rgNDV-Mut HN(F277L)/M(G165W)/F(F117S)/F(F190L)/L(V757I)/ L(F1551S)/L(R1700L)-IL-12 (= rgNDV-NoThaBene-2-IL-12,
- rgNDV-Mut HN(F277L)/M(G165W)/F(F117S)/F(F190L)/L(V757I)/ L(F1551S)/L(R1700L)-NS1 (= rgNDV-NoThaBene-2-NS1,
- rgNDV-Mut HN(F277L)/M(G165W)/F(F117S)/F(F190L)/L(V757I)/ L(F1551S)/L(R1700L)-Apoptm (= rgNDV-NoThaBene-2-Apoptin,
- rgNDV-Mut HN(F277L)/M(G165W)/F(F117S)/F(F190L)/L(V757I)/ L(F1551S)/L(R1700L)-B18R (= rgNDV-NoThaBene-2-B18R,
- rgNDV-Mut HN(F277L)/M(G165W)/F(F117S)/F(F190L)/L(V757I)/ L(F1551S)/L(R1700L)-CD40 (= rgNDV-NoThaBene-2-CD40,
- rgNDV-Mut HN(F277L)/M(G165W)/F(F117S)/F(F190L)/L(V757I)/ L(F1551S)/L(R1700L)-Theralizumab (= rgNDV-NoThaBene-2-Theralizumab,
- rgNDV-Mut HN(F277L)/M(G165W)/F(F117S)/F(F190L)/L(V757I)/ L(F1551S)/L(R1700L)-anti-CD40 (= rgNDV-NoThaBene-2-anti-CD40),
- rgNDV-Mut HN(F277L)/M(G165W)/F(F117S)/F(F190L)/L(V757I)/ L(F1551S)/L(R1700L)-anti-CD80 (= rgNDV-NoThaBene-2-anti-CD80),
- rgNDV-Mut HN(F277L)/M(G165W)/F(F117S)/F(F190L)/L(V757I)/ L(F1551S)/L(R1700L)-anti-CA 15-3 (= rgNDV-NoThaBene-2-anti-CA 15-3),
- rgNDV-Mut HN(F277L)/M(G165W)/F(F117S)/F(F190L)/L(V757I)/ L(F1551S)/L(R1700L)-anti CA 19-9 (= rgNDV-NoThaBene-2-anti CA 19-9),
- rgNDV-Mut HN(F277L)/M(G165W)/F(F117S)/F(F190L)/L(V757I)/ L(F1551S)/L(R1700L)-Sofituzumab (= rgNDV-NoThaBene-2-Sofituzumab),
- rgNDV-Mut HN(F277L)/M(G165W)/F(F117S)/F(F190L)/L(V757I)/ L(F1551S)/L(R1700L)-Cetuximab (= rgNDV-NoThaBene-2-Cetuximab),
- rgNDV-Mut HN(F277L)/M(G165W)/F(F117S)/F(F190L)/L(V757I)/ L(F1551S)/L(R1700L)-Trastuzumab (= rgNDV-NoThaBene-2-Trastuzumab),
- rgNDV-Mut HN(F277L)/M(G165W)/F(F117S)/F(F190L)/L(V757I)/ L(F1551S)/L(R1700L)-BIL=3s (= rgNDV-NoThaBene-2-BIL=3s),
- rgNDV-Mut HN(F277L)/M(G165W)/F(F117S)/F(F190L)/L(V757I)/ L(F1551S)/L(R1700L)-J591 (= rgNDV-NoThaBene-2-J591),
- rgNDV-Mut HN(F277L)/M(G165W)/F(F117S)/F(F190L)/L(V757I)/ L(F1551S)/L(R1700L)-Ramucircumab (= rgNDV-NoThaBene-2-Ramucircumab),
- rgNDV-Mut HN(F277L)/M(G165W)/F(F117S)/F(F190L)/L(V757I)/ L(F1551S)/L(R1700L)-TRAIL (= rgNDV-NoThaBene-2-TRAIL).

A NDV or rgNDV according to the present invention can be provided in a suitable cell or cell line, for example a HeLa cell line or in cancer cells, or an embryonated egg that is susceptible to a NDV infection.

In these infected cells the virus is grown to sufficient quantities, and preferably under sufficient conditions such that the virus is free from exogenous contamination, and the progeny virus particles are collected by suitable methods well known to those skilled in the art.

In a further aspect the present invention as pertains to a vector comprising any of the nucleic acid sequences as disclosed herein or comprising a nucleic acid sequence encoding a protein as disclosed herein. The protein can for example be a HN protein preferably having an amino acid sequence as shown in SEQ ID No. 32 of the sequence listing, or a F protein preferably having an amino acid sequence as shown in SEQ ID No. 33 or 39 of the sequence listing, or M protein preferably having an amino acid sequence as shown in SEQ ID No. 34 of the sequence listing, or a L protein preferably having an amino acid sequence as shown in SEQ ID No. 35 of the sequence listing. The vector can also encode an NDV according to the present invention.

Moreover, the present invention also pertains to a host cell comprising any of the nucleic acid sequences according to the present invention or a vector according to the present invention.

In a further aspect the present invention is concerned with the medical use of the NDV strains and the recombinant NDV strains (the virus particles) of the present invention. In this regard the invention provides an oncolytic NDV for use in oncological treatment (this term is used herein also replaceable with "for use as a medicament, especially in a method of treatment of cancer"; or the use of said oncolytic NDV in the preparation of a pharmaceutical formulation for use in said method of treatment) in humans (or more generically animals, such as mammalian animals).

In this regard the present invention is first concerned with a NDV or a recombinant NDV according to the present invention for use in medicine. Secondly, the present invention is more particulary concerned with a NDV or a recombinant NDV according to the present invention for use in a method of treating cancer in a subject considered in need thereof.

In particular one or more of the NDV strains and/or the recombinant NDV strains of the present invention are used in a method for treating one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.

In a preferred embodiment of the present invention the subject to be treated is a mammal, particularly a mammalian animal or a human subject. Still more preferred the one or more oncolytic NDV strains of the present invention may be used for the treatment of adults and/or children, preferably human adults and/or human children.

In one embodiment only one NDV or rgNDV strain or recombinant NDV or recombinant rgNDV strain according to the present invention is used in a method for treating cancer, in particular a cancer selected from the specific cancers as described and/or mentioned in the claims herein. Alternatively in another preferred embodiment of the present invention a combination of at least two of the NDV or rgNDV strains according to the present invention, which are preferably recombinant, is used in a method for treating cancer, in particular a cancer selected from the specific cancers as described and/or mentioned in the claims herein. In particular it is preferred to use a combination of virus particles, wherein each virus particle encodes and expresses another foreign gene, part or variant thereof selected from the group of the foreign genes according to group A or group B. The use of such a combination of foreign genes results in an active combination of proteins beneficially improving the immunological response with the cancer.

Also within the scope of the present invention is the use of one or more NDV or rgNDV strains according to the present invention in combination with one or more other therapies suitable for the treatment of cancer. In a preferred embodiment the one or more NDV or rgNDV strains or a pharmaceutical formulation comprising the same is used in a method for treating cancer, especially a cancer selected from the specific cancers as described and/or mentioned in the claims herein.

The one or more NDV or rgNDV strains and one or more other therapies can be used concurrently or sequentially. In certain embodiments, the one or more NDV or rgNDV and the one or more other therapies are administered in the same ("fixed") pharmaceutical formulation. In other embodiments, the one or more NDV or rgNDV strains and the one or more other therapies are administered in different formulations. The one or more NDV or rgNDV strains of the present invention and the one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention or having other mutations in the NDV genome. Particularly preferred NDV strains and rgNDV which may be used in combination are disclosed in the copending European patent application no. 18166400.4 and the copending European patent application no. 19154204.2. For details of these strains reference to these patent applications is made.

Particulary preferred is the combined use of a recombinant NDV or rgNDV strain encoding and expressing as foreign genes a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab, a recombinant NDV or rgNDV strain encoding and expressing as foreign genes a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab, and a recombinant NDV or rgNDV strain encoding and expressing as foreign genes a gene encoding an antibody directed to protein PD-L1 or an antigen-binding part directed to protein PD-L1 (anti-PD-Ll), preferably a gene encoding Atezolizumab, an antigen-binding part of Atezolizumab, a variant of Atezolizumab or a variant of an antigen-binding part of Atezolizumab. Such a combination results in decreased side effects, as compared to directly administering the respective proteins to a patient in need thereof. For example the combination of the following NDV strains may be used in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F1551S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F1551S)/L(R1700L)-Atezolizumab

In a further aspect the present invention also provides a pharmaceutical formulation comprising a NDV strain, preferably an rgNDV, as described herein, without or preferably with at least one pharmaceutically acceptable carrier material.

For the indications mentioned herein, the appropriate dosage will, of course, vary depending upon, for example, the particular molecule of the invention to be employed, the mode of administration and the nature and severity of the condition being treated. In general, the dosage, in one embodiment of the invention, can preferably be in the range of 10⁷ to 10⁹ pfu per dose.

The NDV strains according to the present invention provide on the one hand an improved replication capacity of the NDV particles in cancer cells and a good or even an improved safety profile, preferably in humans, which is associated with increased cancer cell lysis and increased anti-cancer activitity, because the recombinant and mutated NDV strains of the present invention are still selective for cancer cells. The low to sero activity towards normal cells is associated with an increased therapeutic window or safety margin, as is commonly determined for cancer therapeutics. Beside the virological activity the recombinant mutated NDV strains of the present invention are in addition beneficial for the treatment of cancer from an immunological point of view. This is because the foreign gene carried by NDV strains of the present invention beneficially influences the ability of the immune system of the subject to be treated to attack and destroy cancer cells. Advantageuously the foreign gene is expressed inside the tumor to be treated, that is directly in the side of need.

The pharmaceutical formulations or pharmaceutical compositions of the present invention may be manufactured in any conventional manner, which are known to those skilled in the art. For example a pharmaceutical composition according to the present invention comprising a recombinant virus particle as decribed within this description can be provided in a dried, preferably in a lyophilized form and can be complemented with a suitable solvent, e.g. an aqueous carrier for injection at the time when used for administration. The dried form is very stable. Thus, in one embodiment the pharmaceutical composition is present as a dried form, still more preferred in a lyophilized form, and is reconstituted before administration by adding a suitable solvent. That is the present invention also relates to the above described dried form of the pharmaceutical composition for use as a medicament, preferably for use in a method of treating cancer. The process parameters for drying, preferably lyophilisation must be chosen such that the dried/lyophilized product contains live, replication competent viral particles, which can be grown to standardized titers, when needed. A person skilled in the art knows how to set these parameters.

For administration of a dried form, the dried form is directly dissolved in a suitable solvent or pharmaceutically acceptable carrier for injection and/or stabilization of the NDV or rg NDV and/or for improving its delivery to cancer cells. The solvent or carrier is preferably an aqueous carrier, for example sterile water for injection or sterile buffered physiological saline or another, preferably isotonic buffer system having a pH in the range of 7.2 to 7.6. The reconstitution can be performed ideally at or close to the intended time of administration in order to avoid any contaminations with microbes, etc. The skilled person is familiar with the handling of pharmaceutical compositions for reconstitution and reconstituted solutions.

In another embodiment, the present invention is concerned with pharmaceutical formulations comprising cancer cells infected with a transgene-expressing NDV or transgene-expressing rgNDV strain as described herein, and a pharmaceutically acceptable carrier. In specific embodiments, the cancer cells have been treated with gamma radiation prior to incorporation into the pharmaceutical formulation. In specific embodiments, the cancer cells have been treated with gamma radiation before infection with the NDV strain (e.g., rgNDV). In other specific embodiments, the cancer cells have been treated with gamma radiation after infection with the NDV (e.g., rgNDV). In another embodiment, presented herein are pharmaceutical formulations comprising a protein concentrate from lysed NDV-infected cancer cells (e.g., rgNDV infected cancer cells), and a pharmaceutically acceptable carrier.

The pharmaceutical formulation of the present invention can be prepared by a method comprising or consisting of the steps of:
- propagating an NDV, preferably an rgNDV, according to the present invention in at least one cell or embryonated egg that is susceptible to a NDV infection;
- collecting the progeny virus particles, wherein the virus is grown to sufficient quantities and under sufficient conditions that the virus is free from exogenous contamination, such that the progeny virus is suitable for formulation into a pharmaceutical formulation; and
- preferably manufacturing a pharmaceutical formulation comprising said progeny virus particles in the absence of or after addition of at least one pharmaceutically acceptable carrier material.

Thus in a further aspect the present invention also provides a method for producing the above mentioned pharmaceutical formulation. It is also within the scope of the present invention that for manufacturing the said pharmaceutical formulation a nucleic acid according to the present invention is used, from which virus particles can be rescued which are then propagated in at least one cell or embryonated egg that is susceptible to a NDV infection.

The step of collecting progeny virus particles may in one embodiment also comprise a step of processing the collected virus-containing material to enrich virus particles and/or to eliminate host cell DNA.

In a further aspect the present invention also provides a cell or cell line or an embryonated egg comprising a NDV, preferably a rgNDV, according to the present invention.

In still a further aspect the present invention is also concerned with a method for treating cancer, especially a cancer selected from the specific cancers as described and/or mentioned in the claims herein, in a subject considered in need thereof. The method for treating cancer utilizes a NDV, a transgene-expressing NDV or transgene-expressing rgNDV described herein or any combination thereof or a pharmaceutical formulation comprising such a NDV or rgNDV or any combination thereof, especially in a therapeutically effective amount. Within the meaning of the present patent application a therapeutically effective amount also includes an effective amount for preventing a cancer, in particular a cancer selected from the specific cancers as described and/or mentioned in the claims herein.

The method of treatment comprises or consists of administering to a subject in need thereof a NDV or an rgNDV according to the present invention or any combination thereof in a sufficient amount for infecting and destroying some or all of the cancer cells. In a specific embodiment the method for treating cancer comprises or consists of infecting a cancer cell in a subject with a NDV or rgNDV of the present invention or any combination thereof or with a pharmaceutical composition of the present invention. In another embodiment the one or more NDV or rgNDV or a pharmaceutical composition comprising the same is administered to a subject in need thereof by intravenous, intra-arterial, intratumoral, intramuscular, intradermal, subcutaneous, or any other medically relevant route of administration. According to another embodiment of the present invention the NDV or rgNDV according to the present invention or any combination thereof is administering to a subject in need thereof by administering cancer cells infected with a NDV or rgNDV according to the present invention, especially in a therapeutically effective amount, or pharmaceutical formulation thereof by intravenous, intra-arterial, intratumoral, intramuscular, intradermal, subcutaneous, or any other medically relevant route of administration. In specific embodiments, the cancer cells have been treated with gamma radiation prior to administration to the subject or incorporation into the pharmaceutical formulation. In still another embodiment, a method for treating cancer comprises or consists of administering to a subject in need thereof protein concentrates or plasma membrane fragments from cancer cells infected with a NDV or rgNDV or a pharmaceutical formulation according to the present invention.

In a particular preferred embodiment the method for treating a cancer, in particular a cancer selected from the specific cancers as described and/or mentioned in the claims herein, comprises administering a mixture of at least two recombinant and mutated NDV or rgNDV strains according to the present invention. In particular it is preferred to use a combination of virus particles, wherein each virus particle encodes and expresses another foreign gene, preferably a foreign gene according to group A. The administration of such a combination of foreign genes results in an active combination of proteins beneficially improving the immunological response with the cancer. The administration of this mixture of NDVs or rgNDVs according to the present invention can be achieved as specified supra.

Also within the scope of the present invention is a method of treating cancer, especially a cancer selected from the specific cancers as described and/or mentioned in the claims herein, which utilizes a NDV or rgNDV according to the present invention in combination with one or more other therapies. In a preferred embodiment the NDV or rgNDV or a pharmaceutical formulation comprising the same is administered to a subject in need thereof in a therapeutically effective amount. The NDV or rgNDV and one or more other therapies can be administered concurrently or sequentially to the subject (meaning they are jointly therapeutically active). In certain embodiments, the NDV or rgNDV and one or more other therapies are administered in the same ("fixed") pharmaceutical formulation. In other embodiments, the NDV or rgNDV and one or more other therapies are administered in different formulations. The NDV or rgNDV of the present invention and one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention or having other mutations in the NDV genome. Particularly preferred NDV strains and rgNDV which may be used in combination are disclosed in the copending European patent application no. 18166400.4 and the copending European patent application no. 19154204.2. For details of these strains reference to these patent applications is made. Particulary preferred is the combined use of recombinant NDV or rgNDV strains encoding and expressing as foreign genes anti-PD-1, preferably Nivolumab, anti-CTLA-4, preferably Ipilimumab, anti-PD-Ll, preferably Atezolizumab, or parts or variants thereof. For example the following combinations are in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F1551S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F1551S)/L(R1700L)-Atezolizumab

Another therapy may also comprise or may be radiotherapy for cancer.

### Figures

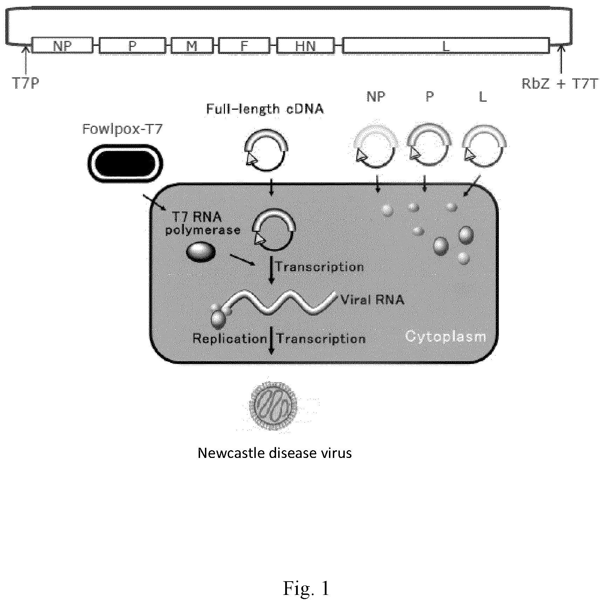

Figure 1 is a schematic presentation of the NDV reverse genetics system. The upper part shows the composition of the full-length cDNA plasmid which contains the full-length NDV cDNA (encoding the nucleocapsid protein (NP), the phosphoprotein (P), the matrix protein (M), the fusion protein (F), the hemagglutinin-neuraminidase (HN), and the RNA-dependent RNA polymerase (L)) cloned behind the bacteriophage T7 RNA Polymerase promoter (T7P; yellow triangle) and followed by a ribozyme sequence (Rz) and T7 transcription termination signal (T7T).
   A suitable host cell (shaded round-cornered box) is infected with a recombinant Fowlpox virus that expresses T7 DNA-dependent RNA polymerase (Fowlpox-T7) and subsequently co-transfected with the full-length cDNA plasmid and three helper plasmids containing the genes encoding the NDV NP, P and L proteins, respectively. Transcription of the full-length cDNA results in the generation of the NDV antigenome RNA which is encapsidated by NP protein then transcribed and replicated by the RNA-leading to the generation of infectious NDV.
Figure 2 is a schematic presentation of the genome of the rgNDV-mutants according to the present invention. The figure shows the position of the respective foreign gene which is inserted as extra transcription unit into the rgNDV-mutant genome between the P and M genes.

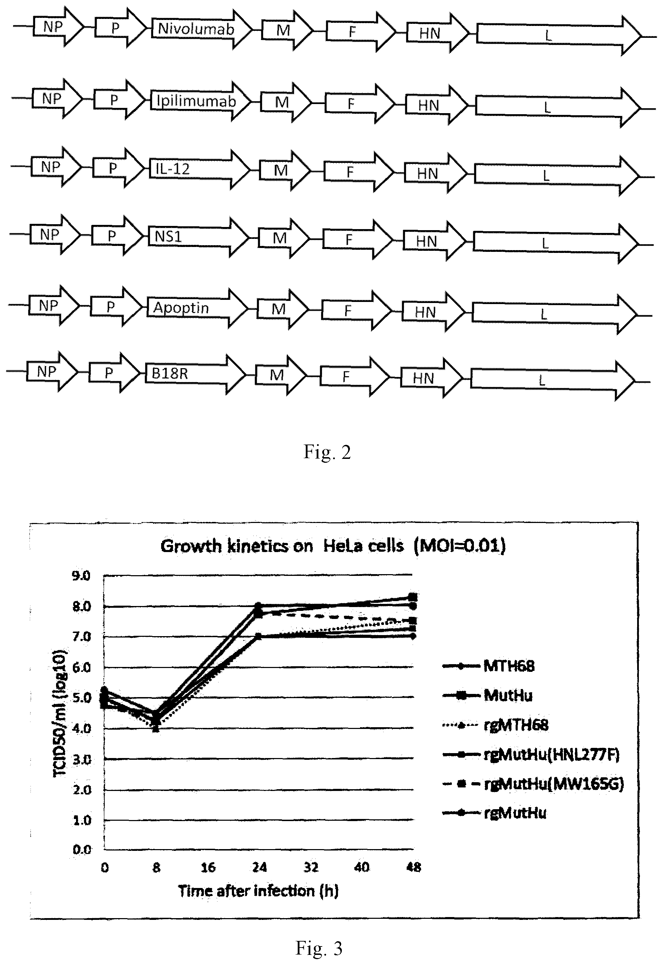

Figure 3 shows growth kinetics in HeLa cells. HeLa cells were infected at a multiplicity of infection (MOI) of 0.01 with the indicated viruses. At 0h, 8h, 24h and 48h after infection, the amount of infectious virus in the supernatant was determined by end-point titration on QM5 cells. MTH68: NDV strain MTH-68/H; MutHu: NDV-Mut HN(F277L)/M(G165W); rgMTH68: NDV strain MTH-68/H derived by reverse genetics from cloned full-length cDNA; rgMutHu: NDV strain NDV-Mut HN(F277L)/M(G165W) derived by reverse genetics from cloned full-length cDNA; rgMutHu(HNL277F): rgMutHu in which the amino acid mutation at position 277 in the HN gene was converted from L back to F; rgMutHu(MW165G): rgMutHu in which the amino acid mutation at position 165 in the M gene was converted from W back to G.

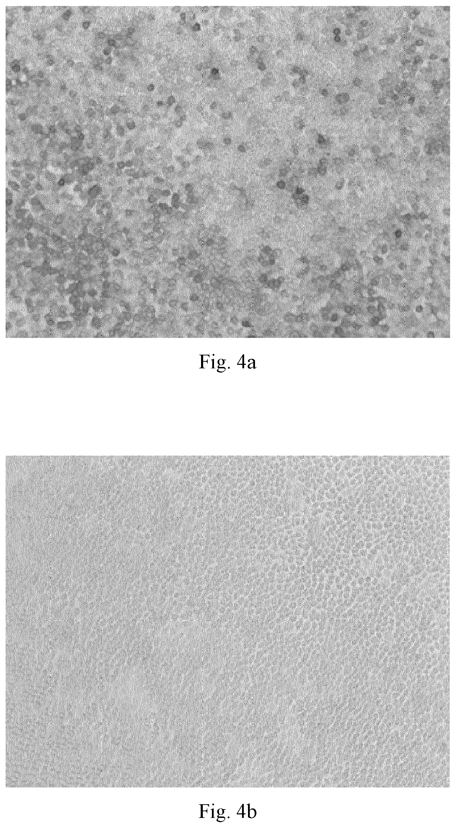

Figure 4 shows the expression of CD80 in a recombinant Nothabene-2-strain. Fig. 4a shows the recombinant strain, Fig. 4b is the negative control. For detection an anti-CD80 antibody was used.

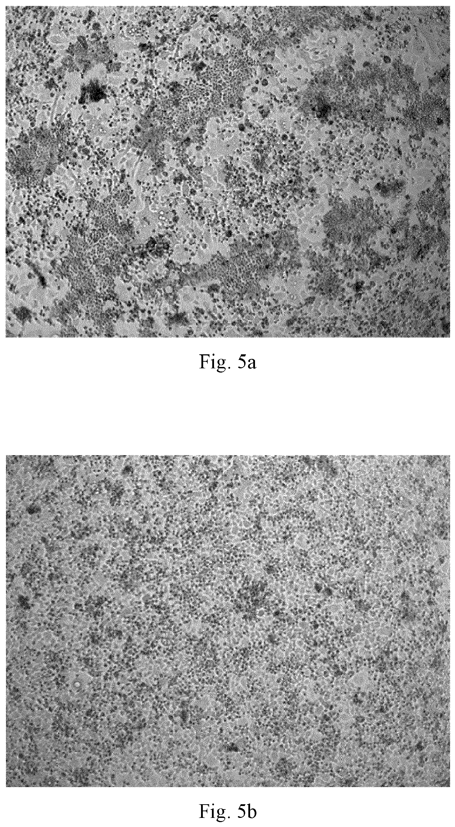

Figure 5 shows syncytium formation due to the F(L289A) mutation. Fig. 5a is rgNothabene-2-F2L3+F(L289A) (i.e. F3L3) according to the present invention, Fig. 5b is rgNothabene-2-F2L3.

### Examples

### Example 1. Nucleotide sequence analysis of mutant NDV-Mut HN(F277L)/M(G165W).

We identified a spontaneous mutant of an oncolytic NDV strain MTH-68/H (Csatary et al., 1999, Anticancer Res. 19:635-638.; further called MTH68). The replication capacity of the mutant strain (designated NDV-Mut HN(F277L)/M(G165W) in a variety of human neoplastic cell lines, as well as autologous primary tumors, is greatly enhanced as compared to the original MTH-68/H strain (also referred to as MTH68 strain). We analyzed its nucleotide sequence and found that, compared to MTH68, NDV-HN(F277L)/M(G165W) has two nucleotide mutations, one leading to an amino acid substitution in the M protein (G165W) and the other in the HN protein (F277L).

### Example 2. A reverse genetics system that allows genetic modification of NDV-strains.

### 2.1 Reverse genetics

In order to be able to genetically modify the genome of an RNA virus such as NDV, a manipulatable genetic system must be developed that uses a copy of the full viral RNA (vRNA) genome in the form of DNA. This full-length cDNA is amenable to genetic modification by using recombinant DNA techniques. The authentic or modified cDNA can be converted back into vRNA in cells, which in the presence of the viral replication proteins results in the production of a new modified infectious virus. Such 'reverse genetics systems' have been developed in the last few decades for different classes of RNA viruses. This system enables the rapid and facile introduction of mutations and deletions and the insertion of a transgene transcriptional unit, thereby enabling the changing of the biological properties of the virus.

Reverse genetics systems for several NDV strains, including lentogenic as well as velogenic strains, were developed by the Central Veterinary Institute (CVI), part of Wageningen University and Research, currently Wageningen Bioveterinary Research (WBVR) under the supervision of Dr. Ben Peeters (Peeters et al., 1999, J. Virol. 73:5001-9; de Leeuw et al., 2005, J. Gen. Virol. 86:1759-69; Dortmans et al., 2009, J. Gen. Virol. 90:2746-50). In order to generate a reverse genetics system for providing the NDV nucleic acids and strains according to the present invention, a similar approach was used. Details of the procedure can be found in the above cited papers and in the paragraphs below. Briefly, the system consists of 4 components, i.e., a transcription plasmid containing the full-length (either authentic or genetically modified) cDNA of the virus, which is used to generate the vRNA, and 3 expression plasmids ('helper plasmids') containing the NP, P and L genes of NDV respectively, which are used to generate the vRNA-replication complex (consisting of NP, P and L proteins). Transcription of the cDNA (i.e. conversion of the cDNA into vRNA) and expression of the NP, P and L genes by the helper plasmids is driven by a T7 promoter. The corresponding T7 DNA-dependent RNA polymerase (T7-RNAPol) is provided by a helper-virus (Fowlpox-T7).

In order to rescue virus, the 4 plasmids are co-transfected into Fowlpox-T7 infected cells (Fig. 1). Three to five days after transfection, the supernatant is inoculated into specific-pathogen-free embryonated chicken eggs (ECE) and incubated for 3 days. Infectious virus that is produced by transfected cells will replicate in the ECE and progeny virus can be harvested from the allantois fluid.

In order to develop a reverse genetics system for NDV the following steps were followed:
- Generation of sub-genomic NDV and foreign gene cDNA's by RT-PCR
- Assembly of full-length cDNA in a transcription vector
- Cloning of each of the NP, P and L genes into an expression vector
- Verify nucleotide sequence of full-length cDNA and helper-plasmids
- Repair nucleotide differences resulting from the cloning procedure, if necessary
- Rescue of infectious virus from cDNA using co-transfection (Fig. 1)

### 2.2 Construction of full-length NDV-Mut HN(F277L)/M(G165W) cDNA, NDV-Mut HN(F277L)/M(G165W)/ F(F117S)/F(F190L)/ L(V757I)/ L(F1551S)/ L(R1700L) and helper plasmids

NDV-Mut HN(F277L)/M(G165W) and NDV-Mut HN(F277L)/M(G165W)/F(F117S)/ F(F190L)/L(V757I)/L(F1551S)/L(R1700L) (passage 28 HeLa cells) were used for the isolation of vRNA using standard procedures. The vRNA was used to generate first-strand cDNA by means of Reverse Transcriptase followed by PCR to generate 4 sub-genomic cDNA fragments (designated C1, C2, C3 and C8). The full-length cDNA of NDV-MutHu was assembled from these fragments and cloned in the transcription vector pOLTV5 (Peeters et al., 1999, J. Virol. 73:5001-5009) by a combination of In-Fusion® cloning and classical cloning using restriction enzymes. An overview of the procedure is shown in Fig. 2, and further details can be found in Appendix 1 of this reference. The NP, P and L-genes of NDV-MutHu were obtained by RT-PCR (Appendix 1) and cloned in the expression plasmid pCVI which was derived by deletion of a ClaI restriction fragment from pCI-neo (Promega).

### 2.3 Nucleotide sequence analysis

Nucleotide sequence analysis was used to verify that the sequence of pFL-NDV Mut HN(F277L)/M(G165W) and pFL-NDV Mut HN(F277L)/M(G165W)/F(F117S)/ F(F190L)/L(V757I)/L(F1551S)/L(R1700L) were correct. A few nucleotides which differed from the Reference sequence were repaired. Silent mutations (i.e., not leading to an amino acid change) may be left unchanged.

### 2.4 Rescue of infectious virus from pFL-NDV Mut HN(F277L)/M(G165W)

In order to generate infectious virus, we used the co-transfection system described above (and illustrated in Fig. 1) to transfect QM5 cells (derived from Quail) using plasmid pFL-NDV_Mut HN(F277L)/M(G165W) and the helper plasmids pCVI-NP^{MutHN(F277L)/M(G165W)}, pCVI-P^{MutHN(F277L)/M(G165W)} and pCVI-LMut HN(F277L)/M(G165W) Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMut HN(F277L)/ M(G165W). NDV-Mut HN(F277L)/M(G165W) and rgMut HN(F277L)/M(G165W) have similar growth kinetics in HeLa cells.

### 2.5 Rescue of infectious virus from pFL-NDV Mut HN(F277L)/M(G165W)/ F(F117S)/F(F190L)/L(V757I)/L(F1551S)/L(R1700L)

In order to generate infectious virus, we used the co-transfection system described above (and illustrated in Fig. 1) to transfect QM5 cells (derived from Quail) using plasmid plasmid pFL-NDV_Mut HN(F277L)/M(G165W)/F(F117S)/F(F190L)/ L(V757I)/ L(F1551S)/ L(R1700L) and the helper plasmids pCVI-NP^{Mut HN(F277L)/M(G165W)/F(F117S)/F(F190L)/L(V757I)/L(F1551S)/L(R1700L)}, pCVI-P^{Mut HN(F277L)/M(G165W)/ F(F117S)/F(F190L)/L(V757I)/L(F1551S)/L(R1700L)} and pCVI-L^{Mut HN(F277L)/M(G165W)/F(F117S)/F(F190L)/L(V757I)/ L(F1551S)/L(R1700L)}. Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMut HN(F277L)/M(G165W)/F(F117S)/F(F190L)/L(V757I)/L(F1551S)/L(R1700L).

### Example 3. Identify whether one or both of the amino acid substitutions in Mut HN(F277L)/M(G165W) are responsible for the difference in growth kinetics between Mut HN(F277L)/M(G165W) and the parent strain MTH68.

### 3.1 Growth kinetics in HeLa cells

The rescued rg-viruses (Table 1) as well as the original Mut HN(F277L)/M(G165W) and MTH68 viruses were used to determine their growth-kinetics in HeLa cells. Briefly, 4x10⁶ HeLa cells were seeded in 25 cm² cell culture flasks and grown overnight. The cells were infected using a MOI of 0.01 (i.e., 1 infectious virus particle per 100 cells), and at 8, 24 and 48 hours after infection the virus titer in the supernatant was determined by end-point titration on QM5 cells.

The data (Fig. 3) indicate that strains Mut HN(F277L)/M(G165W) and rgMut HN(F277L)/M(G165W) yield at least 10-fold higher virus titers than MTH68. Furthermore, the data indicate that the mutation at amino acid position 277 in the HN gene is responsible for this effect. The M mutation does not seem to have an effect. This can be best seen when looking at the virus titers 24h after infection, or even better when comparing the increase in virus titer between 8h and 24h (the exponential growth phase). The virus titer shows an increase of 3.5 (log10) for Mut HN(F277L)/M(G165W), rgMut HN(F277L)/M(G165W) and rgMut HN(F277L)/M(W165G), whereas this is 2.5 for MTH68, 2.7 for rgMut HN(L277F) and 3.0 for rgMTH68 (Table 3).

rgMut HN(F277L)/M(W165G) is a strain in which the mutation in the M gene has been restored in accordance with the NDV MTH-68/H.

**Table 1. Virus titers (log10 TCID50/ml)**

| | Time after infection (h) | | | |
|---|---|---|---|---|
| Virus | 0 h | 8 h | 24 h | 48 h |
| MTH68 | 4.8 | 4.5 | 7.0 | 7.0 |
| Mut HN(F277L)/M(GI65W) | 5.0 | 4.3 | 7.8 | 8.3 |
| rgMTH68 | 5.0 | 4.0 | 7.0 | 7.5 |
| rgMut HN(L277F) | 5.0 | 4.3 | 7.0 | 7.3 |
| rgMut M(W165G) | 4.8 | 4.3 | 7.8 | 7.5 |
| rgMutHu | 5.3 | 4.5 | 8.0 | 8.0 |

### Example 4. Generation of recombinant NDV-strains with enhanced oncolytic and immune stimulating properties due to the expression of different therapeutic proteins.

The recombinant NDV-strains expressing one of the foreign genes of the present invention can be generated by means of the previously established reverse genetics system described above. The respective gene is to be inserted into the full-length cDNA of e.g. NDV-Mut HN(F277L)/M(G165W)/F(F117S)/ F(F190L)/L(V757I)/ L(F1551S)/L(R1700L) between the P and the M genes. To this end the open reading frames of the foreign genes may be fused via a 2A sequence. The genes were provided with the necessary NDV gene-start and gene-end sequences in order to allow transcription by the vRNA polymerase. Figure 2 shows the final constellation of the recombinant viruses.

Infectious virus can be rescued, and virus stocks can be prepared by two passages in HeLa cells. Expression of the respective foreign gene can be determined and quantified by using a total human IgG ELISA (Invitrogen).

### References:

- Blach-Olszewska et al., (1977) Why HeLa cells do not produce interferon? Arch Immunol Ther Exp (Warsz) 25:683-91.
- Chng et al., (2015) Cleavage efficient 2A peptides for high level monoclonal antibody expression in CHO cells, mAbs, 7:2, 403-412; http://dx.doi.org/10.1080/19420862.2015.1008351.
- Enoch et al., (1986) Activation of the Human beta-Interferon Gene Requires an Interferon-Inducible Factor, Mol. Cell. Biol. 6:801-10.
- Haryadi et al., (2015) Optimization of Heavy Chain and Light Chain Signal Peptides for High Level Expression of Therapeutic Antibodies in CHO Cells. PLoS ONE 10(2): e0116878. doi:10.1371/journal.pone.0116878.
- Schirrmacher, (2015) Oncolytic Newcastle disease virus as a prospective anti-cancer therapy. A biologic agent with potential to break therapy resistance. Expert Opin. Biol. Ther. 15:17 57-71
- Zamarin et al., (2014) Localized oncolytic virotherapy overcomes systemic tumor resistance to immune checkpoint blockade immunotherapy. Sci. Transl. Med. 6(226).
- Zamarin & Palese, (2017) Oncolytic Newcastle Disease Virus for cancer therapy: old challenges and new directions. Future Microbiol. 7: 347-67.

### Appendix 1: primers used for the generation of cDNA fragments and helper-plasmids

### cDNA fragments

| Fragment | Size | Primer | Sequence (5'-3') |
|---|---|---|---|
| C1 | 3.6 kb | Noss-09 (SEQ ID No. 40) | ACGACTCACTATAGGaccaaacagagaatccgtgag |
| | | Noss-121 (SEQ ID No. 41) | CCGGGAAGATCCAGGgcactcttcttgcatgttac |
| C2 | 3.7 kb | Noss-122 (SEQ ID No. 42) | GGGCCTGCCTCACTAtggtggtaacatgcaagaag |
| | | Noss-123 (SEQ ID No. 43) | TGCATGTTACCACCAatgtgtcattgtatcgcttg |
| C3 | 5.7 kb | Noss-125 (SEQ ID No. 44) | CAAGAAGGGAGATACgtaatatacaagcgatacaatg |
| | | N oss-126 (SEQ ID No. 45) | TCGCTTGT AT ATT ACttgttgtagcaaagagcacc |
| C8 | 2.0 kb | N oss-13 3 (SEQ ID No. 46) | GGCCTGGATCTTCCCattatgctgtctgtatacggtgc |
| | | Noss-10 (SEQ ID No. 47) | ATGCCATGCCGACCCaccaaacaaagacttggtgaatg |
| iPCR pOLTV5 (StuI) | 2.5 kb | Noss-17 (SEQ ID No. 48) | CCTATAGTGAGTCGTATTAATTTC |
| | | Noss-128 (SEQ ID No. 49) | CCTGGATCTTCCCGGGTCGG |
| iPCR pOLTV5 (SmaI) | 2.5 kb | Noss-137 (SEQ ID No. 50) | GGGTCGGCATGGCATCTCCACC |
| | | Noss-138 (SEQ ID No. 51) | GGGAAGATCCAGGCCTATAGTG |

### Helper-plasmids (generated by In-Fusion® cloning in pCVI)

| Gene | primer | sequence |
|---|---|---|
| NP | Noss-22 (SEQ ID No. 52) | CTCTAGAGTCGACCCttctgccaacatgtcttccg |
| | Noss-23 (SEQ ID No. 53) | GGGAAGCGGCCGCCCgtcggtcagtatccccagtc |
| P | Noss-24 (SEQ ID No. 54) | CTCTAGAGTCGACCCcagagtgaagatggccaccttc |
| | Noss-25n (SEQ ID No. 55) | GGGAAGCGGCCGCCCgtagtagtgatcagccattc |
| L | Noss-26 (SEQ ID No. 56) | CTCTAGAGTCGACCCgggtaggacatggcgggctc |
| | Noss-27 (SEQ ID No. 57) | GGGAAGCGGCCGCCCtgcctttaagagtcacagttac |

## Claims

1. A Newcastle Disease Virus (NDV), comprising a viral genome comprising a nucleic acid comprising
a nucleic acid sequence encoding a hemagglutinin-neuramidase protein (HN protein) with an amino acid substitution at position 277 to an amino acid with a hydrophobic side chain other than phenylalanine, preferably where phenylalanine (F) at position 277 is substituted to leucine (L), and
a nucleic acid sequence encoding a fusion protein (F protein) with an amino acid substitution at position 117 to an amino acid with a hydroxylated amino acid side chain, preferably where phenylalanine (F) is substituted to serine (S) at position 117 of the F protein, and/or with an amino acid substitution at position 190 to an amino acid with an aliphatic amino acid side chain, preferably where phenylalanine (F) is substituted to leucine (L) at position 190 of the F protein, and with an amino acid substitution at position 289 to an amino acid with an aliphatic side chain other than leucine (L), preferably where leucine (L) is substituted to alanine (A) at position 289 of the F protein.

2. The NDV according to claim 1, wherein the viral genome further comprises a nucleic acid comprising a nucleic acid sequence encoding a matrix protein (M protein) with an amino acid substitution at position 165 to an amino acid with an aromatic side chain, preferably where glycine (G) is substituted to tryptophane (W) at position 165 of the M protein.

3. The NDV according to claim 1 or claim 2, wherein the viral genome further comprises a nucleic acid comprising a nucleic acid sequence encoding a large polymerase protein (L protein) having
- an amino acid substitution at position 757 to an amino acid with an aliphatic amino acid side chain other than valine (V), preferably where valine (V) is substituted to isoleucine (I) at position 757 of the L protein, and/or
- an amino acid substitution at position 1551 to an amino acid with a hydroxylated side chain, preferably where phenylalanine (F) is substituted to serine (S) at position 1551 of the L protein, and/or
- an amino acid substitution at position 1700 to an amino acid with an aliphatic side chain, preferably where arginine (R) is substituted to leucine (L) at position 1700 of the L protein, preferably wherein the NDV comprises a mutated L gene and the encoded L protein with the amino acid substitution at position 757, position 1551 and position 1700.

4. The NDV according to claim 3, wherein the nucleic acid sequence encoding the large polymerase protein (L protein) having further
- an amino acid substitution at position 1717 to an amino acid with aromatic side chain other than tyrosine (Y), preferably where tyrosine (Y) is substituted to histidine (H) at position 1717 of the L protein, and/or
- an amino acid substitution at position 1910 to an amino acid with a basic side chain, preferably where glutamic acid (E) is substituted to lysine (K) at position 1910 of the L protein.

5. The NDV according to any one of claims 1 to 4, wherein the nucleic acid is at least 70 % identical to a nucleic acid sequence according to SEQ ID No. 1 to SEQ ID No. 3 of the sequence listing.

6. The NDV according to any one of claims 1 to 5, wherein
the HN protein comprises or consists of the amino acid sequence according to SEQ ID No. 32 of the sequence listing, and/or
wherein the F protein comprises or consists of the amino acid sequence according to SEQ ID No. 33 of the sequence listing, and/or
wherein the M protein comprises or consists of the amino acid sequence according to SEQ ID No. 34 of the sequence listing, and/or
wherein the L protein comprises or consists of the amino acid sequence according to SEQ ID No. 35 of the sequence listing.

7. The NDV according to any one of claims 1 to 6, wherein the NDV is a recombinant virus comprising a nucleic acid sequence encoding at least one foreign gene, the at least one foreign gene preferably being selected from the group consisting of:
- a gene encoding an antibody directed to protein PD-L1 or an antigen-binding part directed to protein PD-L1 (anti-PD-Ll), preferably a gene encoding Atezolizumab, an antigen-binding part of Atezolizumab, a variant of Atezolizumab or a variant of an antigen-binding part of Atezolizumab,
- a gene encoding an antibody directed to the growth factor protein VEGF-A or an antigen-binding part directed to the growth factor protein VEGF-A (anti-VEGF-A), preferably a gene encoding Bevacizumab, an antigen-binding part of Bevacizumab, a variant of Bevacizumab or a variant of an antigen-binding part of Bevacizumab,
- a gene encoding an antibody directed to killer-cell immunoglobulin-like receptors (KIRs) or an antigen-binding part directed to killer-cell immunoglobulin-like receptors (KIRs) (anti-KIR), preferably a gene encoding Lirilumab, an antigen-binding part of Lirilumab, a variant of Lirilumab or a variant of an antigen-binding part of Lirilumab,
- a gene encoding an antibody directed to and inhibiting LAG-3 or an antigen-binding part directed to and inhibiting LAG-3 (anti-LAG-3), preferably a gene encoding Relatlimab, an antigen-binding part of Relatlimab, a variant of Relatlimab or a variant of an antigen-binding part of Relatlimab,
- a gene encoding an antibody directed to NKG2A or an antigen-binding part directed to NKG2A (anti-NKG2A), preferably a gene encoding Monalizumab, an antigen-binding part of Monalizumab, a variant of Monalizumab or a variant of an antigen-binding part of Monalizumab,
- a gene encoding an antibody directed to and activating the glucocorticoid-induced TNF-superfamily receptor (GITR) or an antigen-binding part directed to the glucocorticoid-induced TNF-superfamily receptor (GITR) (anti-GITR), preferably a gene encoding TRX518, an antigen-binding part of TRX518, a variant of TRX518 or a variant of an antigen-binding part of TRX518,
- a gene encoding an antibody directed to and activating the protein OX40 or an antigen-binding part directed to and activating the protein OX40 (anti-OX40), preferably a gene encoding BMS 986178, an antigen-binding part of BMS 986178, a variant of BMS 986178 or a variant of an antigen-binding part of BMS 986178,
- a gene encoding the protein CD40 (cluster of differentiation 40), a part of CD40, a variant of CD40 or a variant of a part of CD40,
- a gene encoding a membrane protein which is the receptor for the protein CD28, and which membrane protein is involved in the costimulatory signal essential for T-lymphocyte activation or a part of the membrane protein which is the receptor for the protein CD28, and which membrane protein is involved in the costimulatory signal essential for T-lymphocyte activation, preferably a gene encoding CD80, a part of CD80, a variant of CD80 or a variant of a part of CD80,
- a gene encoding a human interleukin 12 (hIL-12) or a part of a human interleukin 12 (hIL-12), preferably a gene encoding (ns)hIL-12, a part of (ns)hIL-12, a variant of (ns)hIL-12 or a variant of a part of (ns)hIL-12,
- a gene encoding a green fluorescent protein or a part of a green fluorescent protein, preferably a gene encoding eGFP, a part of eGFP, a variant of eGFP or a variant of a part of eGFP,
- a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab,
- a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab,
- a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12, a variant of interleukin-12 or a variant of a part of interleukin-12,
- a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin, a part of Apoptin, a variant of Apoptin or a variant of a part of Apoptin,
- a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus, a variant of the viral protein B18R from vaccinia virus or a variant of a part of the viral protein B18R from vaccinia virus,
- a gene encoding an antibody, preferably an agonistic antibody, directed to CD28 or an antigen-binding part directed to CD28 (anti-CD28), preferably a gene encoding Theralizumab, an antigen-binding part of Theralizumab, a variant of Theralizumab or a variant of an antigen-binding part of Theralizumab,
- a gene encoding an antibody, preferably an agonistic antibody, directed to CD40 or an antigen-binding part directed to CD40 (anti-CD40),
- a gene encoding an antibody, preferably an agonistic antibody, directed to CD80 or an antigen-binding part directed to CD80 (anti-CD80),
- a gene encoding an antibody directed to CA 15-3 or an antigen-binding part directed to CA 15-3 (anti-CA 15-3),
- a gene encoding an antibody directed to CA 19-9 or an antigen-binding part directed to CA 19-9 (anti-CA 19-9),
- a gene encoding an antibody directed to CA 125 or an antigen-binding part directed to CA 125 (anti-CA 125), preferably a gene encoding Sofituzumab, an antigen-binding part of Sofituzumab, a variant of Sofituzumab or a variant of an antigen-binding part of Sofituzumab,
- a gene encoding an antibody, preferably an antagonistic antibody, directed to EGFR or an antigen-binding part directed to EGFR (anti-EGFR), more preferably a gene encoding Cetuximab, an antigen-binding part of Cetuximab, a variant of Cetuximab or a variant of an antigen-binding part of Cetuximab,
- a gene encoding an antibody, preferably an antagonistic antibody, directed to HER2 or an antigen-binding part directed to HER2 (anti-HER2), more preferably a gene encoding Trastuzumab, an antigen-binding part of Trastuzumab, a variant of Trastuzumab or a variant of an antigen-binding part of Trastuzumab,
- a gene encoding an antibody directed to nfP2X₇ or an antigen-binding part directed to nfP2X₇ (anti-nfP2X₇), preferably a gene encoding BIL=3s, an antigen-binding part of BIL=3s, a variant of BIL=3s or a variant of an antigen-binding part of BIL=3s,
- a gene encoding an antibody directed to PSMA or an antigen-binding part directed to PSMA (anti-PSMA), preferably a gene encoding J591, an antigen-binding part of J591, a variant of J591 or a variant of an antigen-binding part of J591,
- a gene encoding an antibody, preferably an antagonistic antibody, directed to VEGFR or an antigen-binding part directed to VEGFR (anti-VEGFR), more preferably a gene encoding Ramucirumab, an antigen-binding part of Ramucirumab, a variant of Ramucirumab or a variant of an antigen-binding part of Ramucirumab,
- a gene encoding a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5, preferably a gene encoding TRAIL, a part of TRAIL, a variant of TRAIL or a variant of a part of TRAIL, and
- any combination of these genes, parts or variants.

8. A nucleic acid comprising a nucleic acid sequence encoding a Newcastle Disease Virus (NDV), preferably a rgNDV, the nucleic acid comprising a nucleic acid sequence encoding a HN protein with an amino acid substitution at position 277 to an amino acid with a hydrophobic side chain other than phenylalanine, preferably where the nucleic acid sequence encodes HN^{F277L}, and
a nucleic acid sequence encoding a F protein with an amino acid substitution at position 117 to an amino acid with a hydroxylated side chain, preferably where the nucleic acid sequence encodes F^{F117S}, and/or with an amino acid substitution at position 190 to an amino acid with an aliphatic side chain, preferably where the nucleic acid sequence encodes F^{F190L}, and with a amino acid substitution at position 289 to an amino acid with a aliphatic side chain other than leucine (L), preferably where the nucleic acid sequence encodes F^{L289A}.

9. The nucleic acid according to claim 8, wherein the nucleic acid sequence encoding the NDV further comprises a nucleic acid sequence encoding a M protein with an amino acid substitution at position 165 to an amino acid with an aromatic side chain, preferably where the nucleic acid sequence encodes M^{G165W}.

10. The nucleic acid according to claim 8 or claim 9, wherein the nucleic acid sequence encoding the NDV further comprises a nucleic acid sequence encoding a L protein with
- an amino acid substitution at position 757 to an amino acid with an aliphatic side chain, preferably where the nucleic acid sequence encodes L^{V757I}, and/or
- an amino acid amino acid substitution at position 1551 to an amino acid with an aliphatic side chain, preferably where the nucleic acid sequence encodes L^{F1551S}, and/or
- with an amino acid amino acid substitution at position 1700 to an amino acid with an aliphatic side chain, preferably where the nucleic acid sequence encodes L^{R1700L}, wherein
more preferably wherein the nucleic acid sequence encodes a L protein with the amino acid substitution at position 757, position 1551 and position 1700.

11. The nucleic acid according to claim 10, wherein the nucleic acid sequence encodes a L protein having in addition an amino acid substitution at position 1717 to an amino acid with aromatic side chain other than tyrosine (Y), preferably where the nucleic acid sequence encodes L^{Y1717H}, and/or an amino acid substitution at position 1910 to an amino acid with a basic side chain, preferably where the nucleic acid sequence encodes L^{E1910K}.

12. The nucleic acid according to any one of claims 8 to 11, wherein the nucleic acid consists of or comprises a nucleic acid sequence according to SEQ ID No. 2 or SEQ ID No. 3 of the sequence listing or variants thereof, preferably the variants having a sequence identity of at least 70 %, particularly of at least 90 %, more of at least particularly 95 % to the nucleic acid sequence according to SEQ ID No. 2 or SEQ ID No. 3.

13. The nucleic acid according to any one of claims 8 to 12, additionally comprising a transgenic construct, wherein said transgenic construct comprises or consists of a nucleic acid sequence encoding a protein selected from the group consisting:
- an antibody directed to protein PD-L1 or an antigen-binding part directed to protein PD-L1 (anti-PD-Ll), preferably Atezolizumab, an antigen-binding part of Atezolizumab, a variant of Atezolizumab or a variant of an antigen-binding part of Atezolizumab,
- an antibody directed to the growth factor protein VEGF-A or an antigen-binding part directed to the growth factor protein VEGF-A (anti-VEGF-A), preferably Bevacizumab, an antigen-binding part of Bevacizumab, a variant of Bevacizumab or a variant of an antigen-binding part of Bevacizumab,
- an antibody directed to killer-cell immunoglobulin-like receptors (KIRs) or an antigen-binding part directed to killer-cell immunoglobulin-like receptors (KIRs) (anti-KIR), preferably Lirilumab, an antigen-binding part of Lirilumab, a variant of Lirilumab or a variant of an antigen-binding part of Lirilumab,
- an antibody directed to and inhibiting LAG-3 or an antigen-binding part directed to and inhibiting LAG-3 (anti-LAG-3), preferably Relatlimab, an antigen-binding part of Relatlimab, a variant of Relatlimab or a variant of an antigen-binding part of Relatlimab,
- an antibody directed to NKG2A or an antigen-binding part directed to NKG2A (anti-NKG2A), preferably Monalizumab, an antigen-binding part of Monalizumab, a variant of Monalizumab or a variant of an antigen-binding part of Monalizumab,
- an antibody directed to the glucocorticoid-induced TNF-superfamily receptor (GITR) or an antigen-binding part directed to the glucocorticoid-induced TNF-superfamily receptor (GITR) (anti-GITR), preferably TRX518, an antigen-binding part of TRX518, a variant of TRX518 or a variant of an antigen-binding part of TRX518,
- an antibody directed to and activating the protein OX40 or an antigen-binding part directed to and activating the protein OX40 (anti-OX40), preferably BMS 986178, an antigen-binding part of BMS 986178, a variant of BMS 986178 or a variant of an antigen-binding part of BMS 986178,
- a protein CD40 (cluster of differentiation 40), a part of CD40, a variant of CD40 or a variant of a part of CD40,
- a membrane protein which is the receptor for the protein CD28, and which membrane protein is involved in the costimulatory signal essential for T-lymphocyte activation or a part of the membrane protein which is the receptor for the protein CD28, and which membrane protein is involved in the costimulatory signal essential for T-lymphocyte activation, preferably CD80, a part of CD80, a variant of CD80 or a variant of a part of CD80,
- a human interleukin 12 (hIL-12) or a part of a human interleukin 12 (hIL-12), preferably (ns)hIL-12, a part of (ns)hIL-12, a variant of (ns)hIL-12 or a variant of a part of (ns)hIL-12,
- a green fluorescent protein or a part of a green fluorescent protein, preferably a eGFP, a part of eGFP, a variant of eGFP or a variant of a part of eGFP,
- an antibody directed to protein PD-1 or an antigen-binding part directed to protein PD-1 (anti-PD-1), preferably Nivolumab, an antigen-binding part of Nivolumab, a variant of Nivolumab or a variant of an antigen-binding part of Nivolumab,
- an antibody directed to the surface protein CTLA-4 or an antigen-binding part directed to protein CTLA-4 (anti-CTLA-4), preferably Ipilimumab, an antigen-binding part of Ipilimumab, a variant of Ipilimumab or a variant of an antigen-binding part of Ipilimumab,
- a protein which improves the cellular immune response and the ability of T cells to enter tumor cells, or a part thereof which improves the cellular immune response and the ability of T cells to enter tumor cells, preferably interleukin-12 (IL-12), a part of interleukin-12, a variant of interleukin-12 or a variant of a part of interleukin-12,
- a protein with the ability to modulate the virus replication cycle, or a part thereof with the ability to modulate the virus replication cycle, preferably the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a protein with the ability to selectively induce apoptosis in human tumor cells, but not in normal human cells, or a part thereof with the ability to selectively induce apoptosis in human tumor cells, but not in normal human cells, preferably Apoptin, a part of Apoptin, a variant of Apoptin or a variant of a part of Apoptin,
- a protein that reduces or inhibits IFN expression such as an IFN-beta receptor, or a part thereof that reduces or inhibits IFN expression such as an IFN-beta receptor, preferably the viral protein B18R from vaccinia virus, a part of the viral protein B18R from vaccinia virus, a variant of the viral protein B18R from vaccinia virus or a variant of a part of the viral protein B18R from vaccinia virus,
- an antibody directed to CD28 or an antigen-binding part directed to CD28 (anti-CD28), preferably Theralizumab, an antigen-binding part of Theralizumab, a variant of Theralizumab or a variant of an antigen-binding part of Theralizumab,
- an antibody directed to CD40 or an antigen-binding part directed to CD40 (anti-CD40),
- an antibody directed to CD80 or an antigen-binding part directed to CD80 (anti-CD80),
- an antibody directed to CA 15-3 or an antigen-binding part directed to CA 15-3 (anti-CA 15-3),
- an antibody directed to CA 19-9 or an antigen-binding part directed to CA 19-9 (anti-CA 19-9),
- an antibody directed to CA 125 or an antigen-binding part directed to CA 125 (anti-CA 125), preferably Sofituzumab, an antigen-binding part of Sofituzumab, a variant of Sofituzumab or a variant of an antigen-binding part of Sofituzumab,
- an antibody directed to EGFR or an antigen-binding part directed to EGFR (anti-EGFR), preferably Cetuximab, an antigen-binding part of Cetuximab, a variant of Cetuximab or a variant of an antigen-binding part of Cetuximab,
- an antibody directed to HER2 or an antigen-binding part directed to HER2 (anti-HER2), preferably Trastuzumab, an antigen-binding part of Trastuzumab, a variant of Trastuzumab or a variant of an antigen-binding part of Trastuzumab,
- an antibody directed to nfP2X₇ or an antigen-binding part directed to nfP2X₇ (anti- nfP2X₇), preferably BIL=3s, an antigen-binding part of BIL=3s, a variant of BIL=3s or a variant of an antigen-binding part of BIL=3s,
- an antibody directed to PSMA or an antigen-binding part directed to PSMA (anti-PSMA), preferably J591, an antigen-binding part of J591, a variant of J591 or a variant of an antigen-binding part of J591,
- an antibody directed to VEGFR or an antigen-binding part directed to VEGFR (anti-VEGFR), preferably Ramucirumab, an antigen-binding part of Ramucirumab, a variant of Ramucirumab or a variant of an antigen-binding part of Ramucirumab,
- a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5, preferably TRAIL, a part of TRAIL, a variant of TRAIL or a variant of a part of TRAIL,
- any combination of these genes, parts or variants.

14. A recombinant Newcastle Disease Virus (NDV), wherein the recombinant NDV comprises a viral genome comprising a nucleic acid comprising a nucleic acid sequence encoding at least one foreign gene, the at least one foreign gene preferably being selected from the group consisting of:
- a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab,
- a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab,
- a gene encoding a protein which improves the cellular immune response and the ability of T cells to enter tumor cells, or a part thereof which improves the cellular immune response and the ability of T cells to enter tumor cells, preferably a gene encoding interleukin-12 (IL-12), a part of interleukin-12, a variant of interleukin-12 or a variant of a part of interleukin-12,
- a gene encoding a protein with the ability to modulate the virus replication cycle, or a part thereof with the ability to modulate the virus replication cycle, preferably a gene encoding the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a gene encoding a protein with the ability to selectively induce apoptosis in human tumor cells, but not in normal human cells, or a part thereof with the ability to selectively induce apoptosis in human tumor cells, but not in normal human cells, preferably a gene encoding Apoptin, a part of Apoptin, a variant of Apoptin or a variant of a part of Apoptin,
- a gene encoding a protein that reduces or inhibits IFN expression such as an IFN-beta receptor, or a part thereof that reduces or inhibits IFN expression such as an IFN-beta receptor, preferably a gene encoding the viral protein B18R from vaccinia virus, a part of the viral protein B18R from vaccinia virus, a variant of the viral protein B18R from vaccinia virus or a variant of a part of the viral protein B18R from vaccinia virus,
- a gene encoding the protein CD40 (cluster of differentiation 40), a part of CD40, a variant of CD40 or a variant of a part of CD40,
- a gene encoding an antibody, preferably an agonistic antibody, directed to CD28 or an antigen-binding part directed to CD28 (anti-CD28), more preferably a gene encoding Theralizumab, an antigen-binding part of Theralizumab, a variant of Theralizumab or a variant of an antigen-binding part of Theralizumab,
- a gene encoding an antibody, preferably an agonistic antibody, directed to CD40 or an antigen-binding part directed to CD40 (anti-CD40),
- a gene encoding an antibody, preferably an agonistic antibody, directed to CD80 or an antigen-binding part directed to CD80 (anti-CD80),
- a gene encoding an antibody directed to CA 15-3 or an antigen-binding part directed to CA 15-3 (anti-CA 15-3),
- a gene encoding an antibody directed to CA 19-9 or an antigen-binding part directed to CA 19-9 (anti-CA 19-9),
- a gene encoding an antibody directed to CA 125 or an antigen-binding part directed to CA 125 (anti-CA 125), preferably a gene encoding Sofituzumab, an antigen-binding part of Sofituzumab, a variant of Sofituzumab or a variant of an antigen-binding part of Sofituzumab,
- a gene encoding an antibody, preferably an antagonistic antibody, directed to EGFR or an antigen-binding part directed to EGFR (anti-EGFR), more preferably a gene encoding Cetuximab, an antigen-binding part of Cetuximab, a variant of Cetuximab or a variant of an antigen-binding part of Cetuximab,
- a gene encoding an antibody, preferably an antagonistic antibody, directed to HER2 or an antigen-binding part directed to HER2 (anti-HER2), more preferably a gene encoding Trastuzumab, an antigen-binding part of Trastuzumab, a variant of Trastuzumab or a variant of an antigen-binding part of Trastuzumab,
- a gene encoding an antibody directed to nfP2X₇ or an antigen-binding part directed to nfP2X₇ (anti-nfP2X₇), preferably a gene encoding BIL=3s, an antigen-binding part of BIL=3s, a variant of BIL=3s or a variant of an antigen-binding part of BIL=3s,
- a gene encoding an antibody directed to PSMA or an antigen-binding part directed to PSMA (anti-PSMA), preferably a gene encoding J591, an antigen-binding part of J591, a variant of J591 or a variant of an antigen-binding part of J591,
- a gene encoding an antibody, preferably an antagonistic antibody, directed to VEGFR or an antigen-binding part directed to VEGFR (anti-VEGFR), more preferably a gene encoding Ramucirumab, an antigen-binding part of Ramucirumab, a variant of Ramucirumab or a variant of an antigen-binding part of Ramucirumab,
- a gene encoding a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5, preferably a gene encoding TRAIL, a part of TRAIL, a variant of TRAIL or a variant of a part of TRAIL, and
- and any combination of these genes, parts or variants, wherein
the viral genome comprises a nucleic acid sequence encoding a hemagglutinin-neuramidase protein (HN protein) with an amino acid substitution at position 277 to an amino acid with a hydrophobic side chain other than phenylalanine, preferably where phenylalanine (F) is substituted to leucine (L) at position 277, and
a nucleic acid sequence encoding a fusion protein (F protein) with an amino acid substitution at position 117 to an amino acid with a hydroxylated amino acid side chain, preferably where phenylalanine (F) is substituted to serine (S) at position 117 of the F protein, and/or with an amino acid substitution at position 190 to an amino acid with an aliphatic amino acid side chain, preferably where phenylalanine (F) is substituted to leucine (L) at position 190 of the F protein.

15. The recombinant NDV according to claim 14, wherein the viral genome comprises a nucleic acid sequence encoding a matrix protein (M protein) with an amino acid substitution at position 165 to an amino acid with an aromatic side chain, preferably where glycine (G) is substituted to tryptophane (W) at position 165 of the M protein.

16. The recombinant NDV according to claim 14 or claim 15, wherein the viral genome comprises a nucleic acid sequence encoding a L protein having
- an amino acid substitution at position 757 to an amino acid with an aliphatic amino acid side chain other than valine (V), preferably where valine (V) is substituted to isoleucine (I) at position 757 of the L protein, and/or
- an amino acid substitution at position 1551 to an amino acid with a hydroxylated side chain, preferably where phenylalanine (F) is substituted to serine (S) at position 1551 of the L protein, and/or
- an amino acid substitution at position 1700 to an amino acid with an aliphatic side chain, preferably where arginine (R) is substituted to leucine (L) at position 1700 of the L protein,
preferably wherein the viral genome comprises a nucleic acid sequence encoding a L protein with the amino acid substitution at position 757, position 1551 and position 1700.

17. The recombinant NDV according to claim 16, wherein the nucleic acid sequence encoding the large polymerase protein (L protein) having further
- an amino acid substitution at position 1717 to an amino acid with aromatic side chain other than tyrosine (Y), preferably where tyrosine (Y) is substituted to histidine (H) at position 1717 of the L protein, and/or
- an amino acid substitution at position 1910 to an amino acid with a basic side chain, preferably where glutamic acid (E) is substituted to lysine (K) at position 1910 of the L protein.

18. The recombinant NDV according to any one of claims 14 to 17, wherein the nucleic acid is at least 70 % identical to a nucleic acid sequence according to SEQ ID No. 1, SEQ ID No. 36, SEQ ID No. 137 or SEQ ID No. 38 of the sequence listing.

19. The recombinant NDV according to any one of claims 14 to 18, wherein
the HN protein comprises or consists of the amino acid sequence according to SEQ ID No. 32 of the sequence listing, and/or
wherein the F protein comprises or consists of the amino acid sequence according to SEQ ID No. 39 of the sequence listing, and/or
wherein the M protein comprises or consists of the amino acid sequence according to SEQ ID No. 34 of the sequence listing, and/or
wherein the L protein comprises or consists of the amino acid sequence according to SEQ ID No. 35 of the sequence listing.

20. A nucleic acid encoding a recombinant NDV, preferably a rgNDV, the nucleic acid comprising a transgenic construct, wherein said transgenic construct comprises a nucleic acid sequence encoding at least one protein selected from the group consisting of:
- an antibody directed to protein PD-1 or an antigen-binding part directed to protein PD-1 (anti-PD-1), preferably Nivolumab, an antigen-binding part of Nivolumab, a variant of Nivolumab or a variant of an antigen-binding part of Nivolumab,
- an antibody directed to the surface protein CTLA-4 or an antigen-binding part directed to protein CTLA-4 (anti-CTLA-4), preferably Ipilimumab, an antigen-binding part of Ipilimumab, a variant of Ipilimumab or a variant of an antigen-binding part of Ipilimumab,
- a protein which improves the cellular immune response and the ability of T cells to enter tumor cells, or a part thereof which improves the cellular immune response and the ability of T cells to enter tumor cells, preferably interleukin-12 (IL-12), a part of interleukin-12, a variant of interleukin-12 or a variant of a part of interleukin-12,
- a protein with the ability to modulate the virus replication cycle, or a part thereof with the ability to modulate the virus replication cycle, preferably the non-structural protein NS1 of influenza A virus, a part of the non-structural protein NS1 of influenza A virus, a variant of the non-structural protein NS1 of influenza A virus or a variant of a part of the non-structural protein NS1 of influenza A virus,
- a protein with the ability to selectively induce apoptosis in human tumor cells, but not in normal human cells, or a part thereof with the ability to selectively induce apoptosis in human tumor cells, but not in normal human cells, preferably Apoptin, a part of Apoptin, a variant of Apoptin or a variant of a part of Apoptin,
- a protein that reduces or inhibits IFN expression such as an IFN-beta receptor, or a part thereof that reduces or inhibits IFN expression such as an IFN-beta receptor, preferably the viral protein B18R from vaccinia virus, a part of the viral protein B18R from vaccinia virus, a variant of the viral protein B18R from vaccinia virus or a variant of a part of the viral protein B18R from vaccinia virus,
- a protein CD40 (cluster of differentiation 40), a part of CD40, a variant of CD40 or a variant of a part of CD40,
- an antibody, preferably an agonistic antibody, directed to CD28 or an antigen-binding part directed to CD28 (anti-CD28), more preferably Theralizumab, an antigen-binding part of Theralizumab, a variant of Theralizumab or a variant of an antigen-binding part of Theralizumab,
- an antibody, preferably an agonistic antibody, directed to CD40 or an antigen-binding part directed to CD40 (anti-CD40),
- an antibody, preferably an agonistic antibody, directed to CD80 or an antigen-binding part directed to CD80 (anti-CD80),
- an antibody directed to CA 15-3 or an antigen-binding part directed to CA 15-3 (anti-CA 15-3),
- an antibody directed to CA 19-9 or an antigen-binding part directed to CA 19-9 (anti-CA 19-9),
- an antibody directed to CA 125 or an antigen-binding part directed to CA 125 (anti-CA 125), preferably Sofituzumab, an antigen-binding part of Sofituzumab, a variant of Sofituzumab or a variant of an antigen-binding part of Sofituzumab,
- an antibody, preferably an antagonistic antibody, directed to EGFR or an antigen-binding part directed to EGFR (anti-EGFR), more preferably Cetuximab, an antigen-binding part of Cetuximab, a variant of Cetuximab or a variant of an antigen-binding part of Cetuximab,
- an antibody, preferably an antagonistic antibody, directed to HER2 or an antigen-binding part directed to HER2 (anti-HER2), more preferably Trastuzumab, an antigen-binding part of Trastuzumab, a variant of Trastuzumab or a variant of an antigen-binding part of Trastuzumab,
- an antibody directed to nfP2X₇ or an antigen-binding part directed to nfP2X₇ (anti-nfP2X₇), preferably BIL=3s, an antigen-binding part of BIL=3s, a variant of BIL=3s or a variant of an antigen-binding part of BIL=3s,
- an antibody directed to PSMA or an antigen-binding part directed to PSMA (anti-PSMA), preferably J591, an antigen-binding part of J591, a variant of J591 or a variant of an antigen-binding part of J591,
- an antibody, preferably an antagonistic antibody, directed to VEGFR or an antigen-binding part directed to VEGFR (anti-VEGFR), more preferably Ramucirumab, an antigen-binding part of Ramucirumab, a variant of Ramucirumab or a variant of an antigen-binding part of Ramucirumab,
- a protein that induces the process of apoptosis by binding to a death receptor, such as death receptor DR4 and/or death receptor DR5, or a part thereof that induces the process of apoptosis by binding to a death receptor, such as death receptor DR4 and/or death receptor DR5, preferably TRAIL, a part of TRAIL, a variant of TRAIL or a variant of a part of TRAIL,
- any combination of these genes, parts or variants,
wherein the nucleic acid in addition comprises a nucleic acid sequence encoding hemagglutinin-neuramidase protein (HN protein) with an amino acid substitution at position 277 to an amino acid with a hydrophobic side chain other than phenylalanine, preferably where the nucleic acid sequence encodes HN^{F277L}, and
a nucleic acid sequence encoding a fusion protein (F protein) with an amino acid substitution at position 117 to an amino acid with a hydroxylated side chain, preferably where the nucleic acid sequence encodes F^{F117S}, and/or with an amino acid substitution at position 190 to an amino acid with an aliphatic side chain, preferably where the nucleic acid seqeunce encodes F^{F190L}.

21. The nucleic acid according to claim 20, further comprising a nucleic acid sequence encoding a matrix protein (M protein) with an amino acid substitution at position 165 to an amino acid with an aromatic side chain, preferably where the nucleic acid sequence encodes M^{G165W}.

22. The nucleic acid according to claim 20 or claim 21, further comprising a nucleic acid sequence encoding a L protein having
- an amino acid substitution at position 757 to an amino acid with an aliphatic side chain, preferably where the nucleic acid sequence encodes L^{V757I}, and/or
- an amino acid amino acid substitution at position 1551 to an amino acid with an aliphatic side chain, preferably where the nucleic acid sequence encodes L^{F1551S}, and/or
- an amino acid amino acid substitution at position 1700 to an amino acid with an aliphatic side chain, preferably where the the nucleic acid sequence encodes L^{R1700L}, more preferably wherein the the nucleic acid sequence encodes a L protein with the amino acid substitutions at position 757, position 1551 and position 1700.

23. The nucleic acid according to claim 22, wherein the nucleic acid sequence encodes a L protein having in addition an amino acid substitution at position 1717 to an amino acid with aromatic side chain other than tyrosine (Y), preferably where the nucleic acid sequence encodes L^{Y1717H}, and/or an amino acid substitution at position 1910 to an amino acid with a basic side chain, preferably where the nucleic acid sequence encodes L^{E1910K}.

24. The nucleic acid according to any one of claims 20 to 23, wherein the nucleic acid comprises at least one of the nucleic acids according to SEQ ID No. 1, SEQ ID No. 36, SEQ ID No. 37 or SEQ ID No. 38 of the sequence listing or variants thereof, preferably the variants having a sequence identity of at least 75 %, particularly of at least 90 %, more of at least particularly 95 % to the respective sequence.

25. The NDV according to claim 7 or the recombinant NDV according to any one of claims 14 to 19 claim, wherein the nucleic acid comprising the nucleic acid sequence encoding the at least one foreign gene comprises or is a nucleic acid sequence according to any one the nucleic acid sequences according to SEQ ID No. 5 to 30 or to SEQ ID No. 15 to 30 of the sequence listing.

26. The nucleic acid according to any one of claims 8 to 13 or the recombinant nucleic acid according to any one of claims 20 to 24 is derived from a NDV according to any one of claims 1 to 7 or from a recombinant NDV according to any one of claims 14 to 19.

27. The nucleic acid according to claim 14 or any one of claims 20 to 24 or claim 26, wherein the transgenic construct comprises or consists of a nucleic acid sequence according to SEQ ID No. 5 to 30 or SEQ ID No. 15 to 30 of the sequence listing.

28. A NDV or a recombinant NDV according to any one of claims 1 to 7 or any one of claims 14 to 19 or claim 25 for use in medicine.

29. A NDV or a recombinant NDV according to any one of claims 1 to 7 or any one of claims 14 to 19 or claim 25 for use in a method of treating cancer in a subject, preferably in a mammal, more preferably a mammalian animal or a human subject, considered in need thereof, in particular for the treatment of one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer,
gastrointestinal tumors, like esophageal tumors, stomach tumors, colon tumors, pancreas tumors, prostate tumors, lung tumors, ear, nose, throat tumors, tongue tumors, and skin tumors, like melanoma.

30. A pharmaceutical formulation comprising particles of a NDV or a recombinant NDV according to any one of claims 1 to 7 or any one of claims 14 to 19 and/or a nucleic acid according to any one of claims 8 to 13 or any one of claims 20 to 24 or claim 26 or claim 27, preferably for use in oncological treatment of a subject considered in need thereof, in particular for the treatment of one or more indications selected from the group consisting of brain tumors, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.


SEQUENCE LISTING

<110> Dr. Arno Thaller

<120> New oncolytic Newcastle Disease Viruses and recombinant NDV strains

<160> 57

<210> 1
<211> 15186
<212> DNA
<213> Newcastle disease virus

<220>
<223> STRAIN MTH-68/H

<400> 1
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gccccccacc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgagccctc ctctataaac ccaagattca aggctccaag cgataacctt 3180
   ctcccgcctc ttccatccca ctgaatgatc gcgcagctgc aattaatcta gcaacattaa 3240
   agattaagaa aaaatacggg tagaatcgga gtgcaccgat tgtgccaaga tggactcatc 3300
   taggacaatt gggttatact ttgattctgc ccttccttct agcaacctgt tagcattccc 3360
   gattgtctta cgagatgtag gagatggaaa gaagcaaatt actccgcaat ataggatccg 3420
   gcgtcttgac tcgtggacag acagtaaaga agactcggta ttcatcacta cctacggatt 3480
   catcttccag gttgggaatg aagaagtcac tgtcggcatg atcaatgata atcccaagcg 3540
   agagttactc tccgctgcga tgctctgcct aggaagcgtc ccgaatgttg gagatcctgt 3600
   tgagctggca agggcctgcc tcactatggt ggtaacatgc aagaagagtg caactaatac 3660
   tgagagaatg gtcttctcag tagtgcaggc accccgggtg ctgcaaagct gtagggtcgt 3720
   ggcagacaaa tactcgtcag tgaatgcagt taagcacgtg aaagcaccag agaagatccc 3780
   tgggagtgaa accctagagt acaaggtgaa ttttgtctct ttgactgtgg tgccgaagaa 3840
   ggatgtctac aagatcccaa ccgcagtatt gaaggtatcc ggctcgagcc tgtataatct 3900
   tgcgctcaat gtcactattg atgtggaggt agacccaaag agcccgttag tcaagtccct 3960
   ttctaggtcc gacagtggat actatgctaa tctcttctta catatcggac ttatgtccac 4020
   tgtagataag aggggaaaga aagtgacatt tgaccagctg gagaggaaga taaggagact 4080
   tgatctatct gtcgggctca gtgatgtgct cggaccttct gtgcttgtga aggcgagagg 4140
   tgcacggact aggctgctgg cacctttctt ctctaacagc gggacagcct gctaccctat 4200
   agcaaatgcc tctcctcagg tagctaagat actctggagt cagaccgcgt gcctgcggag 4260
   tgtaaaaatc attattcaag cgggcaccca acgcgctgtc gcagtgactg ctgaccatga 4320
   ggttacctct actaagatag agaagaggca taccatcgct aaatacaatc ccttcaagaa 4380
   ataggccgta tccctgagac tgcaattcac ccgccttccc aaaacaccat gacaccagat 4440
   aatgatctgt cttgattact tacagttagt ttccctgtct atcaaattag aaaaaacacg 4500
   ggtagaagag ttcggatccc ggccggcgca ccaaaagcgc aagatgggcc ccagatcttc 4560
   taccaggatc ccagtacctc taatgctgac catacggatc acgctggcac tgagttatgt 4620
   ccgtctgaca agttctcttg atggcaggcc tcttgcagcc gcagggatcg tggtaacagg 4680
   ggataaagca gttaacatat acacctcatc ccagacaggg tcaatcatag tcaagttact 4740
   cccaaatatg cccaaggaca aagaggcatg tgcaaaagcc ccattggagg cttacaacag 4800
   gacactgact actttgctta ccccccttgg tgattctatc cgcaggatac aagagtctgt 4860
   gactacatcc ggaggaagga gacagagacg ctttataggt gccattattg gcagtgtagc 4920
   tctaggggtt gcaacagctg cacagataac ggcagcctcg gctctgatac aagccaacca 4980
   gaatgctgct aacatcctcc ggcttaagga gagcattgct gcaaccaatg aagctgtaca 5040
   cgaggtcact ggcggattgt cacagttagc agtggcagtt ggaaagatgc agcaatttgt 5100
   taatgaccag tttaataaca cagctcaaga gttggactgt ataaaaatta cacagcaggt 5160
   tggtgtagaa ctcaacttat acttaactga attgactaca gtgttcgggc cacaaatcac 5220
   ttcccctgcc ttaactcagt tgactgttca ggctctttac aatctggctg gtggtaatgt 5280
   agattacttg ttgactaagt taggtgtagg gaacaaccag ctcagctcat tgattggtag 5340
   cggcttgatc accggtaacc ctatttttta cgactcacag actcaactct tgggcataca 5400
   ggtgacttta ccctcagtcg ggaacctaaa taatatgcgt gccacctact tggagacctt 5460
   gtctgtaagc acaacaaagg gatttgcctc agcacttgtc ccaaaagtag tgacacaggt 5520
   cggttctgtg atagaagagc ttgacacctc atactgtata gaagctgatt tggatttata 5580
   ttgtacaaga atagtgacat tccctatgtc ccctggtatt tattcctgtt tgagcggcaa 5640
   tacatcggct tgcatgtatt caaggactga aggcgcactt actacaccat acatgactct 5700
   caaaggctca gttgttgcca attgccagat gacaacatgt agatgtgcag acccccgggg 5760
   tatcatatca caaaattatg gagaggctgt gtctctaata gataagcact catgcaatgt 5820
   cgtatcctta gacgggataa ctttgaggct cagtggggaa tttgatgcaa cttatcaaaa 5880
   gaatatctca atattagatt ctcaagtact agtgacaggc aatctcgata tctcaactga 5940
   gcttgggaat gtcaaccact caataagtaa tgctttggat aagttagagg aaagcaacag 6000
   caaactagac aaagtcaatg tcaaactgac cagcacatct gctctcatta cctatattgt 6060
   tttaactgtc atatctcttg ttcttggtat gcttagcctg gttctagcat gctatctgat 6120
   gtacaagcaa aaagcgcaac gaaagacctt gttgtggctt ggaaataata ccctagatca 6180
   gatgagagct actacaaaaa tgtgaatgca gatgagaggc agaggtatcc ccaatagcaa 6240
   tctgtgtgtc aattctggca gcctgttaat cagaagaatt aagaaaaaac taccggatgt 6300
   aggtgaacaa aagggaatat acgggtagaa cggcctgaga ggccacccct caatcgggag 6360
   ccaggcccca ctacgtccgc tctaccgcat caccaacagc agtcttcagt catggacagc 6420
   gcagttagcc aagttgcgct agagaatgat agaagagaag cgaataatac atggcgcttg 6480
   gttttccgga tcgcagcctt acttttactg gtaataacct tagccgtctc tgcagtcgcc 6540
   ctggcatata gtatggaggc tagcacacct ggcgaccttg taagcatacc gactgcgatc 6600
   tctagggcag aggaaaggat tacatctgca ctcggttcca atcaagatgt agtagatagg 6660
   atatacaagc aagtggccct tgagtctcca ttggcactgc taaataccga atctataatt 6720
   atgaatgcaa taacgtctct ctcttatcaa atcaatggag ccacaaataa tagcgggtgt 6780
   ggggcacctg ttcatgaccc agattacatc ggggggatag gtcaagaact tattgtagat 6840
   gatacgagtg acgtcacatc attttatccc tctgcattcc aagaacacct gaattttatc 6900
   ccggcgccca ctacaggatc aggctgcact cggataccct cattcgacat gagtgctacc 6960
   cattactgtt acactcacaa tgtgatattg tctggctgca gagatcactc acactcacat 7020
   cagtatttag cacttggtgt gcttcggaca tctgcaacag ggagggtatt cttttctact 7080
   ctgcgttcca tcaacttgga tgacgcccaa aatcggaagt cttgcagtgt gagtgcaact 7140
   cctttaggtt gtgatatgct gtgctctaaa atcacagaga ctgaggaaga ggattataaa 7200
   tcagttatcc ccacatcgat ggtacatgga aggttagggt ttgacggcca ataccatgag 7260
   aaggacctag acgtcacaac actatttagg gactgggtgg caaattaccc aggagtagga 7320
   ggtgggtctt ttattaacaa tcgcgtatgg ttcccggtct acggagggct aaaacccagc 7380
   tcgcctagtg acactgcaca agaaggagga tacgtaatat acaagcgata caatgacaca 7440
   tgcccggatg agcaagatta ccagattcgg atggctaagt catcgtataa gcctaggcgg 7500
   tttggtggaa aacgcgtaca gcaggccatc ctatccatca aggtgtcaac atccttgggt 7560
   gaggacccgg tgttgactgt accgcctaat acggtcgcac tcatgggggc cgaaggcaga 7620
   gttctcacag tggggacatc tcatttctta tatcagcgag ggtcatcata cttctctccc 7680
   gctttgttat accctatgac agtcaacaac aaaacagcca ctcttcataa tccttataca 7740
   ttcaatgctt tcactcggcc aggtagtgtc ccttgccagg cttcagcaag atgccccaac 7800
   tcatgtgtta ccggagtcta tactgatcca tatcccttag tcttccatag gaaccacacc 7860
   ttgcgagggg tattcggaac aatgcttgat gacgaacaag caagactcaa ccctgtatct 7920
   gcagtatttg ataacatatc ccgcagtcgc ataactcggg taagttcaag cagtaccagg 7980
   gcagcataca cgacatcaac gtgttttaaa gttgtcaaga ccaataaaac ctattgcctc 8040
   agcattgcag aaatatccaa taccctcttc ggggaattca gaattgtccc cttactagtt 8100
   gagattctca aggatggtgg ggtttaagaa gctaggaatg gtcggttgag tcaaccgtga 8160
   gaaggcgggg aagatgatat tgcatcatct atcttctgta acaccaagaa tcaaatcgaa 8220
   taccagtgcg agctcgaatc ctacgctgcc agtcagccat aatcggctgg tgctaatgtg 8280
   gttagcctga atcttgtcga tagtcacttg attaagaaaa aatgtggacg gtagtgaaat 8340
   ataaggcaaa gcagctcaca gaggacagca cgggtaggac atggcgggct ccggacccga 8400
   aagggcagag catcagatta tcctaccaga gtcacatctg tcctcaccat tggtcaagca 8460
   taaattgctt tattactgga aattaactgg gctaccgctt cctgacgaat gtgacttcga 8520
   ccatcttatt atcagccgac aatggaagaa ggtacttgaa tcggccaccc ctgacattga 8580
   gagaatgata aaactagggc gggcagtaca ccagactctc aaccacaatt ccaggataac 8640
   cggagtactc catccccggt gtttagagga actggctagt attgaggttc ctgattcaac 8700
   caacaaattt cggaagatcg aaaaaaagat ccagattcac aacacaaggt atggagaact 8760
   gttcacaaga ttatgcacgc atgtagaaaa gaaactattg gggtcatctt ggtctaacaa 8820
   tgtcccacgg tcagaggaat tcaacagcat ccgtacagat ccggcatttt ggtttcactc 8880
   aaaatggtcc acagccaaat ttgcatggct ccatataaaa caggtccaga ggcatctgat 8940
   tgtagcagca agaacaaggt ccgcagtcaa caaattagtg acgctgaccc ataaggtagg 9000
   ccaaatcttt gttactcctg agcttgtcat tgtgacacat acagatgaga acaagttcac 9060
   gtgtcttacc caggaacttg tgttgatgta tgcagatatg atggagggca gagatatggt 9120
   cagcataata tcatccacgg cggcacatct taggagctta tcagagaaaa ttgatgatat 9180
   tctgcggtta gtagatgctc tggcaagaga tttgggcaat caagtctacg atgttgtagc 9240
   actaatggag ggattcgcat acggcgctgt tcagctgctt gaaccgtcag gtacatttgc 9300
   gggggatttc ttcgcattca acctgcagga gctcaaagat actctaaccg gactcctccc 9360
   caaggatatc gcagaatctg tgactcacgc aatcgcaacc atattctctg gcttagaaca 9420
   aaatcaagca gctgagatgt tgtgcctgtt gcgtctgtgg ggtcacccac tactcgagtc 9480
   ccgtattgca gcaaaagcag ttaggagtca aatgtgcgca ccaaaaatgg tagactttga 9540
   tatgatcctc caggtattat ctttctttaa aggaacaatc atcaatggat atagaaagaa 9600
   gaatgcaggc gtgtggccac gtgtcaagat agatacgata tacgggaagg tcatagggca 9660
   gctacacgca gattctgcag agatttcaca tgatgtcatg ttgagggaat acaagagttt 9720
   atctgcactt gaattcgagc catgtataga gtatgaccct gtcaccaatc tgagcatgtt 9780
   tttaaaagac aaggcaatcg cacacccgaa agacaactgg ctcgcttcgt ttaggcgaaa 9840
   ccttctctct gaggaccaga agaaacatgt aagagaggca acctcaacta accgcctctt 9900
   gatagagttc ttagagtcaa atgattttga tccatataaa gagatggaat atctgacgac 9960
   ccttgagtac ctaagagatg ataatgtggc agtatcatac tcactcaaag aaaaagaggt 10020
   gaaagttaat gggcggattt tcgcaaagct aacaaataga ttaaggaatt gtcaggtaat 10080
   ggcagaaggg atcctagctg accagattgc acctttcttc cagggaaatg gggtcattca 10140
   ggatagcata tctttgacta aaagtatgct agcgatgagt caactgtctt tcaacagcaa 10200
   caagaaacgt attactgact gcaaagaaag agtatcctca aaccgcaatc atgatccgaa 10260
   gagcaagaat cgtcggagag ttgccacttt tgtaactacc gacctgcaaa agtattgtct 10320
   caattggaga tatcagacag tcaagctgtt cgctcatgcc atcaatcagc tgatgggctt 10380
   acctcacttc ttcgagtgga ttcatcttag actaatggat actacgatgt ttgtagggga 10440
   ccctttcaat cctccaagtg accctacgga ctgtgatcta tcaagagtcc caaatgatga 10500
   catatatatt gtcagtgcta ggggaggcat tgagggatta tgccagaagc tatggacaat 10560
   gatctcaatt gctgcaatcc agcttgctgc agcaagatca cattgtcgcg ttgcctgtat 10620
   ggtacaaggc gacaatcaag taatagctgt aacgagagag gtaagatcag atgactcccc 10680
   agagatggtg ttaacacaat tgcatcaagc cagtgataat ttcttcaagg aattgattca 10740
   tgtcaatcat ttgatcggcc ataatttgaa ggatcgtgaa accatcaggt cagacacgtt 10800
   cttcatatac agcaaacgaa tattcaaaga tggagcaata ctcagtcagg tcctcaaaaa 10860
   ctcatctaaa ttagtgctaa tatcaggcga ccttagtgaa aacactgtaa tgtcttgtgc 10920
   caacattgca tctactgtag cacggttatg cgagaacggg cttcctaagg atttctgtta 10980
   ttacttaaac tacttaatga gttgcgtgca gacatacttc gattccgaat tttccatcac 11040
   caacaactcg caactcgatt ctaaccagtc gtggatagag gacatttctt ttgtgcactc 11100
   atatgtcctg acccctgctc aactgggggg actgagtaac cttcaatact caaggctcta 11160
   cacaaggaac atcggcgacc cgggaaccac tgctttcgca gagatcaaga gattagaggc 11220
   agtggggtta ctgagtccta gcattatgac taacatctta actaggccgc ctggaaatgg 11280
   agattgggcc agtctgtgca acgatccata ctcctttaat tttgagactg tcgcaagccc 11340
   aaatattgtc cttaagaaac atacacaaag agtcctattt gaaacttgct cgaatccctt 11400
   attatctgga gtgcacacag aggataatga ggcagaagag aaggcattgg ctgaattctt 11460
   gctcaatcaa gaaatgattc atccacgtgt cgcgcatgct atcatggaag caagctctgt 11520
   aggtaggaga aagcaaattc aggggcttgt tgacacaaca aacaccgtga ttaagattgc 11580
   actgactagg aggccactcg gcatcaagag gctgatgcgg atagtcaatt actcgagcat 11640
   gcatgcaatg ctatttagag atgatgtttt ctcgtctaat agatccaacc atcccttagt 11700
   ctcttctgat atgtgttctc tgacactggc agactatgca cggaacagaa gctggtcacc 11760
   tttgacaggg ggtagaaaaa tactgggtgt atctaatcct gataccatag aacttgtaga 11820
   gggtgagatc cttagtgtca gtggagggtg cactagatgt gacagtgggg atgaacagtt 11880
   tacttggttc catcttccaa gcaatataga gctaaacgat gacaccagca agaatcctcc 11940
   aatgagagtg ccatatctcg ggtcaaagac tcaagagagg agagccgcct cgcttgcgaa 12000
   aatagctcat atgtcaccac acgtgaaggc ggctctaagg gcgtcatctg tgttaatctg 12060
   ggcttatggg gacaacgaaa taaactggac tgctgccctt aagattgcaa ggtctcggtg 12120
   caacataagc tcagagtatc ttcgactatt gtcacccttg cctacagctg ggaatctcca 12180
   acatagattg gacgacggca taactcagat gacattcacc cctgcatctc tatacagggt 12240
   gtcaccttac attcatatat ctaatgattc tcaaaggcta tttactgaag aaggagtcaa 12300
   agaggggaat gtggtttatc agcaaatcat gctcttgggt ttgtccttaa ttgagtcact 12360
   cttcccaatg acaacaacca agacatatga tgaaatcaca ttgcacctcc acagtaaatt 12420
   tagctgctgt atcagggaag cacctgttgc agttcctttc gagctactcg gggtggcacc 12480
   ggaactaagg gcagtaacct caaataagtt tatgtatgat cctagccctg tatcagaggg 12540
   agactttgcg agacttgact tagctatctt taagagttat gaacttaatt tagagtcata 12600
   ttccacaata gagctaatga acgttctttc aatatctagt gggaagttga ttggccagtc 12660
   tgtggtttct tatgatgaag atacctccat aaagaatgac gctataatag tgtatgacaa 12720
   cacacggaat tggatcagcg aagctcagaa ttcagatgtg gtccgcctat tcgagtatgc 12780
   ggcactcgaa gtgctcctcg actgttctta tcaactctac tatctgagag taagaggcct 12840
   aaacaatatc gtcctgtaca tgagtgattt atacaagaat atgccaggaa ttctactctc 12900
   taatattgca gccacaatat ctcaccctgt catccattca aggttgaatg cagtaggtct 12960
   ggtcaaccat gacgggtcac accaacttgc agacacagat ttcattgaaa tgtctgcaaa 13020
   gctgctagtc tcttgcactc gacgcgtggt ctcaggttta catgcaggga ataagtatga 13080
   cctgctgttt ccatctgtct tggatgataa cctaagtgag aagatgcttc agttgatatc 13140
   ccaattatgc tgtctgtata cggtgctctt tgctacaaca agagaaatcc cgaaaataag 13200
   aggcttatct gcagaagaga aatgttcagt acttactgag tacctactgt cagatgctgt 13260
   gaaaccgtta cttgggtccg agcaagtgag ctctatcatg tctcccaaca tagttacgtt 13320
   cccagccaat ctgtattaca tgtctaggaa gagccttaat ttgatcaggg agagagagga 13380
   cagggatact atcttggcat tgttgttccc tcaagaaccg ctgctcgagt ttcctctggt 13440
   acgagatatt ggtgctcgtg taaaagatcc atttacccga caacctgcgg cgtttttaca 13500
   agagttagat ttgagtgctc cggcaaggta tgacgcattc acaatcagtc aggcgcattc 13560
   tgaacacata ttgccaaacc cagaggaaga tcacttagta cgatacttgt tcagaggaat 13620
   agggactgcg tcctcctctt ggtataaggc atcccatctt ctttctgtac ccgaggtcag 13680
   atgtgcaagg catgggaact ccttatattt agcagaagga agtggagcca tcatgagtct 13740
   tctcgaattg catataccac acgaaactat ctattacaat acacttttct cgaacgagat 13800
   gaacccccca cagcgacatt tcggaccaac tccaacacag tttctgaatt cggttgtttt 13860
   taggaattta caggcggaag taccatgcaa ggatggattt gtccaggagt tccgtccgtt 13920
   atggagagag aatacagaag aaagcgatct gacctcagat aaagcagtgg gatatatcac 13980
   atctgtagtg ccgtacaggt ctgtatcatt gctgcattgt gacattgaaa tccctccagg 14040
   atctaatcaa agcttactag atcagctggc taccaatctg tctctgattg ccatgcattc 14100
   tgtgaaggag ggcggggtcg tgattatcaa agtactgtat gcaatgggat attacttcca 14160
   tctactcatg aacttgttta ctccatgttc cacgaaagga tatattctct ctaatggcta 14220
   tgcctgtaga ggggatatgg agtgttacct gatatttgtc atgggctacc taggcgggcc 14280
   tacatttgta catgaggtgg tgaggatggc aaaaacgcta gtacagcggc atggcacact 14340
   tttgtctaag tcagatgaga ttacactgac taggttattt acttcacagc aacagcgtgt 14400
   aacagacatc ctatccagcc ctttaccgag actaatgaag tacttgagag agaatattga 14460
   tactgcactg attgaagccg gggacagccc tgtccgtcca ttctgtgcag agagtttagt 14520
   gagcacacta acagacatga ctcagacaac ccagatcatc gccagccaca ttgatacagt 14580
   cattcgatct gtgatctata tggaagcaga gggtgatctt gctgacacag tattcttatt 14640
   taccccttac aacctctcta tggacgggaa aaagagaaca tcacttaaac agtgcacaag 14700
   acagatctta gaggtcacaa tactgggtct cagagtcaaa aatctcaata aagtaggtga 14760
   tgtaatcagc ctagtactca gaggtatgat ttctctggag gaccttctcc cactgagaac 14820
   ctacttgaag tgtagtacct gccctaagta tttgaaggct gtcctaggta ttaccaaact 14880
   caaagaaatg ttcacagacg cctctttatt atacttgact cgtgctcaac aaaaattcta 14940
   catgaaaact ataggcaatg cagtcaaggg atactatagt aactgtgact cttaaaggca 15000
   accacatatc aataggccct ctttctagcc gatcgtattc ttgttgactt cattatacca 15060
   tattagaaaa aaattgaatt ccgacccttt aagactcgta ttcggattca aataattatc 15120
   tcagaaaaaa agtgcacgta gttgttcttg attatagtcc cgtcattcac caagtctttg 15180
   tttggt 15186

<210> 2
<211> 15186
<212> DNA
<213> Newcastle Disease Virus

<220>
<223> Mut
HN(F277L)/M(G165W)/F(F117S)/F(F190L)/F(L289A)/L(V757I)/L(F1551S)/L(R1700L)

<400> 2
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat tttagatttt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gcccccaccc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

ttggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620
ccgtctgaca agttctcttg atggcaggcc tcttgcagcc gcagggatcg tggtaacagg 4680
ggataaagca gttaacatat acacctcatc ccagacaggg tcaatcatag tcaagttact 4740
cccaaatatg cccaaggaca aagaggcatg tgcaaaagcc ccattggagg cttacaacag 4800
gacactgact actttgctta ccccccttgg tgattctatc cgcaggatac aagagtctgt 4860
gactacatcc ggaggaagga gacagagacg ctctataggt gccattattg gcagtgtagc 4920
tctaggggtt gcaacagctg cacagataac ggcagcctcg gctctgatac aagccaacca 4980
gaatgctgct aacatcctcc ggcttaagga gagcattgct gcaaccaatg aagctgtaca 5040
cgaggtcact ggcggattgt cacagttagc agtggcagtt ggaaagatgc agcaatttgt 5100
taatgaccag cttaataaca cagctcaaga gttggactgt ataaaaatta cacagcaggt 5160
tggtgtagaa ctcaacttat acttaactga attgactaca gtgttcggcc cacaaatcac 5220
ttcccctgcc ttaactcagt tgactgttca ggctctttac aatctggctg gtggtaatgt 5280
agattacttg ttgactaagt taggtgtagg gaacaaccag ctcagctcat tgattggtag 5340
cggcttgatc accggtaacc ctatttttta cgactcacag actcaactct tgggcataca 5400
ggtgactgca ccctcagtcg ggaacctaaa taatatgcgt gccacctact tggagacctt 5460
gtctgtaagc acaacaaagg gatttgcctc agcacttgtc ccaaaagtag tgacacaggt 5520
cggttctgtg atagaagagc ttgacacctc atactgtata gaagctgatt tggatttata 5580
ttgtacaaga atagtgacat tccctatgtc ccctggtatt tattcctgtt tgagcggcaa 5640
tacatcggct tgcatgtatt caaggactga aggcgcactt actacaccat acatgactct 5700
caaaggctca gttgttgcca attgccagat gacaacatgt agatgtgcag accccccggg 5760
tatcatatca caaaattatg gagaggctgt gtctctaata gataagcact catgcaatgt 5820
cgtatcctta gacgggataa ctttgaggct cagtggggaa tttgatgcaa cttatcaaaa 5880
gaatatctca atattagatt ctcaagtact agtgacaggc aatctcgata tctcaactga 5940
gcttgggaat gtcaaccact caataagtaa tgctttggat aagttagagg aaagcaacag 6000
caaactagac aaagtcaatg tcaaactgac cagcacatct gctctcatta cctatattgt 6060
tttaactgtc atatctcttg ttcttggtat gcttagcctg gttctagcat gctatctgat 6120
gtacaagcaa aaagcgcaac gaaagacctt gttgtggctt ggaaataata ccctagatca 6180
gatgagagct actacaaaaa tgtgaatgca gatgagaggc agaggtatcc ccaatagcaa 6240
tctgtgtgtc aattctggca gcctgttaat cagaagaatt aagaaaaaac taccggatgt 6300
aggtgaacaa aagggaatat acgggtagaa cggcctgaga ggccacccct caatcgggag 6360
ccaggcccca ctacgtccgc tctaccgcat caccaacagc agtcttcagt catggacagc 6420
gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020

cagtatttag cacttggtgt gcttcggaca tctgcaacag ggagggtatt cttttctact 7080

ctgcgttcca tcaacttgga tgacgcccaa aatcggaagt cttgcagtgt gagtgcaact 7140

cctttaggtt gtgatatgct gtgctctaaa atcacagaga ctgaggaaga ggattataaa 7200

tcagttatcc ccacatcgat ggtacatgga aggttagggc ttgacggcca ataccatgag 7260

aaggacctag acgtcacaac actatttagg gactgggtgg caaattaccc aggagtagga 7320

ggtgggtctt ttattaacaa tcgcgtatgg ttcccggtct acggagggct aaaacccagc 7380

tcgcctagtg acactgcaca agaagggaga tacgtaatat acaagcgata caatgacaca 7440

tgcccggatg agcaagatta ccagattcgg atggctaagt catcgtataa gcctaggcgg 7500

tttggtggaa aacgcgtaca gcaggccatc ctatccatca aggtgtcaac atccttgggt 7560

gaggacccgg tgttgactgt accgcctaat acggtcgcac tcatgggggc cgaaggcaga 7620

gttctcacag tggggacatc tcatttctta tatcagcgag ggtcatcata cttctctccc 7680

gctttgttat accctatgac agtcaacaac aaaacagcca ctcttcataa tccttataca 7740

ttcaatgctt tcactcggcc aggtagtgtc ccttgccagg cttcagcaag atgccccaac 7800

tcatgtgtta ccggagtcta tactgatcca tatcccttag tcttccatag gaaccacacc 7860

ttgcgagggg tattcggaac aatgcttgat gacgaacaag caagactcaa ccctgtatct 7920

gcagtatttg ataacatatc ccgcagtcgc ataactcggg taagttcaag cagtaccagg 7980

gcagcataca cgacatcaac gtgttttaaa gttgtcaaga ccaataaaac ctattgcctc 8040

agcattgcag aaatatccaa taccctcttc ggggaattca gaattgtccc cttactagtt 8100

gagattctca aggatggtgg ggtttaagaa gctaggaatg gtcggttgag tcaaccgtga 8160

gaaggcgggg aagatgatat tgcatcatct atcttctgta acaccaagaa tcaaatcgaa 8220

taccagtgcg agctcgaatc ctacgctgcc agtcagccat aatcggctgg tgctaatgtg 8280

gttagcctga atcttgtcga tagtcacttg attaagaaaa aatgtggacg gtagtgaaat 8340

ataaggcaaa gcagctcaca gaggacagca cgggtaggac atggcgggct ccggacccga 8400

aagggcagag catcagatta tcctaccaga gtcacatctg tcctcaccat tggtcaagca 8460

taaattgctt tattactgga aattaactgg gctaccgctt cctgacgaat gtgacttcga 8520

ccatcttatt atcagccgac aatggaagaa ggtacttgaa tcggccaccc ctgacattga 8580

gagaatgata aaactagggc gggcagtaca ccagactctc aaccacaatt ccaggataac 8640

cggagtactc catccccggt gtttagagga actggctagt attgaggttc ctgattcaac 8700

caacaaattt cggaagatcg aaaaaaagat ccagattcac aacacaaggt atggagaact 8760

gttcacaaga ttatgcacgc atgtagaaaa gaaactattg gggtcatctt ggtctaacaa 8820

tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacgggaagg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgagggatta tgccagaagc tatggacaat 10560

gatctcaatt gctgcaatcc agcttgctgc agcaagatca cattgtcgcg ttgcctgtat 10620

ggtacaaggc gacaatcaag taatagctat aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800

cttcatatac agcaaacgaa tattcaaaga tggagcaata ctcagtcagg tcctcaaaaa 10860

ctcatctaaa ttagtgctaa tatcaggcga ccttagtgaa aacactgtaa tgtcttgtgc 10920

caacattgca tctactgtag cacggttatg cgagaacggg cttcctaagg atttctgtta 10980

ttacttaaac tacttaatga gttgcgtgca gacatacttc gattccgaat tttccatcac 11040

caacaactcg caactcgatt ctaaccagtc gtggatagag gacatttctt ttgtgcactc 11100

atatgtcctg acccctgctc aactgggggg actgagtaac cttcaatact caaggctcta 11160

cacaaggaac atcggcgacc cgggaaccac tgctttcgca gagatcaaga gattagaggc 11220

agtggggtta ctgagtccta gcattatgac taacatctta actaggccgc ctggaaatgg 11280

agattgggcc agtctgtgca acgatccata ctcctttaat tttgagactg tcgcaagccc 11340

aaatattgtc cttaagaaac atacacaaag agtcctattt gaaacttgct cgaatccctt 11400

attatctgga gtgcacacag aggataatga ggcagaagag aaggcattgg ctgaattctt 11460

gctcaatcaa gaaatgattc atccacgtgt cgcgcatgct atcatggaag caagctctgt 11520

aggtaggaga aagcaaattc aggggcttgt tgacacaaca aacaccgtga ttaagattgc 11580

actgactagg aggccactcg gcatcaagag gctgatgcgg atagtcaatt actcgagcat 11640

gcatgcaatg ctatttagag atgatgtttt ctcgtctaat agatccaacc atcccttagt 11700

ctcttctgat atgtgttctc agacactggc agactatgca cggaacagaa gctggtcacc 11760

tttgacaggg ggtagaaaaa tactgggtgt atctaatcct gataccatag aacttgtaga 11820

gggtgagatc cttagtgtca gtggagggtg cactagatgt gacagtgggg atgaacagtt 11880

tacttggttc catcttccaa gcaatataga gctaaacgat gacaccagca agaatcctcc 11940

aatgagagtg ccatatctcg ggtcaaagac tcaagagagg agagccgcct cgcttgcgaa 12000

aatagctcat atgtcaccac acgtgaaggc ggctctaagg gcgtcatctg tgttaatctg 12060

ggcttatggg gacaacgaaa taaactggac tgctgccctt aagattgcaa ggtctcggtg 12120

caacataagc tcagagtatc ttcgactatt gtcacccttg cctacagctg ggaatctcca 12180

acatagattg gacgacggca taactcagat gacattcacc cctgcatctc tatacagggt 12240

gtcaccttac attcatatat ctaatgattc tcaaaggcta tttactgaag aaggagtcaa 12300

agaggggaat gtggtttatc agcaaatcat gctcttgggt ttgtccttaa ttgagtcact 12360

cttcccaatg acaacaacca agacatatga tgaaatcaca ttgcacctcc acagtaaatt 12420

tagctgctgt atcagggaag cacctgttgc agttcctttc gagctactcg gggtggcacc 12480

ggaactaagg gcagtaacct caaataagtt tatgtatgat cctagccctg tatcagaggg 12540

agactttgcg agacttgact tagctatctt taagagttat gaacttaatt tagagtcata 12600

ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

cagggatact atcttggcat tgttgttccc tcaagaaccg ctgctcgagt ttcctctggt 13440

acgagatatt ggtgctcgtg taaaagatcc atttacccta caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 3
<211> 15198
<212> DNA
<213> Newcastle Disease Virus

<220>
<223> Mut
HN(F277L)/M(G165W)/F(F117S)/F(F190L)/F(L289A)/L(V757I)/L(F1551S)/L(R1700L)+Af
eI

<400> 3
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagcgct gaccgagccc tcctctataa acccaagatt caaggctcca 3180

agcgataacc ttctcccgcc tcttccatcc cactgaatga tcgcgcagct gcaattaatc 3240

tagcaacatt aaagattaag aaaaaatacg ggtagaatcg gagtgcaccg attgtgccaa 3300

gatggactca tctaggacaa ttgggttata ctttgattct gcccttcctt ctagcaacct 3360

gttagcattc ccgattgtct tacgagatgt aggagatgga aagaagcaaa ttactccgca 3420

atataggatc cggcgtcttg actcgtggac agacagtaaa gaagactcgg tattcatcac 3480

tacctacgga ttcatcttcc aggttgggaa tgaagaagtc actgtcggca tgatcaatga 3540

taatcccaag cgagagttac tctccgctgc gatgctctgc ctaggaagcg tcccgaatgt 3600

tggagatcct gttgagctgg caagggcctg cctcactatg gtggtaacat gcaagaagag 3660

tgcaactaat actgagagaa tggtcttctc agtagtgcag gcaccccggg tgctgcaaag 3720

ctgtagggtc gtggcagaca aatactcgtc agtgaatgca gttaagcacg tgaaagcacc 3780

agagaagatc ccttggagtg aaaccctaga gtacaaggtg aattttgtct ctttgactgt 3840

ggtgccgaag aaggatgtct acaagatccc aaccgcagta ttgaaggtat ccggctcgag 3900

cctgtataat cttgcgctca atgtcactat tgatgtggag gtagacccaa agagcccgtt 3960

agtcaagtcc ctttctaggt ccgacagtgg atactatgct aatctcttct tacatatcgg 4020

acttatgtcc actgtagata agaggggaaa gaaagtgaca tttgaccagc tggagaggaa 4080

gataaggaga cttgatctat ctgtcgggct cagtgatgtg ctcggacctt ctgtgcttgt 4140

gaaggcgaga ggtgcacgga ctaggctgct ggcacctttc ttctctaaca gcgggacagc 4200

ctgctaccct atagcaaatg cctctcctca ggtagctaag atactctgga gtcagaccgc 4260

gtgcctgcgg agtgtaaaaa tcattattca agcgggcacc caacgcgctg tcgcagtgac 4320

tgctgaccat gaggttacct ctactaagat agagaagagg cataccatcg ctaaatacaa 4380

tcccttcaag aaataggccg tatccctgag actgcaattc acccgccttc ccaaaacacc 4440

atgacaccag ataatgatct gtcttgatta cttacagtta gtttccctgt ctatcaaatt 4500

agaaaaaaca cgggtagaag agttcggatc ccggccggcg caccaaaagc gcaagatggg 4560

ccccagatct tctaccagga tcccagtacc tctaatgctg accatacgga tcacgctggc 4620

actgagttat gtccgtctga caagttctct tgatggcagg cctcttgcag ccgcagggat 4680

cgtggtaaca ggggataaag cagttaacat atacacctca tcccagacag ggtcaatcat 4740

agtcaagtta ctcccaaata tgcccaagga caaagaggca tgtgcaaaag ccccattgga 4800

ggcttacaac aggacactga ctactttgct taccccccct ggtgattcta tccgcaggat 4860

acaagagtct gtgactacat ccggaggaag gagacagaga cgctctatag gtgccattat 4920

tggcagtgta gctctagggg ttgcaacagc tgcacagata acggcagcct cggctctgat 4980

acaagccaac cagaatgctg ctaacatcct ccggcttaag gagagcattg ctgcaaccaa 5040

tgaagctgta cacgaggtca ctggcggatt gtcacagtta gcagtggcag ttggaaagat 5100

gcagcaattt gttaatgacc agcttaataa cacagctcaa gagttggact gtataaaaat 5160

tacacagcag gttggtgtag aactcaactt atacttaact gaattgacta cagtgttcgg 5220

gccacaaatc acttcccctg ccttaactca gttgactgtt caggctcttt acaatctggc 5280

tggtggtaat gtagattact tgttgactaa gttaggtgta gggaacaacc agctcagctc 5340

attgattggt agcggcttga tcaccggtaa ccctattttt tacgactcac agactcaact 5400

cttgggcata caggtgactg caccctcagt cgggaaccta aataatatgc gtgccaccta 5460

cttggagacc ttgtctgtaa gcacaacaaa gggatttgcc tcagcacttg tcccaaaagt 5520

agtgacacag gtcggttctg tgatagaaga gcttgacacc tcatactgta tagaagctga 5580

tttggattta tattgtacaa gaatagtgac attccctatg tcccctggta tttattcctg 5640

tttgagcggc aatacatcgg cttgcatgta ttcaaggact gaaggcgcac ttactacacc 5700

atacatgact ctcaaaggct cagttgttgc caattgccag atgacaacat gtagatgtgc 5760

agaccccccg ggtatcatat cacaaaatta tggagaggct gtgtctctaa tagataagca 5820

ctcatgcaat gtcgtatcct tagacgggat aactttgagg ctcagtgggg aatttgatgc 5880

aacttatcaa aagaatatct caatattaga ttctcaagta ctagtgacag gcaatctcga 5940

tatctcaact gagcttggga atgtcaacca ctcaataagt aatgctttgg ataagttaga 6000

ggaaagcaac agcaaactag acaaagtcaa tgtcaaactg accagcacat ctgctctcat 6060

tacctatatt gttttaactg tcatatctct tgttcttggt atgcttagcc tggttctagc 6120

atgctatctg atgtacaagc aaaaagcgca acgaaagacc ttgttgtggc ttggaaataa 6180

taccctagat cagatgagag ctactacaaa aatgtgaatg cagatgagag gcagaggtat 6240

ccccaatagc aatctgtgtg tcaattctgg cagcctgtta atcagaagaa ttaagaaaaa 6300

actaccggat gtaggtgaac aaaagggaat atacgggtag aacggcctga gaggccaccc 6360

ctcaatcggg agccaggccc cactacgtcc gctctaccgc atcaccaaca gcagtcttca 6420

gtcatggaca gcgcagttag ccaagttgcg ctagagaatg atagaagaga agcgaataat 6480

acatggcgct tggttttccg gatcgcagcc ttacttttac tggtaataac cttagccgtc 6540

tctgcagtcg ccctggcata tagtatggag gctagcacac ctggcgacct tgtaagcata 6600

ccgactgcga tctctagggc agaggaaagg attacatctg cactcggttc caatcaagat 6660

gtagtagata ggatatacaa gcaagtggcc cttgagtctc cattggcact gctaaatacc 6720

gaatctataa ttatgaatgc aataacgtct ctctcttatc aaatcaatgg agccacaaat 6780

aatagcgggt gtggggcacc tgttcatgac ccagattaca tcggggggat aggtcaagaa 6840

cttattgtag atgatacgag tgacgtcaca tcattttatc cctctgcatt ccaagaacac 6900

ctgaatttta tcccggcgcc cactacagga tcaggctgca ctcggatacc ctcattcgac 6960

atgagtgcta cccattactg ttacactcac aatgtgatat tgtctggctg cagagatcac 7020

tcacactcac atcagtattt agcacttggt gtgcttcgga catctgcaac agggagggta 7080

ttcttttcta ctctgcgttc catcaacttg gatgacgccc aaaatcggaa gtcttgcagt 7140

gtgagtgcaa ctcctttagg ttgtgatatg ctgtgctcta aaatcacaga gactgaggaa 7200

gaggattata aatcagttat ccccacatcg atggtacatg gaaggttagg gcttgacggc 7260

caataccatg agaaggacct agacgtcaca acactattta gggactgggt ggcaaattac 7320

ccaggagtag gaggtgggtc ttttattaac aatcgcgtat ggttcccggt ctacggaggg 7380

ctaaaaccca gctcgcctag tgacactgca caagaaggga gatacgtaat atacaagcga 7440

tacaatgaca catgcccgga tgagcaagat taccagattc ggatggctaa gtcatcgtat 7500

aagcctaggc ggtttggtgg aaaacgcgta cagcaggcca tcctatccat caaggtgtca 7560

acatccttgg gtgaggaccc ggtgttgact gtaccgccta atacggtcgc actcatgggg 7620

gccgaaggca gagttctcac agtggggaca tctcatttct tatatcagcg agggtcatca 7680

tacttctctc ccgctttgtt ataccctatg acagtcaaca acaaaacagc cactcttcat 7740

aatccttata cattcaatgc tttcactcgg ccaggtagtg tcccttgcca ggcttcagca 7800

agatgcccca actcatgtgt taccggagtc tatactgatc catatccctt agtcttccat 7860

aggaaccaca ccttgcgagg ggtattcgga acaatgcttg atgacgaaca agcaagactc 7920

aaccctgtat ctgcagtatt tgataacata tcccgcagtc gcataactcg ggtaagttca 7980

agcagtacca gggcagcata cacgacatca acgtgtttta aagttgtcaa gaccaataaa 8040

acctattgcc tcagcattgc agaaatatcc aataccctct tcggggaatt cagaattgtc 8100

cccttactag ttgagattct caaggatggt ggggtttaag aagctaggaa tggtcggttg 8160

agtcaaccgt gagaaggcgg ggaagatgat attgcatcat ctatcttctg taacaccaag 8220

aatcaaatcg aataccagtg cgagctcgaa tcctacgctg ccagtcagcc ataatcggct 8280

ggtgctaatg tggttagcct gaatcttgtc gatagtcact tgattaagaa aaaatgtgga 8340

cggtagtgaa atataaggca aagcagctca cagaggacag cacgggtagg acatggcggg 8400

ctccggaccc gaaagggcag agcatcagat tatcctacca gagtcacatc tgtcctcacc 8460

attggtcaag cataaattgc tttattactg gaaattaact gggctaccgc ttcctgacga 8520

atgtgacttc gaccatctta ttatcagccg acaatggaag aaggtacttg aatcggccac 8580

ccctgacatt gagagaatga taaaactagg gcgggcagta caccagactc tcaaccacaa 8640

ttccaggata accggagtac tccatccccg gtgtttagag gaactggcta gtattgaggt 8700

tcctgattca accaacaaat ttcggaagat cgaaaaaaag atccagattc acaacacaag 8760

gtatggagaa ctgttcacaa gattatgcac gcatgtagaa aagaaactat tggggtcatc 8820

ttggtctaac aatgtcccac ggtcagagga attcaacagc atccgtacag atccggcatt 8880

ttggtttcac tcaaaatggt ccacagccaa atttgcatgg ctccatataa aacaggtcca 8940

gaggcatctg attgtagcag caagaacaag gtccgcagtc aacaaattag tgacgctgac 9000

ccataaggta ggccaaatct ttgttactcc tgagcttgtc attgtgacac atacagatga 9060

gaacaagttc acgtgtctta cccaggaact tgtgttgatg tatgcagata tgatggaggg 9120

cagagatatg gtcagcataa tatcatccac ggcggcacat cttaggagct tatcagagaa 9180

aattgatgat attctgcggt tagtagatgc tctggcaaga gatttgggca atcaagtcta 9240

cgatgttgta gcactaatgg agggattcgc atacggcgct gttcagctgc ttgaaccgtc 9300

aggtacattt gcgggggatt tcttcgcatt caacctgcag gagctcaaag atactctaac 9360

cggactcctc cccaaggata tcgcagaatc tgtgactcac gcaatcgcaa ccatattctc 9420

tggcttagaa caaaatcaag cagctgagat gttgtgcctg ttgcgtctgt ggggtcaccc 9480

actactcgag tcccgtattg cagcaaaagc agttaggagt caaatgtgcg caccaaaaat 9540

ggtagacttt gatatgatcc tccaggtatt atctttcttt aaaggaacaa tcatcaatgg 9600

atatagaaag aagaatgcag gcgtgtggcc acgtgtcaag atagatacga tatacgggaa 9660

ggtcataggg cagctacacg cagattctgc agagatttca catgatgtca tgttgaggga 9720

atacaagagt ttatctgcac ttgaattcga gccatgtata gagtatgacc ctgtcaccaa 9780

tctgagcatg tttttaaaag acaaggcaat cgcacacccg aaagacaact ggctcgcttc 9840

gtttaggcga aaccttctct ctgaggacca gaagaaacat gtaagagagg caacctcaac 9900

taaccgcctc ttgatagagt tcttagagtc aaatgatttt gatccatata aagagatgga 9960

atatctgacg acccttgagt acctaagaga tgataatgtg gcagtatcat actcactcaa 10020

agaaaaagag gtgaaagtta atgggcggat tttcgcaaag ctaacaaata gattaaggaa 10080

ttgtcaggta atggcagaag ggatcctagc tgaccagatt gcacctttct tccagggaaa 10140

tggggtcatt caggatagca tatctttgac taaaagtatg ctagcgatga gtcaactgtc 10200

tttcaacagc aacaagaaac gtattactga ctgcaaagaa agagtatcct caaaccgcaa 10260

tcatgatccg aagagcaaga atcgtcggag agttgccact tttgtaacta ccgacctgca 10320

aaagtattgt ctcaattgga gatatcagac agtcaagctg ttcgctcatg ccatcaatca 10380

gctgatgggc ttacctcact tcttcgagtg gattcatctt agactaatgg atactacgat 10440

gtttgtaggg gaccctttca atcctccaag tgaccctacg gactgtgatc tatcaagagt 10500

cccaaatgat gacatatata ttgtcagtgc taggggaggc attgagggat tatgccagaa 10560

gctatggaca atgatctcaa ttgctgcaat ccagcttgct gcagcaagat cacattgtcg 10620

cgttgcctgt atggtacaag gcgacaatca agtaatagct ataacgagag aggtaagatc 10680

agatgactcc ccagagatgg tgttaacaca attgcatcaa gccagtgata atttcttcaa 10740

ggaattgatt catgtcaatc atttgatcgg ccataatttg aaggatcgtg aaaccatcag 10800

gtcagacacg ttcttcatat acagcaaacg aatattcaaa gatggagcaa tactcagtca 10860

ggtcctcaaa aactcatcta aattagtgct aatatcaggc gaccttagtg aaaacactgt 10920

aatgtcttgt gccaacattg catctactgt agcacggtta tgcgagaacg ggcttcctaa 10980

ggatttctgt tattacttaa actacttaat gagttgcgtg cagacatact tcgattccga 11040

attttccatc accaacaact cgcaactcga ttctaaccag tcgtggatag aggacatttc 11100

ttttgtgcac tcatatgtcc tgacccctgc tcaactgggg ggactgagta accttcaata 11160

ctcaaggctc tacacaagga acatcggcga cccgggaacc actgctttcg cagagatcaa 11220

gagattagag gcagtggggt tactgagtcc tagcattatg actaacatct taactaggcc 11280

gcctggaaat ggagattggg ccagtctgtg caacgatcca tactccttta attttgagac 11340

tgtcgcaagc ccaaatattg tccttaagaa acatacacaa agagtcctat ttgaaacttg 11400

ctcgaatccc ttattatctg gagtgcacac agaggataat gaggcagaag agaaggcatt 11460

ggctgaattc ttgctcaatc aagaaatgat tcatccacgt gtcgcgcatg ctatcatgga 11520

agcaagctct gtaggtagga gaaagcaaat tcaggggctt gttgacacaa caaacaccgt 11580

gattaagatt gcactgacta ggaggccact cggcatcaag aggctgatgc ggatagtcaa 11640

ttactcgagc atgcatgcaa tgctatttag agatgatgtt ttctcgtcta atagatccaa 11700

ccatccctta gtctcttctg atatgtgttc tcagacactg gcagactatg cacggaacag 11760

aagctggtca cctttgacag ggggtagaaa aatactgggt gtatctaatc ctgataccat 11820

agaacttgta gagggtgaga tccttagtgt cagtggaggg tgcactagat gtgacagtgg 11880

ggatgaacag tttacttggt tccatcttcc aagcaatata gagctaaacg atgacaccag 11940

caagaatcct ccaatgagag tgccatatct cgggtcaaag actcaagaga ggagagccgc 12000

ctcgcttgcg aaaatagctc atatgtcacc acacgtgaag gcggctctaa gggcgtcatc 12060

tgtgttaatc tgggcttatg gggacaacga aataaactgg actgctgccc ttaagattgc 12120

aaggtctcgg tgcaacataa gctcagagta tcttcgacta ttgtcaccct tgcctacagc 12180

tgggaatctc caacatagat tggacgacgg cataactcag atgacattca cccctgcatc 12240

tctatacagg gtgtcacctt acattcatat atctaatgat tctcaaaggc tatttactga 12300

agaaggagtc aaagagggga atgtggttta tcagcaaatc atgctcttgg gtttgtcctt 12360

aattgagtca ctcttcccaa tgacaacaac caagacatat gatgaaatca cattgcacct 12420

ccacagtaaa tttagctgct gtatcaggga agcacctgtt gcagttcctt tcgagctact 12480

cggggtggca ccggaactaa gggcagtaac ctcaaataag tttatgtatg atcctagccc 12540

tgtatcagag ggagactttg cgagacttga cttagctatc tttaagagtt atgaacttaa 12600

tttagagtca tattccacaa tagagctaat gaacgttctt tcaatatcta gtgggaagtt 12660

gattggccag tctgtggttt cttatgatga agatacctcc ataaagaatg acgctataat 12720

agtgtatgac aacacacgga attggatcag cgaagctcag aattcagatg tggtccgcct 12780

attcgagtat gcggcactcg aagtgctcct cgactgttct tatcaactct actatctgag 12840

agtaagaggc ctaaacaata tcgtcctgta catgagtgat ttatacaaga atatgccagg 12900

aattctactc tctaatattg cagccacaat atctcaccct gtcatccatt caaggttgaa 12960

tgcagtaggt ctggtcaacc atgacgggtc acaccaactt gcagacacag atttcattga 13020

aatgtctgca aagctgctag tctcttgcac tcgacgcgtg gtctcaggtt tacatgcagg 13080

gaataagtat gacctgctgt ttccatctgt cttggatgat aacctaagtg agaagatgct 13140

tcagttgata tcccaattat gctgtctgta tacggtgctc tttgctacaa caagagaaat 13200

cccgaaaata agaggcttat ctgcagaaga gaaatgttca gtacttactg agtacctact 13260

gtcagatgct gtgaaaccgt tacttgggtc cgagcaagtg agctctatca tgtctcccaa 13320

catagttacg ttcccagcca atctgtatta catgtctagg aagagcctta atttgatcag 13380

ggagagagag gacagggata ctatcttggc attgttgttc cctcaagaac cgctgctcga 13440

gtttcctctg gtacgagata ttggtgctcg tgtaaaagat ccatttaccc tacaacctgc 13500

ggcgttttta caagagttag atttgagtgc tccggcaagg tatgacgcat tcacaatcag 13560

tcaggcgcat tctgaacaca tattgccaaa cccagaggaa gatcacttag tacgatactt 13620

gttcagagga atagggactg cgtcctcctc ttggtataag gcatcccatc ttctttctgt 13680

acccgaggtc agatgtgcaa ggcatgggaa ctccttatat ttagcagaag gaagtggagc 13740

catcatgagt cttctcgaat tgcatatacc acacgaaact atctattaca atacactttt 13800

ctcgaacgag atgaaccccc cacagcgaca tttcggacca actccaacac agtttctgaa 13860

ttcggttgtt tttaggaatt tacaggcgga agtaccatgc aaggatggat ttgtccagga 13920

gttccgtccg ttatggagag agaatacaga agaaagcgat ctgacctcag ataaagcagt 13980

gggatatatc acatctgtag tgccgtacag gtctgtatca ttgctgcatt gtgacattga 14040

aatccctcca ggatctaatc aaagcttact agatcagctg gctaccaatc tgtctctgat 14100

tgccatgcat tctgtgaagg agggcggggt cgtgattatc aaagtactgt atgcaatggg 14160

atattacttc catctactca tgaacttgtt tactccatgt tccacgaaag gatatattct 14220

ctctaatggc tatgcctgta gaggggatat ggagtgttac ctgatatttg tcatgggcta 14280

cctaggcggg cctacatttg tacatgaggt ggtgaggatg gcaaaaacgc tagtacagcg 14340

gcatggcaca cttttgtcta agtcagatga gattacactg actaggttat ttacttcaca 14400

gcaacagcgt gtaacagaca tcctatccag ccctttaccg agactaatga agtacttgag 14460

agagaatatt gatactgcac tgattgaagc cgggggacag cctgtccgtc cattctgtgc 14520

agagagttta gtgagcacac taacagacat gactcagaca acccagatca tcgccagcca 14580

cattgataca gtcattcgat ctgtgatcta tatggaagca gagggtgatc ttgctgacac 14640

agtattctta tttacccctt acaacctctc tatggacggg aaaaagagaa catcacttaa 14700

acagtgcaca agacagatct tagaggtcac aatactgggt ctcagagtca aaaatctcaa 14760

taaagtaggt gatgtaatca gcctagtact cagaggtatg atttctctgg aggaccttct 14820

cccactgaga acctacttga agtgtagtac ctgccctaag tatttgaagg ctgtcctagg 14880

tattaccaaa ctcaaagaaa tgttcacaga cgcctcttta ttatacttga ctcgtgctca 14940

acaaaaattc tacatgaaaa ctataggcaa tgcagtcaag ggatactata gtaactgtga 15000

ctcttaaagg caaccacata tcaataggcc ctctttctag ccgatcgtat tcttgttgac 15060

ttcattatac catattagaa aaaaattgaa ttccgaccct ttaagactcg tattcggatt 15120

caaataatta tctcagaaaa aaagtgcacg tagttgttct tgattatagt cccgtcattc 15180

accaagtctt tgtttggt 15198

<210> 4
<211> 12
<212> DNA
<213> Artificial Sequence

<220>
<223> 12bp insert containing a AfeI restriction site

<400> 4
agcgctgacc ga                                                          12

<210> 5
<211> 2238
<212> DNA
<213> Artificial Sequence

<220>
<223> transgenic construct/Atezolizumab

<400> 5
   ttaagaaaaa atacgggtag aagcagtttg agagctccgt tcgccaccat ggagttcggc      60
   ctgtcctggg tgttcctggt cgccctgttc cggggagtgc agtgtgaggt gcagctggtc     120
   gagtctgggg ggggcctggt gcagccagga ggctccctga ggctgtcttg cgcagcaagc     180
   ggcttcacct tttctgacag ctggatccac tgggtgagac aggcaccagg caagggcctg     240
   gagtgggtgg catggatcag cccttacggc ggctccacct actatgccga cagcgtgaag     300
   ggcaggttta caatctccgc cgatacctct aagaacacag cctatctgca gatgaattcc     360
   ctgcgcgccg aggataccgc cgtgtactat tgcgcacgga gacactggcc tggaggattt     420
   gattactggg gccaggggac cctggtgaca gtgagctccg cctctaccaa gggaccaagc     480
   gtgttcccac tggcaccttc tagcaagtcc acctctggag gaacagccgc cctgggctgt     540
   ctggtgaagg attactttcc tgagccagtg accgtgagct ggaactccgg cgccctgacc     600
   agcggagtgc acacattccc agccgtgctg cagtcctctg gcctgtactc cctgagctcc     660
   gtggtgaccg tgccctctag ctccctgggc acccagacat atatctgcaa cgtgaatcac     720
   aagccatcca atacaaaggt ggacaagaag gtggagccca agtcttgtga taagacccac     780
   acatgcccac cttgtccagc acctgagctg ctgggaggac caagcgtgtt cctgtttcca     840
   cccaagccta aggacaccct gatgatcagc aggacccctg aggtgacctg cgtggtggtg     900
   gacgtgtccc acgaggatcc agaggtgaag ttcaactggt acgtggatgg cgtggaggtg     960
   cacaatgcca agaccaagcc tcgggaggag cagtacgcct ccacctatag agtggtgtct    1020
   gtgctgacag tgctgcacca ggactggctg aacggcaagg agtataagtg caaggtgagc    1080
   aataaggccc tgccagcccc catcgagaag accatctcca aggcaaaggg acagccaagg    1140
   gagccacagg tgtacacact gcctccatct cgcgaggaga tgaccaagaa ccaggtgagc    1200
   ctgacatgtc tggtgaaggg cttttatcca agcgacatcg ccgtggagtg ggagtccaat 1260
   ggccagcccg agaacaatta caagaccaca ccccctgtgc tggactctga tggcagcttc 1320
   tttctgtatt ctaagctgac cgtggataag agcaggtggc agcagggcaa cgtgttcagc 1380
   tgctccgtga tgcacgaggc cctgcacaat cactacacac agaagtctct gagcctgtcc 1440
   ccaggcaaga ggaggaagag gggaagcgga gagggaagag gctccctgct gacctgtggc 1500
   gacgtggagg agaaccctgg cccaatgaag tatctgctgc ccacagcagc cgccggcctg 1560
   ctgctgctgg cagcacagcc tgccatggcc gatatccaga tgacccagag cccatctagc 1620
   ctgtctgcca gcgtgggcga cagggtgacc atcacatgca gggcaagcca ggacgtgtct 1680
   accgccgtgg cctggtacca gcagaagcct ggcaaggccc caaagctgct gatctactcc 1740
   gcctctttcc tgtattctgg agtgccaagc cgctttagcg gatccggatc tggaaccgac 1800
   ttcaccctga caatctcctc tctgcagccc gaggattttg ccacatacta ttgccagcag 1860
   tacctgtatc accctgccac cttcggccag gggaccaagg tggagatcaa gaggaccgtg 1920
   gcagcaccat ccgtgttcat ctttccaccc tctgacgagc agctgaagtc cggcacagcc 1980
   tctgtggtgt gcctgctgaa caatttctac cctagagagg ccaaggtgca gtggaaggtg 2040
   gataacgccc tgcagtccgg caattctcag gagagcgtga ccgagcagga ctccaaggat 2100
   tctacatata gcctgagctc caccctgaca ctgtccaagg ccgactacga gaagcacaag 2160
   gtgtatgcct gtgaggtcac ccatcagggg ctgtcctcac cagtcacaaa atccttcaat 2220
   cggggggaat gctaataa 2238

<210> 6
<211> 2250
<212> DNA
<213> Artificial Sequence

<220>
<223> transgenic construct/anti-VEGF-A

<400> 6
ttaagaaaaa atacgggtag aagcagtttg agagctccgt tcgccaccat ggagttcggc 60

ctgtcctggg tgttcctggt ggccctgttc cgcggcgtgc agtgcgaggt gcagctggtg 120

gagagcggcg gcggcctggt gcagcccggc ggcagcctga ggctgagctg cgccgccagc 180

ggctacacct tcaccaacta cggcatgaac tgggtgaggc aggcccccgg caagggcctg 240

gagtgggtgg gctggatcaa cacctacacc ggcgagccca cctacgccgc cgacttcaag 300

aggaggttca ccttcagcct ggacaccagc aagagcaccg cctacctgca gatgaacagc 360

ctgagggccg aggacaccgc cgtgtactac tgcgccaagt acccccacta ctacggcagc 420

agccactggt acttcgacgt gtggggccag ggcaccctgg tgaccgtgag cagcgccagc 480

accaagggcc ccagcgtgtt ccccctggcc cccagcagca agagcaccag cggcggcacc 540

gccgccctgg gctgcctggt gaaggactac ttccccgagc ccgtgaccgt gagctggaac 600

agcggcgccc tgaccagcgg cgtgcacacc ttccccgccg tgctgcagag cagcggcctg 660

tacagcctga gcagcgtggt gaccgtgccc agcagcagcc tgggcaccca gacctacatc 720

tgcaacgtga accacaagcc cagcaacacc aaggtggaca agaaggtgga gcccaagagc 780

tgcgacaaga cccacacctg ccccccctgc cccgcccccg agctgctggg cggccccagc 840

gtgttcctgt tcccccccaa gcccaaggac accctgatga tcagcaggac ccccgaggtg 900

acctgcgtgg tggtggacgt gagccacgag gaccccgagg tgaagttcaa ctggtacgtg 960

gacggcgtgg aggtgcacaa cgccaagacc aagcccaggg aggagcagta caacagcacc 1020

tacagggtgg tgagcgtgct gaccgtgctg caccaggact ggctgaacgg caaggagtac 1080

aagtgcaagg tgagcaacaa ggccctgccc gcccccatcg agaagaccat cagcaaggcc 1140

aagggccagc ccagggagcc ccaggtgtac accctgcccc ccagcaggga ggagatgacc 1200

aagaaccagg tgagcctgac ctgcctggtg aagggcttct accccagcga catcgccgtg 1260

gagtgggaga gcaacggcca gcccgagaac aactacaaga ccaccccccc cgtgctggac 1320

agcgacggca gcttcttcct gtacagcaag ctgaccgtgg acaagagcag gtggcagcag 1380

ggcaacgtgt tcagctgcag cgtgatgcac gaggccctgc acaaccacta cacccagaag 1440

agcctgagcc tgagccccgg caagcgccgc aagcgcggct ccggcgaggg ccgcggctcc 1500

ctgctgacct gcggcgacgt ggaggagaac cccggcccca tgaagtacct gctgcccacc 1560

gccgccgccg gcctgctgct gctggccgcc cagcccgcca tggccgacat ccagatgacc 1620

cagagcccca gcagcctgag cgccagcgtg ggcgacaggg tgaccatcac ctgcagcgcc 1680

agccaggaca tcagcaacta cctgaactgg taccagcaga agcccggcaa ggcccccaag 1740

gtgctgatct acttcaccag cagcctgcac agcggcgtgc ccagcaggtt cagcggcagc 1800

ggcagcggca ccgacttcac cctgaccatc agcagcctgc agcccgagga cttcgccacc 1860

tactactgcc agcagtacag caccgtgccc tggaccttcg gccagggcac caaggtggag 1920

atcaagagga ccgtggccgc ccccagcgtg ttcatcttcc cccccagcga cgagcagctg 1980

aagagcggca ccgccagcgt ggtgtgcctg ctgaacaact tctaccccag ggaggccaag 2040

gtgcagtgga aggtggacaa cgccctgcag agcggcaaca gccaggagag cgtgaccgag 2100

caggacagca aggacagcac ctacagcctg agcagcaccc tgaccctgag caaggccgac 2160

tacgagaagc acaaggtgta cgcctgcgag gtgacccacc agggcctgag cagccccgtg 2220

accaagagct tcaacagggg cgagtgctga 2250

<210> 7

<211> 2244
<212> DNA
<213> Artificial Sequence

<220>
<223> transgenic construct/anti-KIR
<400> 7
ttaagaaaaa atacgggtag aagcagtttg agagctccgt tcgccaccat ggagttcggc 60

ctgtcctggg tgttcctggt ggccctgttc cgcggcgtgc agtgccaagt gcaactggtg 120

cagagcggtg cagaggtgaa gaagccagga tcctctgtga aagtctcatg caaagccagt 180

ggaggtacgt tcagctttta cgcaatcagc tgggtgagac aggctccggg ccaaggtctg 240

gaatggatgg gcggattcat tccaattttc ggcgcagcaa actacgcaca gaagttccag 300

gggcgggtca cgattactgc tgatgagtcg acaagcacag catacatgga gctcagttct 360

ttgagaagcg atgatacagc agtttattac tgtgctagaa ttccaagcgg ttcttattac 420

tatgactacg atatggatgt ctggggacag ggaacaacag tcaccgtatc ttccgcgagc 480

accaagggac cttccgtgtt tcccctggcc ccttgctcca gatcaacctc agagtcgact 540

gctgctctcg gttgcttggt gaaagattat tttccagaac ccgttaccgt gtcttggaac 600

tccggtgccc ttacttcagg ggttcatacc ttccccgcag tcctacagtc atctggtctg 660

tattctctca gctccgtggt caccgtccca agcagctcac tgggcaccaa gacctatacc 720

tgcaacgtcg accataagcc tagtaataca aaggtcgaca agcgagtcga atccaaatac 780

ggacctcctt gtccaccttg ccccgctcct gaattcctgg gtggaccaag cgtctttctg 840

tttccaccaa aacctaaaga cacacttatg atttcccgca cacccgaagt cacctgcgtg 900

gtggtggatg tcagccagga agaccctgag gtgcaattca actggtacgt tgatggcgta 960

gaagtccaca acgccaagac aaaacccaga gaggagcagt ttaatagtac ctatagggtg 1020

gtgtccgtcc tgaccgttct ccaccaggat tggttgaatg gcaaggaata caagtgtaaa 1080

gtgagcaaca aaggcctacc ttcgagcata gaaaagacta tctccaaggc aaagggacag 1140

ccaagggagc cccaggtgta cacgctgcca ccctctcagg aggagatgac taagaaccag 1200

gtgtccttga cctgcctcgt aaaggggttt tacccctccg acatagccgt cgaatgggag 1260

agtaacggac aacccgagaa caattataaa actacacctc ccgtgctcga ctctgacgga 1320

tccttcttcc tgtactctag gctgacagtg gataaatccc gttggcagga aggaaatgta 1380

ttttcctgta gtgtgatgca cgaggctctg cacaatcatt atacacagaa atcactgtcc 1440

ttaagcctcg gcaaacgccg caagcgcggc tccggcgagg gccgcggctc cctgctgacc 1500

tgcggcgacg tggaggagaa ccccggcccc atgaagtacc tgctgcccac cgccgccgcc 1560

ggcctgctgc tgctggccgc ccagcccgcc atggccgaaa tcgtcctcac acagagcccg 1620

gttacgcttt cattgtctcc aggcgagcgc gctaccctca gctgtagggc gtctcagtcc 1680

gtttccagct atttagcttg gtaccagcaa aaacctggcc aggcccctag actgcttatt 1740

tacgacgcct ctaaccgagc gactggtata cccgcaaggt tcagcggctc gggctcaggc 1800

accgacttta ctctcaccat ttcatcactg gagcctgagg acttcgccgt gtattactgt 1860

cagcagagga gtaactggat gtacaccttc ggccaaggga ccaaattgga aatcaaaagg 1920

acagtggccg cgccaagcgt gtttatattt cccccctccg acgaacagtt aaagagtgga 1980

accgcatctg tggtatgtct gcttaacaat ttttaccctc gcgaggcaaa ggttcaatgg 2040

aaagtcgaca acgccttaca gtctggtaac agtcaagagt ctgttacgga gcaagacagc 2100

aaggactcca cctattcgct gtcttcaaca ctaaccctca gcaaggctga ttacgagaag 2160

cacaaagttt acgcctgtga ggtcacccat caggggcttt ctagccctgt cacaaagtcg 2220

ttcaacaggg gagagtgcta atga 2244

<210> 8
<211> 2232
<212> DNA
<213> Artificial Sequence

<220>
<223> transgenic construct/anti-LAG-3

<400> 8
ttaagaaaaa atacgggtag aagcagtttg agagctccgt tcgccaccat ggagttcggc 60

ctgtcctggg tgttcctggt ggccctgttc cgcggcgtgc agtgccaggt gcaactgcag 120

cagtgggggg ccggactcct gaaaccatcc gaaactctct cattgacttg cgccgtatac 180

ggcgggtcat tttccgatta ctactggaat tggatccgtc agcctccagg taaaggactt 240

gaatggatag gtgaaataaa tcatcgtgga agtactaaca gcaatcctag tctgaaatca 300

agagtaacgc tgagtttgga cacatctaag aaccagtttt cccttaaact tagaagtgtt 360

accgctgctg acacagctgt atactactgt gctttcggtt actccgatta tgagtacaat 420

tggttcgacc cttggggaca gggcacacta gtgacagtgt ccagtgcttc aaccaagggg 480

ccaagcgtgt ttcccttggc cccctgttca cgctccacta gcgaaagcac agccgcgtta 540

ggctgtctgg tgaaagacta ttttccagag ccagtcacgg tgtcttggaa ctcgggtgcc 600

ctgacatcag gtgttcatac tttccccgct gtcctccagt ccagtgggct gtactcatta 660

tcttcggtgg tgactgtccc ctcctcatcg ctggggacga agacctatac ctgtaacgtg 720

gaccacaaac caagcaacac taaggtggac aagcgcgtgg aatccaaata cggcccccca 780

tgcccaccgt gccccgcacc tgagttcctg ggcggtccta gcgtcttttt gttcccaccg 840

aaaccaaagg ataccctcat gatttccagg acgccggagg tcacctgcgt ggtcgtggac 900

gtgagtcagg aggaccccga agtgcaattt aactggtatg tggacggtgt ggaagtccat 960

aatgcaaaga ccaagcctag agaggaacaa tttaatagca cgtaccgggt ggttagtgtc 1020

cttaccgtct tgcaccagga ttggctgaac gggaaggagt ataagtgcaa ggtgtcaaac 1080

aagggactgc cctcatcaat cgagaagaca ataagtaagg cgaaggccca gccacgggag 1140

cctcaggtct acacattgcc gccaagtcaa gaggagatga ccaaaaacca ggtgtctttg 1200

acttgcctcg tgaagggttt ttacccaagt gacatcgccg tagagtggga gtctaacggc 1260

cagccagaga ataactataa aacgacacct ccagtgcttg attctgatgg atcttttttc 1320

ttgtactcaa ggttgaccgt ggataagagc aggtggcaag aaggcaacgt ttttagttgt 1380

tccgttatgc acgaggcctt gcataatcac tatactcaga agtccctatc cctgtctctc 1440

gggcgccgca agcgcggctc cggcgagggc cgcggctccc tgctgacctg cggcgacgtg 1500

gaggagaacc ccggccccat gaagtacctg ctgcccaccg ccgccgccgg cctgctgctg 1560

ctggccgccc agcccgccat ggccgaaatt gtgctgacac aatcgccagc cactctgtct 1620

ctgagccctg gtgagcgggc cactctgtcc tgcagggcca gtcagtctat ttccagctac 1680

ttggcttggt atcagcaaaa acccggccaa gctcctagat tgcttattta cgacgcgagc 1740

aatagggcca ccggtattcc agctcgcttt agcggctccg gaagtggtac tgatttcacg 1800

ctgacaattt caagcttgga acccgaggat tttgcagtgt attactgcca gcagagaagc 1860

aattggccgc tgaccttcgg ccaggggact aatcttgaaa taaaacgcac ggtggctgcc 1920

ccctccgtct tcatctttcc accttctgac gagcaattga agagcggtac cgctagcgta 1980

gtgtgtcttc tgaacaattt ctacccacgc gaggccaaag tgcaatggaa agtcgacaat 2040

gctctgcagt caggaaactc ccaggagagc gtcacagagc aagattctaa agattccact 2100

tacagtctgt catcgacatt gacactgagc aaggccgatt acgaaaagca taaagtgtac 2160

gcttgtgagg tcactcacca aggactgagc tcaccagtta ccaaatcgtt taatagaggt 2220

gagtgttaat ga 2232

<210> 9
<211> 2250
<212> DNA
<213> Artificial Sequence

<220>
<223> transgenic construct/anti-NKG2A
<400> 9
ttaagaaaaa atacgggtag aagcagtttg agagctccgt tcgccaccat ggagttcggc 60

ctgtcctggg tgttcctggt ggccctgttc cgcggcgtgc agtgccaggt tcagttggta 120

cagtctggcg cggaggtgaa aaaacctgga gcatctgtca aggtaagttg taaagcttcg 180

ggctatacct ttacctcata ttggatgaac tgggtgcggc aggcccccgg tcaagggctt 240

gaatggatgg gcagaattga tccttatgat tccgaaacac attacgctca gaagctgcag 300
ggcagggtta ccatgacgac agacaccagc acttccactg cttacatgga gctgcgatct 360
ctgcggtcag atgacacggc tgtgtactat tgcgccagag gcggatacga ttttgacgtg 420
gggactctgt attggttctt tgacgtctgg gggcagggaa ccactgtgac agtctcatcg 480
gcgtcaacta aagggccaag cgtgttcccc ttggcaccat gtagtcggtc caccagtgaa 540
agtaccgctg cactcggttg tctggtaaaa gattattttc cagagcccgt tactgtaagc 600
tggaacagtg gggccttgac gtctggagtc catacctttc cggcggtcct tcagtcatct 660
gggctgtaca gcctttccag cgttgtcact gttccaagct cctccctggg cacgaagacc 720
tatacatgta atgtcgacca taagccttca aatacaaaag tagacaaacg tgtggaatca 780
aaatacggac ccccatgccc gccctgtcca gcgcctgaat tcttgggagg cccgtctgtc 840
tttttattcc ccccgaaacc gaaggacacc ctcatgatct caaggacacc agaggtgacc 900
tgcgtggtgg tggatgttag tcaagaggat ccagaggtgc agtttaactg gtatgtcgat 960
ggcgtcgaag tccacaacgc taaaaccaag ccacgggagg agcagtttaa ctctacctat 1020
cgagtggtgt ccgtactgac tgtgttgcac caggactggc ttaatgggaa agaatacaag 1080
tgtaaagttt caaacaaagg cctgccttca agtatcgaaa agactattag caaagctaaa 1140
gggcagccaa gagaaccaca ggtgtacact ttgccccctt cccaagagga gatgactaag 1200
aatcaagttt ctctgacatg tctggtaaaa ggcttctacc cttctgatat agcagtggag 1260
tgggagtcta acggacagcc cgaaaacaat tacaagacga caccccctgt gctagattca 1320
gacggctctt tttttcttta cagtcggctc acagtggata aatcacgctg gcaggaaggg 1380
aatgtcttta gctgcagcgt catgcatgag gctttgcaca accattacac tcagaaatct 1440
ctgagcctct cacttggtaa acgccgcaag cgcggctccg gcgagggccg cggctccctg 1500
ctgacctgcg gcgacgtgga ggagaacccc ggccccatga agtacctgct gcccaccgcc 1560
gccgccggcc tgctgctgct ggccgcccag cccgccatgg ccgatatcca gatgacccag 1620
agcccctcca gccttagcgc ttcagtcggg gatagagtga caattacctg cagagcgtct 1680
gaaaatatct acagctactt ggcctggtac caacagaaac caggcaaagc tcctaagctc 1740
ctgatttata acgcaaaaac cttggctgag ggggtgccca gtcgcttctc gggctctggc 1800
tcaggaaccg actttacact caccatttct tcactgcagc ctgaggactt cgctacgtac 1860
tactgccagc atcactatgg taccccacgc acttttggag gggggactaa ggtggaaatc 1920
aagaggaccg tggccgcccc atccgtattt atcttccccc catccgatga gcagttaaaa 1980
tcagggaccg cctcggttgt gtgtctgctt aacaactttt acccccgcga agctaaggtg 2040
cagtggaagg ttgataacgc cttgcagtct ggaaacagcc aggagagcgt cacagaacaa 2100
gattcaaaag acagtactta ttccctgagt agtacgctga ccctgtccaa agccgattac 2160

gaaaagcaca aagtatacgc gtgtgaggtc actcaccagg gactatcaag cccggtcact 2220

aaatcattca acagggggga gtgctaatga 2250

<210> 10
<211> 2238
<212> DNA
<213> Artificial Sequence

<220>
<223> transgenic construct/anti-GITR

<400> 10
ttaagaaaaa atacgggtag aagcagtttg agagctccgt tcgccaccat ggagttcggc 60

ctgtcctggg tgttcctggt ggccctgttc cgcggcgtgc agtgccaagt gaccttacgg 120

gaatcaggtc cagcgctggt aaagcccacc caaactctaa cattgacctg tactttcagc 180

ggcttttccc tttcgacaag tggcatgggg gtgggatgga tccggcaacc tcctgggaag 240

gccctcgaat ggctggccca catttggtgg gacgatgaca agtactacaa cccctcactg 300

aagtcacggc tgacgatttc aaaggacacc agcaaaaatc aagtggtact gacgatgacc 360

aatatggatc ccgtcgacac tgctacatac tactgcgctc gcacaaggcg atactttccc 420

ttcgcctatt ggggacaggg aactctggtt accgtgagtt ccgcatccac taagggccca 480

agcgtcttcc cgctggcccc ttccagtaag agcacctccg gagggaccgc tgcacttgga 540

tgcctggtta aagactattt tcctgaacct gttacagtca gttggaactc gggggcacta 600

acctctggag tgcatacctt cccagctgta ctccaatcat ctggactata ctcactttcg 660

tctgtcgtga ctgtgccctc ttcttcactt gggacccaga catatatttg caacgtcaat 720

cataagccat cgaacaccaa ggtggataag aaggtggagc cgaaatcttg tgataagacc 780

cacacatgtc ctccatgccc agcccctgag ctgttgggag gtccctctgt gttcctgttc 840

cccccaaaac caaaagacac cctcatgatc tccagaacgc ctgaggtcac ctgtgtcgtt 900

gtggacgttt ctcacgaaga cccagaggtc aaattcaatt ggtatgtcga cggggtagag 960

gtccacaatg ctaagaccaa gcctcgagaa gagcaatatg cttccactta cagagtggtg 1020

tctgttctga ccgtcctgca ccaagactgg ctaaatggaa aagagtataa atgcaaggtc 1080

agcaacaaag ctctgcccgc cccaattgaa aagactatca gcaaagcgaa aggccagcca 1140

cgggaacctc aggtctatac gctgccccca agccgtgatg agctcacaaa gaaccaggtg 1200

agtctgactt gtctagtgaa aggcttttac cccagtgaca ttgcagtgga gtgggagtcc 1260

aacggacaac ccgaaaacaa ctataaaact accccccccg ttctcgattc tgatggcagc 1320

ttcttcttat acagcaaact tacggtggat aaatcgcggt ggcagcaggg aaatgtgttt 1380

tcctgctctg tcatgcatga ggccctacat aatcactata cacaaaagtc tctctctttg 1440

tcccccggaa aacgccgcaa gcgcggctcc ggcgagggcc gcggctccct gctgacctgc 1500

ggcgacgtgg aggagaaccc cggccccatg aagtacctgc tgcccaccgc cgccgccggc 1560

ctgctgctgc tggccgccca gcccgccatg gccgagatag tgatgactca gtcccccgcc 1620

actctctcag taagcccagg agagagagct acgctgagct gcaaggcctc ccagaacgtg 1680

gggaccaacg tagcctggta ccaacaaaag cccggtcagg ccccccggct gctcatttat 1740

tccgcaagtt atagatattc aggcattccg gccagattta gtggatccgg ctcaggcacg 1800

gagtttacct tgaccattag ctctctccaa tctgaggatt tcgcggtgta ttattgtcag 1860

cagtataaca cagacccctt gaccttcggc ggtggcacaa aagtcgaaat taagaggaca 1920

gtcgccgccc cttccgtgtt catttccccc ccctcagacg aacagctgaa atctggcaca 1980

gcttcgtggt gtttactgaa caacttctat cccagagaag caaaggtcca gtggaaggtc 2040

gataatgccc tgcagtctgg taatagtcag gagtccgtaa ctgagcagga cagcaaggac 2100

tccacctaca gcctgtcctc taccctgaca ctgagcaaag cagactacga aaaacacaaa 2160

gtgtacgcct gtgaggtcac tcaccagggg ctgagctctc cagtgactaa gtcttttaat 2220

cgcggcgagt gctaatga 2238

<210> 11
<211> 2232
<212> DNA
<213> Artificial Sequence

<220>
<223> transgenic construct/anti-OX40

<400> 11
ttaagaaaaa atacgggtag aagcagtttg agagctccgt tcgccaccat ggagttcggc 60

ctgtcctggg tgttcctggt ggccctgttc cgcggcgtgc agtgcgaagt gcagctagtg 120

cagagtgggg gcgggctagt gcagccaggg gggtctcttc gcttgtcttg cgccggtagc 180

ggttttacct tttcatcgta tgcaatgtac tgggtgaggc aagcccctgg caagggattg 240

gagtgggtga gcgcgataga cactgacgcc ggtaccttct atgcggatag tgtaaggggg 300

cgcttcacca ttagtcgcga caacgccaag aactctcttt atctccagat gaattcactc 360

cgagctgagg acaccgcagt ctacttttgt gctaggctgg gggaaggcta cttctttgat 420

tactggggac agggaacctt ggtgactgtg agctctgcct ccacgaaagg tccttctgtt 480

ttccccttgg cccctcctc aaaatctacc tctggaggta ccgccgctct aggctgccta 540

gtaaaggatt acttccctga acccgtgacg gtgtcttgga acagtggtgc tcttacaagt 600

ggcgtgcaca cctttcctgc cgtgctccaa agttctgggc tttattctct gtcctccgtg 660

gtgaccgtgc ccagcagctc tcttggtacc cagacttaca tctgtaatgt taaccacaaa 720

ccctcaaata caaaggtgga caagagagtg gagccaaaga gctgcgataa aacccacacc 780

tgtcccccct gtcccgcacc tgagcttctt gggggcccta gtgtgtttct gttcccacct 840

aagcccaaag acaccctgat gatctctagg accccggagg ttacctgcgt agttgtggac 900

gtctcccacg aggatcccga ggtgaaattc aattggtacg tcgacggcgt tgaagtccat 960

aacgccaaga ccaagcctcg cgaagaacag tataactcta cctatcgcgt ggtgtcggtc 1020

ctgactgtgc ttcaccagga ctggcttaac gggaaggaat ataaatgcaa ggtcagcaat 1080

aaggccctcc ccgctccaat tgagaagacc atttccaaag ctaaaggcca gccacgggag 1140

ccccaggtct acactctacc accctcccgc gaggagatga ccaaaaacca ggtgtccctg 1200

acctgtttgg tgaagggatt ttacccgtcc gatatcgctg tggagtggga aagtaatggc 1260

caacctgaaa acaactataa gacaaccccg ccagtcctag atagcgatgg atccttcttc 1320

ctgtatagta aacttacagt ggataaaagt aggtggcagc agggtaacgt tttttcgtgt 1380

agtgtgatgc atgaagcact ccacaatcac tatacccaaa aaagcctttc gctgtcccct 1440

ggccgccgca agcgcggctc cggcgagggc cgcggctccc tgctgacctg cggcgacgtg 1500

gaggagaacc ccggccccat gaagtacctg ctgcccaccg ccgccgccgg cctgctgctg 1560

ctggccgccc agcccgccat ggccgagatt gtgctgacgc agagcccagc aactttgagt 1620

ctctcgcccg gcgagcgcgc cacactttcc tgtcgagcct cccaatccgt gagcagttac 1680

ctggcatggt accaacagaa gcctgggcaa gcccccagat tgctgattta tgatgcctcc 1740

aatcgagcaa caggaatacc agctcggttc agtgggagcg gaagcggtac agatttcact 1800

ctgacaatca gctcgctgga gccagaggac ttcgctgttt attactgcca gcagcgttcg 1860

aactggcccc caacgtttgg aggaggcacc aaagtggaaa taaaacgaac agtagcagcc 1920

ccatctgtct tcatcttccc ccccagcgat gaacagctga agtcaggcac tgcttccgtc 1980

gtctgcctac tgaacaattt ttatcctaga gaggccaagg tccaatggaa ggtagacaac 2040

gcactccaat cagggaactc acaagagtct gtcaccgagc aggactctaa ggactcgaca 2100

tactcattat ctagtaccct gacactgagc aaggccgact acgaaaaaca taaagtctac 2160

gcctgtgaag tgacgcacca gggactcagc tctcccgtca ctaagagctt caatcggggg 2220

gagtgttaat ga 2232

<210> 12
<211> 924
<212> DNA
<213> Artificial Sequence

<220>
<223> transgenic construct/CD80

<400> 12
ttaagaaaaa atacgggtag aagtggcttt cacttttgac cctaagcatc tgccaccatg 60

ggccacacac ggaggcaggg aacatcacca tccaagtgtc catacctcaa tttctttcag 120

ctcttggtgc tggctggtct ttctcacttc tgttcaggtg ttatccacgt gaccaaggaa 180

gtgaaagaag tggcaacgct gtcctgtggt cacaatgttt ctgttgaaga gctggcacaa 240

actcgcatct actggcaaaa ggagaagaaa atggtgctga ctatgatgtc tggggacatg 300

aatatatggc ccgagtacaa gaaccggacc atctttgata tcactaataa cctctccatt 360

gtgatcctgg ctctgcgccc atctgacgag ggcacatacg agtgtgttgt tctgaagtat 420

gaaaaagacg ctttcaagcg ggaacacctg gctgaagtga cgttatcagt caaagctgac 480

ttccctacac ctagtatatc tgactttgaa attccaactt ctaatattag aaggataatt 540

tgctcaacct ctggaggttt tccagagcct cacctctcct ggttggaaaa tggagaagaa 600

ttaaatgcca tcaacacaac agtttcccaa gatcctgaaa ctgagctcta tgctgttagc 660

agcaaactgg atttcaatat gacaaccaac cacagcttca tgtgtctcat caagtatgga 720

catttaagag tgaatcagac cttcaactgg aatacaacca agcaagagca ttttcctgat 780

aacctgctcc catcctgggc cattacctta atctcagtaa atggaatttt tgtgatatgc 840

tgcctgacct actgctttgc cccaagatgc agagagagaa ggaggaatga gagattgaga 900

agggaaagtg tacgccctgt ataa 924

<210> 13
<211> 1626
<212> DNA
<213> Artificial Sequence

<220>
<223> transgenic construct/(ns)hIL-12

<400> 13
ttaagaaaaa atacgggtag aagcagtttg agagctccgt tcgccaccat gagaaacctc 60

cccgtggcca ctccagaccc aggaatgttc ccatgccttc accactccca aaacctgctg 120

agggccgtca gcaacatgct ccagaaggcc agacaaactc tagaatttta cccttgcact 180

tctgaagaga ttgatcatga agatatcaca aaagataaaa ccagcacagt ggaggcctgt 240

ttaccattgg aattaaccaa gaatgagagt tgcctaaatt ccagagagac ctctttcata 300

actaatggga gttgcctggc ctccagaaag acctctttta tgatggccct gtgccttagt 360

agtatttatg aagacttgaa gatgtaccag gtggagttca agaccatgaa tgcaaagctg 420

ctgatggatc ctaagaggca gatctttcta gatcaaaaca tgctggcagt tattgatgag 480

ctgatgcagg ccctgaattt caacagtgag actgtgccac aaaaatcctc ccttgaagaa 540

ccggattttt ataaaactaa aatcaagctc tgcatacttc ttcatgcttt cagaattcgg 600

gcagtgacta ttgatagagt gatgagctat ctgaatgctt ccggctccgg cgagggccgc 660

ggctccctgc tgacctgcgg cgacgtggag gagaaccccg gccccatatg ggaactgaag 720

aaagatgttt atgtcgtaga attggattgg tatccggatg cccctggaga aatggtggtc 780

ctcacctgtg acacccctga agaagatggt atcacctgga ccttggacca gagcagtgag 840

gtcttaggct ctggcaaaac cctgaccatc caagtcaaag agtttggaga tgctggccag 900

tacacctgtc acaaaggagg cgaggttcta agccattcgc tcctgctgct tcacaaaaag 960

gaagatggaa tttggtccac tgatatttta aaggaccaga aagaacccaa aaataagacc 1020

tttctaagat gcgaggccaa gaattattct ggacgtttca cctgctggtg gctgacgaca 1080

atcagtactg atttgacatt cagtgtcaaa agcagcagag gctcttctga cccccaaggg 1140

gtgacgtgcg gagctgctac actctctgca gagagagtca gaggggacaa caaggagtat 1200

gagtactcag tggagtgcca ggaggacagt gcctgcccag ctgctgagga gagtctgccc 1260

attgaggtca tggtggatgc cgttcacaag ctcaagtatg aaaactacac cagcagcttc 1320

ttcatcaggg acatcatcaa acctgaccca cccaagaact tgcagctgaa gccattaaag 1380

aattctcggc aggtggaggt cagctgggag taccctgaca cctggagtac tccacattcc 1440

tacttctccc tgacattctg cgttcaggtc cagggcaaga gcaagagaga aaagaaagat 1500

agagtcttca cggacaagac ctcagccacg gtcatctgcc gcaaaaatgc cagcattagc 1560

gtgcgggccc aggaccgcta ctatagctca tcttggagcg aatgggcatc tgtgccctgc 1620

agttaa 1626

<210> 14
<211> 768
<212> DNA
<213> Artificial Sequence

<220>
<223> transgenic construct/eGFP

<400> 14
ttaagaaaaa atacgggtag aattggagcg gccgcattgt gccaagatgg tgagcaaggg 60

cgaggagctg ttcaccgggg tggtgcccat cctggtcgag ctggacggcg acgtaaacgg 120

ccacaagttc agcgtgtccg gcgagggcga gggcgatgcc acctacggca agctgaccct 180

gaagttcatc tgcaccaccg gcaagctgcc cgtgccctgg cccaccctcg tgaccaccct 240

gacctacggc gtgcagtgct tcagccgcta ccccgaccac atgaagcagc acgacttctt 300

caagtccgcc atgcccgaag gctacgtcca ggagcgcacc atcttcttca aggacgacgg 360

caactacaag acccgcgccg aggtgaagtt cgagggcgac accctggtga accgcatcga 420

gctgaagggc atcgacttca aggaggacgg caacatcctg gggcacaagc tggagtacaa 480

ctacaacagc cacaacgtct atatcatggc cgacaagcag aagaacggca tcaaggtgaa 540

cttcaagatc cgccacaaca tcgaggacgg cagcgtgcag ctcgccgacc actaccagca 600

gaacaccccc atcggcgacg gccccgtgct gctgcccgac aaccactacc tgagcaccca 660

gtccgccctg agcaaagacc ccaacgagaa gcgcgatcac atggtcctgc tggagttcgt 720

gaccgccgcc gggatcactc tcggcatgga cgagctgtac aagtaaga 768

<210> 15
<211> 2214
<212> DNA
<213> Artificial Sequence

<220>
<223> transgenic construct/Nivolumab

<400> 15
ttaagaaaaa atacgggtag aagcagtttg agagctccgt tcgccaccat ggagttcggc 60

ctgtcctggg tgttcctggt ggccctgttt agaggcgtgc agtgccaggt gcagctggtg 120

gagagcggag gaggagtggt gcagccaggc cggagcctgc gcctggactg taaggccagc 180

ggcatcacct ttagcaactc cggaatgcac tgggtgaggc aggcaccagg caagggcctg 240

gagtgggtgg ccgtgatctg gtacgacggc tctaagcggt actatgccga tagcgtgaag 300

ggccggttca caatctccag agacaactct aagaataccc tgttcctcca gatgaacagc 360

ctgagggccg aggatacagc cgtgtactat tgcgccacca atgacgatta ctggggccag 420

ggcacactgg tgaccgtgag cagcgccagc accaagggac caagcgtgtt cccactggca 480

ccttgctctc gcagcacatc cgagtctacc gccgccctgg gatgtctggt gaaggattat 540

ttccccgagc ctgtgaccgt gagctggaac agcggcgccc tgacatccgg agtgcacacc 600

tttcccgccg tgctccagag cagcggcctg tacagcctgt cctctgtggt gacagtgccc 660

agctcctctc tgggcaccaa gacatatacc tgcaacgtgg accacaagcc tagcaatacc 720

aaggtggata agcgggtgga gtccaagtac ggaccacctt gcccaccatg tccagcacca 780

gagttcctgg gaggaccaag cgtgttcctg tttcctccaa agcccaagga caccctgatg 840

atctccagaa caccagaggt gacctgcgtg gtggtggacg tgtctcagga ggaccccgag 900

gtgcagttca actggtatgt ggatggcgtg gaggtgcaca atgccaagac caagcccagg 960

gaggagcagt ttaactccac ataccgcgtg gtgtctgtgc tgaccgtgct gcaccaggac 1020

tggctgaacg gcaaggagta taagtgcaag gtgtccaata agggcctgcc tagctccatc 1080

gagaagacaa tcagcaaggc aaagggacag ccaagggagc cacaggtgta caccctgccc 1140

ccttctcagg aggagatgac aaagaaccag gtgagcctga cctgtctggt gaagggcttc 1200

tatccttccg acatcgccgt ggagtgggag tctaatggcc agccagagaa caattacaag 1260

accacaccac ccgtgctgga ctctgatggc agcttctttc tgtattccag gctgacagtg 1320

gataagtctc gctggcagga gggcaacgtg tttagctgct ccgtgatgca cgaggccctg 1380

cacaatcact acacccagaa gtctctgagc ctgtccctgg gcaagcggag aaagaggggc 1440

agcggagagg gaaggggctc cctgctgaca tgtggcgacg tggaggagaa tcctggccca 1500

atgaagtatc tgctgcccac cgccgccgcc ggcctgctgc tgctggcagc acagcctgct 1560

atggccgaga tcgtgctgac acagtcccca gccaccctgt ctctgagccc tggagagagg 1620

gccaccctga gctgtagagc atcccagagc gtgagcagct acctggcctg gtatcagcag 1680

aagcctggac aggcaccaag gctgctgatc tacgatgcaa gcaacagggc aacaggcatc 1740

cccgcaagat tcagcggctc cggctctggc accgacttta cactgaccat ctcctctctg 1800

gagcctgagg atttcgccgt gtactattgc cagcagagca gcaattggcc acggacattt 1860

ggccagggca ccaaggtgga gatcaagaga acagtggccg cccctagcgt gttcatcttt 1920

cctccatccg acgagcagct gaagagcggc accgcatccg tggtgtgcct gctgaacaat 1980

ttctacccac gggaggccaa ggtgcagtgg aaggtggata acgccctcca gtccggcaat 2040

tctcaggaga gcgtgacaga gcaggactcc aaggattcta cctatagcct gtctagcaca 2100

ctgaccctgt ccaaggccga ctacgagaag cacaaggtgt atgcctgcga ggtgacacac 2160

cagggcctgt cctctcccgt gaccaagagc tttaacagag gcgagtgttg atga 2214

<210> 16
<211> 2238
<212> DNA
<213> Artificial Sequence

<220>
<223> transgenic construct/anti-CTLA-4

<400> 16
ttaagaaaaa atacgggtag aagcagtttg agagctccgt tcgccaccat ggagtttggg 60

ctgtcatggg tctttctggt cgctctgttt aggggggtgc agtgtcaggt gcagctggtc 120

gagtcagggg ggggcgtggt gcagccaggc aggtccctga ggctgtcttg cgcagccagc 180

ggcttcacct ttagctccta cacaatgcac tgggtgcggc aggcacctgg caagggcctg 240

gagtgggtga ccttcatctc ctatgacggc aacaataagt actatgccga ttctgtgaag 300

ggccggttta ccatctccag agacaactct aagaatacac tgtacctgca gatgaacagc 360

ctgagggccg aggacaccgc catctactat tgcgcaagga caggatggct gggacctttc 420

gattattggg gccagggcac cctggtgaca gtgtctagcg cctctaccaa gggcccaagc 480

gtgtttcccc tggccccttc ctctaagtcc acctctggag gcacagccgc cctgggctgt 540

ctggtgaagg attacttccc tgagccagtg accgtgagct ggaactccgg cgccctgacc 600

agcggagtgc acacatttcc agccgtgctg cagagcagcg gcctgtactc cctgtctagc 660

gtggtgaccg tgccctcctc tagcctgggc acccagacat atatctgcaa cgtgaatcac 720

aagcctagca atacaaaggt ggacaagcgg gtggagccaa agtcctgtga taagacccac 780

acatgcccac cttgtccagc acctgagctg ctgggaggac catccgtgtt cctgtttcca 840

cccaagccca aggacaccct gatgatcagc cggacccctg aggtgacatg cgtggtggtg 900

gacgtgagcc acgaggaccc cgaggtgaag ttcaactggt acgtggatgg cgtggaggtg 960

cacaatgcca agaccaagcc ccgggaggag cagtacaact ccacctatag agtggtgtct 1020

gtgctgacag tgctgcacca ggactggctg aacggcaagg agtataagtg caaggtgtcc 1080

aataaggccc tgccagcccc catcgagaag accatctcta aggcaaaggg acagccacgg 1140

gagccacagg tgtacacact gcctcccagc cgggacgagc tgaccaagaa ccaggtgagc 1200

ctgacatgtc tggtgaaggg cttctatccc agcgacatcg ccgtggagtg ggagtccaat 1260

ggccagcctg agaacaatta caagaccaca ccccctgtgc tggactctga tggcagcttc 1320

tttctgtatt ctaagctgac cgtggacaag agcagatggc agcagggcaa cgtgtttagc 1380

tgctccgtga tgcacgaggc cctgcacaat cactacacac agaagtctct gagcctgtcc 1440

cctggcaagc ggagaaagag gggcagcgga gagggaaggg gctccctgct gacctgtggc 1500

gatgtggagg agaatcctgg cccaatgaag tatctgctgc caacagctgc cgccggcctg 1560

ctgctgctgg ccgcacagcc cgctatggcc gagatcgtgc tgacccagag cccaggcaca 1620

ctgtctctga gccctggaga gagggccacc ctgtcctgca gagcctccca gtctgtgggc 1680

tcctcttacc tggcctggta tcagcagaag cctggccagg ccccaaggct gctgatctac 1740

ggagccttca gcagggccac cggcatcccc gaccgcttca gcggctccgg ctctggcaca 1800

gacttcaccc tgacaatctc caggctggag cccgaggact tcgccgtgta ctattgccag 1860

cagtacggca gctccccttg gacctttggc cagggcacaa aggtggagat caagaggacc 1920

gtggcagcac catccgtgtt catctttcca ccctctgacg agcagctgaa gagcggcaca 1980

gcctccgtgg tgtgcctgct gaacaacttc tacccccgcg aggccaaggt gcagtggaag 2040

gtggataacg ccctgcagtc cggcaattct caggagagcg tgaccgagca ggactccaag 2100

gattctacat atagcctgtc tagcaccctg acactgagca aggccgatta cgagaagcac 2160

aaggtgtatg cctgtgaggt cacccaccag ggactgtcat cacccgtcac caaaagtttc 2220

aatagaggag agtgctga 2238

<210> 17
<211> 1662
<212> DNA

<213> Artificial Sequence

<220>
<223> transgenic construct/IL-12

<400> 17
ttaagaaaaa atacgggtag aagcagtttg agagctccgt tcgccaccat gggtcaccag 60

cagttggtca tctcttggtt ttccctggtt tttctggcat ctcccctcgt ggccatatgg 120

gaactgaaga aagatgttta tgtcgtagaa ttggattggt atccggatgc ccctggagaa 180

atggtggtcc tcacctgtga cacccctgaa gaagatggta tcacctggac cttggaccag 240

agcagtgagg tcttaggctc tggcaaaacc ctgaccatcc aagtcaaaga gtttggagat 300

gctggccagt acacctgtca caaaggaggc gaggttctaa gccattcgct cctgctgctt 360

cacaaaaagg aagatggaat ttggtccact gatattttaa aggaccagaa agaacccaaa 420

aataagacct ttctaagatg cgaggccaag aattattctg gacgtttcac ctgctggtgg 480

ctgacgacaa tcagtactga tttgacattc agtgtcaaaa gcagcagagg ctcttctgac 540

ccccaagggg tgacgtgcgg agctgctaca ctctctgcag agagagtcag aggggacaac 600

aaggagtatg agtactcagt ggagtgccag gaggacagtg cctgcccagc tgctgaggag 660

agtctgccca ttgaggtcat ggtggatgcc gttcacaagc tcaagtatga aaactacacc 720

agcagcttct tcatcaggga catcatcaaa cctgacccac ccaagaactt gcagctgaag 780

ccattaaaga attctcggca ggtggaggtc agctgggagt accctgacac ctggagtact 840

ccacattcct acttctccct gacattctgc gttcaggtcc agggcaagag caagagagaa 900

aagaaagata gagtcttcac ggacaagacc tcagccacgg tcatctgccg caaaaatgcc 960

agcattagcg tgcgggccca ggaccgctac tatagctcat cttggagcga atgggcatct 1020

gtgccctgca gtgttcctgg agtaggggta cctggggtgg gcgccagaaa cctccccgtg 1080

gccactccag acccaggaat gttcccatgc cttcaccact cccaaaacct gctgagggcc 1140

gtcagcaaca tgctccagaa ggccagacaa actctagaat tttacccttg cacttctgaa 1200

gagattgatc atgaagatat cacaaaagat aaaaccagca cagtggaggc ctgtttacca 1260

ttggaattaa ccaagaatga gagttgccta aattccagag agacctcttt cataactaat 1320

gggagttgcc tggcctccag aaagacctct tttatgatgg ccctgtgcct tagtagtatt 1380

tatgaagact tgaagatgta ccaggtggag ttcaagacca tgaatgcaaa gctgctgatg 1440

gatcctaaga ggcagatctt tctagatcaa aacatgctgg cagttattga tgagctgatg 1500

caggccctga atttcaacag tgagactgtg ccacaaaaat cctcccttga agaaccggat 1560

ttttataaaa ctaaaatcaa gctctgcata cttcttcatg ctttcagaat tcgggcagtg 1620

actattgata gagtgatgag ctatctgaat gcttcctaat ga 1662

<210> 18
<211> 708
<212> DNA
<213> Artificial Sequence

<220>
<223> transgenic construct/NS1

<400> 18
ttaagaaaaa atacgggtag aagcagtttg agagctccgt tcgccaccat ggatagcaat 60

accatgtcct cctttcaggt tgactgtttc ctgtggcata ttcgcaagcg tttcgccgat 120

aatggtctgg gtgacgcccc gttcctggac cgtctgcgtc gcgatcagaa gagcctgaaa 180

ggccgcggta acaccctggg cctggacatc gaaaccgcga ccctggtggg caagcagatc 240

gtggagtgga ttctgaaaga ggaaagcagc gaaaccctgc gtatgaccat tgccagcgtg 300

ccgaccagcc gttacctgag cgacatgacc ctggaggaaa tgagccgtga ttggttcatg 360

ctgatgccgc gccagaagat cattggtcca ctgtgcgtgc gtctggacca ggctatcatg 420

gagaagaaca ttgtgctgaa agccaacttc agcgtgatct ttaaccgtct ggaaaccctg 480

attctgctgc gcgcctttac cgaggaaggc gcgatcgtgg gcgagattag cccgctgccg 540

agcctgccag gtcacaccta tgaagatgtg aaaaatgccg tgggcgtgct gattggcggt 600

ctggagtgga acggtaacac cgtgcgtgtg agcgaaaaca ttcagcgctt cgcatggcgt 660

aactgtgatg agaatggtcg tccaagcctg ccaccggaac aaaaataa 708

<210> 19
<211> 414
<212> DNA
<213> Artificial Sequence

<220>
<223> transgenic construct/Apoptin

<400> 19
ttaagaaaaa atacgggtag aagcagtttg agagctccgt tcgccaccat gaacgctctc 60

caagaagata ctccacccgg accatcaacg gtgttcaggc caccaacaag ttcacggccg 120

ttggaaaccc ctcactgcag agagatccgg attggtatcg ctggaattac aatcactcta 180

tcgctgtgtg gctgcgcgaa tgctcgcgct cccacgctaa gatctgcaac tgcggacaat 240

tcagaaagca ctggtttcaa gaatgtgccg gacttgagga ccgatcaacc caagcctccc 300

tcgaagaagc gatcctgcga cccctccgag tacagggtaa gcgagctaaa agaaagcttg 360

attaccacta ctcccagccg accccgaacc gcaaaaaggc gtataagact gtaa 414

<210> 20
<211> 1104

<212> DNA
<213> Artificial Sequence

<220>
<223> transgenic construct/B18R

<400> 20
ttaagaaaaa atacgggtag aatacaatta acgatcttta tagccaccat gacgatgaaa 60

atgatggtac atatatattt cgtatcatta ttgttattgc tattccacag ttacgccata 120

gacatcgaaa atgaaatcac agaattcttc aataaaatga gagatactct accagctaaa 180

gactctaaat ggttgaatcc agcatgtatg ttcggaggca caatgaatga tatagccgct 240

ctaggagagc cattcagcgc aaagtgtcct cctattgaag acagtctttt atcgcacaga 300

tataaagact atgtggttaa atgggaaagg ctagaaaaaa atagacggcg acaggtttct 360

aataaacgtg ttaaacatgg tgatttatgg atagccaact atacatctaa attcagtaac 420

cgtaggtatt tgtgcaccgt aactacaaag aatggtgact gtgttcaggg tatagttaga 480

tctcatatta gaaaacctcc ttcatgcatt ccaaaaacat atgaactagg tactcatgat 540

aagtatggca tagacttata ctgtggaatt ctttacgcaa aacattataa taatataact 600

tggtataaag ataataagga aattaatatc gacgacatta agtattcaca aacgggaaag 660

gaattaatta ttcataatcc agagttagaa gatagcggaa gatacgactg ttacgttcat 720

tacgacgacg ttagaatcaa gaatgatatc gtagtatcaa gatgtaaaat acttacggtt 780

ataccgtcac aagaccacag gtttaaacta atactagatc caaaaatcaa cgtaacgata 840

ggagaacctg ccaatataac atgcactgct gtgtcaacgt cattattgat tgacgatgta 900

ctgattgaat gggaaaatcc atccggatgg cttataggat tcgattttga tgtatactct 960

gttttaacta gtagaggcgg tattaccgag gcgaccttgt actttgaaaa tgttactgaa 1020

gaatatatag gtaatacata taaatgtcgt ggacacaact attattttga aaaaaccctt 1080

acaactacag tagtattgga gtaa 1104

<210> 21
<211> 2232
<212> DNA
<213> Artificial Sequence

<220>
<223> transgenic construct/anti-CD28

<400> 21
   ttaagaaaaa atacgggtag aagcagtttg agagctccgt tcgccaccat ggagtttggg 60
   ctgagttggg tctttctggt cgctctgttc cggggggtgc agtgtcaggt gcagctggtg 120
   cagtctgggg ccgaggtcaa gaagcccggc gcctccgtga aggtgtcttg caaggccagc 180
   ggctacacct tcacaagcta ctatatccac tgggtgaggc aggcaccagg acagggcctg 240
   gagtggatcg gctgttaccc tggcaacgtg aataccaact ataatgagaa gtttaaggac 300
   cgcgccacaa tcctgacctg ggatacaagc tccaccgcct atatggagct gtccaggctg 360
   cgctctgacg atacagccgt gtacttctgc accaggtccc actatggcct gcagtggaac 420
   tttgacgtgt ggggccaggg caccacagtg acagtgtcta gcgcctctac caagggccct 480
   agcgtgttcc ctctggcccc atgctcccgc tctacaagcg agtccaccgc cgccctggga 540
   tgtctggtga aggattactt ccctgagcca gtgaccgtga gctggaattc cggcgccctg 600
   acaagcggag tgcacacctt tccagccgtg ctgcagtcct ctggcctgta ctccctgagc 660
   tccgtggtga cagtgccttc tagctccctg ggcaccaaga catatacctg caacgtggac 720
   cacaagccaa gcaataccaa ggtggataag cgggtggaga gcaagtacgg accaccttgc 780
   ccatcctgtc cagcacctga gtttctggga ggaccttccg tgttcctgtt tccacccaag 840
   ccaaaggaca cactgatgat ctctagaaca cccgaggtga cctgcgtggt ggtggacgtg 900
   agccaggagg atcctgaggt gcagttcaac tggtacgtgg atggcgtgga ggtgcacaat 960
   gccaagacca agccacggga ggagcagttt aactccacat acagagtggt gtctgtgctg 1020
   accgtgctgc accaggactg gctgaacggc aaggagtata agtgcaaggt gtccaataag 1080
   ggcctgccct ctagcatcga gaagacaatc tctaaggcaa agggacagcc aagggagcct 1140
   caggtgtaca ccctgcctcc aagccaggag gagatgacaa agaaccagtg gtccctgacc 1200
   tgtctggtga agggcttcta tccttctgac atcgccgtgg agtgggagag caatggccag 1260
   ccagagaaca attacaagac cacaccccct gtgctggact ctgatggcag cttctttctg 1320
   tattctaggc tgacagtgga taagagccgc tggcaggagg gcaacgtgtt ttcttgcagc 1380
   gtgatgcacg aggccctgca caatcactac acccagaagt ccctgtctct gagcctgggc 1440
   aagcggagaa agaggggaag cggagaggga agaggctccc tgctgacatg tggcgatgtg 1500
   gaggagaacc caggccccat gaagtatctg ctgcccaccg cagccgccgg cctgctgctg 1560
   ctggcagccc agccagccat ggccgacatc cagatgaccc agagcccatc ctctctgtcc 1620
   gcctctgtgg gcgatagggt gacaatcacc tgccacgcct cccagaacat ctacgtgtgg 1680
   ctgaattggt atcagcagaa gccaggcaag gcccccaagc tgctgatcta caaggcctcc 1740
   aatctgcaca caggagtgcc ttctcgcttc agcggatccg gatctggaac cgactttaca 1800
   ctgaccatca gctccctgca gccagaggat ttcgccacct actattgcca gcagggccag 1860
   acatacccct atacctttgg cggcggcaca aaggtggaga tcaagaggac cgtggcagca 1920
   ccatccgtgt tcatctttcc accctctgac gagcagctga agagcggaac cgcatccgtg 1980
   gtgtgcctgc tgaacaattt ctaccctaga gaggccaagg tgcagtggaa ggtggataac 2040
   gccctgcagt ccggcaattc tcaggagagc gtgacagagc aggactccaa ggattctacc 2100
   tatagcctgt ctagcacact gaccctgagc aaggccgact acgagaagca caaggtgtat 2160
   gcctgtgaag tcacccacca ggggctgagt tcaccttgga caaagagttt caatcgggga 2220
   gagtgttgat aa 2232

<210> 22
<211> 2256
<212> DNA
<213> Artificial Sequence

<220>
<223> transgenic construct/anti-CA 15-3

<400> 22
ttaagaaaaa atacgggtag aagcagtttg agagctccgt tcgccaccat ggagttcggc 60

ctgagttggg tcttcctggt cgccctgttt cggggggtcc agtgtgaggt ccagctgcag 120

cagtccgggg gagagagagc cacccccggc gcctccgtga agatgtcttg caagacaagc 180

ggctacacct tcacaaacta ttggatgcac tgggtgaagc agcggcccgg acagggcctg 240

gagtggatca tcggctacat caaccctagc tccggctaca cccagtataa tcagaagttt 300

aaggacaagg ccaccctgac agccgataag tctagctcca ccgcctatat ccagctgtct 360

agcctgacat ctgaggacag cgccgtgtac tattgctcca cctactatgg cgattacctg 420

ttcccatatt ggggacaggg caccctggtg acagtgtccg ccgcatctac aaagggacca 480

agcgtgtttc cactggcacc ttcctctaag tctaccagcg gaggaacagc cgccctggga 540

tgtctggtga aggactactt ccctgagcca gtgaccgtga gctggaactc cggcgccctg 600

acctccggag tgcacacatt tcctgccgtg ctgcagagct ccggcctgta cagcctgtct 660

agcgtggtga ccgtgccatc ctctagcctg ggcacccaga catatatctg caacgtgaat 720

cacaagcctt ccaatacaaa ggtggacaag cgggtggagc caaagtcttg tgataagacc 780

cacacatgcc caccttgtcc agcacctgag ctgctgggag gaccaagcgt gttcctgttt 840

ccacccaagc ccaaggacac cctgatgatc agcagaaccc ccgaggtgac atgcgtggtg 900

gtggacgtgt cccacgagga tcctgaggtg aagttcaact ggtacgtgga tggcgtggag 960

gtgcacaatg ccaagaccaa gcctcgggag gagcagtaca actccaccta tagagtggtg 1020

tctgtgctga cagtgctgca ccaggactgg ctgaacggca aggagtacaa gtgcaaggtg 1080

tctaataagg ccctgccagc ccccatcgag aagaccatca gcaaggcaaa gggacagcca 1140

agggagccac aggtgtacac actgcctcca tctcgcgacg agctgaccaa gaaccaggtg 1200

agcctgacat gtctggtgaa gggcttctat ccaagcgaca tcgccgtgga gtgggagtcc 1260

aatggccagc ccgagaacaa ttacaagacc acaccccctg tgctggactc tgatggcagc 1320

ttctttctgt attctaagct gaccgtggat aagagccggt ggcagcaggg caacgtgttt 1380

tcctgctctg tgatgcacga ggccctgcac aatcactaca cacagaagag cctgtccctg 1440

tctcccggca agcggagaaa gaggggaagc ggagagggaa gaggctccct gctgacctgt 1500

ggcgacgtgg aggagaaccc tggcccaatg aagtatctgc tgcctacagc agccgccggc 1560

ctgctgctgc tggcagcaca gccagccatg gccgatgtgc tgatgaccca gacaccactg 1620

tccctgccag tgtctctggg cgaccaggcc agcatctcct gcaggtcctc tcaggacatc 1680

gtgtacggca acggcaatac ctacctggag tggtatctgc agaagcccgg ccagtcccct 1740

aagctgctga tctataaggt gagcaatagg ttctccggcg tgccagaccg cttttctggc 1800

agcggctccg gcaccgattt cacactgaag atctccaggg tggaggcaga ggacctgggc 1860

gtgtactatt gcttccaggg cagccacgtg ccctacacct ttggcggcgg cacaaagctg 1920

gagatcaaga ggaggaccgt ggcagcacct tccgtgttca tctttccacc ctctgacgag 1980

cagctgaagt ctggcacagc cagcgtggtg tgcctgctga acaacttcta ccccagggag 2040

gccaaggtgc agtggaaggt ggataacgcc ctgcagtccg gcaattctca ggagagcgtg 2100

accgagcagg actccaagga ttctacatat agcctgagct ccaccctgac actgagcaag 2160

gccgattacg agaagcacaa ggtgtatgcc tgtgaggtca cccaccaggg actgagtagc 2220

cccgtcacca aatccttcaa tagaggcgag tgttga 2256

<210> 23
<211> 2262
<212> DNA
<213> Artificial Sequence

<220>
<223> transgenic construct/ anti-CA19-9

<400> 23
ttaagaaaaa atacgggtag aagcagtttg agagctccgt tcgccaccat ggagtttggg 60

ctgtcatggg tctttctggt cgctctgttt cggggggtgc agtgtcaggt gcagctggtc 120

gagtcagggg ggggaagtgt gcagcctggc cggagcctga gactgagctg cgaggcatcc 180

ggcttcacct ttgaggcata cgcaatgcac tgggtgcggc agccacctgg caagggcctg 240

gagtgggtga gctccatcaa ctggaattcc ggccggatcg cctacgccga ctctgtgaag 300

ggcagattca ccatcagcag ggataacgcc cgcaattccc tgtatctgca gatgaactct 360

ctgagactgg aggacaccgc cttttactat tgcgccaagg acatccggag attcagcaca 420

ggcggcgccg agtttgagta ctggggccag ggcaccctgg tgacagtgtc tagcgccagc 480

accaagggac catccgtgtt ccctctggca ccatcctcta agagcacctc cggaggaaca 540

gccgccctgg gctgtctggt gaaggactat ttcccagagc ccgtgacagt gtcctggaac 600

tctggcgccc tgaccagcgg agtgcacaca tttcctgccg tgctgcagag ctccggcctg 660

tactccctgt ctagcgtggt gaccgtgcca tcctctagcc tgggcaccca gacatatatc 720

tgcaacgtga atcacaagcc atccaataca aaggtggaca agagggtgga gcccaagtct 780

tgtgataaga cccacacatg cccaccctgt cctgcaccag agctgctggg aggaccaagc 840

gtgttcctgt ttcctccaaa gcctaaggac accctgatga tcagccgcac cccagaggtg 900

acatgcgtgg tggtggacgt gtcccacgag gatcccgagg tgaagttcaa ctggtacgtg 960

gatggcgtgg aggtgcacaa tgccaagacc aagcccaggg aggagcagta caactctacc 1020

tatcgcgtgg tgagcgtgct gacagtgctg caccaggact ggctgaacgg caaggagtat 1080

aagtgcaagg tgtctaataa ggccctgccc gcccctatcg agaagaccat ctccaaggca 1140

aagggacagc caagggagcc tcaggtgtac acactgcccc cttctcgcga cgagctgacc 1200

aagaaccagg tgagcctgac atgtctggtg aagggcttct atcctagcga catcgccgtg 1260

gagtgggagt ccaatggcca gccagagaac aattacaaga ccacaccacc cgtgctggac 1320

agcgatggct ccttctttct gtatagcaag ctgaccgtgg acaagtccag gtggcagcag 1380

ggcaacgtgt tttcttgcag cgtgatgcac gaggccctgc acaatcacta cacacagaag 1440

tccctgtctc tgagcccagg caagaggagg aagaggggat ccggagaggg aagaggctct 1500

ctgctgacct gtggcgatgt ggaggagaat ccaggcccca tgaagtatct gctgcccaca 1560

gcagccgccg gcctgctgct gctggcagca cagcctgcca tggcccagag cgtgctgacc 1620

cagcctccat ccgcctctgg aaccccagga cagagggtga caatcagctg ctccggctcc 1680

tctagcaaca tcggcagcaa tttcgtgtac tggtatcagc agctgccagg aaccgcacca 1740

aagctgctga tctacaggaa caatcagcgg ccctccggag tgccagacag gttttctggc 1800

agccgctccg gcacatctgc cagcctggcc atctctggcc tgcggagcga ggacgaggca 1860

gattactatt gcgcagcatg ggacgatagc ctgggaggac actacgtgtt cggaaccgga 1920

acaaaggtga ccgtgctgag aacagtggca gcaccaagcg tgttcatctt tccccctagc 1980

gacgagcagc tgaagtccgg caccgcctct gtggtgtgcc tgctgaacaa cttctaccct 2040

agagaggcca aggtgcagtg gaaggtggat aacgccctgc agtctggcaa tagccaggag 2100

tccgtgaccg agcaggactc taaggatagc acatattccc tgtcctctac cctgacactg 2160

tccaaggccg attacgagaa gcacaaggtg tatgcctgtg aagtgaccca ccagggactg 2220

agcagccccg tgaccaaatc tttcaaccga ggcgaatgtt ga 2262

<210> 24
<211> 2232
<212> DNA

<213> Artificial Sequence

<220>
<223> transgenic construct/anti-CA 125

<400> 24
ttaagaaaaa atacgggtag aagcagtttg agagctccgt tcgccaccat ggagtttggg 60

ctgtcctggg tgttcctggt cgctctgttt aggggggtgc agtgtgaagt gcagctggtc 120

gtcgagtccg gggggggcct ggtgcagcca ggaggcagcc tgaggctgtc ctgcgcagca 180

tccggctact ctatcaccaa cgactatgcc tggaattggg tgagacaggc accaggcaag 240

ggcctggagt gggtgggcta catctcttat agcggctaca ccacatataa ccccagcctg 300

aagtccaggt tcaccatctc tcgcgatacc agcaagaaca cactgtacct gcagatgaat 360

tccctgaggg cagaggacac cgccgtgtac tattgcgcca gatggacaag cggcctggat 420

tattggggcc agggcaccct ggtgacagtg agctccgcca gcaccaaggg cccttccgtg 480

tttcccctgg ccccttctag caagtccacc tctggaggaa cagccgccct gggctgtctg 540

gtgaaggact acttccctga gccagtgacc gtgtcttgga acagcggcgc cctgacctcc 600

ggagtgcaca catttcccgc cgtgctgcag tcctctggcc tgtacagcct gagctccgtg 660

gtgaccgtgc cttctagctc cctgggcacc cagacatata tctgcaacgt gaatcacaag 720

ccatccaata caaaggtgga caagcgggtg gagcccaagt cttgtgataa gacccacaca 780

tgcccacctt gtccagcacc tgagctgctg ggaggaccat ccgtgttcct gtttccaccc 840

aagcctaagg acaccctgat gatcagcaga accccagagg tgacatgcgt ggtggtggac 900

gtgtcccacg aggatcccga ggtgaagttc aactggtacg tggatggcgt ggaggtgcac 960

aatgccaaga ccaagcccag ggaggagcag tacaactcta cctatcgcgt ggtgagcgtg 1020

ctgacagtgc tgcaccagga ctggctgaac ggcaaggagt acaagtgcaa ggtgtctaat 1080

aaggccctgc cagcccccat cgagaagacc atcagcaagg caaagggaca gccaagggag 1140

ccacaggtgt acacactgcc tccatctcgc gacgagctga ccaagaacca ggtgagcctg 1200

acatgtctgg tgaagggctt ctatcccagc gacatcgccg tggagtggga gtccaatggc 1260

cagcctgaga acaattacaa gaccacaccc cctgtgctgg actccgatgg ctctttcttt 1320

ctgtatagca agctgaccgt ggataagtcc cggtggcagc agggcaacgt gtttagctgc 1380

tccgtgatgc acgaggccct gcacaatcac tacacacaga agtctctgag cctgtcccca 1440

ggcaagagga gaaagagggg atccggcgag ggcagaggct ctctgctgac ctgtggcgac 1500

gtggaggaga accctggccc aatgaagtat ctgctgccta cagcagccgc cggcctgctg 1560

ctgctggcag cacagcctgc catggccgac atccagatga cccagtcccc atctagcctg 1620

tctgccagcg tgggcgacag ggtgaccatc acatgcaagg ccagcgatct gatccacaat 1680

tggctggcct ggtaccagca gaagcctggc aaggccccaa agctgctgat ctatggagcc 1740

acctctctgg agacaggagt gccaagccgc ttctccggat ctggaagcgg caccgacttt 1800

accctgacaa tctcctctct gcagcctgag gatttcgcca catactattg ccagcagtac 1860

tggaccacac cattcacctt tggccagggc acaaaggtgg agatcaagag gaccgtggcc 1920

gccccttccg tgttcatctt tccaccctct gacgagcagc tgaagtccgg cacagcctct 1980

gtggtgtgcc tgctgaacaa cttctaccca cgcgaggcca aggtgcagtg gaaggtggat 2040

aacgccctgc agagcggcaa ttcccaggag tctgtgaccg agcaggacag caaggattcc 2100

acatattctc tgagctccac cctgacactg agcaaggccg actacgagaa gcacaaggtg 2160

tatgcctgtg aagtcaccca ccaggggctg tcatcaccag tcactaagag tttcaatcgg 2220

ggggaatgct ga 2232

<210> 25
<211> 2238
<212> DNA
<213> Artificial Sequence

<220>
<223> transgenic construct/anti-EGFR

<400> 25
ttaagaaaaa atacgggtag aagcagtttg agagctccgt tcgccaccat ggagttcggg 60

ctgagttggg tgttcctggt cgctctgttt cggggggtgc agtgtcaggt gcagctgaag 120

cagtctgggc ctgggctggt ccagccctct cagagcctgt ccatcacctg cacagtgtct 180

ggcttcagcc tgaccaatta cggagtgcac tgggtgcggc agagccctgg caagggcctg 240

gagtggctgg gcgtgatctg gtccggaggc aatacagact ataacacccc cttcacctcc 300

agactgtcta tcaataagga taactctaag agccaggtgt tctttaagat gaatagcctg 360

cagtccaacg acaccgccat ctactattgc gccagggccc tgacatacta tgattacgag 420

ttcgcctatt ggggccaggg caccctggtg acagtgtctg ccgccagcac caagggccct 480

agcgtgtttc ccctggcccc tagctccaag tccacctctg gaggaacagc cgccctgggc 540

tgtctggtga aggactactt ccctgagcca gtgaccgtgt cctggaattc tggcgccctg 600

accagcggcg tgcacacatt tcccgccgtg ctgcagtcta gcggcctgta ctccctgtcc 660

tctgtggtga ccgtgcctag ctcctctctg ggcacccaga catatatctg caatgtgaac 720

cacaagccat ccaacacaaa ggtggacaag aaggtggagc ccaagtcttg tgataagacc 780

cacacatgcc caccttgtcc agcacctgag ctgctgggag gaccatccgt gttcctgttt 840

ccacccaagc ctaaggacac cctgatgatc tctagaaccc cagaggtgac atgcgtggtg 900

gtggacgtgt cccacgagga tcccgaggtg aagttcaatt ggtacgtgga tggcgtggag 960

gtgcacaacg ccaagaccaa gccacgggag gagcagtaca actccaccta tagagtggtg 1020

tctgtgctga cagtgctgca ccaggactgg ctgaatggca aggagtacaa gtgcaaggtg 1080

agcaacaagg ccctgccagc ccccatcgag aagaccatct ccaaggcaaa gggacagcca 1140

agggagccac aggtgtacac actgcctcca tctcgcgacg agctgaccaa gaatcaggtg 1200

agcctgacat gtctggtgaa gggcttctat cctagcgaca tcgcagtgga gtgggagtcc 1260

aacggacagc cagagaacaa ttacaagacc acaccccctg tgctggacag cgatggctcc 1320

ttctttctgt attctaagct gaccgtggat aagagccggt ggcagcaggg caacgtgttc 1380

agctgctccg tgatgcacga ggccctgcac aaccactaca cacagaagtc tctgagcctg 1440

tcccctggca agcggagaaa gaggggaagc ggagagggaa ggggctccct gctgacctgt 1500

ggcgacgtgg aggagaatcc tggcccaatg aagtatctgc tgcccacagc agccgccggc 1560

ctgctgctgc tggcagcaca gcctgccatg gccgacatcc tgctgaccca gagccctgtg 1620

atcctgtctg tgagcccagg cgagcgggtg tccttctctt gcagagccag ccagtccatc 1680

ggcaccaata tccactggta ccagcagagg acaaacggca gccctcgcct gctgatcaag 1740

tatgcctctg agagcatctc cggcatccca tccagattct ctggcagcgg ctccggcacc 1800

gactttacac tgtctatcaa cagcgtggag tccgaggaca tcgccgatta ctattgccag 1860

cagaacaata actggccaac cacattcggc gccggcacca agctggagct gaagaggaca 1920

gtggccgccc cctccgtgtt catctttcca ccctctgacg agcagctgaa gtctggcacc 1980

gccagcgtgg tgtgcctgct gaataacttt tacccccgcg aggccaaggt gcagtggaag 2040

gtggataatg ccctgcagtc tggcaacagc caggagtccg tgaccgagca ggactctaag 2100

gatagcacat attccctgag ctccaccctg acactgagca aggccgatta cgagaagcac 2160

aaggtgtatg cctgtgaagt cacccaccag ggcctgtcct cacccgtcac aaaatctttc 2220

aatagggggg agtgttga 2238

<210> 26
<211> 2244
<212> DNA
<213> Artificial Sequence

<220>
<223> transgenic construct/anti-HER2

<400> 26
ttaagaaaaa atacgggtag aagcagtttg agagctccgt tcgccaccat ggagttcggg 60

ctgagttggg tgttcctggt cgctctgttt aggggagtgc agtgtcaggt gcagctggtc 120

gagtctgggg gggggctggt ccagcctgga ggctctctga ggctgagctg cgcagcatcc 180

ggctttaaca tcaaggacac ctacatccac tgggtgcggc aggcaccagg caagggcctg 240

gagtgggtgg ccagaatcta tcctaccaat ggctacacac ggtatgccga cagcgtgaag 300

ggcagattca ccatctctgc cgataccagc aagaacacag cctacctgca gatgaattcc 360

ctgcgggccg aggatacagc cgtgtactat tgctctagat ggggcggcga cggcttttac 420

gccatggatt attggggcca gggcaccctg gtgacagtga gctccgcctc taccaaggga 480

ccaagcgtgt tcccactggc accttctagc aagtctacca gcggaggaac agccgccctg 540

ggatgtctgg tgaaggacta ctttcctgag ccagtgaccg tgtcctggaa ctctggcgcc 600

ctgaccagcg gagtgcacac attcccagcc gtgctgcagt cctctggcct gtactccctg 660

agctccgtgg tgaccgtgcc ctctagctcc ctgggcaccc agacatatat ctgcaacgtg 720

aatcacaagc ctagcaatac caaggtggac aagaaggtgg agccaaagtc ctgtgataca 780

ccacctccat gcccaaggtg tccagcacct gagctgctgg gaggaccaag cgtgttcctg 840

tttccaccta agcctaagga caccctgatg atctctcgca cccctgaggt gacatgcgtg 900

gtggtggacg tgtcccacga ggatccagag gtgaagttta actggtacgt ggatggcgtg 960

gaggtgcaca atgccaagac caagcccagg gaggagcagt acaactccac ctatcgcgtg 1020

gtgtctgtgc tgacagtgct gcaccaggac tggctgaacg gcaaggagta taagtgcaag 1080

gtgagcaata aggccctgcc agcccccatc gagaagacca tctccaaggc aaagggacag 1140

ccaagggagc cacaggtgta cacactgcca ccctctcgcg acgagctgac caagaaccag 1200

gtgagcctga catgtctggt gaagggcttt tatccaagcg acatcgccgt ggagtgggag 1260

tccaatggcc agcccgagaa caattacaag accacacctc cagtgctgga cagcgatggc 1320

tccttctttc tgtattctaa gctgaccgtg gataagagcc ggtggcagca gggcaacgtg 1380

ttctcctgct ctgtgatgca cgaggccctg cacaatcact acacacagaa gagcctgtcc 1440

ctgtctcccg gcaagcggag aaagagggga agcggagagg gaagaggctc cctgctgacc 1500

tgtggcgacg tggaggagaa ccctggccca atgaagtatc tgctgcctac agcagccgcc 1560

ggcctgctgc tgctggcagc acagcctgcc atggccgaca tccagatgac ccagagccca 1620

tctagcctga gcgcctccgt gggcgacagg gtgaccatca catgccgcgc ctcccaggat 1680

gtgaatacag ccgtggcctg gtaccagcag aagcctggca aggccccaaa gctgctgatc 1740

tactctgcca gcttcctgta ttccggcgtg ccatctaggt tttccggctc tcgcagcgga 1800

accgacttca ccctgacaat ctcctctctg cagcccgagg attttgccac atactattgc 1860

cagcagcact acaccacacc ccctaccttc ggccagggca caaaggtgga gatcaagagg 1920

accgtggcag caccttccgt gttcatcttt ccaccctctg acgagcagct gaagagcggc 1980

acagcctccg tggtgtgcct gctgaacaat ttctacccaa gagaggccaa ggtgcagtgg 2040

aaggtggata acgccctgca gtctggcaat agccaggagt ccgtgaccga gcaggactct 2100

aaggatagca catattccct gagctccacc ctgacactga gcaaggccga ctacgagaag 2160

cacaaggtgt atgcctgtga agtcacccac caggggctga gcagtcccgt cacaaaatcc 2220

tttaacagag gcgaatgttg ataa 2244

<210> 27
<211> 1458
<212> DNA
<213> Artificial Sequence

<220>
<223> transgenic construct/anti-nfP2X7

<400> 27
ttaagaaaaa atacgggtag aagcagtttg agagctccgt tcgccaccat ggagttcggg 60

ctgagttggg tgttcctggt cgctctgttt aggggggtgc agtgtgaagt gcagctgctg 120

gagtccgggg ggggcctggt ccagccagga ggcagcctga ggctgtcctg cgcagcatct 180

ggcttcacct ttaggaacca cgacatggga tgggtgcgcc aggcacctgg caagggcctg 240

gagtgggtga gcgccatctc tggaagcgga ggatccacat actatgccaa ctctgtgaag 300

ggccggttca ccatcagcag agacaactcc aagaatacac tgtacctgca gatgaatagc 360

ctgcgggccg aggataccgc cgtgtactat tgcgccgagc caaagcccat ggacacagag 420

ttcgattata gatccccagg caccctggtg acagtgagct ccgcctctac caagggcccc 480

agcgtgtttc ctctggcccc atctagcaag tccacctctg gaggaacagc cgccctgggc 540

tgtctggtga aggattactt ccctgagcca gtgacagtgt cttggaacag cggcgccctg 600

acctctggag tgcacacatt tcccgccgtg ctgcagtcct ctggcctgta ctccctgagc 660

tccgtggtga ccgtgccttc tagctccctg ggcacccaga catatatctg caacgtgaat 720

cacaagcctt ctaatacaaa ggtggacaag agggtggagc caaagagctg tgataagacc 780

cacacatgcc caccttgtcc agcacctgag ctgctgggag gaccaagcgt gttcctgttt 840

ccacccaagc ccaaggacac cctgatgatc tcccgcaccc ctgaggtgac atgcgtggtg 900

gtggacgtgt ctcacgagga tccagaggtg aagtttaact ggtacgtgga tggcgtggag 960

gtgcacaatg ccaagaccaa gccccgggag gagcagtaca acagcaccta tagagtggtg 1020

tccgtgctga cagtgctgca ccaggactgg ctgaacggca aggagtataa gtgcaaggtg 1080

agcaataagg ccctgccagc ccccatcgag aagaccatct ccaaggcaaa gggacagcca 1140

agggagcctc aggtgtacac actgcctcca agccgcgacg agctgaccaa gaaccaggtg 1200

tccctgacat gtctggtgaa gggcttctat ccctccgaca tcgccgtgga gtgggagtct 1260

aatggccagc ctgagaacaa ttacaagacc acaccccctg tgctggactc cgatggctct 1320

ttctttctgt attctaagct gaccgtggat aagagcagat ggcagcaggg caacgtgttc 1380

agctgttccg tgatgcacga agcactgcac aaccactaca ctcagaaatc cctgtcactg 1440

tcacctggca agtgataa                                                    1458

<210> 28
<211> 2232
<212> DNA
<213> Artificial Sequence

<220>
<223> transgenic construct/anti-PSMA

<400> 28
ttaagaaaaa atacgggtag aagcagtttg agagctccgt tcgccaccat ggagtttgga      60

ctgagttggg tgttcctggt cgccctgttt agaggcgtgc agtgtgaagt gcagctggtg     120

cagagcggcc ctgaagtgaa aaagccaggc gccaccgtga agatctcttg caagacaagc     180

ggctacacct tcacagagta taccatccac tgggtgaagc aggcaccagg caagggcctg     240

gagtggatcg gcaacatcaa tcctaacaat ggcggcacca catacaacca gaagtttgag     300

gacaaggcca ccctgacagt ggacaagagc accgatacag cctatatgga gctgagctcc     360

ctgcggtccg aggacaccgc cgtgtactat tgcgccgccg gctggaattt cgattactgg     420

ggccagggca ccctgctgac agtgtctagc gccagcacaa agggaccatc cgtgtttcca     480

ctggcacctt cctctaagtc cacctctgga ggaacagccg ccctgggctg tctggtgaag     540

gattacttcc ctgagccagt gaccgtgtcc tggaactctg gcgccctgac ctccggagtg     600

cacacatttc cagccgtgct gcagagctcc ggcctgtaca gcctgtctag cgtggtgacc     660

gtgccttcct ctagcctggg cacccagaca tatatctgca acgtgaatca caagccatcc     720

aatacaaagg tggacaagcg ggtggagccc aagtcttgtg ataagaccca cacatgccca     780

ccttgtccag cacctgagct gctgggagga ccatccgtgt tcctgtttcc acccaagcct     840

aaggacaccc tgatgatctc tagaacccct gaggtgacat gcgtggtggt ggacgtgagc     900

cacgaggatc cagaggtgaa gttcaactgg tacgtggatg gcgtggaggt gcacaatgcc     960

aagaccaagc ctagggagga gcagtacaac tctacctatc gcgtggtgag cgtgctgaca    1020

gtgctgcacc aggactggct gaacggcaag gagtataagt gcaaggtgtc caataaggcc    1080

ctgccagccc ccatcgagaa gaccatcagc aaggcaaagg gacagccaag ggagccacag    1140

gtgtacacac tgcctccatc tagagacgag ctgaccaaga accaggtgag cctgacatgt    1200

ctggtgaagg gcttctatcc atccgacatc gccgtggagt gggagtctaa tggccagccc    1260

gagaacaatt acaagaccac accccctgtg ctggacagcg atggctcctt ctttctgtat    1320

agcaagctga ccgtggacaa gtccaggtgg cagcagggca acgtgtttag ctgctccgtg    1380

atgcacgagg ccctgcacaa tcactacaca cagaagtctc tgagcctgtc cccaggcaag    1440

aggagaaaga ggggatccgg agagggaagg ggctctctgc tgacctgtgg cgatgtggag    1500

gagaaccctg gcccaatgaa gtatctgctg cctacagcag ccgccggcct gctgctgctg 1560

gcagcacagc ctgccatggc cgacatccag atgacccagt ccccatcctc tctgtctaca 1620

agcgtgggcg atcgcgtgac cctgacatgc aaggcatccc aggacgtggg aaccgcagtg 1680

gattggtacc agcagaagcc cggccctagc ccaaagctgc tgatctattg tgccagcacc 1740

cggcacacag gcatcccatc cagattctcc ggctctggca gcggcaccga ctttaccctg 1800

acaatcatca gctccctgca gccagaggac ttcgcagcag attactattg ccagcagtac 1860

aatagctatc ctctgacctt tggcccaggc acaaaggtgg acatcaagag gaccgtggcc 1920

gcccctagcg tgttcatctt tccacccagc gatgagcagc tgaagtccgg cacagcctct 1980

gtggtgtgcc tgctgaacaa cttctaccca cgcgaggcca aggtgcagtg gaaggtggac 2040

aacgccctgc agtctggcaa tagccaggag tccgtgaccg agcaggactc taaggatagc 2100

acatattccc tgtctagcac cctgacactg agcaaggccg attacgagaa gcacaaggtg 2160

tatgcctgcg aggtcactca tcagggcctg tcatcaccag tcaccaaaag tttcaatagg 2220

ggggaatgtt ga 2232

<210> 29
<211> 2232
<212> DNA
<213> Artificial Sequence

<220>
<223> transgenic construct/anti-VEGFR

<400> 29
ttaagaaaaa atacgggtag aagcagtttg agagctccgt tcgccaccat ggagttcggc 60

ctgtcttggg tgttcctggt cgccctgttt aggggagtgc agtgtgaagt gcagctggtg 120

cagtctgggg ggggcctggt gaagccagga ggcagcctga ggctgtcctg cgcagcatct 180

ggcttcacct ttagctccta cagcatgaac tgggtgcgcc aggcaccagg caagggcctg 240

gagtgggtgt ctagcatctc ctctagctcc tcttacatct actatgccga ctccgtgaag 300

ggcaggttca ccatctctcg cgataacgcc aagaatagcc tgtatctgca gatgaattcc 360

ctgagggccg aggacaccgc cgtgtactat tgcgcccgcg tgacagacgc cttcgacatc 420

tggggccagg gcaccatggt gacagtgagc tccgcctcta ccaagggacc aagcgtgctg 480

ccactggcac catctagcaa gtctaccagc ggaggaacag ccgccctggg atgtctggtg 540

aaggattact tccccgagcc tgtgacagtg tcttggaaca gcggcgccct gaccagcgga 600

gtgcacacat ttcccgccgt gctgcagtcc tctggcctgt actccctgag ctccgtggtg 660

accgtgcctt ctagctccct gggcacccag acatatatct gcaacgtgaa tcacaagcct 720

agcaatacaa aggtggacaa gagggtggag ccaaagtcct gtgataagac ccacacatgc 780

ccaccttgtc cagcaccaga gctgctggga ggaccaagcg tgttcctgtt tccacccaag 840

cccaaggaca ccctgatgat ctctcgcacc cctgaggtga catgcgtggt ggtggacgtg 900

tcccacgagg atccagaggt gaagttcaac tggtacgtgg atggcgtgga ggtgcacaat 960

gccaagacca agcctcggga ggagcagtac aactccacct atagagtggt gtctgtgctg 1020

acagtgctgc accaggactg gctgaacggc aaggagtata agtgcaaggt gagcaataag 1080

gccctgcctg ccccaatcga gaagaccatc tccaaggcaa agggacagcc tagggagcca 1140

caggtgtaca cactgcctcc atccagagag gagatgacca agaaccaggt gtctctgaca 1200

tgtctggtga agggcttcta tccaagcgac atcgccgtgg agtgggagtc caatggccag 1260

cccgagaaca attacaagac cacaccccct gtgctggact ccgatggctc tttctttctg 1320

tattctaagc tgaccgtgga taagagccgg tggcagcagg gcaacgtgtt ttcctgctct 1380

gtgatgcacg aggccctgca caatcactac acacagaaga gcctgtccct gtctcccggc 1440

aagcggagaa agaggggaag cggagaggga agaggctccc tgctgacctg tggcgacgtg 1500

gaggagaacc caggacctat gaagtatctg ctgcctacag cagccgccgg cctgctgctg 1560

ctggcagcac agcccgccat ggccgacatc cagatgaccc agagcccttc tagcgtgagc 1620

gcctccatcg gcgacagggt gaccatcaca tgccgcgcct cccagggcat cgataactgg 1680

ctgggctggt accagcagaa gcccggcaag gcccctaagc tgctgatcta tgacgcctct 1740

aatctggata ccggagtgcc aagccggttc tctggaagcg gctccggcac atactttacc 1800

ctgacaatct cctctctgca ggccgaggac ttcgccgtgt atttttgcca gcaggccaag 1860

gcattcccac ccacctttgg cggaggaaca aaggtggaca tcaagaggac cgtggcagca 1920

ccttccgtgt tcatctttcc tccatctgat gagcagctga agagcggcac agcctccgtg 1980

gtgtgcctgc tgaacaattt ctacccaaga gaggccaagg tgcagtggaa ggtggacaac 2040

gccctgcaga gcggcaattc ccaggagtct gtgaccgagc aggacagcaa ggattccaca 2100

tattctctga gctccaccct gacactgagc aaggccgatt acgagaagca caaggtgtat 2160

gcctgtgagg tcactcacca ggggctgtca agtccagtca ccaagtcctt caatcggggg 2220

gaatgttgat aa 2232

<210> 30
<211> 2232
<212> DNA
<213> Artificial Sequence

<220>
<223> transgenic construct/anti-(ns)hIL-12

<400> 30

ttaagaaaaa atacgggtag aagcagtttg agagctccgt tcgccaccat ggagttcggc 60

ctgtcctggg tgttcctggt ggccctgttc cgcggcgtgc agtgcgaggt ccaattggtc 120

gtggaatcgg ggggaggatt ggtacagccc ggcgggagct tgcggctgag ttgcgcagct 180

agcgggtaca gcattactaa cgattacgct tggaattggg ttagacaggc cccaggtaaa 240

ggcctcgagt gggtgggtta tatctcttac tcaggataca caacgtataa tcccagcctc 300

aaatccaggt ttaccatctc ccgtgataca agcaaaaata cgctctacct gcaaatgaac 360

tctttgaggg cagaggacac cgccgtttac tattgcgctc gatggacaag tggcttggac 420

tattgggggc agggcacgtt agttacagtc agcagcgcca gcaccaaggg ccccagcgtg 480

ttccccctgg cccccagcag caagagcacc agcggcggca ccgccgccct gggctgcctg 540

gtgaaggact acttccccga gcccgtgacc gtgagctgga acagcggcgc cctgaccagc 600

ggcgtgcaca ccttccccgc cgtgctgcag agcagcggcc tgtacagcct gagcagcgtg 660

gtgaccgtgc ccagcagcag cctgggcacc cagacctaca tctgcaacgt gaaccacaag 720

cccagcaaca ccaaggtgga caagagggtg gagcccaaga gctgcgacaa gacccacacc 780

tgccccccct gccccgcccc cgagctgctg ggcggcccca gcgtgttcct gttccccccc 840

aagcccaagg acaccctgat gatcagcagg acccccgagg tgacctgcgt ggtggtggac 900

gtgagccacg aggaccccga ggtgaagttc aactggtacg tggacggcgt ggaggtgcac 960

aacgccaaga ccaagcccag ggaggagcag tacaacagca cctacagggt ggtgagcgtg 1020

ctgaccgtgc tgcaccagga ctggctgaac ggcaaggagt acaagtgcaa ggtgagcaac 1080

aaggccctgc ccgcccccat cgagaagacc atcagcaagg ccaagggcca gcccagggag 1140

ccccaggtgt acaccctgcc ccccagcagg gacgagctga ccaagaacca ggtgagcctg 1200

acctgcctgg tgaagggctt ctaccccagc gacatcgccg tggagtggga gagcaacggc 1260

cagcccgaga acaactacaa gaccaccccc cccgtgctgg acagcgacgg cagcttcttc 1320

ctgtacagca agctgaccgt ggacaagagc aggtggcagc agggcaacgt gttcagctgc 1380

agcgtgatgc acgaggccct gcacaaccac tacacccaga agagcctgag cctgagcccc 1440

ggcaagcgcc gcaagcgcgg ctccggcgag ggccgcggct ccctgctgac ctgcggcgac 1500

gtggaggaga accccggccc catgaagtac ctgctgccca ccgccgccgc cggcctgctg 1560

ctgctggccg cccagcccgc catggccgac atacagatga ctcagagtcc ctcctcactc 1620

tctgcatcag tcggcgaccg ggtcactatt acttgtaaag catccgatct tattcacaat 1680

tggttggctt ggtatcaaca aaaaccaggt aaggctccta aattactcat atacggggct 1740

acctcacttg aaacgggagt gcctagccgc ttttcaggca gcgggtctgg aacagacttt 1800

accctgacca tttctagcct gcagccagaa gacttcgcca catattactg ccaacagtac 1860

tggacaacgc cattcacatt cggccaaggt accaaggtag aaatcaaaag gaccgtggcc 1920

gcccccagcg tgttcatctt cccccccagc gacgagcagc tgaagagcgg caccgccagc 1980

gtggtgtgcc tgctgaacaa cttctacccc agggaggcca aggtgcagtg gaaggtggac 2040

aacgccctgc agagcggcaa cagccaggag agcgtgaccg agcaggacag caaggacagc 2100

acctacagcc tgagcagcac cctgaccctg agcaaggccg actacgagaa gcacaaggtg 2160

tacgcctgcg aggtgaccca ccagggcctg agcagccccg tgaccaagag cttcaacagg 2220

ggcgagtgct ga 2232

<210> 31
<211> 17430
<212> DNA
<213> Newcastle Disease Virus

<220>
<223> Mut
HN(F277L)/M(G165W)/F(F117S)/F(F190L)/F(L289A)/L(V757I)/L(F1551S)/L(R1700L)-So
fituzumab

<400> 31
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cggtagaagg cagtttgaga gctccgttcg 3180

ccaccatgga gtttgggctg tcctgggtgt tcctggtcgc tctgtttagg ggggtgcagt 3240

gtgaagtgca gctggtcgtc gagtccgggg ggggcctggt gcagccagga ggcagcctga 3300

ggctgtcctg cgcagcatcc ggctactcta tcaccaacga ctatgcctgg aattgggtga 3360

gacaggcacc aggcaagggc ctggagtggg tgggctacat ctcttatagc ggctacacca 3420

catataaccc cagcctgaag tccaggttca ccatctctcg cgataccagc aagaacacac 3480

tgtacctgca gatgaattcc ctgagggcag aggacaccgc cgtgtactat tgcgccagat 3540

ggacaagcgg cctggattat tggggccagg gcaccctggt gacagtgagc tccgccagca 3600

ccaagggccc ttccgtgttt cccctggccc cttctagcaa gtccacctct ggaggaacag 3660

ccgccctggg ctgtctggtg aaggactact tccctgagcc agtgaccgtg tcttggaaca 3720

gcggcgccct gacctccgga gtgcacacat ttcccgccgt gctgcagtcc tctggcctgt 3780

acagcctgag ctccgtggtg accgtgcctt ctagctccct gggcacccag acatatatct 3840

gcaacgtgaa tcacaagcca tccaatacaa aggtggacaa gcgggtggag cccaagtctt 3900

gtgataagac ccacacatgc ccaccttgtc cagcacctga gctgctggga ggaccatccg 3960

tgttcctgtt tccacccaag cctaaggaca ccctgatgat cagcagaacc ccagaggtga 4020

catgcgtggt ggtggacgtg tcccacgagg atcccgaggt gaagttcaac tggtacgtgg 4080

atggcgtgga ggtgcacaat gccaagacca agcccaggga ggagcagtac aactctacct 4140

atcgcgtggt gagcgtgctg acagtgctgc accaggactg gctgaacggc aaggagtaca 4200

agtgcaaggt gtctaataag gccctgccag cccccatcga gaagaccatc agcaaggcaa 4260

agggacagcc aagggagcca caggtgtaca cactgcctcc atctcgcgac gagctgacca 4320

agaaccaggt gagcctgaca tgtctggtga agggcttcta tcccagcgac atcgccgtgg 4380

agtgggagtc caatggccag cctgagaaca attacaagac cacaccccct gtgctggact 4440

ccgatggctc tttctttctg tatagcaagc tgaccgtgga taagtcccgg tggcagcagg 4500

gcaacgtgtt tagctgctcc gtgatgcacg aggccctgca caatcactac acacagaagt 4560

ctctgagcct gtccccaggc aagaggagaa agaggggatc cggcgagggc agaggctctc 4620

tgctgacctg tggcgacgtg gaggagaacc ctggcccaat gaagtatctg ctgcctacag 4680

cagccgccgg cctgctgctg ctggcagcac agcctgccat ggccgacatc cagatgaccc 4740

agtccccatc tagcctgtct gccagcgtgg gcgacagggt gaccatcaca tgcaaggcca 4800

gcgatctgat ccacaattgg ctggcctggt accagcagaa gcctggcaag gccccaaagc 4860

tgctgatcta tggagccacc tctctggaga caggagtgcc aagccgcttc tccggatctg 4920

gaagcggcac cgactttacc ctgacaatct cctctctgca gcctgaggat ttcgccacat 4980

actattgcca gcagtactgg accacaccat tcacctttgg ccagggcaca aaggtggaga 5040

tcaagaggac cgtggccgcc ccttccgtgt tcatctttcc accctctgac gagcagctga 5100

agtccggcac agcctctgtg gtgtgcctgc tgaacaactt ctacccacgc gaggccaagg 5160

tgcagtggaa ggtggataac gccctgcaga gcggcaattc ccaggagtct gtgaccgagc 5220

aggacagcaa ggattccaca tattctctga gctccaccct gacactgagc aaggccgact 5280

acgagaagca caaggtgtat gcctgtgaag tcacccacca ggggctgtca tcaccagtca 5340

ctaagagttt caatcggggg gaatgctgag ctgaccgagc cctcctctat aaacccaaga 5400

ttcaaggctc caagcgataa ccttctcccg cctcttccat cccactgaat gatcgcgcag 5460

ctgcaattaa tctagcaaca ttaaagatta agaaaaaata cgggtagaat cggagtgcac 5520

cgattgtgcc aagatggact catctaggac aattgggtta tactttgatt ctgcccttcc 5580

ttctagcaac ctgttagcat tcccgattgt cttacgagat gtaggagatg gaaagaagca 5640

aattactccg caatatagga tccggcgtct tgactcgtgg acagacagta aagaagactc 5700

ggtattcatc actacctacg gattcatctt ccaggttggg aatgaagaag tcactgtcgg 5760

catgatcaat gataatccca agcgagagtt actctccgct gcgatgctct gcctaggaag 5820

cgtcccgaat gttggagatc ctgttgagct ggcaagggcc tgcctcacta tggtggtaac 5880

atgcaagaag agtgcaacta atactgagag aatggtcttc tcagtagtgc aggcaccccg 5940

ggtgctgcaa agctgtaggg tcgtggcaga caaatactcg tcagtgaatg cagttaagca 6000

cgtgaaagca ccagagaaga tcccttggag tgaaacccta gagtacaagg tgaattttgt 6060

ctctttgact gtggtgccga agaaggatgt ctacaagatc ccaaccgcag tattgaaggt 6120

atccggctcg agcctgtata atcttgcgct caatgtcact attgatgtgg aggtagaccc 6180

aaagagcccg ttagtcaagt ccctttctag gtccgacagt ggatactatg ctaatctctt 6240

cttacatatc ggacttatgt ccactgtaga taagagggga aagaaagtga catttgacca 6300

gctggagagg aagataagga gacttgatct atctgtcggg ctcagtgatg tgctcggacc 6360

ttctgtgctt gtgaaggcga gaggtgcacg gactaggctg ctggcacctt tcttctctaa 6420

cagcgggaca gcctgctacc ctatagcaaa tgcctctcct caggtagcta agatactctg 6480

gagtcagacc gcgtgcctgc ggagtgtaaa aatcattatt caagcgggca cccaacgcgc 6540

tgtcgcagtg actgctgacc atgaggttac ctctactaag atagagaaga ggcataccat 6600

cgctaaatac aatcccttca agaaataggc cgtatccctg agactgcaat tcacccgcct 6660

tcccaaaaca ccatgacacc agataatgat ctgtcttgat tacttacagt tagtttccct 6720

gtctatcaaa ttagaaaaaa cacgggtaga agagttcgga tcccggccgg cgcaccaaaa 6780

gcgcaagatg ggccccagat cttctaccag gatcccagta cctctaatgc tgaccatacg 6840

gatcacgctg gcactgagtt atgtccgtct gacaagttct cttgatggca ggcctcttgc 6900

agccgcaggg atcgtggtaa caggggataa agcagttaac atatacacct catcccagac 6960

agggtcaatc atagtcaagt tactcccaaa tatgcccaag gacaaagagg catgtgcaaa 7020

agccccattg gaggcttaca acaggacact gactactttg cttacccccc ttggtgattc 7080

tatccgcagg atacaagagt ctgtgactac atccggagga aggagacaga gacgctctat 7140

aggtgccatt attggcagtg tagctctagg ggttgcaaca gctgcacaga taacggcagc 7200

ctcggctctg atacaagcca accagaatgc tgctaacatc ctccggctta aggagagcat 7260

tgctgcaacc aatgaagctg tacacgaggt cactggcgga ttgtcacagt tagcagtggc 7320

agttggaaag atgcagcaat ttgttaatga ccagcttaat aacacagctc aagagttgga 7380

ctgtataaaa attacacagc aggttggtgt agaactcaac ttatacttaa ctgaattgac 7440

tacagtgttc gggccacaaa tcacttcccc tgccttaact cagttgactg ttcaggctct 7500

ttacaatctg gctggtggta atgtagatta cttgttgact aagttaggtg tagggaacaa 7560

ccagctcagc tcattgattg gtagcggctt gatcaccggt aaccctattt tttacgactc 7620

acagactcaa ctcttgggca tacaggtgac tgcaccctca gtcgggaacc taaataatat 7680

gcgtgccacc tacttggaga ccttgtctgt aagcacaaca aagggatttg cctcagcact 7740

tgtcccaaaa gtagtgacac aggtcggttc tgtgatagaa gagcttgaca cctcatactg 7800

tatagaagct gatttggatt tatattgtac aagaatagtg acattcccta tgtcccctgg 7860

tatttattcc tgtttgagcg gcaatacatc ggcttgcatg tattcaagga ctgaaggcgc 7920

acttactaca ccatacatga ctctcaaagg ctcagttgtt gccaattgcc agatgacaac 7980

atgtagatgt gcagaccccc cgggtatcat atcacaaaat tatggagagg ctgtgtctct 8040

aatagataag cactcatgca atgtcgtatc cttagacggg ataactttga ggctcagtgg 8100

ggaatttgat gcaacttatc aaaagaatat ctcaatatta gattctcaag tactagtgac 8160

aggcaatctc gatatctcaa ctgagcttgg gaatgtcaac cactcaataa gtaatgcttt 8220

ggataagtta gaggaaagca acagcaaact agacaaagtc aatgtcaaac tgaccagcac 8280

atctgctctc attacctata ttgttttaac tgtcatatct cttgttcttg gtatgcttag 8340

cctggttcta gcatgctatc tgatgtacaa gcaaaaagcg caacgaaaga ccttgttgtg 8400

gcttggaaat aataccctag atcagatgag agctactaca aaaatgtgaa tgcagatgag 8460

aggcagaggt atccccaata gcaatctgtg tgtcaattct ggcagcctgt taatcagaag 8520

aattaagaaa aaactaccgg atgtaggtga acaaaaggga atatacgggt agaacggcct 8580

gagaggccac ccctcaatcg ggagccaggc cccactacgt ccgctctacc gcatcaccaa 8640

cagcagtctt cagtcatgga cagcgcagtt agccaagttg cgctagagaa tgatagaaga 8700

gaagcgaata atacatggcg cttggttttc cggatcgcag ccttactttt actggtaata 8760

accttagccg tctctgcagt cgccctggca tatagtatgg aggctagcac acctggcgac 8820

cttgtaagca taccgactgc gatctctagg gcagaggaaa ggattacatc tgcactcggt 8880

tccaatcaag atgtagtaga taggatatac aagcaagtgg cccttgagtc tccattggca 8940

ctgctaaata ccgaatctat aattatgaat gcaataacgt ctctctctta tcaaatcaat 9000

ggagccacaa ataatagcgg gtgtggggca cctgttcatg acccagatta catcgggggg 9060

ataggtcaag aacttattgt agatgatacg agtgacgtca catcatttta tccctctgca 9120

ttccaagaac acctgaattt tatcccggcg cccactacag gatcaggctg cactcggata 9180

ccctcattcg acatgagtgc tacccattac tgttacactc acaatgtgat attgtctggc 9240

tgcagagatc actcacactc acatcagtat ttagcacttg gtgtgcttcg gacatctgca 9300

acagggaggg tattcttttc tactctgcgt tccatcaact tggatgacgc ccaaaatcgg 9360

aagtcttgca gtgtgagtgc aactccttta ggttgtgata tgctgtgctc taaaatcaca 9420

gagactgagg aagaggatta taaatcagtt atccccacat cgatggtaca tggaaggtta 9480

gggcttgacg gccaatacca tgagaaggac ctagacgtca caacactatt tagggactgg 9540

gtggcaaatt acccaggagt aggaggtggg tcttttatta acaatcgcgt atggttcccg 9600

gtctacggag ggctaaaacc cagctcgcct agtgacactg cacaagaagg gagatacgta 9660

atatacaagc gatacaatga cacatgcccg gatgagcaag attaccagat tcggatggct 9720

aagtcatcgt ataagcctag gcggtttggt ggaaaacgcg tacagcaggc catcctatcc 9780

atcaaggtgt caacatcctt gggtgaggac ccggtgttga ctgtaccgcc taatacggtc 9840

gcactcatgg gggccgaagg cagagttctc acagtgggga catctcattt cttatatcag 9900

cgagggtcat catacttctc tcccgctttg ttatacccta tgacagtcaa caacaaaaca 9960

gccactcttc ataatcctta tacattcaat gctttcactc ggccaggtag tgtcccttgc 10020

caggcttcag caagatgccc caactcatgt gttaccggag tctatactga tccatatccc 10080

ttagtcttcc ataggaacca caccttgcga ggggtattcg gaacaatgct tgatgacgaa 10140

caagcaagac tcaaccctgt atctgcagta tttgataaca tatcccgcag tcgcataact 10200

cgggtaagtt caagcagtac cagggcagca tacacgacat caacgtgttt taaagttgtc 10260

aagaccaata aaacctattg cctcagcatt gcagaaatat ccaataccct cttcggggaa 10320

ttcagaattg tccccttact agttgagatt ctcaaggatg gtggggttta agaagctagg 10380

aatggtcggt tgagtcaacc gtgagaaggc ggggaagatg atattgcatc atctatcttc 10440

tgtaacacca agaatcaaat cgaataccag tgcgagctcg aatcctacgc tgccagtcag 10500

ccataatcgg ctggtgctaa tgtggttagc ctgaatcttg tcgatagtca cttgattaag 10560

aaaaaatgtg gacggtagtg aaatataagg caaagcagct cacagaggac agcacgggta 10620

ggacatggcg ggctccggac ccgaaagggc agagcatcag attatcctac cagagtcaca 10680

tctgtcctca ccattggtca agcataaatt gctttattac tggaaattaa ctgggctacc 10740

gcttcctgac gaatgtgact tcgaccatct tattatcagc cgacaatgga agaaggtact 10800

tgaatcggcc acccctgaca ttgagagaat gataaaacta gggcgggcag tacaccagac 10860

tctcaaccac aattccagga taaccggagt actccatccc cggtgtttag aggaactggc 10920

tagtattgag gttcctgatt caaccaacaa atttcggaag atcgaaaaaa agatccagat 10980

tcacaacaca aggtatggag aactgttcac aagattatgc acgcatgtag aaaagaaact 11040

attggggtca tcttggtcta acaatgtccc acggtcagag gaattcaaca gcatccgtac 11100

agatccggca ttttggtttc actcaaaatg gtccacagcc aaatttgcat ggctccatat 11160

aaaacaggtc cagaggcatc tgattgtagc agcaagaaca aggtccgcag tcaacaaatt 11220

agtgacgctg acccataagg taggccaaat ctttgttact cctgagcttg tcattgtgac 11280

acatacagat gagaacaagt tcacgtgtct tacccaggaa cttgtgttga tgtatgcaga 11340

tatgatggag ggcagagata tggtcagcat aatatcatcc acggcggcac atcttaggag 11400

cttatcagag aaaattgatg atattctgcg gttagtagat gctctggcaa gagatttggg 11460

caatcaagtc tacgatgttg tagcactaat ggagggattc gcatacggcg ctgttcagct 11520

gcttgaaccg tcaggtacat ttgcggggga tttcttcgca ttcaacctgc aggagctcaa 11580

agatactcta accggactcc tccccaagga tatcgcagaa tctgtgactc acgcaatcgc 11640

aaccatattc tctggcttag aacaaaatca agcagctgag atgttgtgcc tgttgcgtct 11700

gtggggtcac ccactactcg agtcccgtat tgcagcaaaa gcagttagga gtcaaatgtg 11760

cgcaccaaaa atggtagact ttgatatgat cctccaggta ttatctttct ttaaaggaac 11820

aatcatcaat ggatatagaa agaagaatgc aggcgtgtgg ccacgtgtca agatagatac 11880

gatatacggg aaggtcatag ggcagctaca cgcagattct gcagagattt cacatgatgt 11940

catgttgagg gaatacaaga gtttatctgc acttgaattc gagccatgta tagagtatga 12000

ccctgtcacc aatctgagca tgtttttaaa agacaaggca atcgcacacc cgaaagacaa 12060

ctggctcgct tcgtttaggc gaaaccttct ctctgaggac cagaagaaac atgtaagaga 12120

ggcaacctca actaaccgcc tcttgataga gttcttagag tcaaatgatt ttgatccata 12180

taaagagatg gaatatctga cgacccttga gtacctaaga gatgataatg tggcagtatc 12240

atactcactc aaagaaaaag aggtgaaagt taatgggcgg attttcgcaa agctaacaaa 12300

tagattaagg aattgtcagg taatggcaga aggatcccta gctgaccaga ttgcaccttt 12360

cttccaggga aatggggtca ttcaggatag catatctttg actaaaagta tgctagcgat 12420

gagtcaactg tctttcaaca gcaacaagaa acgtattact gactgcaaag aaagagtatc 12480

ctcaaaccgc aatcatgatc cgaagagcaa gaatcgtcgg agagttgcca cttttgtaac 12540

taccgacctg caaaagtatt gtctcaattg gagatatcag acagtcaagc tgttcgctca 12600

tgccatcaat cagctgatgg gcttacctca cttcttcgag tggattcatc ttagactaat 12660

ggatactacg atgtttgtag gggacccttt caatcctcca agtgacccta cggactgtga 12720

tctatcaaga gtcccaaatg atgacatata tattgtcagt gctaggggag gcattgaggg 12780

attatgccag aagctatgga caatgatctc aattgctgca atccagcttg ctgcagcaag 12840

atcacattgt cgcgttgcct gtatggtaca aggcgacaat caagtaatag ctataacgag 12900

agaggtaaga tcagatgact ccccagagat ggtgttaaca caattgcatc aagccagtga 12960

taatttcttc aaggaattga ttcatgtcaa tcatttgatc ggccataatt tgaaggatcg 13020

tgaaaccatc aggtcagaca cgttcttcat atacagcaaa cgaatattca aagatggagc 13080

aatactcagt caggtcctca aaaactcatc taaattagtg ctaatatcag gcgaccttag 13140

tgaaaacact gtaatgtctt gtgccaacat tgcatctact gtagcacggt tatgcgagaa 13200

cgggcttcct aaggatttct gttattactt aaactactta atgagttgcg tgcagacata 13260

cttcgattcc gaattttcca tcaccaacaa ctcgcaactc gattctaacc agtcgtggat 13320

agaggacatt tcttttgtgc actcatatgt cctgacccct gctcaactgg ggggactgag 13380

taaccttcaa tactcaaggc tctacacaag gaacatcggc gacccgggaa ccactgcttt 13440

cgcagagatc aagagattag aggcagtggg gttactgagt cctagcatta tgactaacat 13500

cttaactagg ccgcctggaa atggagattg ggccagtctg tgcaacgatc catactcctt 13560

taattttgag actgtcgcaa gcccaaatat tgtccttaag aaacatacac aaagagtcct 13620

atttgaaact tgctcgaatc ccttattatc tggagtgcac acagaggata atgaggcaga 13680

agagaaggca ttggctgaat tcttgctcaa tcaagaaatg attcatccac gtgtcgcgca 13740

tgctatcatg gaagcaagct ctgtaggtag gagaaagcaa attcaggggc ttgttgacac 13800

aacaaacacc gtgattaaga ttgcactgac taggaggcca ctcggcatca agaggctgat 13860

gcggatagtc aattactcga gcatgcatgc aatgctattt agagatgatg ttttctcgtc 13920

taatagatcc aaccatccct tagtctcttc tgatatgtgt tctcagacac tggcagacta 13980

tgcacggaac agaagctggt cacctttgac agggggtaga aaaatactgg gtgtatctaa 14040

tcctgatacc atagaacttg tagagggtga gatccttagt gtcagtggag ggtgcactag 14100

atgtgacagt ggggatgaac agtttacttg gttccatctt ccaagcaata tagagctaaa 14160

cgatgacacc agcaagaatc ctccaatgag agtgccatat ctcgggtcaa agactcaaga 14220

gaggagagcc gcctcgcttg cgaaaatagc tcatatgtca ccacacgtga aggcggctct 14280

aagggcgtca tctgtgttaa tctgggctta tggggacaac gaaataaact ggactgctgc 14340

ccttaagatt gcaaggtctc ggtgcaacat aagctcagag tatcttcgac tattgtcacc 14400

cttgcctaca gctgggaatc tccaacatag attggacgac ggcataactc agatgacatt 14460

cacccctgca tctctataca gggtgtcacc ttacattcat atatctaatg attctcaaag 14520

gctatttact gaagaaggag tcaaagaggg gaatgtggtt tatcagcaaa tcatgctctt 14580

gggtttgtcc ttaattgagt cactcttccc aatgacaaca accaagacat atgatgaaat 14640

cacattgcac ctccacagta aatttagctg ctgtatcagg gaagcacctg ttgcagttcc 14700

tttcgagcta ctcggggtgg caccggaact aagggcagta acctcaaata agtttatgta 14760

tgatcctagc cctgtatcag agggagactt tgcgagactt gacttagcta tctttaagag 14820

ttatgaactt aatttagagt catattccac aatagagcta atgaacgttc tttcaatatc 14880

tagtgggaag ttgattggcc agtctgtggt ttcttatgat gaagatacct ccataaagaa 14940

tgacgctata atagtgtatg acaacacacg gaattggatc agcgaagctc agaattcaga 15000

tgtggtccgc ctattcgagt atgcggcact cgaagtgctc ctcgactgtt cttatcaact 15060

ctactatctg agagtaagag gcctaaacaa tatcgtcctg tacatgagtg atttatacaa 15120

gaatatgcca ggaattctac tctctaatat tgcagccaca atatctcacc ctgtcatcca 15180

ttcaaggttg aatgcagtag gtctggtcaa ccatgacggg tcacaccaac ttgcagacac 15240

agatttcatt gaaatgtctg caaagctgct agtctcttgc actcgacgcg tggtctcagg 15300

tttacatgca gggaataagt atgacctgct gtttccatct gtcttggatg ataacctaag 15360

tgagaagatg cttcagttga tatcccaatt atgctgtctg tatacggtgc tctttgctac 15420

aacaagagaa atcccgaaaa taagaggctt atctgcagaa gagaaatgtt cagtacttac 15480

tgagtaccta ctgtcagatg ctgtgaaacc gttacttggg tccgagcaag tgagctctat 15540

catgtctccc aacatagtta cgttcccagc caatctgtat tacatgtcta ggaagagcct 15600

taatttgatc agggagagag aggacaggga tactatcttg gcattgttgt tccctcaaga 15660

accgctgctc gagtttcctc tggtacgaga tattggtgct cgtgtaaaag atccatttac 15720

cctacaacct gcggcgtttt tacaagagtt agatttgagt gctccggcaa ggtatgacgc 15780

attcacaatc agtcaggcgc attctgaaca catattgcca aacccagagg aagatcactt 15840

agtacgatac ttgttcagag gaatagggac tgcgtcctcc tcttggtata aggcatccca 15900

tcttctttct gtacccgagg tcagatgtgc aaggcatggg aactccttat atttagcaga 15960

aggaagtgga gccatcatga gtcttctcga attgcatata ccacacgaaa ctatctatta 16020

caatacactt ttctcgaacg agatgaaccc cccacagcga catttcggac caactccaac 16080

acagtttctg aattcggttg tttttaggaa tttacaggcg gaagtaccat gcaaggatgg 16140

atttgtccag gagttccgtc cgttatggag agagaataca gaagaaagcg atctgacctc 16200

agataaagca gtgggatata tcacatctgt agtgccgtac aggtctgtat cattgctgca 16260

ttgtgacatt gaaatccctc caggatctaa tcaaagctta ctagatcagc tggctaccaa 16320

tctgtctctg attgccatgc attctgtgaa ggagggcggg gtcgtgatta tcaaagtact 16380

gtatgcaatg ggatattact tccatctact catgaacttg tttactccat gttccacgaa 16440

aggatatatt ctctctaatg gctatgcctg tagaggggat atggagtgtt acctgatatt 16500

tgtcatgggc tacctaggcg ggcctacatt tgtacatgag gtggtgagga tggcaaaaac 16560

gctagtacag cggcatggca cacttttgtc taagtcagat gagattacac tgactaggtt 16620

atttacttca cagcaacagc gtgtaacaga catcctatcc agccctttac cgagactaat 16680

gaagtacttg agagagaata ttgatactgc actgattgaa gccgggggac agcctgtccg 16740

tccattctgt gcagagagtt tagtgagcac actaacagac atgactcaga caacccagat 16800

catcgccagc cacattgata cagtcattcg atctgtgatc tatatggaag cagagggtga 16860

tcttgctgac acagtattct tatttacccc ttacaacctc tctatggacg ggaaaaagag 16920

aacatcactt aaacagtgca caagacagat cttagaggtc acaatactgg gtctcagagt 16980

caaaaatctc aataaagtag gtgatgtaat cagcctagta ctcagaggta tgatttctct 17040

ggaggacctt ctcccactga gaacctactt gaagtgtagt acctgcccta agtatttgaa 17100

ggctgtccta ggtattacca aactcaaaga aatgttcaca gacgcctctt tattatactt 17160

gactcgtgct caacaaaaat tctacatgaa aactataggc aatgcagtca agggatacta 17220

tagtaactgt gactcttaaa ggcaaccaca tatcaatagg ccctctttct agccgatcgt 17280

attcttgttg acttcattat accatattag aaaaaaattg aattccgacc ctttaagact 17340

cgtattcgga ttcaaataat tatctcagaa aaaaagtgca cgtagttgtt cttgattata 17400

gtcccgtcat tcaccaagtc tttgtttggt 17430

<210> 32
<211> 571
<212> PRT
<213> Newcastle disease virus

<220>
<223> HN protein

<400> 32
Met Asp Ser Ala Val Ser Gln Val Ala Leu Glu Asn Asp Arg Arg Glu
1               5                   10                  15
Ala Asn Asn Thr Trp Arg Leu Val Phe Arg Ile Ala Ala Leu Leu Leu
            20                  25                  30
Leu Val Ile Thr Leu Ala Val Ser Ala Val Ala Leu Ala Tyr Ser Met
        35                  40                  45
Glu Ala Ser Thr Pro Gly Asp Leu Val Ser Ile Pro Thr Ala Ile Ser
    50                  55                  60

Arg Ala Glu Glu Arg Ile Thr Ser Ala Leu Gly Ser Asn Gln Asp Val
65                  70                  75                  80
Val Asp Arg Ile Tyr Lys Gln Val Ala Leu Glu Ser Pro Leu Ala Leu
                85                  90                  95
Leu Asn Thr Glu Ser Ile Ile Met Asn Ala Ile Thr Ser Leu Ser Tyr
            100                 105                 110
Gln Ile Asn Gly Ala Thr Asn Asn Ser Gly Cys Gly Ala Pro Val His
        115                 120                 125
Asp Pro Asp Tyr Ile Gly Gly Ile Gly Gln Glu Leu Ile Val Asp Asp
    130                 135                 140
Thr Ser Asp Val Thr Ser Phe Tyr Pro Ser Ala Phe Gln Glu His Leu
145                 150                 155                 160
Asn Phe Ile Pro Ala Pro Thr Thr Gly Ser Gly Cys Thr Arg Ile Pro
                165                 170                 175
Ser Phe Asp Met Ser Ala Thr His Tyr Cys Tyr Thr His Asn Val Ile
            180                 185                 190
Leu Ser Gly Cys Arg Asp His Ser His Ser His Gln Tyr Leu Ala Leu
        195                 200                 205
Gly Val Leu Arg Thr Ser Ala Thr Gly Arg Val Phe Phe Ser Thr Leu
    210                 215                 220
Arg Ser Ile Asn Leu Asp Asp Ala Gln Asn Arg Lys Ser Cys Ser Val
225                 230                 235                 240
Ser Ala Thr Pro Leu Gly Cys Asp Met Leu Cys Ser Lys Ile Thr Glu
                245                 250                 255
Thr Glu Glu Glu Asp Tyr Lys Ser Val Ile Pro Thr Ser Met Val His
            260                 265                 270
Gly Arg Leu Gly Leu Asp Gly Gln Tyr His Glu Lys Asp Leu Asp Val
        275                 280                 285
Thr Thr Leu Phe Arg Asp Trp Val Ala Asn Tyr Pro Gly Val Gly Gly
    290                 295                 300
Gly Ser Phe Ile Asn Asn Arg Val Trp Phe Pro Val Tyr Gly Gly Leu
305                 310                 315                 320
Lys Pro Ser Ser Pro Ser Asp Thr Ala Gln Glu Gly Arg Tyr Val Ile
                325                 330                 335
Tyr Lys Arg Tyr Asn Asp Thr Cys Pro Asp Glu Gln Asp Tyr Gln Ile
            340                 345                 350
Arg Met Ala Lys Ser Ser Tyr Lys Pro Arg Arg Phe Gly Gly Lys Arg
        355                 360                 365
Val Gln Gln Ala Ile Leu Ser Ile Lys Val Ser Thr Ser Leu Gly Glu
    370                 375                 380
Asp Pro Val Leu Thr Val Pro Pro Asn Thr Val Ala Leu Met Gly Ala
385                 390                 395                 400
Glu Gly Arg Val Leu Thr Val Gly Thr Ser His Phe Leu Tyr Gln Arg
                405                 410                 415
Gly Ser Ser Tyr Phe Ser Pro Ala Leu Leu Tyr Pro Met Thr Val Asn
            420                 425                 430
Asn Lys Thr Ala Thr Leu His Asn Pro Tyr Thr Phe Asn Ala Phe Thr
        435                 440                 445
Arg Pro Gly Ser Val Pro Cys Gln Ala Ser Ala Arg Cys Pro Asn Ser
    450                 455                 460
Cys Val Thr Gly Val Tyr Thr Asp Pro Tyr Pro Leu Val Phe His Arg
465                 470                 475                 480
Asn His Thr Leu Arg Gly Val Phe Gly Thr Met Leu Asp Asp Glu Gln
                485                 490                 495
Ala Arg Leu Asn Pro Val Ser Ala Val Phe Asp Asn Ile Ser Arg Ser
            500                 505                 510
Arg Ile Thr Arg Val Ser Ser Ser Ser Thr Arg Ala Ala Tyr Thr Thr
        515                 520                 525
Ser Thr Cys Phe Lys Val Val Lys Thr Asn Lys Thr Tyr Cys Leu Ser
    530                 535                 540
Ile Ala Glu Ile Ser Asn Thr Leu Phe Gly Glu Phe Arg Ile Val Pro
545                 550                 555                 560
Leu Leu Val Glu Ile Leu Lys Asp Gly Gly Val

565                 570

<210> 33
<211> 553
<212> PRT
<213> Newcastle disease virus

<220>
<223> F protein (F3)

<400> 33
Met Gly Pro Arg Ser Ser Thr Arg Ile Pro Val Pro Leu Met Leu Thr
1               5                   10                  15
Ile Arg Ile Thr Leu Ala Leu Ser Tyr Val Arg Leu Thr Ser Ser Leu
            20                  25                  30
Asp Gly Arg Pro Leu Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Met Pro Lys Asp Lys Glu Ala Cys Ala Lys Ala Pro
65                  70                  75                  80
Leu Glu Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Ser Ile Gly Ala Ile Ile Gly Ser Val Ala Leu Gly
        115                 120                 125
Val Ala Thr Ala Ala Gln Ile Thr Ala Ala Ser Ala Leu Ile Gln Ala
    130                 135                 140
Asn Gln Asn Ala Ala Asn Ile Leu Arg Leu Lys Glu Ser Ile Ala Ala
145                 150                 155                 160
Thr Asn Glu Ala Val His Glu Val Thr Gly Gly Leu Ser Gln Leu Ala
                165                 170                 175
Val Ala Val Gly Lys Met Gln Gln Phe Val Asn Asp Gln Leu Asn Asn
            180                 185                 190
Thr Ala Gln Glu Leu Asp Cys Ile Lys Ile Thr Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Thr Gln Leu Thr Val Gln Ala Leu Tyr Asn
225                 230                 235                 240
Leu Ala Gly Gly Asn Val Asp Tyr Leu Leu Thr Lys Leu Gly Val Gly
                245                 250                 255
Asn Asn Gln Leu Ser Ser Leu Ile Gly Ser Gly Leu Ile Thr Gly Asn
            260                 265                 270
Pro Ile Phe Tyr Asp Ser Gln Thr Gln Leu Leu Gly Ile Gln Val Thr
        275                 280                 285
Ala Pro Ser Val Gly Asn Leu Asn Asn Met Arg Ala Thr Tyr Leu Glu
    290                 295                 300
Thr Leu Ser Val Ser Thr Thr Lys Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Ala Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Arg Thr Glu Gly Ala Leu Thr Thr Pro Tyr Met
    370                 375                 380
Thr Leu Lys Gly Ser Val Val Ala Asn Cys Gln Met Thr Thr Cys Arg

385                 390                 395                 400
Cys Ala Asp Pro Pro Gly Ile Ile Ser Gln Asn Tyr Gly Glu Ala Val
                405                 410                 415
Ser Leu Ile Asp Lys His Ser Cys Asn Val Val Ser Leu Asp Gly Ile
            420                 425                 430
Thr Leu Arg Leu Ser Gly Glu Phe Asp Ala Thr Tyr Gln Lys Asn Ile
        435                 440                 445
Ser Ile Leu Asp Ser Gln Val Leu Val Thr Gly Asn Leu Asp Ile Ser
    450                 455                 460
Thr Glu Leu Gly Asn Val Asn His Ser Ile Ser Asn Ala Leu Asp Lys
465                 470                 475                 480
Leu Glu Glu Ser Asn Ser Lys Leu Asp Lys Val Asn Val Lys Leu Thr
                485                 490                 495
Ser Thr Ser Ala Leu Ile Thr Tyr Ile Val Leu Thr Val Ile Ser Leu
            500                 505                 510
Val Leu Gly Met Leu Ser Leu Val Leu Ala Cys Tyr Leu Met Tyr Lys
        515                 520                 525
Gln Lys Ala Gln Arg Lys Thr Leu Leu Trp Leu Gly Asn Asn Thr Leu
    530                 535                 540
Asp Gln Met Arg Ala Thr Thr Lys Met
545                 550

<210> 34
<211> 364
<212> PRT
<213> Newcastle disease virus

<220>
<223> M protein

<400> 34
Met Asp Ser Ser Arg Thr Ile Gly Leu Tyr Phe Asp Ser Ala Leu Pro
1               5                   10                  15
Ser Ser Asn Leu Leu Ala Phe Pro Ile Val Leu Arg Asp Val Gly Asp
            20                  25                  30
Gly Lys Lys Gln Ile Thr Pro Gln Tyr Arg Ile Arg Arg Leu Asp Ser
        35                  40                  45
Trp Thr Asp Ser Lys Glu Asp Ser Val Phe Ile Thr Thr Tyr Gly Phe
    50                  55                  60
Ile Phe Gln Val Gly Asn Glu Glu Val Thr Val Gly Met Ile Asn Asp
65                  70                  75                  80
Asn Pro Lys Arg Glu Leu Leu Ser Ala Ala Met Leu Cys Leu Gly Ser
                85                  90                  95
Val Pro Asn Val Gly Asp Pro Val Glu Leu Ala Arg Ala Cys Leu Thr
            100                 105                 110
Met Val Val Thr Cys Lys Lys Ser Ala Thr Asn Thr Glu Arg Met Val
        115                 120                 125
Phe Ser Val Val Gln Ala Pro Arg Val Leu Gln Ser Cys Arg Val Val
    130                 135                 140
Ala Asp Lys Tyr Ser Ser Val Asn Ala Val Lys His Val Lys Ala Pro
145                 150                 155                 160
Glu Lys Ile Pro Trp Ser Glu Thr Leu Glu Tyr Lys Val Asn Phe Val
                165                 170                 175
Ser Leu Thr Val Val Pro Lys Lys Asp Val Tyr Lys Ile Pro Thr Ala
            180                 185                 190
Val Leu Lys Val Ser Gly Ser Ser Leu Tyr Asn Leu Ala Leu Asn Val
        195                 200                 205
Thr Ile Asp Val Glu Val Asp Pro Lys Ser Pro Leu Val Lys Ser Leu
    210                 215                 220
Ser Arg Ser Asp Ser Gly Tyr Tyr Ala Asn Leu Phe Leu His Ile Gly
225                 230                 235                 240

Leu Met Ser Thr Val Asp Lys Arg Gly Lys Lys Val Thr Phe Asp Gln
                245                 250                 255
Leu Glu Arg Lys Ile Arg Arg Leu Asp Leu Ser Val Gly Leu Ser Asp
            260                 265                 270
Val Leu Gly Pro Ser Val Leu Val Lys Ala Arg Gly Ala Arg Thr Arg
        275                 280                 285
Leu Leu Ala Pro Phe Phe Ser Asn Ser Gly Thr Ala Cys Tyr Pro Ile
    290                 295                 300
Ala Asn Ala Ser Pro Gln Val Ala Lys Ile Leu Trp Ser Gln Thr Ala
305                 310                 315                 320
Cys Leu Arg Ser Val Lys Ile Ile Ile Gln Ala Gly Thr Gln Arg Ala
                325                 330                 335
Val Ala Val Thr Ala Asp His Glu Val Thr Ser Thr Lys Ile Glu Lys
            340                 345                 350
Arg His Thr Ile Ala Lys Tyr Asn Pro Phe Lys Lys
        355                 360

<210> 35
<211> 2204
<212> PRT
<213> Newcastle disease virus

<220>
<223> L protein

<220>
<221> misc_feature
<222> (1717)..(1717)
<223> Xaa can be Tyr or His

<220>
<221> misc_feature
<222> (1910)..(1910)
<223> Xaa can be Glu or Lys

<400> 35
Met Ala Gly Ser Gly Pro Glu Arg Ala Glu His Gln Ile Ile Leu Pro
1               5                   10                  15
Glu Ser His Leu Ser Ser Pro Leu Val Lys His Lys Leu Leu Tyr Tyr
            20                  25                  30
Trp Lys Leu Thr Gly Leu Pro Leu Pro Asp Glu Cys Asp Phe Asp His
        35                  40                  45
Leu Ile Ile Ser Arg Gln Trp Lys Lys Val Leu Glu Ser Ala Thr Pro
    50                  55                  60
Asp Ile Glu Arg Met Ile Lys Leu Gly Arg Ala Val His Gln Thr Leu
65                  70                  75                  80
Asn His Asn Ser Arg Ile Thr Gly Val Leu His Pro Arg Cys Leu Glu
                85                  90                  95
Glu Leu Ala Ser Ile Glu Val Pro Asp Ser Thr Asn Lys Phe Arg Lys
            100                 105                 110
Ile Glu Lys Lys Ile Gln Ile His Asn Thr Arg Tyr Gly Glu Leu Phe
        115                 120                 125
Thr Arg Leu Cys Thr His Val Glu Lys Lys Leu Leu Gly Ser Ser Trp
    130                 135                 140
Ser Asn Asn Val Pro Arg Ser Glu Glu Phe Asn Ser Ile Arg Thr Asp
145                 150                 155                 160
Pro Ala Phe Trp Phe His Ser Lys Trp Ser Thr Ala Lys Phe Ala Trp
                165                 170                 175
Leu His Ile Lys Gln Val Gln Arg His Leu Ile Val Ala Ala Arg Thr

180                 185                 190
Arg Ser Ala Val Asn Lys Leu Val Thr Leu Thr His Lys Val Gly Gln
        195                 200                 205
Ile Phe Val Thr Pro Glu Leu Val Ile Val Thr His Thr Asp Glu Asn
    210                 215                 220
Lys Phe Thr Cys Leu Thr Gln Glu Leu Val Leu Met Tyr Ala Asp Met
225                 230                 235                 240
Met Glu Gly Arg Asp Met Val Ser Ile Ile Ser Ser Thr Ala Ala His
                245                 250                 255
Leu Arg Ser Leu Ser Glu Lys Ile Asp Asp Ile Leu Arg Leu Val Asp
            260                 265                 270
Ala Leu Ala Arg Asp Leu Gly Asn Gln Val Tyr Asp Val Val Ala Leu
        275                 280                 285
Met Glu Gly Phe Ala Tyr Gly Ala Val Gln Leu Leu Glu Pro Ser Gly
    290                 295                 300
Thr Phe Ala Gly Asp Phe Phe Ala Phe Asn Leu Gln Glu Leu Lys Asp
305                 310                 315                 320
Thr Leu Thr Gly Leu Leu Pro Lys Asp Ile Ala Glu Ser Val Thr His
                325                 330                 335
Ala Ile Ala Thr Ile Phe Ser Gly Leu Glu Gln Asn Gln Ala Ala Glu
            340                 345                 350
Met Leu Cys Leu Leu Arg Leu Trp Gly His Pro Leu Leu Glu Ser Arg
        355                 360                 365
Ile Ala Ala Lys Ala Val Arg Ser Gln Met Cys Ala Pro Lys Met Val
    370                 375                 380
Asp Phe Asp Met Ile Leu Gln Val Leu Ser Phe Phe Lys Gly Thr Ile
385                 390                 395                 400
Ile Asn Gly Tyr Arg Lys Lys Asn Ala Gly Val Trp Pro Arg Val Lys
                405                 410                 415
Ile Asp Thr Ile Tyr Gly Lys Val Ile Gly Gln Leu His Ala Asp Ser
            420                 425                 430
Ala Glu Ile Ser His Asp Val Met Leu Arg Glu Tyr Lys Ser Leu Ser
        435                 440                 445
Ala Leu Glu Phe Glu Pro Cys Ile Glu Tyr Asp Pro Val Thr Asn Leu
    450                 455                 460
Ser Met Phe Leu Lys Asp Lys Ala Ile Ala His Pro Lys Asp Asn Trp
465                 470                 475                 480
Leu Ala Ser Phe Arg Arg Asn Leu Leu Ser Glu Asp Gln Lys Lys His
                485                 490                 495
Val Arg Glu Ala Thr Ser Thr Asn Arg Leu Leu Ile Glu Phe Leu Glu
            500                 505                 510
Ser Asn Asp Phe Asp Pro Tyr Lys Glu Met Glu Tyr Leu Thr Thr Leu
        515                 520                 525
Glu Tyr Leu Arg Asp Asp Asn Val Ala Val Ser Tyr Ser Leu Lys Glu
    530                 535                 540
Lys Glu Val Lys Val Asn Gly Arg Ile Phe Ala Lys Leu Thr Asn Arg
545                 550                 555                 560
Leu Arg Asn Cys Gln Val Met Ala Glu Gly Ile Leu Ala Asp Gln Ile
                565                 570                 575
Ala Pro Phe Phe Gln Gly Asn Gly Val Ile Gln Asp Ser Ile Ser Leu
            580                 585                 590
Thr Lys Ser Met Leu Ala Met Ser Gln Leu Ser Phe Asn Ser Asn Lys
        595                 600                 605
Lys Arg Ile Thr Asp Cys Lys Glu Arg Val Ser Ser Asn Arg Asn His
    610                 615                 620
Asp Pro Lys Ser Lys Asn Arg Arg Arg Val Ala Thr Phe Val Thr Thr
625                 630                 635                 640
Asp Leu Gln Lys Tyr Cys Leu Asn Trp Arg Tyr Gln Thr Val Lys Leu
                645                 650                 655
Phe Ala His Ala Ile Asn Gln Leu Met Gly Leu Pro His Phe Phe Glu
            660                 665                 670
Trp Ile His Leu Arg Leu Met Asp Thr Thr Met Phe Val Gly Asp Pro
        675                 680                 685

Phe Asn Pro Pro Ser Asp Pro Thr Asp Cys Asp Leu Ser Arg Val Pro
    690                 695                 700
Asn Asp Asp Ile Tyr Ile Val Ser Ala Arg Gly Gly Ile Glu Gly Leu
705                 710                 715                 720
Cys Gln Lys Leu Trp Thr Met Ile Ser Ile Ala Ala Ile Gln Leu Ala
                725                 730                 735
Ala Ala Arg Ser His Cys Arg Val Ala Cys Met Val Gln Gly Asp Asn
            740                 745                 750
Gln Val Ile Ala Ile Thr Arg Glu Val Arg Ser Asp Asp Ser Pro Glu
        755                 760                 765
Met Val Leu Thr Gln Leu His Gln Ala Ser Asp Asn Phe Phe Lys Glu
    770                 775                 780
Leu Ile His Val Asn His Leu Ile Gly His Asn Leu Lys Asp Arg Glu
785                 790                 795                 800
Thr Ile Arg Ser Asp Thr Phe Phe Ile Tyr Ser Lys Arg Ile Phe Lys
                805                 810                 815
Asp Gly Ala Ile Leu Ser Gln Val Leu Lys Asn Ser Ser Lys Leu Val
            820                 825                 830
Leu Ile Ser Gly Asp Leu Ser Glu Asn Thr Val Met Ser Cys Ala Asn
        835                 840                 845
Ile Ala Ser Thr Val Ala Arg Leu Cys Glu Asn Gly Leu Pro Lys Asp
    850                 855                 860
Phe Cys Tyr Tyr Leu Asn Tyr Leu Met Ser Cys Val Gln Thr Tyr Phe
865                 870                 875                 880
Asp Ser Glu Phe Ser Ile Thr Asn Asn Ser Gln Leu Asp Ser Asn Gln
                885                 890                 895
Ser Trp Ile Glu Asp Ile Ser Phe Val His Ser Tyr Val Leu Thr Pro
            900                 905                 910
Ala Gln Leu Gly Gly Leu Ser Asn Leu Gln Tyr Ser Arg Leu Tyr Thr
        915                 920                 925
Arg Asn Ile Gly Asp Pro Gly Thr Thr Ala Phe Ala Glu Ile Lys Arg
    930                 935                 940
Leu Glu Ala Val Gly Leu Leu Ser Pro Ser Ile Met Thr Asn Ile Leu
945                 950                 955                 960
Thr Arg Pro Pro Gly Asn Gly Asp Trp Ala Ser Leu Cys Asn Asp Pro
                965                 970                 975
Tyr Ser Phe Asn Phe Glu Thr Val Ala Ser Pro Asn Ile Val Leu Lys
            980                 985                 990
Lys His Thr Gln Arg Val Leu Phe Glu Thr Cys Ser Asn Pro Leu Leu
        995                 1000                1005
Ser Gly Val His Thr Glu Asp Asn Glu Ala Glu Glu Lys Ala Leu Ala
    1010                1015                1020
Glu Phe Leu Leu Asn Gln Glu Met Ile His Pro Arg Val Ala His Ala
1025                1030                1035                1040
Ile Met Glu Ala Ser Ser Val Gly Arg Arg Lys Gln Ile Gln Gly Leu
                1045                1050                1055
Val Asp Thr Thr Asn Thr Val Ile Lys Ile Ala Leu Thr Arg Arg Pro
            1060                1065                1070
Leu Gly Ile Lys Arg Leu Met Arg Ile Val Asn Tyr Ser Ser Met His
        1075                1080                1085
Ala Met Leu Phe Arg Asp Asp Val Phe Ser Ser Asn Arg Ser Asn His
    1090                1095                1100
Pro Leu Val Ser Ser Asp Met Cys Ser Gln Thr Leu Ala Asp Tyr Ala
1105                1110                1115                1120
Arg Asn Arg Ser Trp Ser Pro Leu Thr Gly Gly Arg Lys Ile Leu Gly
                1125                1130                1135
Val Ser Asn Pro Asp Thr Ile Glu Leu Val Glu Gly Glu Ile Leu Ser
            1140                1145                1150
Val Ser Gly Gly Cys Thr Arg Cys Asp Ser Gly Asp Glu Gln Phe Thr
        1155                1160                1165
Trp Phe His Leu Pro Ser Asn Ile Glu Leu Asn Asp Asp Thr Ser Lys
    1170                1175                1180
Asn Pro Pro Met Arg Val Pro Tyr Leu Gly Ser Lys Thr Gln Glu Arg

1185                1190                1195                1200
Arg Ala Ala Ser Leu Ala Lys Ile Ala His Met Ser Pro His Val Lys
                1205                1210                1215
Ala Ala Leu Arg Ala Ser Ser Val Leu Ile Trp Ala Tyr Gly Asp Asn
            1220                1225                1230
Glu Ile Asn Trp Thr Ala Ala Leu Lys Ile Ala Arg Ser Arg Cys Asn
        1235                1240                1245
Ile Ser Ser Glu Tyr Leu Arg Leu Leu Ser Pro Leu Pro Thr Ala Gly
    1250                1255                1260
Asn Leu Gln His Arg Leu Asp Asp Gly Ile Thr Gln Met Thr Phe Thr
1265                1270                1275                1280
Pro Ala Ser Leu Tyr Arg Val Ser Pro Tyr Ile His Ile Ser Asn Asp
                1285                1290                1295
Ser Gln Arg Leu Phe Thr Glu Glu Gly Val Lys Glu Gly Asn Val Val
            1300                1305                1310
Tyr Gln Gln Ile Met Leu Leu Gly Leu Ser Leu Ile Glu Ser Leu Phe
        1315                1320                1325
Pro Met Thr Thr Thr Lys Thr Tyr Asp Glu Ile Thr Leu His Leu His
    1330                1335                1340
Ser Lys Phe Ser Cys Cys Ile Arg Glu Ala Pro Val Ala Val Pro Phe
1345                1350                1355                1360
Glu Leu Leu Gly Val Ala Pro Glu Leu Arg Ala Val Thr Ser Asn Lys
                1365                1370                1375
Phe Met Tyr Asp Pro Ser Pro Val Ser Glu Gly Asp Phe Ala Arg Leu
            1380                1385                1390
Asp Leu Ala Ile Phe Lys Ser Tyr Glu Leu Asn Leu Glu Ser Tyr Ser
        1395                1400                1405
Thr Ile Glu Leu Met Asn Val Leu Ser Ile Ser Ser Gly Lys Leu Ile
    1410                1415                1420
Gly Gln Ser Val Val Ser Tyr Asp Glu Asp Thr Ser Ile Lys Asn Asp
1425                1430                1435                1440
Ala Ile Ile Val Tyr Asp Asn Thr Arg Asn Trp Ile Ser Glu Ala Gln
                1445                1450                1455
Asn Ser Asp Val Val Arg Leu Phe Glu Tyr Ala Ala Leu Glu Val Leu
            1460                1465                1470
Leu Asp Cys Ser Tyr Gln Leu Tyr Tyr Leu Arg Val Arg Gly Leu Asn
        1475                1480                1485
Asn Ile Val Leu Tyr Met Ser Asp Leu Tyr Lys Asn Met Pro Gly Ile
    1490                1495                1500
Leu Leu Ser Asn Ile Ala Ala Thr Ile Ser His Pro Val Ile His Ser
1505                1510                1515                1520
Arg Leu Asn Ala Val Gly Leu Val Asn His Asp Gly Ser His Gln Leu
                1525                1530                1535
Ala Asp Thr Asp Phe Ile Glu Met Ser Ala Lys Leu Leu Val Ser Cys
            1540                1545                1550
Thr Arg Arg Val Val Ser Gly Leu His Ala Gly Asn Lys Tyr Asp Leu
        1555                1560                1565
Leu Phe Pro Ser Val Leu Asp Asp Asn Leu Ser Glu Lys Met Leu Gln
    1570                1575                1580
Leu Ile Ser Gln Leu Cys Cys Leu Tyr Thr Val Leu Phe Ala Thr Thr
1585                1590                1595                1600
Arg Glu Ile Pro Lys Ile Arg Gly Leu Ser Ala Glu Glu Lys Cys Ser
                1605                1610                1615
Val Leu Thr Glu Tyr Leu Leu Ser Asp Ala Val Lys Pro Leu Leu Gly
            1620                1625                1630
Ser Glu Gln Val Ser Ser Ile Met Ser Pro Asn Ile Val Thr Phe Pro
        1635                1640                1645
Ala Asn Leu Tyr Tyr Met Ser Arg Lys Ser Leu Asn Leu Ile Arg Glu
    1650                1655                1660
Arg Glu Asp Arg Asp Thr Ile Leu Ala Leu Leu Phe Pro Gln Glu Pro
1665                1670                1675                1680
Leu Leu Glu Phe Pro Leu Val Arg Asp Ile Gly Ala Arg Val Lys Asp
                1685                1690                1695

Pro Phe Thr Leu Gln Pro Ala Ala Phe Leu Gln Glu Leu Asp Leu Ser
            1700                1705                1710
Ala Pro Ala Arg Xaa Asp Ala Phe Thr Ile Ser Gln Ala His Ser Glu
        1715                1720                1725
His Ile Leu Pro Asn Pro Glu Glu Asp His Leu Val Arg Tyr Leu Phe
    1730                1735                1740
Arg Gly Ile Gly Thr Ala Ser Ser Ser Trp Tyr Lys Ala Ser His Leu
1745                1750                1755                1760
Leu Ser Val Pro Glu Val Arg Cys Ala Arg His Gly Asn Ser Leu Tyr
                1765                1770                1775
Leu Ala Glu Gly Ser Gly Ala Ile Met Ser Leu Leu Glu Leu His Ile
            1780                1785                1790
Pro His Glu Thr Ile Tyr Tyr Asn Thr Leu Phe Ser Asn Glu Met Asn
        1795                1800                1805
Pro Pro Gln Arg His Phe Gly Pro Thr Pro Thr Gln Phe Leu Asn Ser
    1810                1815                1820
Val Val Phe Arg Asn Leu Gln Ala Glu Val Pro Cys Lys Asp Gly Phe
1825                1830                1835                1840
Val Gln Glu Phe Arg Pro Leu Trp Arg Glu Asn Thr Glu Glu Ser Asp
                1845                1850                1855
Leu Thr Ser Asp Lys Ala Val Gly Tyr Ile Thr Ser Val Val Pro Tyr
            1860                1865                1870
Arg Ser Val Ser Leu Leu His Cys Asp Ile Glu Ile Pro Pro Gly Ser
        1875                1880                1885
Asn Gln Ser Leu Leu Asp Gln Leu Ala Thr Asn Leu Ser Leu Ile Ala
    1890                1895                1900
Met His Ser Val Lys Xaa Gly Gly Val Val Ile Ile Lys Val Leu Tyr
1905                1910                1915                1920
Ala Met Gly Tyr Tyr Phe His Leu Leu Met Asn Leu Phe Thr Pro Cys
                1925                1930                1935
Ser Thr Lys Gly Tyr Ile Leu Ser Asn Gly Tyr Ala Cys Arg Gly Asp
            1940                1945                1950
Met Glu Cys Tyr Leu Ile Phe Val Met Gly Tyr Leu Gly Gly Pro Thr
        1955                1960                1965
Phe Val His Glu Val Val Arg Met Ala Lys Thr Leu Val Gln Arg His
    1970                1975                1980
Gly Thr Leu Leu Ser Lys Ser Asp Glu Ile Thr Leu Thr Arg Leu Phe
1985                1990                1995                2000
Thr Ser Gln Gln Gln Arg Val Thr Asp Ile Leu Ser Ser Pro Leu Pro
                2005                2010                2015
Arg Leu Met Lys Tyr Leu Arg Glu Asn Ile Asp Thr Ala Leu Ile Glu
            2020                2025                2030
Ala Gly Gly Gln Pro Val Arg Pro Phe Cys Ala Glu Ser Leu Val Ser
        2035                2040                2045
Thr Leu Thr Asp Met Thr Gln Thr Thr Gln Ile Ile Ala Ser His Ile
    2050                2055                2060
Asp Thr Val Ile Arg Ser Val Ile Tyr Met Glu Ala Glu Gly Asp Leu
2065                2070                2075                2080
Ala Asp Thr Val Phe Leu Phe Thr Pro Tyr Asn Leu Ser Met Asp Gly
                2085                2090                2095
Lys Lys Arg Thr Ser Leu Lys Gln Cys Thr Arg Gln Ile Leu Glu Val
            2100                2105                2110
Thr Ile Leu Gly Leu Arg Val Lys Asn Leu Asn Lys Val Gly Asp Val
        2115                2120                2125
Ile Ser Leu Val Leu Arg Gly Met Ile Ser Leu Glu Asp Leu Leu Pro
    2130                2135                2140
Leu Arg Thr Tyr Leu Lys Cys Ser Thr Cys Pro Lys Tyr Leu Lys Ala
2145                2150                2155                2160
Val Leu Gly Ile Thr Lys Leu Lys Glu Met Phe Thr Asp Ala Ser Leu
                2165                2170                2175
Leu Tyr Leu Thr Arg Ala Gln Gln Lys Phe Tyr Met Lys Thr Ile Gly
            2180                2185                2190
Asn Ala Val Lys Gly Tyr Tyr Ser Asn Cys Asp Ser

2195                2200

<210> 36
<211> 15186
<212> DNA
<213> Newcastle disease virus

<220>
<223> Mut HN
      (F277L)/M(G165W)/F(F117S)/F(F190L)/L(V757I)/L(F1551S)/L(R1700L)

<400> 36
accaaacaga gaatccgtga gatacgataa aaggcgaagg agcaatcgaa gtcgtacggg      60

tagaaggtgt gaatctcgag tgcgagaccg aagcttaaac tcgagagagc cttctgccaa     120

catgtcttcc gtatttgacg agtatgaaca gctcctcgcg gctcagactc gccctaatgg     180

agctcacgga ggaggagaga aggggagcac cttaaaagtt gaagtcccgg tattcactct     240

taacagtgac gacccagaag atagatggaa ctttgcagta ttctgtctta ggattgctgt     300

tagcgaagat gccaacaaac cactcagaca aggtgctctc atatctctct tatgctccca     360

ttctcaagtg atgaggaacc atgtcgccct tgcagggaaa cagaatgagg ccacactggc     420

tgttcttgag atcgatggtt tcaccaacag cgtgcctcaa tttaacaaca ggagtggagt     480

gtctgaggag agagcacaga gattcttgat gatagcaggg tccctccctc gggcatgcag     540

caacggtacc ccgttcatca cagctggggt tgaagatgat gcaccagaag acatcactga     600

tactctggaa agaatcatct ctatccaggc tcaagtatgg gtcacggtag caaaggccat     660

gactgcatat gagacagcgg atgagtcgga aacaagaaga atcaataagt atatgcagca     720

aggcagagtc caaaagaagt acatcctcca ccccgtatgt aggagcgcaa ttcaactcac     780

gattagacaa tctctggcag tccgcatttt cttggttagc gagcttaaga gaggccgcaa     840

cacggcaggt ggaagttcca cttattatag cttggtaggg gacatagact catacatcag     900

gaacaccggg cttactgcat tcttcctgac actcaggtat ggaattaaca ccaagacatc     960

agcccttgca ctcagcagcc tcgcaggcga tatccaaaaa atgaagcagc tcatgcgtct    1020

atatcggatg aaaggagata atgcgccgta catgacactg ctcggtgaca gtgaccagat    1080

gagctttgca ccggctgagt atgcacaact ctactctttt gcaatgggta tggcatccgt    1140

cctagacaaa ggaactggca agtaccagtt cgccagagac tttatgagca catcattctg    1200

gagacttgga gtagagtatg ctcgggctca gggaagcagc atcaatgagg atatggctgc    1260

cgagctaaag ctgaccccag cggcaaggag gggcctggca gctgctgccc aacgagcgtc    1320

cgaggagacc ggcagcatgg acattcccac tcaacaagct ggggtcctca caggcctcag     1380

cgacggaggc cctcaagccc cacaaggtgg actgaacagg tcgcaagggc aaccggacgc    1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

ttggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620

ccgtctgaca agttctcttg atggcaggcc tcttgcagcc gcagggatcg tggtaacagg 4680

ggataaagca gttaacatat acacctcatc ccagacaggg tcaatcatag tcaagttact 4740

cccaaatatg cccaaggaca aagaggcatg tgcaaaagcc ccattggagg cttacaacag 4800

gacactgact actttgctta ccccccttgg tgattctatc cgcaggatac aagagtctgt 4860

gactacatcc ggaggaagga gacagagacg ctctataggt gccattattg gcagtgtagc 4920

tctaggggtt gcaacagctg cacagataac ggcagcctcg gctctgatac aagccaacca 4980

gaatgctgct aacatcctcc ggcttaagga gagcattgct gcaaccaatg aagctgtaca 5040

cgaggtcact ggcggattgt cacagttagc agtggcagtt ggaaagatgc agcaatttgt 5100

taatgaccag cttaataaca cagctcaaga gttggactgt ataaaaatta cacagcaggt 5160

tggtgtagaa ctcaacttat acttaactga attgactaca gtgttcggcc cacaaatcac 5220

ttcccctgcc ttaactcagt tgactgttca ggctctttac aatctggctg gtggtaatgt 5280

agattacttg ttgactaagt taggtgtagg gaacaaccag ctcagctcat tgattggtag 5340

cggcttgatc accggtaacc ctatttttta cgactcacag actcaactct tgggcataca 5400

ggtgacttta ccctcagtcg ggaacctaaa taatatgcgt gccacctact tggagacctt 5460

gtctgtaagc acaacaaagg gatttgcctc agcacttgtc ccaaaagtag tgacacaggt 5520

cggttctgtg atagaagagc ttgacacctc atactgtata gaagctgatt tggatttata 5580

ttgtacaaga atagtgacat tccctatgtc ccctggtatt tattcctgtt tgagcggcaa 5640

tacatcggct tgcatgtatt caaggactga aggcgcactt actacaccat acatgactct 5700

caaaggctca gttgttgcca attgccagat gacaacatgt agatgtgcag acccccgggg 5760

tatcatatca caaaattatg gagaggctgt gtctctaata gataagcact catgcaatgt 5820

cgtatcctta gacgggataa ctttgaggct cagtggggaa tttgatgcaa cttatcaaaa 5880

gaatatctca atattagatt ctcaagtact agtgacaggc aatctcgata tctcaactga 5940

gcttgggaat gtcaaccact caataagtaa tgctttggat aagttagagg aaagcaacag 6000

caaactagac aaagtcaatg tcaaactgac cagcacatct gctctcatta cctatattgt 6060

tttaactgtc atatctcttg ttcttggtat gcttagcctg gttctagcat gctatctgat 6120

gtacaagcaa aaagcgcaac gaaagacctt gttgtggctt ggaaataata ccctagatca 6180

gatgagagct actacaaaaa tgtgaatgca gatgagaggc agaggtatcc ccaatagcaa 6240

tctgtgtgtc aattctggca gcctgttaat cagaagaatt aagaaaaaac taccggatgt 6300

aggtgaacaa aagggaatat acgggtagaa cggcctgaga ggccacccct caatcgggag 6360

ccaggcccca ctacgtccgc tctaccgcat caccaacagc agtcttcagt catggacagc 6420

gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020

cagtatttag cacttggtgt gcttcggaca tctgcaacag ggagggtatt cttttctact 7080

ctgcgttcca tcaacttgga tgacgcccaa aatcggaagt cttgcagtgt gagtgcaact 7140

cctttaggtt gtgatatgct gtgctctaaa atcacagaga ctgaggaaga ggattataaa 7200

tcagttatcc ccacatcgat ggtacatgga aggttagggc ttgacggcca ataccatgag 7260

aaggacctag acgtcacaac actatttagg gactgggtgg caaattaccc aggagtagga 7320

ggtgggtctt ttattaacaa tcgcgtatgg ttcccggtct acggagggct aaaacccagc 7380

tcgcctagtg acactgcaca agaagggaga tacgtaatat acaagcgata caatgacaca 7440

tgcccggatg agcaagatta ccagattcgg atggctaagt catcgtataa gcctaggcgg 7500

tttggtggaa aacgcgtaca gcaggccatc ctatccatca aggtgtcaac atccttgggt 7560

gaggacccgg tgttgactgt accgcctaat acggtcgcac tcatgggggc cgaaggcaga 7620

gttctcacag tggggacatc tcatttctta tatcagcgag ggtcatcata cttctctccc 7680

gctttgttat accctatgac agtcaacaac aaaacagcca ctcttcataa tccttataca 7740

ttcaatgctt tcactcggcc aggtagtgtc ccttgccagg cttcagcaag atgccccaac 7800

tcatgtgtta ccggagtcta tactgatcca tatcccttag tcttccatag gaaccacacc 7860

ttgcgagggg tattcggaac aatgcttgat gacgaacaag caagactcaa ccctgtatct 7920

gcagtatttg ataacatatc ccgcagtcgc ataactcggg taagttcaag cagtaccagg 7980

gcagcataca cgacatcaac gtgttttaaa gttgtcaaga ccaataaaac ctattgcctc 8040

agcattgcag aaatatccaa taccctcttc ggggaattca gaattgtccc cttactagtt 8100

gagattctca aggatggtgg ggtttaagaa gctaggaatg gtcggttgag tcaaccgtga 8160

gaaggcgggg aagatgatat tgcatcatct atcttctgta acaccaagaa tcaaatcgaa 8220

taccagtgcg agctcgaatc ctacgctgcc agtcagccat aatcggctgg tgctaatgtg 8280

gttagcctga atcttgtcga tagtcacttg attaagaaaa aatgtggacg gtagtgaaat 8340

ataaggcaaa gcagctcaca gaggacagca cgggtaggac atggcgggct ccggacccga 8400

aagggcagag catcagatta tcctaccaga gtcacatctg tcctcaccat tggtcaagca 8460

taaattgctt tattactgga aattaactgg gctaccgctt cctgacgaat gtgacttcga 8520

ccatcttatt atcagccgac aatggaagaa ggtacttgaa tcggccaccc ctgacattga 8580

gagaatgata aaactagggc gggcagtaca ccagactctc aaccacaatt ccaggataac 8640

cggagtactc catccccggt gtttagagga actggctagt attgaggttc ctgattcaac 8700

caacaaattt cggaagatcg aaaaaaagat ccagattcac aacacaaggt atggagaact 8760

gttcacaaga ttatgcacgc atgtagaaaa gaaactattg gggtcatctt ggtctaacaa 8820

tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacgggaagg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgaggattta tgccagaagc tatggacaat 10560

gatctcaatt gctgcaatcc agcttgctgc agcaagatca cattgtcgcg ttgcctgtat 10620

ggtacaaggc gacaatcaag taatagctat aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800

cttcatatac agcaaacgaa tattcaaaga tggagcaata ctcagtcagg tcctcaaaaa 10860

ctcatctaaa ttagtgctaa tatcaggcga ccttagtgaa aacactgtaa tgtcttgtgc 10920

caacattgca tctactgtag cacggttatg cgagaacggg cttcctaagg atttctgtta 10980

ttacttaaac tacttaatga gttgcgtgca gacatacttc gattccgaat tttccatcac 11040

caacaactcg caactcgatt ctaaccagtc gtggatagag gacatttctt ttgtgcactc 11100

atatgtcctg acccctgctc aactgggggg actgagtaac cttcaatact caaggctcta 11160

cacaaggaac atcggcgacc cgggaaccac tgctttcgca gagatcaaga gattagaggc 11220

agtggggtta ctgagtccta gcattatgac taacatctta actaggccgc ctggaaatgg 11280

agattgggcc agtctgtgca acgatccata ctcctttaat tttgagactg tcgcaagccc 11340

aaatattgtc cttaagaaac atacacaaag agtcctattt gaaacttgct cgaatccctt 11400

attatctgga gtgcacacag aggataatga ggcagaagag aaggcattgg ctgaattctt 11460

gctcaatcaa gaaatgattc atccacgtgt cgcgcatgct atcatggaag caagctctgt 11520

aggtaggaga aagcaaattc aggggcttgt tgacacaaca aacaccgtga ttaagattgc 11580

actgactagg aggccactcg gcatcaagag gctgatgcgg atagtcaatt actcgagcat 11640

gcatgcaatg ctatttagag atgatgtttt ctcgtctaat agatccaacc atcccttagt 11700

ctcttctgat atgtgttctc agacactggc agactatgca cggaacagaa gctggtcacc 11760

tttgacaggg ggtagaaaaa tactgggtgt atctaatcct gataccatag aacttgtaga 11820

gggtgagatc cttagtgtca gtggagggtg cactagatgt gacagtgggg atgaacagtt 11880

tacttggttc catcttccaa gcaatataga gctaaacgat gacaccagca agaatcctcc 11940

aatgagagtg ccatatctcg ggtcaaagac tcaagagagg agagccgcct cgcttgcgaa 12000

aatagctcat atgtcaccac acgtgaaggc ggctctaagg gcgtcatctg tgttaatctg 12060

ggcttatggg gacaacgaaa taaactggac tgctgccctt aagattgcaa ggtctcggtg 12120

caacataagc tcagagtatc ttcgactatt gtcacccttg cctacagctg ggaatctcca 12180

acatagattg gacgacggca taactcagat gacattcacc cctgcatctc tatacagggt 12240

gtcaccttac attcatatat ctaatgattc tcaaaggcta tttactgaag aaggagtcaa 12300

agaggggaat gtggtttatc agcaaatcat gctcttgggt ttgtccttaa ttgagtcact 12360

cttcccaatg acaacaacca agacatatga tgaaatcaca ttgcacctcc acagtaaatt 12420

tagctgctgt atcagggaag cacctgttgc agttcctttc gagctactcg gggtggcacc 12480

ggaactaagg gcagtaacct caaataagtt tatgtatgat cctagccctg tatcagaggg 12540

agactttgcg agacttgact tagctatctt taagagttat gaacttaatt tagagtcata 12600

ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

caggatactt atcttggcat tgttgttccc tcaagaaccg ctgctcgagt ttcctctggt 13440

acgagatatt ggtgctcgtg taaaagatcc atttacccta caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 37
<211> 15198
<212> DNA
<213> Newcastle disease virus

<220>
<223> Mut HN
(F277L)/M(G165W)/F(F117S)/F(F190L)/L(V757I)/L(F1551S)/L(R1700L)+AfeI

<400> 37
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat tttagatttt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gcccccaccc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700
tggaagccaa cctgggcatg atgaagattc tggaccctgg ttgtgctaac gtttcatctt 2760
taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagcgct gaccgagccc tcctctataa acccaagatt caaggctcca 3180

agcgataacc ttctcccgcc tcttccatcc cactgaatga tcgcgcagct gcaattaatc 3240

tagcaacatt aaagattaag aaaaaatacg ggtagaatcg gagtgcaccg attgtgccaa 3300

gatggactca tctaggacaa ttgggttata ctttgattct gcccttcctt ctagcaacct 3360

gttagcattc ccgattgtct tacgagatgt aggagatgga aagaagcaaa ttactccgca 3420

atataggatc cggcgtcttg actcgtggac agacagtaaa gaagactcgg tattcatcac 3480

tacctacgga ttcatcttcc aggttgggaa tgaagaagtc actgtcggca tgatcaatga 3540

taatcccaag cgagagttac tctccgctgc gatgctctgc ctaggaagcg tcccgaatgt 3600

tggagatcct gttgagctgg caagggcctg cctcactatg gtggtaacat gcaagaagag 3660

tgcaactaat actgagagaa tggtcttctc agtagtgcag gcaccccggg tgctgcaaag 3720

ctgtagggtc gtggcagaca aatactcgtc agtgaatgca gttaagcacg tgaaagcacc 3780

agagaagatc ccttggagtg aaaccctaga gtacaaggtg aattttgtct ctttgactgt 3840

ggtgccgaag aaggatgtct acaagatccc aaccgcagta ttgaaggtat ccggctcgag 3900

cctgtataat cttgcgctca atgtcactat tgatgtggag gtagacccaa agagcccgtt 3960

agtcaagtcc ctttctaggt ccgacagtgg atactatgct aatctcttct tacatatcgg 4020

acttatgtcc actgtagata agaggggaaa gaaagtgaca tttgaccagc tggagaggaa 4080

gataaggaga cttgatctat ctgtcgggct cagtgatgtg ctcggacctt ctgtgcttgt 4140

gaaggcgaga ggtgcacgga ctaggctgct ggcacctttc ttctctaaca gcgggacagc 4200

ctgctaccct atagcaaatg cctctcctca ggtagctaag atactctgga gtcagaccgc 4260

gtgcctgcgg agtgtaaaaa tcattattca agcgggcacc caacgcgctg tcgcagtgac 4320

tgctgaccat gaggttacct ctactaagat agagaagagg cataccatcg ctaaatacaa 4380

tcccttcaag aaataggccg tatccctgag actgcaattc acccgccttc ccaaaacacc 4440

atgacaccag ataatgatct gtcttgatta cttacagtta gtttccctgt ctatcaaatt 4500

agaaaaaaca cgggtagaag agttcggatc ccggccggcg caccaaaagc gcaagatggg 4560

ccccagatct tctaccagga tcccagtacc tctaatgctg accatacgga tcacgctggc 4620

actgagttat gtccgtctga caagttctct tgatggcagg cctcttgcag ccgcagggat 4680

cgtggtaaca ggggataaag cagttaacat atacacctca tcccagacag ggtcaatcat 4740

agtcaagtta ctcccaaata tgcccaagga caaagaggca tgtgcaaaag ccccattgga 4800

ggcttacaac aggacactga ctactttgct tacccccctt ggtgattcta tccgcaggat 4860

acaagagtct gtgactacat ccggaggaag gagacagaga cgctctatag gtgccattat 4920

tggcagtgta gctctagggg ttgcaacagc tgcacagata acggcagcct cggctctgat 4980

acaagccaac cagaatgctg ctaacatcct ccggcttaag gagagcattg ctgcaaccaa 5040

tgaagctgta cacgaggtca ctggcggatt gtcacagtta gcagtggcag ttggaaagat 5100

gcagcaattt gttaatgacc agcttaataa cacagctcaa gagttggact gtataaaaat 5160

tacacagcag gttggtgtag aactcaactt atacttaact gaattgacta cagtgttcgg 5220

gccacaaatc acttcccctg ccttaactca gttgactgtt caggctcttt acaatctggc 5280

tggtggtaat gtagattact tgttgactaa gttaggtgta gggaacaacc agctcagctc 5340

attgattggt agcggcttga tcaccggtaa ccctattttt tacgactcac agactcaact 5400

cttgggcata caggtgactt taccctcagt cgggaaccta aataatatgc gtgccaccta 5460

cttggagacc ttgtctgtaa gcacaacaaa gggatttgcc tcagcacttg tcccaaaagt 5520

agtgacacag gtcggttctg tgatagaaga gcttgacacc tcatactgta tagaagctga 5580

tttggattta tattgtacaa gaatagtgac attccctatg tcccctggta tttattcctg 5640

tttgagcggc aatacatcgg cttgcatgta ttcaaggact gaaggcgcac ttactacacc 5700

atacatgact ctcaaaggct cagttgttgc caattgccag atgacaacat gtagatgtgc 5760

agaccccccg ggtatcatat cacaaaatta tggagaggct gtgtctctaa tagataagca 5820

ctcatgcaat gtcgtatcct tagacgggat aactttgagg ctcagtgggg aatttgatgc 5880

aacttatcaa aagaatatct caatattaga ttctcaagta ctagtgacag gcaatctcga 5940

tatctcaact gagcttggga atgtcaacca ctcaataagt aatgctttgg ataagttaga 6000

ggaaagcaac agcaaactag acaaagtcaa tgtcaaactg accagcacat ctgctctcat 6060

tacctatatt gttttaactg tcatatctct tgttcttggt atgcttagcc tggttctagc 6120

atgctatctg atgtacaagc aaaaagcgca acgaaagacc ttgttgtggc ttggaaataa 6180

taccctagat cagatgagag ctactacaaa aatgtgaatg cagatgagag gcagaggtat 6240

ccccaatagc aatctgtgtg tcaattctgg cagcctgtta atcagaagaa ttaagaaaaa 6300

actaccggat gtaggtgaac aaaagggaat atacgggtag aacggcctga gaggccaccc 6360

ctcaatcggg agccaggccc cactacgtcc gctctaccgc atcaccaaca gcagtcttca 6420

gtcatggaca gcgcagttag ccaagttgcg ctagagaatg atagaagaga agcgaataat 6480

acatggcgct tggttttccg gatcgcagcc ttacttttac tggtaataac cttagccgtc 6540

tctgcagtcg ccctggcata tagtatggag gctagcacac ctggcgacct tgtaagcata 6600

ccgactgcga tctctagggc agaggaaagg attacatctg cactcggttc caatcaagat 6660

gtagtagata ggatatacaa gcaagtggcc cttgagtctc cattggcact gctaaatacc 6720

gaatctataa ttatgaatgc aataacgtct ctctcttatc aaatcaatgg agccacaaat 6780

aatagcgggt gtggggcacc tgttcatgac ccagattaca tcggggggat aggtcaagaa 6840

cttattgtag atgatacgag tgacgtcaca tcattttatc cctctgcatt ccaagaacac 6900

ctgaatttta tcccggcgcc cactacagga tcaggctgca ctcggatacc ctcattcgac 6960

atgagtgcta cccattactg ttacactcac aatgtgatat tgtctggctg cagagatcac 7020

tcacactcac atcagtattt agcacttggt gtgcttcgga catctgcaac agggagggta 7080

ttcttttcta ctctgcgttc catcaacttg gatgacgccc aaaatcggaa gtcttgcagt 7140

gtgagtgcaa ctcctttagg ttgtgatatg ctgtgctcta aaatcacaga gactgaggaa 7200

gaggattata aatcagttat ccccacatcg atggtacatg gaaggttagg gcttgacggc 7260

caataccatg agaaggacct agacgtcaca acactattta gggactgggt ggcaaattac 7320

ccaggagtag gaggtgggtc ttttattaac aatcgcgtat ggttcccggt ctacggaggg 7380

ctaaaaccca gctcgcctag tgacactgca caagaaggga gatacgtaat atacaagcga 7440

tacaatgaca catgcccgga tgagcaagat taccagattc ggatggctaa gtcatcgtat 7500

aagcctaggc ggtttggtgg aaaacgcgta cagcaggcca tcctatccat caaggtgtca 7560

acatccttgg gtgaggaccc ggtgttgact gtaccgccta atacggtcgc actcatgggg 7620

gccgaaggca gagttctcac agtggggaca tctcatttct tatatcagcg agggtcatca 7680

tacttctctc ccgctttgtt ataccctatg acagtcaaca acaaaacagc cactcttcat 7740

aatccttata cattcaatgc tttcactcgg ccaggtagtg tcccttgcca ggcttcagca 7800

agatgcccca actcatgtgt taccggagtc tatactgatc catatccctt agtcttccat 7860

aggaaccaca ccttgcgagg ggtattcgga acaatgcttg atgacgaaca agcaagactc 7920

aaccctgtat ctgcagtatt tgataacata tcccgcagtc gcataactcg ggtaagttca 7980

agcagtacca gggcagcata cacgacatca acgtgtttta aagttgtcaa gaccaataaa 8040

acctattgcc tcagcattgc agaaatatcc aataccctct tcggggaatt cagaattgtc 8100

cccttactag ttgagattct caaggatggt ggggtttaag aagctaggaa tggtcggttg 8160

agtcaaccgt gagaaggcgg ggaagatgat attgcatcat ctatcttctg taacaccaag 8220

aatcaaatcg aataccagtg cgagctcgaa tcctacgctg ccagtcagcc ataatcggct 8280

ggtgctaatg tggttagcct gaatcttgtc gatagtcact tgattaagaa aaaatgtgga 8340

cggtagtgaa atataaggca aagcagctca cagaggacag cacgggtagg acatggcggg 8400

ctccggaccc gaaagggcag agcatcagat tatcctacca gagtcacatc tgtcctcacc 8460

attggtcaag cataaattgc tttattactg gaaattaact gggctaccgc ttcctgacga 8520

atgtgacttc gaccatctta ttatcagccg acaatggaag aaggtacttg aatcggccac 8580

ccctgacatt gagagaatga taaaactagg gcgggcagta caccagactc tcaaccacaa 8640

ttccaggata accggagtac tccatccccg gtgtttagag gaactggcta gtattgaggt 8700

tcctgattca accaacaaat ttcggaagat cgaaaaaaag atccagattc acaacacaag 8760

gtatggagaa ctgttcacaa gattatgcac gcatgtagaa aagaaactat tggggtcatc 8820

ttggtctaac aatgtcccac ggtcagagga attcaacagc atccgtacag atccggcatt 8880

ttggtttcac tcaaaatggt ccacagccaa atttgcatgg ctccatataa aacaggtcca 8940

gaggcatctg attgtagcag caagaacaag gtccgcagtc aacaaattag tgacgctgac 9000

ccataaggta ggccaaatct ttgttactcc tgagcttgtc attgtgacac atacagatga 9060

gaacaagttc acgtgtctta cccaggaact tgtgttgatg tatgcagata tgatggaggg 9120

cagagatatg gtcagcataa tatcatccac ggcggcacat cttaggagct tatcagagaa 9180

aattgatgat attctgcggt tagtagatgc tctggcaaga gatttgggca atcaagtcta 9240

cgatgttgta gcactaatgg agggattcgc atacggcgct gttcagctgc ttgaaccgtc 9300

aggtacattt gcgggggatt tcttcgcatt caacctgcag gagctcaaag atactctaac 9360

cggactcctc cccaaggata tcgcagaatc tgtgactcac gcaatcgcaa ccatattctc 9420

tggcttagaa caaaatcaag cagctgagat gttgtgcctg ttgcgtctgt ggggtcaccc 9480

actactcgag tcccgtattg cagcaaaagc agttaggagt caaatgtgcg caccaaaaat 9540

ggtagacttt gatatgatcc tccaggtatt atctttcttt aaaggaacaa tcatcaatgg 9600

atatagaaag aagaatgcag gcgtgtggcc acgtgtcaag atagatacga tatacgggaa 9660

ggtcataggg cagctacacg cagattctgc agagatttca catgatgtca tgttgaggga 9720

atacaagagt ttatctgcac ttgaattcga gccatgtata gagtatgacc ctgtcaccaa 9780

tctgagcatg tttttaaaag acaaggcaat cgcacacccg aaagacaact ggctcgcttc 9840

gtttaggcga aaccttctct ctgaggacca gaagaaacat gtaagagagg caacctcaac 9900

taaccgcctc ttgatagagt tcttagagtc aaatgatttt gatccatata aagagatgga 9960

atatctgacg acccttgagt acctaagaga tgataatgtg gcagtatcat actcactcaa 10020

agaaaaagag gtgaaagtta atgggcggat tttcgcaaag ctaacaaata gattaaggaa 10080

ttgtcaggta atggcagaag ggatcctagc tgaccagatt gcacctttct tccagggaaa 10140

tggggtcatt caggatagca tatctttgac taaaagtatg ctagcgatga gtcaactgtc 10200

tttcaacagc aacaagaaac gtattactga ctgcaaagaa agagtatcct caaaccgcaa 10260

tcatgatccg aagagcaaga atcgtcggag agttgccact tttgtaacta ccgacctgca 10320

aaagtattgt ctcaattgga gatatcagac agtcaagctg ttcgctcatg ccatcaatca 10380

gctgatgggc ttacctcact tcttcgagtg gattcatctt agactaatgg atactacgat 10440

gtttgtaggg gaccctttca atcctccaag tgaccctacg gactgtgatc tatcaagagt 10500

cccaaatgat gacatatata ttgtcagtgc taggggaggc attgagggat tatgccagaa 10560

gctatggaca atgatctcaa ttgctgcaat ccagcttgct gcagcaagat cacattgtcg 10620

cgttgcctgt atggtacaag gcgacaatca agtaatagct ataacgagag aggtaagatc 10680

agatgactcc ccagagatgg tgttaacaca attgcatcaa gccagtgata atttcttcaa 10740

ggaattgatt catgtcaatc atttgatcgg ccataatttg aaggatcgtg aaaccatcag 10800

gtcagacacg ttcttcatat acagcaaacg aatattcaaa gatggagcaa tactcagtca 10860

ggtcctcaaa aactcatcta aattagtgct aatatcaggc gaccttagtg aaaacactgt 10920

aatgtcttgt gccaacattg catctactgt agcacggtta tgcgagaacg ggcttcctaa 10980

ggatttctgt tattacttaa actacttaat gagttgcgtg cagacatact tcgattccga 11040

attttccatc accaacaact cgcaactcga ttctaaccag tcgtggatag aggacatttc 11100

ttttgtgcac tcatatgtcc tgacccctgc tcaactgggg ggactgagta accttcaata 11160

ctcaaggctc tacacaagga acatcggcga cccgggaacc actgctttcg cagagatcaa 11220

gagattagag gcagtggggt tactgagtcc tagcattatg actaacatct taactaggcc 11280

gcctggaaat ggagattggg ccagtctgtg caacgatcca tactccttta attttgagac 11340

tgtcgcaagc ccaaatattg tccttaagaa acatacacaa agagtcctat ttgaaacttg 11400

ctcgaatccc ttattatctg gagtgcacac agaggataat gaggcagaag agaaggcatt 11460

ggctgaattc ttgctcaatc aagaaatgat tcatccacgt gtcgcgcatg ctatcatgga 11520

agcaagctct gtaggtagga gaaagcaaat tcaggggctt gttgacacaa caaacaccgt 11580

gattaagatt gcactgacta ggaggccact cggcatcaag aggctgatgc ggatagtcaa 11640

ttactcgagc atgcatgcaa tgctatttag agatgatgtt ttctcgtcta atagatccaa 11700

ccatccctta gtctcttctg atatgtgttc tcagacactg gcagactatg cacggaacag 11760

aagctggtca cctttgacag ggggtagaaa aatactgggt gtatctaatc ctgataccat 11820

agaacttgta gagggtgaga tccttagtgt cagtggaggg tgcactagat gtgacagtgg 11880

ggatgaacag tttacttggt tccatcttcc aagcaatata gagctaaacg atgacaccag 11940

caagaatcct ccaatgagag tgccatatct cgggtcaaag actcaagaga ggagagccgc 12000

ctcgcttgcg aaaatagctc atatgtcacc acacgtgaag gcggctctaa gggcgtcatc 12060

tgtgttaatc tgggcttatg ggacaacgaa aataaactgg actgctgccc ttaagattgc 12120

aaggtctcgg tgcaacataa gctcagagta tcttcgacta ttgtcaccct tgcctacagc 12180

tgggaatctc caacatagat tggacgacgg cataactcag atgacattca cccctgcatc 12240

tctatacagg gtgtcacctt acattcatat atctaatgat tctcaaaggc tatttactga 12300

agaaggagtc aaagagggga atgtggttta tcagcaaatc atgctcttgg gtttgtcctt 12360

aattgagtca ctcttcccaa tgacaacaac caagacatat gatgaaatca cattgcacct 12420

ccacagtaaa tttagctgct gtatcaggga agcacctgtt gcagttcctt tcgagctact 12480

cggggtggca ccggaactaa gggcagtaac ctcaaataag tttatgtatg atcctagccc 12540

tgtatcagag ggagactttg cgagacttga cttagctatc tttaagagtt atgaacttaa 12600

tttagagtca tattccacaa tagagctaat gaacgttctt tcaatatcta gtgggaagtt 12660

gattggccag tctgtggttt cttatgatga agatacctcc ataaagaatg acgctataat 12720

agtgtatgac aacacacgga attggatcag cgaagctcag aattcagatg tggtccgcct 12780

attcgagtat gcggcactcg aagtgctcct cgactgttct tatcaactct actatctgag 12840

agtaagaggc ctaaacaata tcgtcctgta catgagtgat ttatacaaga atatgccagg 12900

aattctactc tctaatattg cagccacaat atctcaccct gtcatccatt caaggttgaa 12960

tgcagtaggt ctggtcaacc atgacgggtc acaccaactt gcagacacag atttcattga 13020

aatgtctgca aagctgctag tctcttgcac tcgacgcgtg gtctcaggtt tacatgcagg 13080

gaataagtat gacctgctgt ttccatctgt cttggatgat aacctaagtg agaagatgct 13140

tcagttgata tcccaattat gctgtctgta tacggtgctc tttgctacaa caagagaaat 13200

cccgaaaata agaggcttat ctgcagaaga gaaatgttca gtacttactg agtacctact 13260

gtcagatgct gtgaaaccgt tacttgggtc cgagcaagtg agctctatca tgtctcccaa 13320

catagttacg ttcccagcca atctgtatta catgtctagg aagagcctta atttgatcag 13380

ggagagagag gacagggata ctatcttggc attgttgttc cctcaagaac cgctgctcga 13440

gtttcctctg gtacgagata ttggtgctcg tgtaaaagat ccatttaccc tacaacctgc 13500

ggcgttttta caagagttag atttgagtgc tccggcaagg tatgacgcat tcacaatcag 13560

tcaggcgcat tctgaacaca tattgccaaa cccagaggaa gatcacttag tacgatactt 13620

gttcagagga atagggactg cgtcctcctc ttggtataag gcatcccatc ttctttctgt 13680

acccgaggtc agatgtgcaa ggcatgggaa ctccttatat ttagcagaag gaagtggagc 13740

catcatgagt cttctcgaat tgcatatacc acacgaaact atctattaca atacactttt 13800

ctcgaacgag atgaaccccc cacagcgaca tttcggacca actccaacac agtttctgaa 13860

ttcggttgtt tttaggaatt tacaggcgga agtaccatgc aaggatggat ttgtccagga 13920

gttccgtccg ttatggagag agaatacaga agaaagcgat ctgacctcag ataaagcagt 13980

gggatatatc acatctgtag tgccgtacag gtctgtatca ttgctgcatt gtgacattga 14040

aatccctcca ggatctaatc aaagcttact agatcagctg gctaccaatc tgtctctgat 14100

tgccatgcat tctgtgaagg agggcggggt cgtgattatc aaagtactgt atgcaatggg 14160

atattacttc catctactca tgaacttgtt tactccatgt tccacgaaag gatatattct 14220

ctctaatggc tatgcctgta gaggggatat ggagtgttac ctgatatttg tcatgggcta 14280

cctaggcggg cctacatttg tacatgaggt ggtgaggatg gcaaaaacgc tagtacagcg 14340

gcatggcaca cttttgtcta agtcagatga gattacactg actaggttat ttacttcaca 14400

gcaacagcgt gtaacagaca tcctatccag ccctttaccg agactaatga agtacttgag 14460

agagaatatt gatactgcac tgattgaagc cgggggacag cctgtccgtc cattctgtgc 14520

agagagttta gtgagcacac taacagacat gactcagaca acccagatca tcgccagcca 14580

cattgataca gtcattcgat ctgtgatcta tatggaagca gagggtgatc ttgctgacac 14640

agtattctta tttacccctt acaacctctc tatggacggg aaaaagagaa catcacttaa 14700

acagtgcaca agacagatct tagaggtcac aatactgggt ctcagagtca aaaatctcaa 14760

taaagtaggt gatgtaatca gcctagtact cagaggtatg atttctctgg aggaccttct 14820

cccactgaga acctacttga agtgtagtac ctgccctaag tatttgaagg ctgtcctagg 14880

tattaccaaa ctcaaagaaa tgttcacaga cgcctcttta ttatacttga ctcgtgctca 14940

acaaaaattc tacatgaaaa ctataggcaa tgcagtcaag ggatactata gtaactgtga 15000

ctcttaaagg caaccacata tcaataggcc ctctttctag ccgatcgtat tcttgttgac 15060

ttcattatac catattagaa aaaaattgaa ttccgaccct ttaagactcg tattcggatt 15120

caaataatta tctcagaaaa aaagtgcacg tagttgttct tgattatagt cccgtcattc 15180

accaagtctt tgtttggt 15198

<210> 38
<211> 17442
<212> DNA
<213> Newcastle Disease Virus

<220>
<223> Mut HN

(F277L)/M(G165W)/F(F117S)/F(F190L)/L(V757I)/L(F1551S)/L(R1700L)-Lirilumab

<400> 38
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaagtgca actggtgcag agcggtgcag aggtgaagaa gccaggatcc tctgtgaaag 3300

tctcatgcaa agccagtgga ggtacgttca gcttttacgc aatcagctgg gtgagacagg 3360

ctccgggcca aggtctggaa tggatgggcg gattcattcc aattttcggc gcagcaaact 3420

acgcacagaa gttccagggg cgggtcacga ttactgctga tgagtcgaca agcacagcat 3480

acatggagct cagttctttg agaagcgatg atacagcagt ttattactgt gctagaattc 3540

caagcggttc ttattactat gactacgata tggatgtctg gggacaggga acaacagtca 3600

ccgtatcttc cgcgagcacc aagggacctt ccgtgtttcc cctggcccct tgctccagat 3660

caacctcaga gtcgactgct gctctcggtt gcttggtgaa agattatttt ccagaacccg 3720

ttaccgtgtc ttggaactcc ggtgccctta cttcaggggt tcataccttc cccgcagtcc 3780

tacagtcatc tggtctgtat tctctcagct ccgtggtcac cgtcccaagc agctcactgg 3840

gcaccaagac ctatacctgc aacgtcgacc ataagcctag taatacaaag gtcgacaagc 3900

gagtcgaatc caaatacgga cctccttgtc caccttgccc cgctcctgaa ttcctgggtg 3960

gaccaagcgt ctttctgttt ccaccaaaac ctaaagacac acttatgatt tcccgcacac 4020

ccgaagtcac ctgcgtggtg gtggatgtca gccaggaaga ccctgaggtg caattcaact 4080

ggtacgttga tggcgtagaa gtccacaacg ccaagacaaa acccagagag gagcagttta 4140

atagtaccta tagggtggtg tccgtcctga ccgttctcca ccaggattgg ttgaatggca 4200

aggaatacaa gtgtaaagtg agcaacaaag gcctaccttc gagcatagaa aagactatct 4260

ccaaggcaaa gggacagcca agggagcccc aggtgtacac gctgccaccc tctcaggagg 4320

agatgactaa gaaccaggtg tccttgacct gcctcgtaaa ggggttttac ccctccgaca 4380

tagccgtcga atgggagagt aacggacaac ccgagaacaa ttataaaact acacctcccg 4440

tgctcgactc tgacggatcc ttcttcctgt actctaggct gacagtggat aaatcccgtt 4500

ggcaggaagg aaatgtattt tcctgtagtg tgatgcacga ggctctgcac aatcattata 4560

cacagaaatc actgtcctta agcctcggca aacgccgcaa gcgcggctcc ggcgagggcc 4620

gcggctccct gctgacctgc ggcgacgtgg aggagaaccc cggccccatg aagtacctgc 4680

tgcccaccgc cgccgccggc ctgctgctgc tggccgccca gcccgccatg gccgaaatcg 4740

tcctcacaca gagcccggtt acgctttcat tgtctccagg cgagcgcgct accctcagct 4800

gtagggcgtc tcagtccgtt tccagctatt tagcttggta ccagcaaaaa cctggccagg 4860

cccctagact gcttatttac gacgcctcta accgagcgac tggtataccc gcaaggttca 4920

gcggctcggg ctcaggcacc gactttactc tcaccatttc atcactggag cctgaggact 4980

tcgccgtgta ttactgtcag cagaggagta actggatgta caccttcggc caagggacca 5040

aattggaaat caaaaggaca gtggccgcgc caagcgtgtt tatatttccc ccctccgacg 5100

aacagttaaa gagtggaacc gcatctgtgg tatgtctgct taacaatttt taccctcgcg 5160

aggcaaaggt tcaatggaaa gtcgacaacg ccttacagtc tggtaacagt caagagtctg 5220

ttacggagca agacagcaag gactccacct attcgctgtc ttcaacacta accctcagca 5280

aggctgatta cgagaagcac aaagtttacg cctgtgaggt cacccatcag ggcctttcta 5340

gccctgtcac aaagtcgttc aacaggggag agtgctaatg agctgaccga gccctcctct 5400

ataaacccaa gattcaaggc tccaagcgat aaccttctcc cgcctcttcc atcccactga 5460

atgatcgcgc agctgcaatt aatctagcaa cattaaagat taagaaaaaa tacgggtaga 5520

atcggagtgc accgattgtg ccaagatgga ctcatctagg acaattgggt tatactttga 5580

ttctgccctt ccttctagca acctgttagc attcccgatt gtcttacgag atgtaggaga 5640

tggaaagaag caaattactc cgcaatatag gatccggcgt cttgactcgt ggacagacag 5700

taaagaagac tcggtattca tcactaccta cggattcatc ttccaggttg ggaatgaaga 5760

agtcactgtc ggcatgatca atgataatcc caagcgagag ttactctccg ctgcgatgct 5820

ctgcctagga agcgtcccga atgttggaga tcctgttgag ctggcaaggg cctgcctcac 5880

tatggtggta acatgcaaga agagtgcaac taatactgag agaatggtct tctcagtagt 5940

gcaggcaccc cgggtgctgc aaagctgtag ggtcgtggca gacaaatact cgtcagtgaa 6000

tgcagttaag cacgtgaaag caccagagaa gatcccttgg agtgaaaccc tagagtacaa 6060

ggtgaatttt gtctctttga ctgtggtgcc gaagaaggat gtctacaaga tcccaaccgc 6120

agtattgaag gtatccggct cgagcctgta taatcttgcg ctcaatgtca ctattgatgt 6180

ggaggtagac ccaaagagcc cgttagtcaa gtccctttct aggtccgaca gtggatacta 6240

tgctaatctc ttcttacata tcggacttat gtccactgta gataagaggg gaaagaaagt 6300

gacatttgac cagctggaga ggaagataag gagacttgat ctatctgtcg ggctcagtga 6360

tgtgctcgga ccttctgtgc ttgtgaaggc gagaggtgca cggactaggc tgctggcacc 6420

tttcttctct aacagcggga cagcctgcta ccctatagca aatgcctctc ctcaggtagc 6480

taagatactc tggagtcaga ccgcgtgcct gcggagtgta aaaatcatta ttcaagcggg 6540

cacccaacgc gctgtcgcag tgactgctga ccatgaggtt acctctacta agatagagaa 6600

gaggcatacc atcgctaaat acaatccctt caagaaatag gccgtatccc tgagactgca 6660

attcacccgc cttcccaaaa caccatgaca ccagataatg atctgtcttg attacttaca 6720

gttagtttcc ctgtctatca aattagaaaa aacacgggta gaagagttcg gatcccggcc 6780

ggcgcaccaa aagcgcaaga tgggccccag atcttctacc aggatcccag tacctctaat 6840

gctgaccata cggatcacgc tggcactgag ttatgtccgt ctgacaagtt ctcttgatgg 6900

caggcctctt gcagccgcag ggatcgtggt aacaggggat aaagcagtta acatatacac 6960

ctcatcccag acagggtcaa tcatagtcaa gttactccca aatatgccca aggacaaaga 7020

ggcatgtgca aaagccccat tggaggctta caacaggaca ctgactactt tgcttacccc 7080

ccttggtgat tctatccgca ggatacaaga gtctgtgact acatccggag gaaggagaca 7140

gagacgctct ataggtgcca ttattggcag tgtagctcta ggggttgcaa cagctgcaca 7200

gataacggca gcctcggctc tgatacaagc caaccagaat gctgctaaca tcctccggct 7260

taaggagagc attgctgcaa ccaatgaagc tgtacacgag gtcactggcg gattgtcaca 7320

gttagcagtg gcagttggaa agatgcagca atttgttaat gaccagctta ataacacagc 7380

tcaagagttg gactgtataa aaattacaca gcaggttggt gtagaactca acttatactt 7440

aactgaattg actacagtgt tcgggccaca aatcacttcc cctgccttaa ctcagttgac 7500

tgttcaggct ctttacaatc tggctggtgg taatgtagat tacttgttga ctaagttagg 7560

tgtagggaac aaccagctca gctcattgat tggtagcggc ttgatcaccg gtaaccctat 7620

tttttacgac tcacagactc aactcttggg catacaggtg actttaccct cagtcgggaa 7680

cctaaataat atgcgtgcca cctacttgga gaccttgtct gtaagcacaa caaagggatt 7740

tgcctcagca cttgtcccaa aagtagtgac acaggtcggt tctgtgatag aagagcttga 7800

cacctcatac tgtatagaag ctgatttgga tttatattgt acaagaatag tgacattccc 7860

tatgtcccct ggtatttatt cctgtttgag cggcaataca tcggcttgca tgtattcaag 7920

gactgaaggc gcacttacta caccatacat gactctcaaa ggctcagttg ttgccaattg 7980

ccagatgaca acatgtagat gtgcagaccc cccgggtatc atatcacaaa attatggaga 8040

ggctgtgtct ctaatagata agcactcatg caatgtcgta tccttagacg ggataacttt 8100

gaggctcagt ggggaatttg atgcaactta tcaaaagaat atctcaatat tagattctca 8160

agtactagtg acaggcaatc tcgatatctc aactgagctt gggaatgtca accactcaat 8220

aagtaatgct ttggataagt tagaggaaag caacagcaaa ctagacaaag tcaatgtcaa 8280

actgaccagc acatctgctc tcattaccta tattgtttta actgtcatat ctcttgttct 8340

tggtatgctt agcctggttc tagcatgcta tctgatgtac aagcaaaaag cgcaacgaaa 8400

gaccttgttg tggcttggaa ataataccct agatcagatg agagctacta caaaaatgtg 8460

aatgcagatg agaggcagag gtatccccaa tagcaatctg tgtgtcaatt ctggcagcct 8520

gttaatcaga agaattaaga aaaaactacc ggatgtaggt gaacaaaagg gaatatacgg 8580

gtagaacggc ctgagaggcc acccctcaat cgggagccag gccccactac gtccgctcta 8640

ccgcatcacc aacagcagtc ttcagtcatg gacagcgcag ttagccaagt tgcgctagag 8700

aatgatagaa gagaagcgaa taatacatgg cgcttggttt tccggatcgc agccttactt 8760

ttactggtaa taaccttagc cgtctctgca gtcgccctgg catatagtat ggaggctagc 8820

acacctggcg accttgtaag cataccgact gcgatctcta gggcagagga aaggattaca 8880

tctgcactcg gttccaatca agatgtagta gataggatat acaagcaagt ggcccttgag 8940

tctccattgg cactgctaaa taccgaatct ataattatga atgcaataac gtctctctct 9000

tatcaaatca atggagccac aaataatagc gggtgtgggg cacctgttca tgacccagat 9060

tacatcgggg ggataggtca agaacttatt gtagatgata cgagtgacgt cacatcattt 9120

tatccctctg cattccaaga acacctgaat tttatcccgg cgcccactac aggatcaggc 9180

tgcactcgga taccctcatt cgacatgagt gctacccatt actgttacac tcacaatgtg 9240

atattgtctg gctgcagaga tcactcacac tcacatcagt atttagcact tggtgtgctt 9300

cggacatctg caacagggag ggtattcttt tctactctgc gttccatcaa cttggatgac 9360

gcccaaaatc ggaagtcttg cagtgtgagt gcaactcctt taggttgtga tatgctgtgc 9420

tctaaaatca cagagactga ggaagaggat tataaatcag ttatccccac atcgatggta 9480

catggaaggt tagggcttga cggccaatac catgagaagg acctagacgt cacaacacta 9540

tttagggact gggtggcaaa ttacccagga gtaggaggtg ggtcttttat taacaatcgc 9600

gtatggttcc cggtctacgg agggctaaaa cccagctcgc ctagtgacac tgcacaagaa 9660

gggagatacg taatatacaa gcgatacaat gacacatgcc cggatgagca agattaccag 9720

attcggatgg ctaagtcatc gtataagcct aggcggtttg gtggaaaacg cgtacagcag 9780

gccatcctat ccatcaaggt gtcaacatcc ttgggtgagg acccggtgtt gactgtaccg 9840

cctaatacgg tcgcactcat gggggccgaa ggcagagttc tcacagtggg gacatctcat 9900

ttcttatatc agcgagggtc atcatacttc tctcccgctt tgttataccc tatgacagtc 9960

aacaacaaaa cagccactct tcataatcct tatacattca atgctttcac tcggccaggt 10020

agtgtccctt gccaggcttc agcaagatgc cccaactcat gtgttaccgg agtctatact 10080

gatccatatc ccttagtctt ccataggaac cacaccttgc gaggggtatt cggaacaatg 10140

cttgatgacg aacaagcaag actcaaccct gtatctgcag tatttgataa catatcccgc 10200

agtcgcataa ctcgggtaag ttcaagcagt accagggcag catacacgac atcaacgtgt 10260

tttaaagttg tcaagaccaa taaaacctat tgcctcagca ttgcagaaat atccaatacc 10320

ctcttcgggg aattcagaat tgtcccctta ctagttgaga ttctcaagga tggtggggtt 10380

taagaagcta ggaatggtcg gttgagtcaa ccgtgagaag gcggggaaga tgatattgca 10440

tcatctatct tctgtaacac caagaatcaa atcgaatacc agtgcgagct cgaatcctac 10500

gctgccagtc agccataatc ggctggtgct aatgtggtta gcctgaatct tgtcgatagt 10560

cacttgatta agaaaaaatg tggacggtag tgaaatataa ggcaaagcag ctcacagagg 10620

acagcacggg taggacatgg cgggctccgg acccgaaagg gcagagcatc agattatcct 10680

accagagtca catctgtcct caccattggt caagcataaa ttgctttatt actggaaatt 10740

aactgggcta ccgcttcctg acgaatgtga cttcgaccat cttattatca gccgacaatg 10800

gaagaaggta cttgaatcgg ccacccctga cattgagaga atgataaaac tagggcgggc 10860

agtacaccag actctcaacc acaattccag gataaccgga gtactccatc cccggtgttt 10920

agaggaactg gctagtattg aggttcctga ttcaaccaac aaatttcgga agatcgaaaa 10980

aaagatccag attcacaaca caaggtatgg agaactgttc acaagattat gcacgcatgt 11040

agaaaagaaa ctattggggt catcttggtc taacaatgtc ccacggtcag aggaattcaa 11100

cagcatccgt acagatccgg cattttggtt tcactcaaaa tggtccacag ccaaatttgc 11160

atggctccat ataaaacagg tccagaggca tctgattgta gcagcaagaa caaggtccgc 11220

agtcaacaaa ttagtgacgc tgacccataa ggtaggccaa atctttgtta ctcctgagct 11280

tgtcattgtg acacatacag atgagaacaa gttcacgtgt cttacccagg aacttgtgtt 11340

gatgtatgca gatatgatgg agggcagaga tatggtcagc ataatatcat ccacggcggc 11400

acatcttagg agcttatcag agaaaattga tgatattctg cggttagtag atgctctggc 11460

aagagatttg ggcaatcaag tctacgatgt tgtagcacta atggagggat tcgcatacgg 11520

cgctgttcag ctgcttgaac cgtcaggtac atttgcgggg gatttcttcg cattcaacct 11580

gcaggagctc aaagatactc taaccggact cctccccaag gatatcgcag aatctgtgac 11640

tcacgcaatc gcaaccatat tctctggctt agaacaaaat caagcagctg agatgttgtg 11700

cctgttgcgt ctgtggggtc acccactact cgagtcccgt attgcagcaa aagcagttag 11760

gagtcaaatg tgcgcaccaa aaatggtaga ctttgatatg atcctccagg tattatcttt 11820

ctttaaagga acaatcatca atggatatag aaagaagaat gcaggcgtgt ggccacgtgt 11880

caagatagat acgatatacg ggaaggtcat agggcagcta cacgcagatt ctgcagagat 11940

ttcacatgat gtcatgttga gggaatacaa gagtttatct gcacttgaat tcgagccatg 12000

tatagagtat gaccctgtca ccaatctgag catgttttta aaagacaagg caatcgcaca 12060

cccgaaagac aactggctcg cttcgtttag gcgaaacctt ctctctgagg accagaagaa 12120

acatgtaaga gaggcaacct caactaaccg cctcttgata gagttcttag agtcaaatga 12180

ttttgatcca tataaagaga tggaatatct gacgaccctt gagtacctaa gagatgataa 12240

tgtggcagta tcatactcac tcaaagaaaa agaggtgaaa gttaatgggc ggattttcgc 12300

aaagctaaca aatagattaa ggaattgtca ggtaatggca gaagggatcc tagctgacca 12360

gattgcacct ttcttccagg gaaatggggt cattcaggat agcatatctt tgactaaaag 12420

tatgctagcg atgagtcaac tgtctttcaa cagcaacaag aaacgtatta ctgactgcaa 12480

agaaagagta tcctcaaacc gcaatcatga tccgaagagc aagaatcgtc ggagagttgc 12540

cacttttgta actaccgacc tgcaaaagta ttgtctcaat tggagatatc agacagtcaa 12600

gctgttcgct catgccatca atcagctgat gggcttacct cacttcttcg agtggattca 12660

tcttagacta atggatacta cgatgtttgt aggggaccct ttcaatcctc caagtgaccc 12720

tacggactgt gatctatcaa gagtcccaaa tgatgacata tatattgtca gtgctagggg 12780

aggcattgag ggattatgcc agaagctatg gacaatgatc tcaattgctg caatccagct 12840

tgctgcagca agatcacatt gtcgcgttgc ctgtatggta caaggcgaca atcaagtaat 12900

agctataacg agagaggtaa gatcagatga ctccccagag atggtgttaa cacaattgca 12960

tcaagccagt gataatttct tcaaggaatt gattcatgtc aatcatttga tcggccataa 13020

tttgaaggat cgtgaaacca tcaggtcaga cacgttcttc atatacagca aacgaatatt 13080

caaagatgga gcaatactca gtcaggtcct caaaaactca tctaaattag tgctaatatc 13140

aggcgacctt agtgaaaaca ctgtaatgtc ttgtgccaac attgcatcta ctgtagcacg 13200

gttatgcgag aacgggcttc ctaaggattt ctgttattac ttaaactact taatgagttg 13260

cgtgcagaca tacttcgatt ccgaattttc catcaccaac aactcgcaac tcgattctaa 13320

ccagtcgtgg atagaggaca tttcttttgt gcactcatat gtcctgaccc ctgctcaact 13380

ggggggactg agtaaccttc aatactcaag gctctacaca aggaacatcg gcgacccggg 13440

aaccactgct ttcgcagaga tcaagagatt agaggcagtg gggttactga gtcctagcat 13500

tatgactaac atcttaacta ggccgcctgg aaatggagat tgggccagtc tgtgcaacga 13560

tccatactcc tttaattttg agactgtcgc aagcccaaat attgtcctta agaaacatac 13620

acaaagagtc ctatttgaaa cttgctcgaa tcccttatta tctggagtgc acacagagga 13680

taatgaggca gaagagaagg cattggctga attcttgctc aatcaagaaa tgattcatcc 13740

acgtgtcgcg catgctatca tggaagcaag ctctgtaggt aggagaaagc aaattcaggg 13800

gcttgttgac acaacaaaca ccgtgattaa gattgcactg actaggaggc cactcggcat 13860

caagaggctg atgcggatag tcaattactc gagcatgcat gcaatgctat ttagagatga 13920

tgttttctcg tctaatagat ccaaccatcc cttagtctct tctgatatgt gttctcagac 13980

actggcagac tatgcacgga acagaagctg gtcacctttg acagggggta gaaaaatact 14040

gggtgtatct aatcctgata ccatagaact tgtagagggt gagatcctta gtgtcagtgg 14100

agggtgcact agatgtgaca gtggggatga acagtttact tggttccatc ttccaagcaa 14160

tatagagcta aacgatgaca ccagcaagaa tcctccaatg agagtgccat atctcgggtc 14220

aaagactcaa gagaggagag ccgcctcgct tgcgaaaata gctcatatgt caccacacgt 14280

gaaggcggct ctaagggcgt catctgtgtt aatctgggct tatggggaca acgaaataaa 14340

ctggactgct gcccttaaga ttgcaaggtc tcggtgcaac ataagctcag agtatcttcg 14400

actattgtca cccttgccta cagctgggaa tctccaacat agattggacg acggcataac 14460

tcagatgaca ttcacccctg catctctata cagggtgtca ccttacattc atatatctaa 14520

tgattctcaa aggctattta ctgaagaagg agtcaaagag gggaatgtgg tttatcagca 14580

aatcatgctc ttgggtttgt ccttaattga gtcactcttc ccaatgacaa caaccaagac 14640

atatgatgaa atcacattgc acctccacag taaatttagc tgctgtatca gggaagcacc 14700

tgttgcagtt cctttcgagc tactcggggt ggcaccggaa ctaagggcag taacctcaaa 14760

taagtttatg tatgatccta gccctgtatc agagggagac tttgcgagac ttgacttagc 14820

tatctttaag agttatgaac ttaatttaga gtcatattcc acaatagagc taatgaacgt 14880

tctttcaata tctagtggga agttgattgg ccagtctgtg gtttcttatg atgaagatac 14940

ctccataaag aatgacgcta taatagtgta tgacaacaca cggaattgga tcagcgaagc 15000

tcagaattca gatgtggtcc gcctattcga gtatgcggca ctcgaagtgc tcctcgactg 15060

ttcttatcaa ctctactatc tgagagtaag aggcctaaac aatatcgtcc tgtacatgag 15120

tgatttatac aagaatatgc caggaattct actctctaat attgcagcca caatatctca 15180

ccctgtcatc cattcaaggt tgaatgcagt aggtctggtc aaccatgacg ggtcacacca 15240

acttgcagac acagatttca ttgaaatgtc tgcaaagctg ctagtctctt gcactcgacg 15300

cgtggtctca ggtttacatg cagggaataa gtatgacctg ctgtttccat ctgtcttgga 15360

tgataaccta agtgagaaga tgcttcagtt gatatcccaa ttatgctgtc tgtatacggt 15420

gctctttgct acaacaagag aaatcccgaa aataagaggc ttatctgcag aagagaaatg 15480

ttcagtactt actgagtacc tactgtcaga tgctgtgaaa ccgttacttg ggtccgagca 15540

agtgagctct atcatgtctc ccaacatagt tacgttccca gccaatctgt attacatgtc 15600

taggaagagc cttaatttga tcagggagag agaggacagg gatactatct tggcattgtt 15660

gttccctcaa gaaccgctgc tcgagtttcc tctggtacga gatattggtg ctcgtgtaaa 15720

agatccattt accctacaac ctgcggcgtt tttacaagag ttagatttga gtgctccggc 15780

aaggtatgac gcattcacaa tcagtcaggc gcattctgaa cacatattgc caaacccaga 15840

ggaagatcac ttagtacgat acttgttcag aggaataggg actgcgtcct cctcttggta 15900

taaggcatcc catcttcttt ctgtacccga ggtcagatgt gcaaggcatg ggaactcctt 15960

atatttagca gaaggaagtg gagccatcat gagtcttctc gaattgcata taccacacga 16020

aactatctat tacaatacac ttttctcgaa cgagatgaac cccccacagc gacatttcgg 16080

accaactcca acacagtttc tgaattcggt tgtttttagg aatttacagg cggaagtacc 16140

atgcaaggat ggatttgtcc aggagttccg tccgttatgg agagagaata cagaagaaag 16200

cgatctgacc tcagataaag cagtgggata tatcacatct gtagtgccgt acaggtctgt 16260

atcattgctg cattgtgaca ttgaaatccc tccaggatct aatcaaagct tactagatca 16320

gctggctacc aatctgtctc tgattgccat gcattctgtg aaggagggcg gggtcgtgat 16380

tatcaaagta ctgtatgcaa tgggatatta cttccatcta ctcatgaact tgtttactcc 16440

atgttccacg aaaggatata ttctctctaa tggctatgcc tgtagagggg atatggagtg 16500

ttacctgata tttgtcatgg gctacctagg cgggcctaca tttgtacatg aggtggtgag 16560

gatggcaaaa acgctagtac agcggcatgg cacacttttg tctaagtcag atgagattac 16620

actgactagg ttatttactt cacagcaaca gcgtgtaaca gacatcctat ccagcccttt 16680

accgagacta atgaagtact tgagagagaa tattgatact gcactgattg aagccggggg 16740

acagcctgtc cgtccattct gtgcagagag tttagtgagc acactaacag acatgactca 16800

gacaacccag atcatcgcca gccacattga tacagtcatt cgatctgtga tctatatgga 16860

agcagagggt gatcttgctg acacagtatt cttatttacc ccttacaacc tctctatgga 16920

cgggaaaaag agaacatcac ttaaacagtg cacaagacag atcttagagg tcacaatact 16980

gggtctcaga gtcaaaaatc tcaataaagt aggtgatgta atcagcctag tactcagagg 17040

tatgatttct ctggaggacc ttctcccact gagaacctac ttgaagtgta gtacctgccc 17100

taagtatttg aaggctgtcc taggtattac caaactcaaa gaaatgttca cagacgcctc 17160

tttattatac ttgactcgtg ctcaacaaaa attctacatg aaaactatag gcaatgcagt 17220

caagggatac tatagtaact gtgactctta aaggcaacca catatcaata ggccctcttt 17280

ctagccgatc gtattcttgt tgacttcatt ataccatatt agaaaaaaat tgaattccga 17340

ccctttaaga ctcgtattcg gattcaaata attatctcag aaaaaaagtg cacgtagttg    17400

ttcttgatta tagtcccgtc attcaccaag tctttgtttg gt                       17442

<210> 39
<211> 553
<212> PRT
<213> Newcastle disease virus

<220>
<223> F protein (F2)

<400> 39
Met Gly Pro Arg Ser Ser Thr Arg Ile Pro Val Pro Leu Met Leu Thr
1               5                   10                  15
Ile Arg Ile Thr Leu Ala Leu Ser Tyr Val Arg Leu Thr Ser Ser Leu
            20                  25                  30
Asp Gly Arg Pro Leu Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Met Pro Lys Asp Lys Glu Ala Cys Ala Lys Ala Pro
65                  70                  75                  80
Leu Glu Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Ser Ile Gly Ala Ile Ile Gly Ser Val Ala Leu Gly
        115                 120                 125
Val Ala Thr Ala Ala Gln Ile Thr Ala Ala Ser Ala Leu Ile Gln Ala
    130                 135                 140
Asn Gln Asn Ala Ala Asn Ile Leu Arg Leu Lys Glu Ser Ile Ala Ala
145                 150                 155                 160
Thr Asn Glu Ala Val His Glu Val Thr Gly Gly Leu Ser Gln Leu Ala
                165                 170                 175
Val Ala Val Gly Lys Met Gln Gln Phe Val Asn Asp Gln Leu Asn Asn
            180                 185                 190
Thr Ala Gln Glu Leu Asp Cys Ile Lys Ile Thr Gln Gln Val Gly Val
        195                 200                 205
Glu Leu Asn Leu Tyr Leu Thr Glu Leu Thr Thr Val Phe Gly Pro Gln
    210                 215                 220
Ile Thr Ser Pro Ala Leu Thr Gln Leu Thr Val Gln Ala Leu Tyr Asn
225                 230                 235                 240
Leu Ala Gly Gly Asn Val Asp Tyr Leu Leu Thr Lys Leu Gly Val Gly
                245                 250                 255
Asn Asn Gln Leu Ser Ser Leu Ile Gly Ser Gly Leu Ile Thr Gly Asn
            260                 265                 270
Pro Ile Phe Tyr Asp Ser Gln Thr Gln Leu Leu Gly Ile Gln Val Thr
        275                 280                 285
Leu Pro Ser Val Gly Asn Leu Asn Asn Met Arg Ala Thr Tyr Leu Glu
    290                 295                 300
Thr Leu Ser Val Ser Thr Thr Lys Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Ala Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Arg Thr Glu Gly Ala Leu Thr Thr Pro Tyr Met

370                 375                 380
Thr Leu Lys Gly Ser Val Val Ala Asn Cys Gln Met Thr Thr Cys Arg
385                 390                 395                 400
Cys Ala Asp Pro Pro Gly Ile Ile Ser Gln Asn Tyr Gly Glu Ala Val
                405                 410                 415
Ser Leu Ile Asp Lys His Ser Cys Asn Val Val Ser Leu Asp Gly Ile
            420                 425                 430
Thr Leu Arg Leu Ser Gly Glu Phe Asp Ala Thr Tyr Gln Lys Asn Ile
        435                 440                 445
Ser Ile Leu Asp Ser Gln Val Leu Val Thr Gly Asn Leu Asp Ile Ser
    450                 455                 460
Thr Glu Leu Gly Asn Val Asn His Ser Ile Ser Asn Ala Leu Asp Lys
465                 470                 475                 480
Leu Glu Glu Ser Asn Ser Lys Leu Asp Lys Val Asn Val Lys Leu Thr
                485                 490                 495
Ser Thr Ser Ala Leu Ile Thr Tyr Ile Val Leu Thr Val Ile Ser Leu
            500                 505                 510
Val Leu Gly Met Leu Ser Leu Val Leu Ala Cys Tyr Leu Met Tyr Lys
        515                 520                 525
Gln Lys Ala Gln Arg Lys Thr Leu Leu Trp Leu Gly Asn Asn Thr Leu
    530                 535                 540
Asp Gln Met Arg Ala Thr Thr Lys Met
545                 550

<210> 40
<211> 36
<212> DNA
<213> Artificial Sequence

<220>
<223> Primer

<400> 40
acgactcact ataggaccaa acagagaatc cgtgag                                36

<210> 41
<211> 35
<212> DNA
<213> Artificial Sequence

<220>
<223> Primer

<400> 41
ccgggaagat ccagggcact cttcttgcat gttac                                 35

<210> 42
<211> 35
<212> DNA
<213> Artificial Sequence

<220>
<223> Primer

<400> 42
gggcctgcct cactatggtg gtaacatgca agaag                                35

<210> 43
<211> 35
<212> DNA
<213> Artificial Sequence

<220>
<223> Primer

<400> 43
tgcatgttac caccaatgtg tcattgtatc gcttg                                35

<210> 44
<211> 37
<212> DNA
<213> Artificial Sequence

<220>
<223> Primer

<400>44
caagaaggga gatacgtaat atacaagcga tacaatg                              37

<210> 45
<211> 35
<212> DNA
<213> Artificial Sequence

<220>
<223> Primer

<400> 45
tcgcttgtat attacttgtt gtagcaaaga gcacc                                35

<210> 46
<211> 38
<212> DNA
<213> Artificial Sequence

<220>
<223> Primer

<400> 46
ggcctggatc ttcccattat gctgtctgta tacggtgc                             38

<210> 47
<211> 38
<212> DNA
<213> Artificial Sequence

<220>
<223> Primer

<400> 47
atgccatgcc gacccaccaa acaaagactt ggtgaatg 38

<210> 48
<211> 24
<212> DNA
<213> Artificial Sequence

<220>
<223> Primer

<400> 48
cctatagtga gtcgtattaa tttc 24

<210> 49
<211> 20
<212> DNA
<213> Artificial Sequence

<220>
<223> Primer

<400> 49
cctggatctt cccgggtcgg 20

<210> 50
<211> 22
<212> DNA
<213> Artificial Sequence

<220>
<223> Primer

<400> 50
gggtcggcat ggcatctcca cc 22

<210> 51
<211> 22
<212> DNA
<213> Artificial Sequence

<220>
<223> Primer

<400> 51
gggaagatcc aggcctatag tg                                                22

<210> 52
<211> 35
<212> DNA
<213> Artificial Sequence

<220>
<223> Primer

<400> 52
ctctagagtc gacccttctg ccaacatgtc ttccg                                  35

<210> 53
<211> 35
<212> DNA
<213> Artificial Sequence

<220>
<223> Primer

<400> 53
gggaagcggc cgcccgtcgg tcagtatccc cagtc                                  35

<210> 54
<211> 37
<212> DNA
<213> Artificial Sequence

<220>
<223> Primer

<400> 54
ctctagagtc gaccccagag tgaagatggc caccttc                                37

<210> 55
<211> 35
<212> DNA
<213> Artificial Sequence

<220>
<223> Primer

<400> 55
gggaagcggc cgcccgtagt agtgatcagc cattc                                  35

<210> 56
<211> 35
<212> DNA
<213> Artificial Sequence

<220>
<223> Primer

<400> 56
ctctagagtc gacccgggta ggacatggcg ggctc                                35

<210> 57
<211> 37
<212> DNA
<213> Artificial Sequence

<220>
<223> Primer

<400> 57
gggaagcggc cgccctgcct ttaagagtca cagttac                              37